(19) Europäisches Patentamt / European Patent Office / Office européen des brevets

(11) **EP 2 563 936 B1**

(12) **EUROPEAN PATENT SPECIFICATION**

(45) Date of publication and mention
of the grant of the patent:
**14.03.2018 Bulletin 2018/11**

(21) Application number: **11741685.9**

(22) Date of filing: **29.04.2011**

(51) Int Cl.:
***C12Q 1/68*** (2018.01)

(86) International application number:
**PCT/IB2011/001405**

(87) International publication number:
**WO 2011/135459 (03.11.2011 Gazette 2011/44)**

(54) **METHODS AND DEVICES FOR PREDICTING TREATMENT EFFICACY**

VERFAHREN UND VORRICHTUNGEN ZUR VORHERSAGE DER BEHANDLUNGSWIRKSAMKEIT

MÉTHODES ET DISPOSITIFS PERMETTANT DE PRÉDIRE L'EFFICACITÉ D'UN TRAITEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.04.2010 DK 201000382**

(43) Date of publication of application:
**06.03.2013 Bulletin 2013/10**

(73) Proprietor: **Medical Prognosis Institute A/S
2970 Horsholm (DK)**

(72) Inventor: **KNUDSEN, Steen
DK-3460 Birkroed (DK)**

(74) Representative: **Lahrtz, Fritz
Isenbruck Bösl Hörschler LLP
Patentanwälte
Prinzregentenstrasse 68
81675 München (DE)**

(56) References cited:
**WO-A1-2009/080437     WO-A2-2008/112283**

• **SYLVAIN PRADERVAND ET AL: "Concordance among digital gene expression, microarrays, and qPCR when measuring differential expression of microRNAs", BIOTECHNIQUES, vol. 48, no. 3, 1 March 2010 (2010-03-01), pages 219-222, XP055012457, ISSN: 0736-6205, DOI: 10.2144/000113367**

• **SUNG-CHOU LI ET AL: "Intronic MicroRNA: Discovery and Biological Implications", DNA AND CELL BIOLOGY, MARY ANN LIEBERT, NEW YORK, NY, US, vol. 26, no. 4, 1 April 2007 (2007-04-01), pages 195-207, XP008124956, ISSN: 1044-5498**

• **"SEQ ID NO 486 of EP2112235 disclosing has-miR-766", , 31 October 2009 (2009-10-31), XP055012964, Retrieved from the Internet: URL:http:// [retrieved on 2011-11-23]**

• **FRIIS-HANSEN L ET AL: "1072 Mir-449 Inhibits Growth of Gastric Cancer Cells Partly By Inhibiting the Expression of Met and Amphiregulin", GASTROENTEROLOGY, ELSEVIER, PHILADELPHIA, PA, vol. 136, no. 5, 1 May 2009 (2009-05-01), pages A-165, XP026111292, ISSN: 0016-5085, DOI: 10.1016/S0016-5085(09)60745-9 [retrieved on 2009-05-01]**

• **"NCode(TM) Multi-species miRNA Microarray Probe Set Version 2.0", , 1 May 2009 (2009-05-01), XP055013130, Retrieved from the Internet: URL:http://www.invitrogen.com/site/us/en/h ome/Products-and-Services/Applications/epi genetics-noncoding-rna-research/miRNA-Prof iling-/miRNA-Probe-Set-Files.html [retrieved on 2011-11-25]**

**Description**

**FIELD OF THE INVENTION**

The invention features the use of devices to predict the growth inhibition of cancer cells of a patient to a medical treatment, e.g., a chemotherapeutic agent.

**BACKGROUND OF THE INVENTION**

DNA microarrays have been used to measure gene expression in tumor samples from patients and to facilitate diagnosis. Gene expression can reveal the presence of cancer in a patient, its type, stage, and origin, and whether genetic mutations are involved. Gene expression may even have a role in predicting the efficacy of chemotherapy. Over recent decades, the National Cancer Institute (NCI) has tested compounds, including chemotherapy agents, for their effect in limiting the growth of 60 human cancer cell lines. The NCI has also measured gene expression in those 60 cancer cell lines using DNA microarrays. Various studies have explored the relationship between gene expression and compound effect using the NCI datasets. During chemotherapy for cancers critical time is often lost due to a trial and error approach to finding an effective therapy. In addition, cancer cells often develop resistance to a previously effective therapy. In such situations, patient outcome would be greatly improved by early detection of such resistance.

Pradervand et al (Biotechniques, 48:219-222, 2010) discloses measurement of differential microRNA (miRNA) expression using microarray platforms of various vendors, as well as qPCR and ultra high-throughput sequencing.

Li et al. (DNA and Cell Biology, 26(4):195-207, 2007) discloses hypothetical models of feedback regulation by intronic miRNA on host-coding genes by selecting transcription factors as miRNA targets or by protein-protein interactions between intronic miRNA host gene product and miRNA target gene products.

EP21112235 discloses the use of miRNA expression profiling to identify mammalian cells having a predisposition to develop nasopharyngeal carcinoma.

Friis-Hanses et al (Gastroenterology, 136:A165, 2009) discloses that miR449b is a potential tumor suppressor miRNA and the reduction in expression of miR449b during carcinogenesis in gastrin knockout mice.

NCodeTM Multi-species miRNA Microarray Prob Set Version 2.0 (Invitrogen TM) discloses a probe set that includes several miRNAs.

WO 2008/112283 discloses methods of predicting responsiveness of prostate cancer to therapeutic treatment by using a specific androgen responsiveness miRNA expression profile.

WO 2009/080437 discloses methods of determining or predicting the phenotype of a cancer patient with respect to the resistance or susceptibility of the patient to treatment using specific miRNAs.

There remains a need for proven methods and devices that predict the sensitivity or resistance of cancer patients to a medical treatment.

**SUMMARY OF THE INVENTION**

The present invention relates to the use of a device for predicting the growth inhibition of cancer cells of a cancer patient by at least one treatment for cancer,
wherein said device comprises at least one single-stranded nucleic acid molecule that is complementary or identical to at least 5 consecutive nucleotides of hsa-miR-766_st,
wherein said at least one single stranded nucleic acid molecule is capable of specifically hybridizing with hsa-miR-766_st or its complement, said device allowing determination of a level of expression of hsa-miR-766_st in a biological sample from said patient,
wherein said use further comprises assaying a level of expression of hsa-miR-766_st in a sample from said patient using said device, and wherein:

said patient is predicted to exhibit growth inhibition of cancer cells by said at least one treatment if said level of expression of hsa-miR-766_st is substantially similar to the level of expression of hsa-miR-766_st in a cell or tissue known to exhibit growth inhibition of cancer cells by said at least one treatment,

wherein said at least one treatment for cancer is selected from the group consisting of etoposide, adriamycin, paclitaxel, dexamethasone, methylprednisolone, belinostat, 5-Aza-2'-deoxycytidine (decitabine), melphalan, suberoylanilide hydroxamic acid (SAHA, vorinostat), leukeran, fludarabine, busulfan, dacarbazine, hydroxyurea, daunorubicin, mechlorethamine, carmustine, lomustine, mercaptopurine, teniposide, dactinomycin, ifosfamide, irinotecan, and thioguanine.

The application discloses methods and devices for predicting the sensitivity or resistance of a patient, e.g., a cancer patient, to a treatment, e.g., treatment with a compound, such as a chemotherapeutic agent, or radiation. In particular, the methods and devices can be used to predict the sensitivity or resistance of a cancer patient to any medical treatment, including, e.g., treatment with a compound, drug, or radiation. The devices and methods have been used to accurately predict treatment efficacy in cancer patients (e.g., patients with lung, lymphoma, and brain cancer) and can be used to predict treatment efficacy in patients diagnosed with any cancer.

Disclosed is a method of predicting sensitivity of a cancer patient to a treatment for cancer by determining the expression level of at least one gene or noncoding RNA (e.g. microRNA (miRNA), such as hsa-miR-766_st) in a cell (e.g., a cancer cell) of the patient in which the level of expression of the gene and/or RNA (e.g., miRNA) indicates the patient is sensitive or resistant to at least one treatment for cancer. In an embodiment, the method involves assaying for the level of expression of one or more (e.g., two, three, four, five, ten, twenty, thirty, forty, or fifty or more) biomarkers from one or more of Tables 1-65 and/or one or more of Tables 66-129) in a patient. For example, the method includes assaying the level of expression of hsa-miR-766_st in a biological sample from the patient (e.g., a cell, tissue, or organ sample from a patient) or in a sample prepared from a biological sample from the patient.

In another embodiment, the method includes assaying the level of expression of at least hsa-miR-766_st (alone or in combination with one or more (e.g., two, three, four, or more) of the biomarkers listed in one or more of Tables 1-65 and/or one or more of the biomarkers listed in one or more of Tables 66-129). In an embodiment, the method includes determining the expression level of two of the listed biomarkers, more preferably three, four, five, six, seven, eight, nine, or ten of the listed biomarkers, and most preferably twenty, thirty, forty, fifty, sixty, seventy, eighty, ninety, or one hundred or more of the listed biomarkers. It is disclosed that the change in the level of biomarker expression (e.g., an increase or decrease) is determined relative to the level of biomarker expression in a cell or tissue known to be sensitive to the treatment, such that a similar level of biomarker expression exhibited by a cell or tissue of the patient indicates the patient is sensitive to the treatment. It is disclosed that the change in the level of biomarker expression (e.g., an increase or decrease) is determined relative to the level of biomarker expression in a cell or tissue known to be resistant to the treatment, such that a similar level of biomarker expression exhibited by a cell or tissue of the patient indicates the patient is resistant to the treatment.

Disclosed is a method for determining the development of resistance by a patient (i.e., a cell, such as a cancer cell, in the patient) to a treatment that the patient was previously sensitive to. The method includes determining the level of expression of one or more of the biomarkers set forth above , such that the expression level of a biomarker(s) which is decreased in a cell or tissue known to be sensitive to the treatment indicates that the patient is resistant to or has a propensity to become resistant to the treatment. Alternatively, a decrease in the expression level of a biomarker(s) which is increased in a cell or tissue known to be sensitive to the treatment indicates that the patient is resistant to or has a propensity to become resistant to the treatment.

Disclosed are devices for assaying the level of expression of one or more biomarkers described herein (e.g., one or more of the biomarkers listed in Tables 1-129, e.g., at least hsa-miR-766_st (alone or in combination with one or more (e.g., two, three, four, five, ten, twenty, thirty, forty, or fifty or more) additional biomarkers from one or more of Tables 1-65 and/or one or more of Tables 66-129)) in a patient; the devices include probes capable of hybridizing to the biomarkers. For example, the method includes assaying the level of expression of hsa-miR-766_st in a biological sample from the patient (e.g., a cell, tissue, or organ sample from a patient) or in a sample prepared from a biological sample from the patient. It is disclosed that the method can be used to determine the sensitivity of a patient to at least one treatment for cancer (e.g,. a chemotherapy drug, such as vincristine, cisplatin, ctoposide, azaguanine, carboplatin, adriamycin, aclarubicin, mitoxantrone, mitoxantrone, mitomycin, paclitaxel, gemcitabine, taxotere, dexamethasone, ara-c, methylprednisolone, methotrexate, bleomycin, methyl-gag, belinostat, carboplatin, 5-fu (5-fluorouracil), idarubicin, melphalan, IL4-PR38, valproic acid, all-trans retinoic acid (ATRA), cytoxan, topotecan, suberoylanilide hydroxamic acid (SAHA, vorinostat), depsipeptide (FR901228), bortezomib, leukeran, fludarabine, vinblastine, busulfan, dacarbazine, oxaliplatin, hydroxyurea, tegafur, daunorubicin, estramustine, mechlorethamine, streptozocin, carmustine, lomustine, mercaptopurine, teniposide, dactinomycin, tretinoin, ifosfamide, tamoxifen, irinotecan, floxuridine, thioguanine, PSC 833, erlotinib, herceptin, celecoxib, fulvestrant, iressa, anastrozole, letrozole, cetuximab, rituximab, radiation, histone deacetylase (HDAC) inhibitors, and/or 5-Aza-2'-deoxycytidine (decitabine)). It is disclosed that the method can be used to determine the sensitivity of a patient to two or more (e.g., three, four, five, ten, twenty, or more) of the treatments for cancer listed above. It is disclosed that the method can be used to assay the sensitivity of a patient to one or more (e.g., two or more, or even all) of the treatments for cancer listed above in a single assay.

Used devices of the invention include probes , the device includes at least one or more probes directed to hsa-miR-766_st and probes having at least 85% identify (e.g., 90%, 95%, 99%, or 100% identity) to a target nucleic acid molecule with a sequence that is complementary or identical to at least 5-100 (e.g., 10-20 (e.g., 15), 20-50 (e.g., 25 and/or 40), and 25-60) consecutive nucleotides of one or more of the biomarkers of the invention (e.g., two, three, four, five, ten, twenty, thirty, forty, fifty or more, or all) of the biomarkers listed in one or more of Tables 1-65 for the chemotherapy drugs

etoposide, adriamycin, paclitaxel, dexamethasone, methylprednisolone, belinostat, melphalan, suberoylanilide hydroxamic acid (SAHA, vorinostat), leukeran, fludarabine, busulfan, dacarbazine, hydroxyurea, daunorubicin, mechlorethamine, carmustine, lomustine, mercaptopurine, teniposide, dactinomycin, ifosfamide, irinotecan, thioguanine, and/or 5-Aza-2'-deoxycytidine (decitabine) are also provided. In other embodiments, the used device includes at least one nucleic acid molecule that is complementary to or identical to at least 5 (e.g., 10, 15, 20, or 22) consecutive nucleotides of hsa-miR-766_st (e.g., at least 5 (e.g., 10, 15, 20, or 22) consecutive nucleotides of the sequence 5'-GCTGAGGCTGTGGGGCTGGAGT-3'). It is disclosed that the device includes probes (e.g., the device includes at least one or more probes directed to hsa-miR-766_st) having at least 85% identify (e.g., 90%, 95%, 99%, or 100% identity) to a target nucleic acid molecule with a sequence that is complementary or identical to at least 5-100 (e.g., 10-20 (e.g., 15), 20-50 (e.g., 25 and/or 40), and 25-60) consecutive nucleotides of those biomarkers listed in one or more (or all) of Tables 1-65 having a mean score correlation coefficient (positive correlation) of equal to or greater than 0.25, 0.30, 0.35, 0.40, 0.45, 0.50, 0.55, 0.60, 0.65, and/or 0.7 or more (preferably the devices include probes directed to those biomarkers in Tables 1-65 having a mean score correlation coefficient of 0.4 or greater and preferably the probes have a sequence that is complementary or identical to the sequences in shown Tables 1-65 for the indicated biomarkers). Other devices of the disclosure include probes (e.g., one or more probes directed to hsa-miR-766_st) having at least 85% identify (e.g., 90%, 95%, 99%, or 100% identity) to a target nucleic acid molecule with a sequence that is complementary or identical to at least 5-100 (e.g., 10-20 (e.g., 15), 20-50 (e.g., 25 and/or 40), and 25-60) consecutive nucleotides of those biomarkers listed in one or more (or all) of Tables 66-129 having a mean score correlation coefficient (negative correlation) of equal to or greater than -0.7, -0.65, -0.6, -0.55, -0.5, -0.45, -0.4, -0.35, - 0.3, and/or -0.25 (preferably the devices include probes directed to those biomarkers in Tables 66-129 having a mean score correlation coefficient of -0.5 or greater and preferably the probes have a sequence that is complementary or identical to the sequences in shown Tables 66-129 for the indicated biomarkers). In yet other disclosure , devices include probes having at least 85% identify (e.g., 90%, 95%, 99%, or 100% identity) to a target nucleic acid molecule with a sequence that is complementary or identical to at least 5-100 (e.g., 10-20 (e.g., 15), 20-50 (e.g., 25 and/or 40), and 25-60) consecutive nucleotides of those biomarkers listed in one or more of Tables 1-65 having a mean score correlation coefficient of greater than 0.25, 0.30, 0.35, 0.40, 0.45, 0.50, 0.55, 0.60, 0.65, and/or 0.7 or more (e.g., preferably the mean score correlation coefficient is 0.3 or greater) and probes having at least 85% identify (e.g., 90%, 95%, 99%, or 100% identity) to a target nucleic acid molecule with a sequence that is complementary or identical to at least 5-100 (e.g., 10-20 (e.g., 15), 20-50 (e.g., 25 and/or 40), and 25-60) consecutive nucleotides of those biomarkers listed in one or more of Tables 66-129 having a mean score correlation coefficient of at least -0.7, -0.65, -0.6, -0.55, -0.5, -0.45, -0.4, -0.35, - 0.3, and/or -0.25 (e.g., preferably the mean score correlation coefficient is -0.3 or lower). In a disclosure , the used devices include probes directed to those biomarkers in Tables 1-65 having a mean score correlation coefficient of 0.4 or greater, and preferably the probes have a sequence that is complementary or identical to the sequences in shown Tables 1-65 for the indicated biomarkers, and probes directed to those biomarkers in Tables 66-129 having a mean score correlation coefficient of -0.5 or greater, preferably the probes have a sequence that is complementary or identical to the sequences in shown Tables 66-129 for the indicated biomarkers.

Disclosed are devices above, which include probes having at least 99% or 100% identity to a target nucleic acid molecule with a sequence that is complementary or identical to the biomarker sequences of Tables 1-65 and/or Tables 66-129. Disclosed are devices that include probes having at least 99% or 100% identity to a target nucleic acid molecule with a sequence that is complementary or identical to the biomarker sequences of Tables 1-65 that have a correlation coefficient of 0.3 or greater and/or biomarker sequences of Tables 66-129 that have a correlation coefficient of -0.3 or lower. For example, a device of the use of the invention includes a probe for hsa-miR-766_st (alone or in combination with one or more (e.g., two, three, four, five, ten, twenty, thirty, forty, or fifty or more) additional biomarkers from one or more of Tables 1-65 and/or one or more of Tables 66-129)) in a patient; the devices include probes capable of hybridizing to the biomarkers. For example, the use includes assaying the level of expression of hsa-miR-766_st in a biological sample from the patient (e.g., a cell, tissue, or organ sample from a patient) or in a sample prepared from a biological sample from the patient. It is disclosed that the method can be used to determine the sensitivity of a patient to at least one treatment for cancer (e.g,. a chemotherapy drug, such as vincristine, cisplatin, etoposide, azaguanine, carboplatin, adriamycin, aclarubicin, mitoxantrone, mitoxantrone, mitomycin, paclitaxel, gemcitabine, taxotere, dexamethasone, ara-c, methylprednisolone, methotrexate, bleomycin, methyl-gag, belinostat, carboplatin, 5-fu (5-fluorouracil), idarubicin, melphalan, IL4-PR38, valproic acid, all-trans retinoic acid (ATRA), cytoxan, topotecan, suberoylanilide hydroxamic acid (SAHA, vorinostat), depsipeptide (FR901228), bortezomib, leukeran, fludarabine, vinblastine, busulfan, dacarbazine, oxaliplatin, hydroxyurea, tegafur, daunorubicin, estramustine, mechlorethamine, streptozocin, carmustine, lomustine, mercaptopurine, teniposide, dactinomycin, tretinoin, ifosfamide, tamoxifen, irinotecan, floxuridine, thioguanine, PSC 833, erlotinib, herceptin, celecoxib, fulvestrant, iressa, anastrozole, letrozole, cetuximab, rituximab, radiation, histone deacetylase (HDAC) inhibitors, and/or 5-Aza-2'-deoxycytidine (decitabine)). In other disclosure, the method can be used to determine the sensitivity of a patient to two or more (e.g., three, four, five, ten, twenty, or more) of the treatments for cancer listed above. In yet other disclosure , the method can be used to assay the sensitivity of a patient to one or more (e.g., two or more, or even all) of the treatments for cancer listed above in a single assay.

For example, a device of the use of the invention includes a probe for hsa-miR-766_st (e.g., a probe having a sequence that is complementary or identical to 5'-GCTGAGGCTGTGGGGCTGGAGT-3') and any one additional probe for a biomarker selected from hsa-miR-106b-star_st, hsa-miR-25-star_st, HBII-85-2_x_st, U48_st, U55_x_st, hsa-mir-124_st, hsa-miR-1281_st, hsa-mir-181-a-star_st, hsa-miR-181b_st, hsa-miR-342-3p_st, hsa-miR-432_st, hsa-miR-938_st, hsa-miR-100-st, hsa-miR-140-3p_st, hsa-miR-140-5p_st, hsa-miR-146a_st, hsa-miR-155_st, hsa-miR-506_st, hsa-miR-508-5p_st, hsa-miR-509-3-5p_st, hsa-miR-509-3p_st, hsa-miR-510_st, hsa-miR-513a-5p_st, hsa-miR-513b_st, hsa-miR-663_st, hsa-miR-1271_st, hsa-miR-143_st, hsa-miR-370_st, hsa-miR-433_st, hsa-miR-654-3p_st, hsa-miR-758_st, U55_st, hsa-miR-106b_st, hsa-miR-1299_st, hsa-miR-29b-2-star_st, hsa-miR-33b-star_st, hsa-miR-629-star_st, hsa-miR-652_st, hsa-miR-671-5p_st, hsa-miR-768-3p_st, hsa-miR-93_st, ACA10_s_st, ACA18_x_st, ACA44_st, ACA51_x_st, ACA61_st, ENSG00000200394_st, ENSG00000202252_st, HBII-180A_x_st, HBII-429_st, U104_st, U13_st, U17b_st, U17b_x_st, U26_st, U3-2_s_st, U35A_st, U49A_st, U49A_x_st, U49B_s_st, U67_st, U68_st, U68_x_st, U74_x_st, hsa-miR-1275_st, hsa-miR-18a-star_st, hsa-miR-18a_st, 14qII-14_st, 14qII-1_st, 14qII-26_st, 14qII-26_x_st, hsa-miR-127-3p_st, hsa-miR-181a_st, hsa-miR-181c_st, hsa-miR-342-5p_st, hsa-miR-409-3p_st, hsa-miR-487b_st, hsa-miR-768-5p_st, hsa-miR-92b_st, hsa-miR-1228_st, hsa-miR-185_st, hsa-miR-188-5p_st, hsa-miR-18b_st, hsa-miR-20b_st, hsa-miR-25_st, hsa-miR-320c_st, hsa-miR-320d_st, hsa-miR-362-5p_st, hsa-miR-500-star st, hsa-miR-500_st, hsa-miR-501-3p_st, hsa-miR-502-3p_st, hsa-miR-532-3p_st, HBII-438A_s_st, hsa-miR-181astar_st, hsa-miR-503_st, hsa-miR-1307 st, hsa-miR-505_st, hsa-miR-769-3p_st, hsa-miR-769-5p_st, U49B_x_st, hsa-miR-10a-star_st, hsa-miR-1207-5p_st, hsa-miR-128_st, hsa-miR-150_st, hsa-miR-424-star_st, hsa-miR-424_st, HBII-202_st, HBII-85-11_st, U78_s_st, U78_x_st, ACA23_st, ACA24_x_st, ACA54_st, U31_st, hsa-let-7d-star_st, hsa-miR-1183_st, hsa-miR-1268_st, hsa-miR-15a_st, hsa-miR-198_st, hsa-miR-20b-star_st, hsa-miR-223_st, hsa-miR-297_st, hsa-miR-363_st, hsa-miR-588_st, hsa-miR-631_st, hsa-miR-92a-2-star_st, hsa-miR-940_st, U30_st, U31_x_st, U56_st, U67_x_st, hsa-miR-106a_st, hsa-miR-1254_st, hsa-miR-17_st, hsa-miR-19b_st, hsa-miR-34b_st, hsa-miR-663b_st, hsa-miR-92a_st, 14qII-14_x_st, 14qII-1_x_st, 14qII-3_st, hsa-miR-125b-1-star_st, hsa-miR-134_st, hsa-miR-193a-5p_st, hsa-miR-21-star st, hsa-miR-22_st, hsa-mi R-299-3p_st, hsa-miR-337-5p_st, hsa-miR-376c_st, hsa-miR-377-star_st, hsa-miR-379_st, hsa-miR-381_st, hsa-miR-382_st, hsa-miR-409-5p_st, hsa-miR-411_st, hsa-miR-431_st, hsa-miR-455-3p_st, hsa-miR-485-5p_st, hsa-miR-493_st, hsa-miR-494_st, hsa-miR-543_st, ENSG00000200879_st, U25_st, U28_x_st, U29_st, hsa-miR-92a-1-star_st, ACA9_st, ACA9_x_st, HBII-336_st, HBII-55_st, U27 st, U38A_st, U56_x_st, U57_st, U60_st, hsa-miR-1246_st, hsa-miR-142-5p_st, hsa-miR-17-star_st, hsa-miR-195-star_st, hsa-miR-19a_st, hsa-miR-20a_st, hsa-miR297_st, hsa-miR-330-3p_st, hsa-miR-346_st, hsa-miR-595_st, hsa-miR-647_st, ENSG00000199411_s_st, U36A_x_st, U36C_st, U8_x_st, hsa-miR-194-star_st, hsa-miR-200c-star st, hsa-miR-324-3p_st, hsa-miR-371-5p_st, hsa-miR-93-star_st, ACA41_x_st, ACA48_x_st, ACA7_s_st, ENSG00000207002_x_st, HBII-85-23_x_st, HBII-85-26_st, HBII-85-6_x_st, U33_st, U34_st, U41_st, U52_st, U83_st, U95_st, hsa-miR-541-star_st, hsa-miR-610_st, hsa-miR-885-5p_st, hsa-miR-339-3p_st, hsa-miR-339-5p_st, hsa-miR-1308_st, hsa-miR-148a_st, hsa-miR-152_st, hsa-miR-34a-star_st, hsa-miR-34a_st, HBII-142_st, HBII-142_x_st, hsa-miR-1202_st, hsa-miR-148a-star_st, hsa-miR-184_st, hsa-miR-191_st, hsa-miR-425-star_st, hsa-miR-425_st, hsa-miR-449a_st, hsa-miR-449b_st, hsa-miR-551b_st, hsa-miR-877_st, hsa-miR-196a st, hsa-miR-205_st, hsa-miR-29c-star_st, hsa-miR-375_st, hsa-miR-489_st, hsa-miR-126_st, hsa-miR-153_st, hsa-miR-30c_st, hsa-miR-30e_st, hsa-miR-363-star st, hsa-miR-532-5p_st, hsa-miR-660_st, hsa-miR-638_st, HBII-276_st, HBII-52-32_x_st, hsa-miR-130a_st, hsa-miR-34c-3p_st, hsa-miR-554_st, hsa-miR-923_st, U17a_st, hsa-miR-181d_st, ACA15_s_st, ACA21_st, HBII-99_st, U50B_st, U71d_x_st, U73a_st, hsa-miR-301a_st, ENSG00000199282_st, ENSG00000201859_x_st, hsa-miR-491-3p_st, U43_x_st, U51_st, hsa-miR-1228-star_st, hsa-miR-149-star_st, hsa-miR-16_st, hsa-miR-106a-star_st, hsa-miR-106bstar_st, hsa-miR-877-star st, hsa-miR-1226_st, hsa-miR-193a-3p_st, hsa-miR-330-5p_st, hsa-miR-378_st, hsa-miR-586_st, ACA13_st, HBII-239_st, U75_st, hsa-miR-331-5p_st, hsa-miR-296-3p_st, ACA52_st, HBII-382_s_st, U17a_x_st, U70_x_st, U83B_st, hsa-miR-1274a_st, hsa-miR-1280 st, hsa-miR-130b_st, hsa-miR-146b-3p_st, hsa-miR-146b-5p_st, hsa-miR-185-star_st, hsa-miR-202_st, hsa-miR-373_st, hsa-miR-378-star_st, hsa-miR-422a_st, hsa-miR-423-5p_st, hsa-miR-451_st, hsa-miR-486-3p_st, hsa-miR-486-5p_st, hsa-miR-504_st, hsa-miR-550_st, hsa-miR-616_st, hsa-miR-671-3p_st, hsa-miR-615-3p_st, ACA43_st, ACA57 st, U59B_st, hsa-miR25-star_st, hsa-miR-501-5p_st, hsa-miR-629_st, hsa-miR-181c-star st, HBII-180C_st, HBII-180C_x_st, U32A_x_st, U43_st, U46_x_st, U71d_st, hsa-miR-593-star_st, hsa-miR-874_st, ENSG00000202093_x_st, U36A_st, U54_st, HBII-180B_x_st, U38B_st, U50B_x_st, hsa-miR-1292_st, hsa-miR-149_st, hsa-miR-27a_st, hsa-miR-30a-star_st, hsa-miR-30a_st, hsa-miR-30c-2-star_st, hsa-miR-30e-star_st, hsa-miR-34c-5p_st, hsa-miR-15b_st, hsa-miR-203_st, hsa-miR-421_st, hsa-miR-492_st, hsa-miR-622_st, hsa-miR-1826_st, hsa-miR-675_st, hsa-miR-934_st, hsa-miR-1323_st, hsa-miR-187_st, hsa-miR-197_st, hsa-miR-498_st, hsa-miR-509-5p_st, hsa-miR-512-3p_st, hsa-miR-513c st, hsa-miR-516b_st, hsa-miR-517a_st, hsa-miR-517b_st, hsa-miR-525-5p_st, hsa-miR-526b_st, hsa-miR-551a_st, hsa-miR-873_st, hsa-miR-361-5p_st, hsa-miR-498_st, hsa-let-7i-star_st, hsa-miR-338-3p_st, and/or hsa-miR-34b-star_st, HBII-85-29_st, hsa-miR-130a_st, hsa-miR-148b_st, hsa-miR-184_st, hsa-miR-26a_st, hsa-miR-30a-star_st, hsa-miR-30a_st, hsa-miR-30c-2-star st, hsa-miR-30c_st, hsa-miR-34a-star_st, hsa-miR-34a_st, hsa-miR-34c-5p_st, hsa-miR-449a_st, hsa-miR-449b_st, hsa-miR-10a_st, hsa-miR-151-3p_st, hsa-miR-151-5p_st, hsa-miR-192-star_st, hsa-miR-192_st, hsa-miR-194_st, hsa-miR-200b_st, hsa-miR-203_st, hsa-miR-29b_st, hsa-miR-30b_st, hsa-miR-30d_st, hsa-miR-625_st, hsa-let-7e_st, hsa-miR-10b_st, hsa-miR-125a-5p_st, hsa-miR-141 st, hsa-miR-200a-star_st, hsa-miR-200a_st, hsa-miR-200b-star_st, hsa-miR-200c_st, hsa-miR-429_st, hsa-miR-516a-5p_st, hsa-miR-934 st, hsa-miR-99b_st, hsa-miR-1244_st, hsa-miR-1303_st, hsa-miR-141-star_st, hsa-miR-182_st, hsa-miR-183-star_st, hsa-miR-183_st, hsa-miR-200c-star_st, hsa-miR-205_st, hsa-miR-215_st, hsa-miR-27b_st, hsa-miR-331-3p_st, hsa-miR-375_st, hsa-miR-622_st, hsa-miR-99b-star_st, hsa-miR-7_st, hsa-miR-23a_st, hsa-miR-518d-5p_st, hsa-miR-518e-star_st, hsa-miR-519a-star_st, hsa-miR-519a_st, hsa-miR-519b-5p_st, hsa-miR-519c-5p_st, hsa-miR-522-star_st, hsa-miR-523-star_st, hsa-miR-526a_st, hsa-let-7a_st, hsa-let-7c_st, hsa-let-7f_st, hsa-miR-193a-5p_st, hsa-miR-22-star_st, hsa-miR-22_st, hsa-miR-24-2-star_st, hsa-miR-24_st, hsa-miR-506_st, hsa-miR-508-5p_st, hsa-miR-509-3-5p_st, hsa-miR-509-3p_st, hsa-miR-509-5p_st, hsa-miR-510_st, hsa-miR-513a-5p_st, hsa-miR-513c_st, hsa-miR-584_st, hsa-miR-885-3p_st, HBII-85-29-st, hsa-miR-193b_st, hsa-miR-27a_st, hsa-miR-29a_st, hsa-miR-34b-star_st, hsa-miR-34c-3p_st, HBII-438A_s_st, HBII-85-11_st, HBII-85-15_x_st, HBII-85-23_x_st, HBII-85-29_x_st, hsa-miR-424-star_st, hsa-miR-23b_st, hsa-miR-23b-star st, hsa-miR-1308_st, hsa-miR-21-star_st, hsa-miR-21_st, hsa-miR-27a-star_st, hsa-miR-339-3p_st, hsa-miR-28-5p_st, hsa-miR-516b_st, hsa-miR-517a st, hsa-miR-525-5p_st, hsa-miR-128_st, hsa-miR-1292_st, hsa-miR-15b_st, hsa-miR-185-star_st, hsa-miR-188-5p_st, hsa-miR-18b_st, hsa-miR-20b_st, hsa-miR-215_st, hsa-miR-25_st, hsa-miR-362-5p_st, hsa-miR-378-star_st, hsa-miR-421_st, hsa-miR-425_st, hsa-miR-532-5p_st, hsa-miR-93_st, hsa-miR-941_st, hsa-let-7i_st, hsa-miR-10b_st, hsa-miR-1301_st, hsa-miR-140-5p_st, 14qII-14 st, 14qII-l_x_st, hsa-let-7b_st, hsa-miR-125a-3p_st, hsa-miR-125b-l-star_st, hsa-miR-125b_st, hsa-miR-1287_st, hsa-miR-134_st, hsa-miR-23a-star_st, hsa-miR-28-3p_st, hsa-miR-299-5p_st, hsa-miR-337-5p_st, hsa-miR-370_st, hsa-miR-376a_st, hsa-miR-376c_st, hsa-miR-379_st, hsa-miR-381_st, hsa-miR-382_st, hsa-miR-409-3p_st, hsa-miR-409-5p_st, hsa-miR-411_st, hsa-miR-431_st, hsa-miR-452_st, hsa-miR-485-5p_st, hsa-miR-487a_st, hsa-miR-487b_st, hsa-miR-494_st, hsa-miR-495_st, hsa-miR-543_st, hsa-miR-654-5p_st, hsa-miR-100_st, hsa-miR-181a-2-star_st, hsa-miR-197_st, hsa-miR-221-star_st, hsa-miR-S2S-5p_st, hsa-miR-1207-5p_st, hsa-miR-766_st, hsa-miR-31-star st, hsa-miR-31_st, hsa-miR-320d_st, hsa-miR-146a st, HBII-85-26_st, HBII-85-6_x_st, hsa-miR-491-5p_st, hsa-miR-589-star_st, hsa-miR-744_st, hsa-let-7b_st, hsa-miR-221_st, hsa-miR-222_st, hsa-miR-29b-1-star_st, hsa-miR-455-3p_st, hsa-let-7d_st, hsa-let-7g_st, hsa-miR-138_st, hsa-miR-503_st, HBII-289_st, hsa-miR-373_st, hsa-miR-512-3p_st, 14qII-14_x_st, 14qII-1_x_st, 14qII-26_st, 14qII-26_x_st, hsa-miR-125b-l-star_st, hsa-miR-127-3p_st, hsa-miR-127-5p_st, hsa-miR-143-star_st, hsa-miR-143_st, hsa-miR-193a-3p_st, hsa-miR-193b-star_st, hsa-miR-199a-5p_st, hsa-miR-210_st, hsa-miR-214_st, hsa-miR-299-3p_st, hsa-miR-339-5p_st, hsa-miR-377-star_st, hsa-miR-410_st, hsa-miR-493_st, hsa-miR-542-5p_st, hsa-miR-758_st, hsa-miR-935_st, HBII-85-1_x_st, U28_st, hsa-miR-155_st, hsa-miR-196b_st, hsa-miR-371-3p_st, hsa-miR-371-5p_st, hsa-miR-372_st, hsa-miR-886-3p_st, HBII-52-32_x_st, hsa-miR-149_st, hsa-miR-9-star_st, hsa-miR-98_st, U91_s_st, hsa-miR-27b-star_st, hsa-miR-320a_st, hsa-miR-320b st, hsa-miR-320c_st, hsa-miR-139-5p_st, hsa-miR-16_st, hsa-miR-675_st, hsa-miR-24-1-star st, hsa-miR-532-3p_st, hsa-miR-103_st, hsa-miR-107_st, hsa-miR-1180_st, hsa-miR-1231_st, hsa-miR-132_st, hsa-miR-324-5p_st, hsa-miR-589_st, hsa-miR-1269_st, hsa-miR-1270_st, hsa-miR-145_st, hsa-miR-217_st, hsa-miR-574-3p_st, hsa-miR-195_st, hsa-miR-30b-star_st, hsa-miR-501-3p_st, 14qII-14_st, 14qII-1_st, hsa-miR-222-star_st, hsa-miR-30e-star_st, HBII-180C_st, HBII-180C_x_st, hsa-miR-106a_st, hsa-miR-106b-star_st, hsa-miR-106b_st, hsa-miR-1306_st, hsa-miR-1307_st, hsa-miR-130b_st, hsa-miR-17_st, hsa-miR-185_st, hsa-miR-18a-star_st, hsa-miR-18a_st, hsa-miR-19b_st, hsa-miR-20a_st, hsa-miR-25-star_st, hsa-miR-422a_st, hsa-miR-500-star_st, hsa-mi R-500_st, hsa-miR-660_st, hsa-miR-720_st, hsa-miR-93-star_st, hsa-miR-135b-star_st, hsa-miR-194-star_st, hsa-miR-552_st, hsa-miR-592_st, hsa-miR-874_st, hsa-miR-181a_st, hsa-miR-497_st, hsa-miR-9_st, hsa-miR-502-3p_st, hsa-miR-652_st, hsa-miR-671-5p_st, hsa-miR-505-star_st, U27_st, U29_st, and/or hsa-miR-191_st.

Another device of the use of the invention includes at least a probe for hsa-miR-766_st (e.g., a probe having a sequence that is complementary or identical to 5'-GCTGAGGCTGTGGGGCTGGAGT-3') and any one additional probe for a biomarker specified in Tables 1-65. Preferably the one additional probe is for a biomarker having a correlation coefficient of equal to or greater than 0.3. In another embodiment, the one additional probe is for a biomarker having a correlation coefficient of equal to or greater than 0.4.

Another device of the use of the invention includes at least a probe for hsa-miR-766_st (e.g., a probe having a sequence that is complementary or identical to 5'-GCTGAGGCTGTGGGGCTGGAGT-3') and any two additional probes for a biomarker specified in Tables 1-65. Preferably the two additional probes are for biomarkers having a correlation coefficient of equal to or greater than 0.3.

For example, a device of the use of the invention may include one or more probes directed to those biomarkers in Tables 1-65 having a mean score correlation coefficient of 0.3 or greater, in which the probes have a sequence that is complementary or identical to the sequences in shown Tables 1-65 for the selected biomarkers, and can be used to predict growth inhibition of cancer cells of a cancer patient by treatment with one or more of etoposide, adriamycin, paclitaxel, dexamethasone, methylprednisolone, belinostat, 5-Aza-2'-deoxycytidine (decitabine), melphalan, suberoylanilide hydroxamic acid (SAHA, vorinostat), leukeran, fludarabine, busulfan, dacarbazine, hydroxyurea, daunorubicin, mechlorethamine, carmustine, lomustine, mercaptopurine, teniposide, dactinomycin, ifosfamide, irinotecan, and thioguanine in a patient diagnosed with a cancer of the breast, prostate, lung and bronchus, colon and rectum, urinary bladder, skin, kidney, pancreas, oral cavity and pharynx, ovary, thyroid, parathyroid, stomach, brain, esophagus, liver and intrahepatic bile duct, cervix larynx, heart, testis, small and large intestine, anus, anal canal and anorectum, vulva, gallbladder, pleura, bones and joints, hypopharynx, eye and orbit, nose, nasal cavity and middle ear, nasopharynx, ureter, peritoneum, omentum and mesentery, and/or gastrointestines, as well as any form of cancer including, e.g., chronic myeloid leukemia, acute lymphocytic leukemia, non-Hodgkin lymphoma, melanoma, carcinoma, basal cell carcinoma, malignant mesothelioma, neuroblastoma, multiple myeloma, leukemia, retinoblastoma, acute myeloid leukemia, chronic lymphocytic leukemia, Hodgkin lymphoma, carcinoid tumors, acute tumor, and/or soft tissue sarcoma (e.g., preferably the cancer is a cancer of the bladder, breast, colon, rectum, uterus, kidney, lung, skin (e.g., melanoma), pancreas, prostate, blood and/or bone marrow (e.g., leukemia), lymphocytes (e.g., non-Hodgkin lymphoma), and/or thyroid).

The device includes at least a probe for hsa-miR-766_st (e.g., a probe having a sequence that is complementary or identical to 5'-GCTGAGGCTGTGGGGCTGGAGT-3').

In another example, a disclosed device may include one or more probes directed to those biomarkers in Tables 24, 28, 44-47, and/or 52 having a mean score correlation coefficient of 0.3 or greater, in which the probes have a sequence that is complementary or identical to the sequences in shown Tables 24, 28, 44-47, and/or 52 for the selected biomarkers, and can be used to predict the efficacy of treatment with an alkylating agent as correlated in Tables 24, 28, 44-47, and/or 52, such as mechlorethamine, cyclophosphamide (Cytoxan), ifosfamide, melphalan, streptozocin, lomustine, carmustine (BCNU), busulfan, and/or dacarbazine in a patient diagnosed with, e.g., chronic leukemia, lymphoma, Hodgkin disease, sarcoma, multiple myeloma, lung cancer, breast cancer, and/or ovarian cancer. The device may also include one or more probes directed to those biomarkers in Tables 2, 5, and 38 having a mean score correlation coefficient of 0.3 or greater, in which the probes have a sequence that is complementary or identical to the sequences in shown Tables 2, 5, and 38 for the selected biomarkers, and can be used to predict the efficacy of treatment with an agent as correlated in Tables 2, 5, and 38, such as cisplatin, carbonplatin, and/or oxalaplatin in a patient diagnosed with, e.g., chronic leukemia, lymphoma, Hodgkin disease, sarcoma, multiple myeloma, lung cancer, breast cancer, and/or ovarian cancer. Preferably, the device also includes at least a probe for hsa-miR-766_st (e.g., a probe having a sequence that is complementary or identical to 5'-GCTGAGGCTGTGGGGCTGGAGT-3').

In another example, a disclosed device may include all probes directed to those biomarkers in Tables 24, 28, 44-47, and/or 52 having a mean score correlation coefficient of 0.3 or greater, in which the probes have a sequence that is complementary or identical to the sequences in shown Tables 24,28, 44-47, and/or 52 for the selected biomarkers, and can be used to predict the efficacy of treatment with an alkylating agent as correlated in Tables 24, 28, 44-47, and/or 52, such as mechlorethamine, cyclophosphamide (Cytoxan), ifosfamide, melphalan, streptozocin, lomustine, carmustine (BCNU), busulfan, and/or dacarbazine in a patient diagnosed with, e.g., chronic leukemia, lymphoma, Hodgkin disease, sarcoma, multiple myeloma, lung cancer, breast cancer, and/or ovarian cancer. The device may also include one or more probes directed to those biomarkers in Tables 2, 5, and 38 having a mean score correlation coefficient of 0.3 or greater, in which the probes have a sequence that is complementary or identical to the sequences in shown Tables 2, 5, and 38 for the selected biomarkers, and can be used to predict the efficacy of treatment with an agent as correlated in Tables 2, 5, and 38, such as cisplatin, carbonplatin, and/or oxalaplatin in a patient diagnosed with, e.g., chronic leukemia, lymphoma, Hodgkin disease, sarcoma, multiple myeloma, lung cancer, breast cancer, and/or ovarian cancer. Preferably, the device also includes at least a probe for hsa-miR-766_st (e.g., a probe having a sequence that is complementary or identical to 5'-GCTGAGGCTGTGGGGCTGGAGT-3').

Another disclosed device may include one or more probes directed to those biomarkers in Tables 11, 14, 16, 20, 34, 48, and/or 56 having a mean score correlation coefficient of 0.3 or greater, in which the probes have a sequence that is complementary or identical to the sequences in shown Tables 11, 14, 16, 20, 34, 48, and/or 56 for the selected biomarkers, and can be used to predict the efficacy of treatment with an antimetabolite as correlated in Tables 11, 14, 16, 20, 34, 48, and/or 56, such as 5-fluorouracil (5-FU), methotrexate, 6-mercaptopurine, thioguanine, cytarabine, gemcitabine, and/or fludarabine in a patient diagnosed with, e.g., chronic and/or acute leukemia, breast cancer, ovarian cancer, esophageal cancer, head and/or neck cancer, and/or cancer of the intestinal tract. Preferably, the device also includes at least a probe for hsa-miR-766_st (e.g., a probe having a sequence that is complementary or identical to 5'-GCTGAGGCTGTGGGGCTGGAGT-3').

Another disclosed device may include all probes directed to those biomarkers in Tables 11, 14, 16, 20, 34, 48, and/or 56 having a mean score correlation coefficient of 0.3 or greater, in which the probes have a sequence that is complementary or identical to the sequences in shown Tables 11, 14, 16, 20, 34, 48, and/or 56 for the selected biomarkers, and can be used to predict the efficacy of treatment with an antimetabolite selected as correlated in Tables 11, 14, 16,20, 34, 48, and/or 56, such as 5-fluorouracil (5-FU), methotrexate, 6-mercaptopurine, thioguanine, cytarabine, gemcitabine, and/or fludarabine in a patient diagnosed with, e.g., chronic and/or acute leukemia, breast cancer, ovarian cancer, esophageal cancer, head and/or neck cancer, and/or cancer of the intestinal tract. Preferably, the device also includes at least a probe for hsa-miR-766_st (e.g., a probe having a sequence that is complementary or identical to 5'-GCTGAGGCTGTGGGGCTGGAGT-3').

Yet another disclosed device may include one or more probes directed to those biomarkers in Tables 9, 23, 41, 42, 50, and/or 59 having a mean score correlation coefficient of 0.3 or greater, in which the probes have a sequence that is complementary or identical to the sequences in shown Tables 9, 23, 41, 42, 50, and/or 59 for the selected biomarkers, and can be used to predict the efficacy of treatment with an anthracycline as correlated in Tables 9, 23, 41, 42, 50, and/or 59, such as daunorubicin, idarubicin, bleomycin, actinomycin, and/or mitomycin, in a patient diagnosed with, e.g., a leukemia, a lymphoma, breast cancer, uterine cancer, ovarian cancer, and/or lung cancer. Preferably, the device also includes at least a probe for hsa-miR-766_st (e.g., a probe having a sequence that is complementary or identical to 5'-GCTGAGGCTGTGGGGCTGGAGT-3').

Yet another disclosed device may include all probes directed to those biomarkers in Tables 9, 23, 41, 42, 50, and/or 59 having a mean score correlation coefficient of 0.3 or greater, in which the probes have a sequence that is complementary or identical to the sequences in shown Tables 9, 23, 41, 42, 50, and/or 59 for the selected biomarkers, and can be used to predict the efficacy of treatment with an anthracycline as correlated in Tables 9, 23, 41, 42, 50, and/or 59, such as daunorubicin, idarubicin, bleomycin, actinomycin, and/or mitomycin, in a patient diagnosed with, e.g., a leukemia, a lymphoma, breast cancer, uterine cancer, ovarian cancer, and/or lung cancer. Preferably, the device also includes at least a probe for hsa-miR-766_st (e.g., a probe having a sequence that is complementary or identical to 5'-GCTGAGGCTGTGGGGCTGGAGT-3').

Yet another disclosed device may include one or more probes directed to those biomarkers in Tables 3, 29, 49, 54, and/or 65 having a mean score correlation coefficient of 0.3 or greater, in which the probes have a sequence that is complementary or identical to the sequences in shown Tables 3, 29, 49, 54, and/or 65 for the selected biomarkers, and can be used to predict the efficacy of treatment with a topoisomerase inhibitor as correlated in Tables 3, 29, 49, 54, and/or 65, such as topotecan, irinotecan, etoposide, teniposide, and/or cetuximab in a patient diagnosed with, e.g., a leukemia, small cell lung cancer, colorectal cancer, ovarian cancer, and/or gastrointestinal cancer. Preferably, the device also includes at least a probe for hsa-miR-766_st (e.g., a probe having a sequence that is complementary or identical to 5'-GCTGAGGCTGTGGGGCTGGAGT-3').

Yet another disclosed device may include all probes directed to those biomarkers in Tables 3, 29, 49, 54, and/or 65 having a mean score correlation coefficient of 0.3 or greater, in which the probes have a sequence that is complementary or identical to the sequences in shown Tables 3, 29,49, 54, and/or 65 for the selected biomarkers, and can be used to predict the efficacy of treatment with a topoisomerase inhibitor as correlated in Tables 3, 29, 49, 54, and/or 65, such as topotecan, irinotecan, etoposide, teniposide, and/or cetuximab in a patient diagnosed with, e.g., a leukemia, small cell lung cancer, colorectal cancer, ovarian cancer, and/or gastrointestinal cancer. Preferably, the device also includes at least a probe for hsa-miR-766_st (e.g., a probe having a sequence that is complementary or identical to 5'-GCTGAGGCTGTGGGGCTGGAGT-3').

Yet another disclosed device may include one or more probes directed to those biomarkers in Tables 1, 3, 10, 12, 35, 43, and/or 49 having a mean score correlation coefficient of 0.3 or greater, in which the probes have a sequence that is complementary or identical to the sequences in shown Tables 1, 3, 10, 12, 35, 43, and/or 49 for the selected biomarkers, and can be used to predict the efficacy of treatment with a plant alkaloid as correlated in Tables 1, 3, 10, 12, 35, 43, and/or 49, such as vincristine, vinblastine, paclitaxel, docetaxcl, estramustine, etoposide, and/or teniposide in a patient diagnosed with, e.g., breast cancer, lung cancer, ovarian cancer, a myeloma, a lymphoma, a leukemia, and/or a mesothelioma. Preferably, the device also includes at least a probe for hsa-miR-766_st (e.g., a probe having a sequence that is complementary or identical to 5'-GCTGAGGCTGTGGGGCTGGAGT-3').

Yet another disclosed device may include all probes directed to those biomarkers in Tables 1, 3, 10, 12, 35, 43, and/or 49 having a mean score correlation coefficient of 0.3 or greater, in which the probes have a sequence that is complementary or identical to the sequences in shown Tables 1, 3, 10, 12, 35, 43, and/or 49 for the selected biomarkers, and can be used to predict the efficacy of treatment with a plant alkaloid as correlated in Tables 1, 3, 10, 12, 35, 43, and/or 49, such as vincristine, vinblastine, paclitaxel, docetaxel, estramustine, etoposide, and/or teniposide in a patient diagnosed with, e.g., breast cancer, lung cancer, ovarian cancer, a myeloma, a lymphoma, a leukemia, and/or a mesothelioma. Preferably, the device also includes at least a probe for hsa-miR-766_st (e.g., a probe having a sequence that is complementary or identical to 5-GCTGAGGCTGTGGGGCTGGAGT-3').

Yet another disclosed device may include one or more probes directed to those biomarkers in Tables 13 and/or 15 having a mean score correlation coefficient of 0.3 or greater, in which the probes have a sequence that is complementary or identical to the sequences in shown Tables 13 and/or 15 for the selected biomarkers, and can be used to predict the efficacy of treatment with a corticosteroid as correlated in Tables 13 and/or 15, such as dexamethasone and/or methylprednisolone in a patient diagnosed with, e.g., lung cancer, breast cancer lymphoma, leukemias, and/or multiple myeloma. Preferably, the device also includes at least a probe for hsa-miR-766_st (e.g., a probe having a sequence that is complementary or identical to 5'-GCTGAGGCTGTGGGGCTGGAGT-3').

Yet another disclosed device may include all probes directed to those biomarkers in Tables 13 and/or 15 having a mean score correlation coefficient of 0.3 or greater, in which the probes have a sequence that is complementary or identical to the sequences in shown Tables 13 and/or 15 for the selected biomarkers, and can be used to predict the efficacy of treatment with a corticosteroid as correlated in Tables 13 and/or 15, such as dexamethasone and/or methylprednisolone in a patient diagnosed with, e.g., lung cancer, breast cancer lymphoma, leukemias, and/or multiple myeloma. Preferably, the device also includes at least a probe for hsa-miR-766_st (e.g., a probe having a sequence that is complementary or identical to 5'-GCTGAGGCTGTGGGGCTGGAGT-3').

Yet another disclosed device may include one or more probes directed to those biomarkers in Tables 2, 5, and/or 38 having a mean score correlation coefficient of 0.3 or greater, in which the probes have a sequence that is complementary or identical to the sequences in shown Tables 2, 5, and/or 38 for the selected biomarkers, and can be used to predict the efficacy of treatment with a platinum compound as correlated in Tables 2, 5, and/or 38, such as cisplatin, carbonplatin, and/or oxalaplatin in a patient diagnosed with, e.g., chronic leukemia, lymphoma, Hodgkin disease, sarcoma, multiple myeloma, mesothelioma, lung cancer, breast cancer, testicular cancer, colon cancer, and/or ovarian cancer. Preferably, the device also includes at least a probe for hsa-miR-766_st (e.g., a probe having a sequence that is complementary or identical to 5'-GCTGAGGCTGTGGGGCTGGAGT-3').

Yet another disclosed device may include all probes directed to those biomarkers in Tables 2, 5, and/or 38 having a mean score correlation coefficient of 0.3 or greater, in which the probes have a sequence that is complementary or identical to the sequences in shown Tables 2, 5, and/or 38 for the selected biomarkers, and can be used to predict the efficacy of treatment with a platinum compound as correlated in Tables 2, 5, and/or 38, such as cisplatin, carbonplatin, and/or oxalaplatin in a patient diagnosed with, e.g., chronic leukemia, lymphoma, Hodgkin disease, sarcoma, multiple myeloma, mesothelioma, lung cancer, breast cancer, testicular cancer, colon cancer, and/or ovarian cancer. Preferably, the device also includes at least a probe for hsa-miR-766_st (e.g., a probe having a sequence that is complementary or identical to 5'-GCTGAGGCTGTGGGGCTGGAGT-3').

Yet another disclosed device may include one or more probes having a sequence that is complementary or identical to at least 5 (e.g., 10, 15, 20, or 22) consecutive nucleotides of hsa-miR-766_st (e.g., preferably the probe is complementary or identical to the 22 consecutive nucleotides of the sequence 5'-GCTGAGGCTGTGGGGCTGGAGT-3'), which can be used to predict the efficacy of treatment with a drug selected from one or more of etoposide, adriamycin, paclitaxel, dexamethasone, methylprednisolone, belinostat, aza-2'-deoxycytidine, melphalan, suberoylanilide hydroxamic acid, leukeran, fludarabine, busulfan, dacarbazine, hydroxyurea, daunorubicin, mechlorethamine, carmustine, lomustine, mercaptopurine, teniposide, dactinomycin, ifosamide, irinotecan, and/or thioguanine in a patient (e.g., a patient diagnosed with a cancer, e.g., a cancer of the breast, prostate, lung and bronchus, colon and rectum, urinary bladder, skin, kidney, pancreas, oral cavity and pharynx, ovary, thyroid, parathyroid, stomach, brain, esophagus, liver and intrahepatic bile duct, cervix larynx, heart, testis, small and large intestine, anus, anal canal and anorectum, vulva, gallbladder, pleura, bones and joints, hypopharynx, eye and orbit, nose, nasal cavity and middle ear, nasopharynx, ureter, peritoneum, omentum and mesentery, and/or gastrointestines, as well as any form of cancer including, e.g., chronic myeloid leukemia, acute lymphocytic leukemia, non-Hodgkin lymphoma, melanoma, carcinoma, basal cell carcinoma, malignant mesothelioma, neuroblastoma, multiple myeloma, leukemia, retinoblastoma, acute myeloid leukemia, chronic lymphocytic leukemia, Hodgkin lymphoma, carcinoid tumors, acute tumor, and/or soft tissue sarcoma).

In an embodiment, the devices of the use of the invention include at least one probe having a sequence that is complementary to or identical to at least 5 (e.g., 10, 15, 20, or 22) consecutive nucleotides of hsa-miR-766_st (e.g., preferably the probe has a sequence that is complementary or identical to the 22 consecutive nucleotides of the sequence 5'-GCTGAGGCTGTGGGGCTGGAGT-3').

Also disclosed are methods of using one or more of the devices described above to predict the efficacy of treatment with the indicated chemotherapeutic agent in a patient having one or more of the specified cancers.

The methods and devices can be used to predict the sensitivity or resistance of a subject (e.g., a cancer patient) diagnosed with a disease condition, e.g., cancer (e.g., cancers of the breast, prostate, lung and bronchus, colon and rectum, urinary bladder, skin, kidney, pancreas, oral cavity and pharynx, ovary, thyroid, parathyroid, stomach, brain, esophagus, liver and intrahepatic bile duct, cervix larynx, heart, testis, small and large intestine, anus, anal canal and anorectum, vulva, gallbladder, pleura, bones and joints, hypopharynx, eye and orbit, nose, nasal cavity and middle ear, nasopharynx, ureter, peritoneum, omentum and mesentery, or gastrointestines, as well as any form of cancer including, e.g., chronic myeloid leukemia, acute lymphocytic leukemia, non-Hodgkin lymphoma, melanoma, carcinoma, basal cell carcinoma, malignant mesothelioma, neuroblastoma, multiple myeloma, leukemia, retinoblastoma, acute myeloid leukemia, chronic lymphocytic leukemia, Hodgkin lymphoma, carcinoid tumors, acute tumor, or soft tissue sarcoma) to a treatment, e.g., treatment with a compound or drug, e.g., a chemotherapeutic agent, or radiation, such as those treatments listed above.

Disclosed is a kit that includes a device having at least one or more single-stranded nucleic acid molecules (e.g., deoxyribonucleic acid (DNA) or ribonucleic acid (RNA) molecules) that are complementary to or identical to at least 5 consecutive nucleotides (more preferably at least 10, 15, 20, 25, 30, 35, 40, 45, 50, 55, 60, 65, 70, 75, 80, 85, 90,95, 100, 150,200, 250, 300, or more consecutive nucleotides; the nucleic acid molecule can also be 5-20, 25, 5-50, 50-100, or over 100 consecutive nucleotides long) of at least one of the biomarkers set forth above, such that the single stranded nucleic acid molecules are sufficient for the detection of expression of the biomarkers (e.g., in a sample from a patient or prepared from a patient sample) by allowing specific hybridization between the single stranded nucleic acid molecules and one or more of the nucleic acid molecules corresponding to the biomarker, or a complement thereof. In a disclosure , the kit includes one or more of the devices (e.g., a device that can be used to predict the efficacy of therapy with an alkylating agent, an antimetabolite, an anthracycline, a topoisomerase inhibitor, a plant alkaloid, a corticosteroid, and/or a platinum agent). The kit further includes instructions for applying nucleic acid molecules collected from a sample from a cancer patient (e.g., from a cell, tissue, or organ of the patient), determining the level of expression of the biomarkers in the sample that hybridize to the single stranded nucleic acid molecule(s), and predicting the patient's sensitivity to at least one or more treatments for cancer when use of the kit establishes that the expression level of the biomarkers is changed (i.e., increased or decreased relative to a control sample (i.e., a cell, tissue, and/or organ) known to be sensitive or resitant to the treatment(s), as is discussed above in connection with the first aspect of the invention). It is disclosed that the instructions further indicate that an alteration in the expression level of the biomarker(s) relative to the expression of the biomarker(s) in a control sample (e.g., a cell, tissue, and/or organ known to be sensitive or resistant to the treatment(s)) indicates a change in sensitivity of the patient to the treatment(s) (i.e., a decrease in the level of expression of a biomarker (e.g., a gene and/or RNA (e.g., a miRNA)) known to be expressed in cells sensitive to the treatment(s) indicates that the patient is becoming resistant to the treatment(s) or is likely to become resistant to the treatment(s), and vice versa).

The kit can be utilized to determine a patient's resistance or sensitivity to at least one or more of vincristine, cisplatin, etoposide, azaguanine, carboplatin, adriamycin, aclarubicin, mitoxantrone, mitoxantrone, mitomycin, paclitaxel, gemcitabine, taxotere, dexamethasone, ara-c, methylprednisolone, methotrexate, bleomycin, methyl-gag, belinostat, carboplatin, 5-fu (5-fluorouracil), idarubicin, melphalan, IL4-PR38, valproic acid, all-trans retinoic acid (ATRA), cytoxan, topotecan, suberoylanilide hydroxamic acid (SAHA, vorinostat), depsipeptide (FR901228), bortezomib, leukeran, fludarabine, vinblastine, busulfan, dacarbazine, oxaliplatin, hydroxyurea, tegafur, daunorubicin, estramustine, mechlorethamine, streptozocin, carmustine, lomustine, mercaptopurine, teniposide, dactinomycin, tretinoin, ifosfamide, tamoxifen, irinotecan, floxuridine, thioguanine, PSC 833, erlotinib, herceptin, celecoxib, fulvestrant, iressa, anastrozole, letrozole, cetuximab, rituximab, radiation, histone deacetylase (HDAC) inhibitors, and/or 5-Aza-2'-deoxycytidine (decitabine) by determining the expression level of one or more of the biomarkers set forth above and known to be increased in a patient sensitive to treatment with one or more of these agents (i.e., a patient is determined to be sensitive, or is likely to be sensitive, to the indicated treatment if the level of expression of one or more of the biomarkers increases relative to the level of expression of the biomarkers in a control sample (i.e., a cell, tissue, or organ) in which increased expression of the biomarkers indicates sensitivity to the treatment, and vice versa).

In a disclosure, the nucleic acid molecules of the methods and/or devices are characterized by their ability to specifically identify nucleic acid molecules complementary to or identical to the biomarkers in a sample collected from a cancer patient.

Disclosed is a method of identifying biomarkers indicative of sensitivity of a cancer patient to a treatment for cancer by obtaining pluralities of measurements of the expression level of one or more gene(s) and/or RNA(s) (e.g., a miRNA)). For example, the measurements can be obtained by detecting the expression of one or more genes and/or RNAs using a single probe or by using multiple probes directed to a single gene or RNA. The methods can include assaying the expression levels of the gene(s) and/or RNA(s) in one or more different cell types and/or measuring the growth of those cell types in the presence of a treatment for cancer relative to the growth of the cell types in the absence of the treatment for cancer. The method further includes correlating each plurality of measurements of the expression level of the gene and/or RNAs in cells with the growth of the cells to obtain a correlation coefficient; selecting the median correlation coefficient calculated for the gene(s) and/or RNA(s); and identifying the gene(s) and/or RNA(s) as a biomarker for use in determining the sensitivity of a cancer patient to one or more treatments for cancer if said median correlation coefficient exceeds 0.3 (preferably the gene and/or RNA is identified as a biomarker for a patient's sensitivity to a treatment if the correlation coefficient exceeds 0.4, 0.5, 0.6, 0.7, 0.8. 0.9, 0.95, or 0.99 or more). In a disclosure, the method is performed in the presence of a second treatment.

Disclosed is a method of predicting sensitivity of a patient (e.g., a cancer patient) to a treatment for cancer by obtaining a measurement of the expression of a biomarker gene or RNA (e.g., a miRNA) from a sample (e.g., a cell or tissue) from the patient; applying a model predictive of sensitivity to a treatment for cancer to the measurement, in which the model is developed using an algorithm selected from the group consisting of linear sums, nearest neighbor, nearest centroid, linear discriminant analysis, support vector machines, and neural networks; and predicting whether or not the patient will be responsive to the treatment for cancer. In a disclosure , the measurement is obtained by assaying expression of the gene and/or RNA biomarker in a cell known to be sensitive or resistant to the treatment. In another disclosure , the model combines the outcomes of linear sums, linear discriminant analysis, support vector machines, neural networks, k-nearest neighbors, and nearest centroids, or the model is cross-validated using a random sample of multiple measurements. In another disclosure , treatment, e.g., a compound, has previously failed to show efficacy in a patient. In several disclosures , the linear sum is compared to a sum of a reference population with known sensitivity; the sum of a reference population is the median of the sums derived from the population members' biomarker gene and/or RNA expression. In another disclosure , the model is derived from the components of a data set obtained by independent component analysis or is derived from the components of a data set obtained by principal component analysis.

Disclosed is a kit, apparatus, and software used to implement the method.

According to an embodiment of the invention, the expression level of the gene(s) is determined by detecting the level of mRNA transcribed from the gene(s), or by detecting the level of RNA(s) (e.g., miRNA(s)) expressed from noncoding regions.

In further disclosure, an increase or decrease in the expression level of the gene(s) and/or RNA(s), relative to the expression level of the gene(s) and/or RNA(s) in a cell, tissue, or organ sensitive to the treatment(s), indicates increased sensitivity of the cancer patient to the treatment(s). Alternatively, an increase or decrease in the expression level of the gene(s) and/or RNA(s), relative to the expression level of the gene(s) and/or RNA(s) in a cell, tissue, or organ resistant to the treatment(s), indicates increased resistance of the cancer patient to the treatment(s). According to the use of the invention, the cell is a cancer cell. In another embodiment of all aspects of the invention, the expression level of one or more of the biomarkers described herein is measured using a quantitative reverse transcription-polymerase chain reaction (qRT-PCR). It is disclosed that the level of expression of two or more of the biomarkers described herein (e.g., two of more of the biomarkers in one or more of Tables 1-65 and/or one or more of Tables 66-129) is performed, more preferably the level of expression of three, four, five, six, seven, eight, nine, or ten of the biomarkers described herein is performed, and most preferably twenty, thirty, forty, fifty, sixty, seventy, eighty, ninety, or one hundred or more of the biomarkers described herein is performed (e.g., any combination of two or more of the biomarkers (e.g., the combination of hsa-miR-766_st with one one or more of the other biomarkers described herein). In another embodiment of all aspects of the invention, the expression level of the biomarker(s) is determined usine the device as specified in the claims.

In another embodiment of all aspects of the invention, the treatment is a compound selected from etoposide, adriamycin, paclitaxel, dexamethasone, methylprednisolone, belinostat, 5-Aza-2'-deoxycytidine (decitabine), melphalan, suberoylanilide hydroxamic acid (SAHA, vorinostat), leukeran, fludarabine, busulfan, dacarbazine, hydroxyurea, daunorubicin, mechlorethamine, carmustine, lomustine, mercaptopurine, teniposide, dactinomycin, ifosfamide, irinotecan, and thioguanine.

It is disclosed that one or more of the treatments has previously failed to show effect in a subject (e.g., a subject selected from a subpopulation predicted to be sensitive to the treatment(s), a subject selected from a subpopulation predicted to die without treatment(s), a subject selected from a subpopulation predicted to have disease symptoms without treatment(s), a subject selected from a subpopulation predicted to be cured without treatments).

Disclosed is that the treatment is, e.g., administration of a compound, a protein, an antibody, an oligonucleotide, a chemotherapeutic agent, or radiation to a patient. It is disclosed that the treatment is, e.g., a chemotherapeutic agent, such as, e.g., Vincristine, Cisplatin, Azaguanine, Etoposide, Adriamycin, Aclarubicin, Mitoxantrone, Mitomycin, Paclitaxel, Gemcitabine, Taxotere, Dexamethasone, Ara-C, Methylprednisolone, Methotrexate, Bleomycin, Methyl-GAG, Carboplatin, 5-FU (5-Fluorouracil), MABTHERA™ (Rituximab), histone deacetylase (HDAC) inhibitors, 5-Aza-2'-deoxycytidine (Decitabine), alpha emitters such as astatine-211, bismuth-212, bismuth-213, lead-212, radium-223, actinium-225, and thorium-227, beta emitters such as tritium, strontium-90, cesium-137, carbon-11, nitrogen-13, oxygen-15, fluorine-18, iron-52, cobalt-55, cobalt-60, copper-61, copper-62, copper-64, zinc-62, zinc-63, arsenic-70, arsenic-71, arsenic-74, bromine-76, bromine-79, rubidium-82, yttrium-86, zirconium-89, indium-110, iodine-120, iodine-124, iodine-129, iodine-131, iodine-125, xenon-122, technetium-94m, technetium-94, technetium-99m, and technetium-99, gamma emitters such as cobalt-60, cesium-137, and technetium-99m, Alemtuzumab, Daclizumab, Rituximab (MABTHERA™), Trastuzumab (HERCEPTIN™), Gemtuzumab, Ibritumomab, Edrecolomab, Tositumomab, CeaVac, Epratuzumab, Mitumomab, Bevacizumab, Cetuximab, Edrecolomab, Lintuzumab, MDX-210, IGN-101, MDX-010, MAb, AME, ABX-EGF, EMD 72 000, Apolizumab, Labetuzumab, ior-t1, MDX-220, MRA, H-11 scFv, Oregovomab, huJ591 MAb, BZL, Visilizumab, TriGem, TriAb, R3, MT-201, G-250, unconjugated, ACA-125, Onyvax-105, CDP-860, BrevaRex MAb, AR54, IMC-1C11, GlioMAb-H, ING-1, Anti-LCG MAbs, MT-103, KSB-303, Therex, KW-2871, Anti-HMI.24, Anti-PTHrP, 2C4 antibody, SGN-30, TRAIL-RI MAb, CAT, Prostate cancer antibody, H22xKi-4, ABX-MA1, Imuteran, Monopharm-C, Acivicin, Aclarubicin, Acodazole Hydrochloride, Acronine, Adozelesin, Adriamycin, Aldesleukin, Altretamine, Ambomycin, A. metantronc Acetate, Aminoglutethimide, Amsacrine, Anastrozole, Anthramycin, Asparaginase, Asperlin, Azacitidine, Azetepa, Azotomycin, Batimastat, Benzodepa, Bicalutamide, Bisantrene Hydrochloride, Bisnafide Dimesylate, Bizelesin, Bleomycin Sulfate, Brequinar Sodium, Bropirimine, Busulfan, Cactinomycin, Calusterone, Camptothecin, Caracemide, Carbetimer, Carboplatin, Carmustine, Carubicin Hydrochloride, Carzelesin, Cedefingol, Chlorambucil, Cirolemycin, Cisplatin, Cladribine, Combretestatin A-4, Crisnatol Mesylate, Cyclophosphamide, Cytarabine, Dacarbazine, DACA (N- [2- (Dimethyl-amino) ethyl] acridine-4-carboxamide), Dactinomycin, Daunorubicin Hydrochloride, Daunomycin, Decitabine, Dexormaplatin, Dezaguanine, Dezaguanine Mesylate, Diaziquone, Docetaxel, Dolasatins, Doxorubicin, Doxorubicin Hydrochloride, Droloxifene, Droloxifene Citrate, Dromostanolone Propionate, Duazomycin, Edatrexate, Eflornithine Hydrochloride, Ellipticine, Elsamitrucin, Enloplatin, Enpromate, Epipropidine, Epirubicin Hydrochloride, Erbulozole, Esorubicin Hydrochloride, Estramustine, Estramustine Phosphate Sodium,

Etanidazole, Ethiodized Oil I 131, Etoposide, Etoposide Phosphate, Etoprinc, Fadrozole Hydrochloride, Fazarabine, Fenretinide, Floxuridine, Fludarabine Phosphate, Fluorouracil, 5-FdUMP, Flurocitabine, Fosquidone, Fostriecin Sodium, Gemcitabine, Gemcitabine Hydrochloride, Gold Au 198, Homocamptothecin, Hydroxyurea, Idarubicin Hydrochloride, Ifosfamide, Ilmofosine, Interferon Alfa-2a, Interferon Alfa-2b, Interferon Alfa-nl, Interferon Alfa-n3, Interferon Beta-I a, Interferon Gamma-I b, Iproplatin, Irinotecan Hydrochloride, Lanreotide Acetate, Letrozole, Leuprolide Acetate, Liarozole Hydrochloride, Lometrexol Sodium, Lomustine, Losoxantrone Hydrochloride, Masoprocol, Maytansine, Mechlorethamine Hydrochloride, Megestrol Acetate, Melengestrol Acetate, Melphalan, Menogaril, Mercaptopurine, Methotrexate, Methotrexate Sodium, Metoprine, Meturedepa, Mitindomide, Mitocarcin, Mitocromin, Mitogillin, Mitomalcin, Mitomycin, Mitosper, Mitotane, Mitoxantrone Hydrochloride, Mycophenolic Acid, Nocodazole, Nogalamycin,Ormaplatin, Oxisuran, Paclitaxel, Pegaspargase, Peliomycin, Pentamustine, PeploycinSulfate, Perfosfamide, Pipobroman, Piposulfan, Piroxantrone Hydrochloride, Plicamycin, Plomestane, Porfimer Sodium, Porfiromycin, Prednimustine, Procarbazine Hydrochloride, Puromycin, Puromycin Hydrochloride, Pyrazofurin, Rhizoxin, Rhizoxin D, Riboprine, Rogletimide, Safingol, Safingol Hydrochloride, Semustine, Simtrazene, Sparfosate Sodium, Sparsomycin, Spirogermanium Hydrochloride, Spiromustine, Spiroplatin, Streptonigrin, Streptozocin, Strontium Chloride Sr 89, Sulofenur, Talisomycin, Taxane, Taxoid, Tecogalan Sodium, Tegafur, Teloxantrone Hydrochloride, Temoporfin, Teniposide, Teroxirone, Testolactone, Thiamiprine, Thioguanine, Thiotepa, Thymitaq, Tiazofurin, Tirapazamine, Tomudex, TOP53, Topotecan Hydrochloride, Toremifene Citrate, Trestolone Acetate, Triciribine Phosphate, Trimetrexate, Trimetrexate Glucuronate, Triptorelin, Tubulozole Hydrochloride, Uracil Mustard, Uredepa, Vapreotide, Verteporfin, Vinblastine, Vinblastine Sulfate, Vincristine, Vincristine Sulfate, Vindesine, Vindesine Sulfate, Vinepidine Sulfate, Vinglycinate Sulfate, Vinleurosine Sulfate, Vinorelbine Tartrate, Vinrosidine Sulfate, Vinzolidine Sulfate, Vorozole, Zeniplatin, Zinostatin, Zorubicin Hydrochloride, 2-Chlorodeoxyadenosine, 2'Deoxyformycin, 9-aminocamptothecin, raltitrexed, N-propargyl-5,8-dideazafolic acid, 2chloro-2'-arabino-fluoro-2'-deoxyadenosine, 2-chloro-2'-deoxyadenosine, anisomycin, trichostatin A, hPRL-G129R, CEP-751, linomide, sulfur mustard, nitrogen mustard (mechlor ethamine), cyclophosphamide, melphalan, chlorambucil, ifosfamide, busulfan, N-methyl-Nnitrosourea (MNU), N, N'-Bis(2-chloroethyl)-N-nitrosourea (BCNU), N-(2-chloroethyl)-N'cyclohexyl-N-nitrosourea (CCNU), N-(2-chloroethyl)-N'-(trans-4-methylcyclohexyl-N-nitrosourea (MeCCNU), N-(2-chloroethyl)-N'-(diethyl) ethylphosphonate-N-nitrosourea (fotemustine), streptozotocin, diacarbazine (DTIC), mitozolomide, temozolomide, thiotepa, mitomycin C, AZQ, adozelesin, Cisplatin, Carboplatin, Ormaplatin, Oxaliplatin,C1-973, DWA 2114R, JM216, JM335, Bis (platinum), tomudex, azacitidine, cytarabine, gemcitabine, 6-Mercaptopurine, 6-Thioguanine, Hypoxanthine, teniposide 9-amino camptothecin, Topotecan, CPT-11, Doxorubicin, Daunomycin, Epirubicin, darubicin, mitoxantrone, losoxantrone, Dactinomycin (Actinomycin D), amsacrine, pyrazoloacridine, all-trans retinol, 14-hydroxy-retro-retinol, all-trans retinoic acid, N-(4-Hydroxyphenyl)retinamide, 13-cis retinoic acid, 3-Methyl TTNEB, 9-cis retinoic acid, fludarabine (2-F-ara-AMP), 2-chlorodeoxyadenosine (2-Cda), 20-pi-1,25 dihydroxyvitamin D3, 5-ethynyluracil, abiraterone, aclarubicin, acylfulvene, adecypenol, adozelesin, aldesleukin, ALL-TK antagonists, altretamine, ambamustine, amidox, amifostine, aminolevulinic acid, amrubicin, amsacrine, anagrelide, anastrozole, andrographolide, angiogenesis inhibitors, antagonist D, antagonist G, antarelix, anti-dorsalizing morphogenetic protein-1, antiandrogen, prostatic carcinoma, antiestrogen, antineoplaston, antisense oligonucleotides, aphidicolin glycinate, apoptosis gene modulators, apoptosis regulators, apurinic acid, ara-CDP-DL-PTBA, argininedeaminase, asulacrine, atamestane, atrimustine, axinastatin 1, axinastatin 2, axinastatin 3, azasetron, azatoxin, azatyrosine, baccatin III derivatives, balanol, batimastat, BCR/ABL antagonists, benzochlorins, benzoylstaurosporine, beta lactam derivatives, beta-alethine, betaclamycin B, betulinic acid, bFGF inhibitor, bicalutamide, bisantrene, bisaziridinylspermine, bisnafide, bistratene A, bizelesin, breflate, bleomycin A2, bleomycin B2, bropirimine, budotitane, buthionine sulfoximine, calcipotriol, calphostin C, camptothecin derivatives (e.g., 10-hydroxy-camptothecin), canarypox IL-2, capecitabine, carboxamide-amino-triazole, carboxyamidotriazole, CaRest M3, CARN 700, cartilage derived inhibitor, carzelesin, casein kinase inhibitors (ICOS), castanospermine, cecropin B, cetrorelix, chlorins, chloroquinoxaline sulfonamide, cicaprost, cis-porphyrin, cladribine, clomifene analogues, clotrimazole, collismycin A , collismycin B, combretastatin A4, combretastatin analogue, conagenin, crambescidin 816, crisnatol, cryptophycin 8, cryptophycin A derivatives, curacin A, cyclopentanthraquinones, cycloplatam, cypemycin, cytarabine ocfosfate, cytolytic factor, cytostatin, dacliximab, decitabine, dehydrodidemnin B, 2'deoxycoformycin (DCF), deslorelin, dexifosfamide, dexrazoxane, dexverapamil, diaziquone, didemnin B, didox, diethylnorspermine, dihydro-5-azacytidine, dihydrotaxol, 9-, dioxamycin, diphenyl spiromustine, discodermolide, docosanol, dolasetron, doxifluridine, droloxifene, dronabinol, duocarmycin SA, ebselen, ecomustine, edelfosine, edrecolomab, eflornithine, elemene, emitefur, epirubicin, epothilones (A, R = H, B, R = Me), epithilones, epristeride, estramustine analogue, estrogen agonists, estrogen antagonists, etanidazole, etoposide, etoposide 4'-phosphate (etopofos), exemestane, fadrozole, fazarabine, fenretinide, filgrastim, finasteride, flavopiridol, flezelastine, fluasterone, fludarabine, fluorodaunorunicin hydrochloride, forfenimex, formestane, fostriecin, fotemustine, gadolinium texaphyrin, gallium nitrate, galocitabine, ganirelix, gelatinase inhibitors, gemcitabine, glutathione inhibitors, hepsulfam, heregulin, hexamethylene bisacetamide, homoharringtonine (HHT), hypericin, ibandronic acid, idarubicin, idoxifene, idramantone, ilmofosine, ilomastat, imidazoacridones, imiquimod, immunostimulant peptides, insulin-like growth factor-1 receptor inhibitor, interferon agonists, interferons, interleukins, iobenguane, iododoxorubicin, ipomeanol, 4-, irinotecan, iroplact, irsogladine, isobengazole, isohomohalicondrin B, itasetron,

jasplakinolide, kahalalide F, lamellarin-N triacetate, lanreotide, leinamycin, lenograstim, lentinan sulfate, leptolstatin, letrozole, leukemia inhibiting factor, leukocyte alpha interferon, leuprolide + estrogen + progesterone, leuprorelin, levamisole, liarozole, linear polyamine analogue, lipophilic disaccharide peptide, lipophilic platinum compounds, lissoclinamide 7, lobaplatin, lombricine, lometrexol, lonidamine, losoxantrone, lovastatin, loxoribine, lurtotecan, lutetium texaphyrin, lysofylline, lytic peptides, maytansine, mannostatin A, marimastat, masoprocol, maspin, matrilysin inhibitors, matrix metalloproteinase inhibitors, menogaril, rnerbarone, meterelin, methioninase, metoclopramide, MIF inhibitor, ifepristone, miltefosine, mirimostim, mismatched double stranded RNA, mithracin, mitoguazone, mitolactol, mitomycin analogues, mitonafide, mitotoxin fibroblast growth factor-saporin, mitoxantrone, mofarotene, molgramostim, monoclonal antibody, human chorionic gonadotrophin, monophosphoryl lipid A + myobacterium cell wall sk, mopidamol, multiple drug resistance gene inhibitor, multiple tumor suppressor 1-based therapy, mustard anticancer agent, mycaperoxide B, mycobacterial cell wall extract, myriaporone, N-acetyldinaline, N-substituted benzamides, nafarelin, nagrestip, naloxone + pentazocine, napavin, naphterpin, nartograstim, nedaplatin, nemorubicin, neridronic acid, neutral endopeptidase, nilutamide, nisamycin, nitric oxide modulators, nitroxide antioxidant, nitrullyn, 06-benzylguanine, octreotide, okicenone, oligonucleotides, onapristone, ondansetron, ondansetron, oracin, oral cytokine inducer, ormaplatin, osaterone, oxaliplatin, oxaunomycin, paclitaxel analogues, paclitaxel derivatives, palauamine, palmitoylrhizoxin, pamidronic acid, panaxytriol, panomifene, parabactin, pazelliptine, pegaspargase, peldesine, pentosan polysulfate sodium, pentostatin, pentrozole, perflubron, perfosfamide, perillyl alcohol, phenazinomycin, phenylacetate, phosphatase inhibitors, picibanil, pilocarpine hydrochloride, pirarubicin, piritrexim, placetin A, placetin B, plasminogen activator inhibitor, platinum complex, platinum compounds, platinum-triamine complex, podophyllotoxin, porfimer sodium, porfiromycin, propyl bis-acridone, prostaglandin J2, proteasome inhibitors, protein A-based immune modulator, protein kinase C inhibitor, protein kinase C inhibitors, microalgal, protein tyrosine phosphatase inhibitors, purine nucleoside phosphorylase inhibitors, purpurins, pyrazoloacridine, pyridoxylated hemoglobin polyoxyethylene conjugate, raf antagonists, raltitrexed, ramosetron, ras farnesyl protein transferase inhibitors, ras inhibitors, ras-GAP inhibitor, retelliptine demethylated, rhenium Re 186 etidronate, rhizoxin, ribozymes, RII retinamide, rogletimide, rohitukine, romurtide, roquinimex, rubiginone B 1, ruboxyl, safingol, saintopin, SarCNU, sarcophytol A, sargramostim, Sdi 1 mimetics, semustine, senescence derived inhibitor 1, sense oligonucleotides, signal transduction inhibitors, signal transduction modulators, single chain antigen binding protein, sizofiran, sobuzoxane, sodium borocaptate, sodium phenylacetate, solverol, somatomedin binding protein, sonermin, sparfosic acid, spicamycin D, spiromustine, splenopentin, spongistatin 1, squalamine, stem cell inhibitor, stem-cell division inhibitors, stipiamide, stromelysin inhibitors, sulfinosine, superactive vasoactive intestinal peptide antagonist, suradista, suramin, swainsonine, synthetic glycosaminoglycans, tallimustine, tamoxifen methiodide, tauromustine, tazarotene, tecogalan sodium, tegafur, tellurapyrylium, telomerase inhibitors, temoporfin, temozolomide, teniposide, tetrachlorodecaoxide, tetrazomine, thaliblastine, thalidomide, thiocoraline, thrombopoietin, thrombopoietin mimetic, thymalfasin, thymopoietin receptor agonist, thymotrinan, thyroid stimulating hormone, tin ethyl etiopurpurin, tirapazamine, titanocene dichloride, topotecan, topsentin, toremifene, totipotent stem cell factor, translation inhibitors, tretinoin, triacetyluridine, triciribine, trimetrexate, triptorelin, tropisetron, turosteride, tyrosine kinase inhibitors, tyrphostins, UBC inhibitors, ubenimex, urogenital sinus-derived growth inhibitory factor, urokinase receptor antagonists, vapreotide, variolin B, vector system, erythrocyte gene therapy, velaresol, veramine, verdins, verteporfin, vinorelbine, vinxaltine, vitaxin, vorozole, zanoterone, zeniplatin, zilascorb, or zinostatin stimalamer. In another disclosure, a second treatment is utilized to determine gene expression in a sample from the patient.

## DEFINITIONS

"Resistant" or "resistance" as used herein means that a cell, a tumor, a person, or a living organism is able to withstand treatment, e.g., with a compound, such as a chemotherapeutic agent or radiation treatment, in that the treatment inhibits the growth of a cell, e.g., a cancer cell, *in vitro* or in a tumor, person, or living organism by less than 10%, 20%, 30%, 40%, 50%, 60%, or 70% relative to the growth of a cell not exposed to the treatment. Resistance to treatment may be determined by a cell-based assay that measures the growth of treated cells as a function of the cells' absorbance of an incident light beam as used to perform the NCI60 assays described herein. In this example, greater absorbance indicates greater cell growth, and thus, resistance to the treatment. A smaller reduction in growth indicates more resistance to a treatment. By "chemoresistant" or "chemoresistance" is meant resistance to a compound.

"Sensitive" or "sensitivity" as used herein means that a cell, a tumor, a person, or a living organism is responsive to treatment, e.g., with a compound, such as a chemotherapeutic agent or radiation treatment, in that the treatment inhibits the growth of a cell, e.g., a cancer cell, *in vitro* or in a tumor, person, or living organism by 70%, 80%, 90%, 95%, 99% or 100%. Sensitivity to treatment may be determined by a cell-based assay that measures the growth of treated cells as a function of the cells' absorbance of an incident light beam as used to perform the NCI60 assays described herein. In this example, lesser absorbance indicates lesser cell growth, and thus, sensitivity to the treatment. A greater reduction in growth indicates more sensitivity to the treatment. By "chemosensitive" or "chemosensitivity" is meant sensitivity to a compound.

"Complement" of a nucleic acid sequence or a "complementary" nucleic acid sequence as used herein refers to an oligonucleotide which is in "antiparallel association" when it is aligned with the nucleic acid sequence such that the 5' end of one sequence is paired with the 3' end of the other. Nucleotides and other bases may have complements and may be present in complementary nucleic acids. Bases not commonly found in natural nucleic acids that may be included in the nucleic acids of the present invention include, for example, inosine and 7-deazaguanine. "Complementarity" may not be perfect; stable duplexes of complementary nucleic acids may contain mismatched base pairs or unmatched bases. Those skilled in the art of nucleic acid technology can determine duplex stability empirically considering a number of variables including, for example, the length of the oligonucleotide, percent concentration of cytosine and guanine bases in the oligonucleotide, ionic strength, and incidence of mismatched base pairs.

When complementary nucleic acid sequences form a stable duplex, they are said to be "hybridized" or to "hybridize" to each other or it is said that "hybridization" has occurred. Nucleic acids are referred to as being "complementary" if they contain nucleotides or nucleotide homologues that can form hydrogen bonds according to Watson-Crick base-pairing rules (e.g., G with C, A with T or A with U) or other hydrogen bonding motifs such as for example diaminopurine with T, 5-methyl C with G, 2-thiothymidine with A, inosine with C, pseudoisocytosine with G, etc. Anti-sense RNA may be complementary to other oligonucleotides, e.g., mRNA.

"Gene" as used herein indicates a a coding or noncoding gene whose activity can be determined by measuring the produced RNA. Examples include protein coding genes, microRNAs, small nuclear RNAs and other RNAs with catalytic, regulatory or coding properties.

"Biomarker" as used herein indicates a gene or RNA (e.g., a miRNA) whose expression indicates sensitivity or resistance to a treatment.

"Compound" as used herein means a chemical or biological substance, e.g., a drug, a protein, an antibody, or an oligonucleotide, which may be used to treat a disease or which has biological activity *in vivo* or *in vitro.* Preferred compounds may or may not be approved by the U.S. Food and Drug Administration (FDA). Preferred compounds include, e.g., chemotherapy agents that may inhibit cancer growth. Preferred chemotherapy agents include, e.g., Vincristine, Cisplatin, Azaguanine, Etoposide, Adriamycin, Aclarubicin, Mitoxantrone, Mitomycin, Paclitaxel, Gemcitabine, Taxotere, Dexamethasone, Ara-C, Methylprednisolone, Methotrexate, Bleomycin, Methyl-GAG, Carboplatin, 5-FU (5-Fluorouracil), MABTHERA™ (Rituximab), radiation, histone deacetylase (HDAC) inhibitors, and 5-Aza-2'-deoxycytidine (Decitabine). Exemplary radioactive chemotherapeutic agents include compounds containing alpha emitters such as astatine-211, bismuth-212, bismuth-213, lead-212, radium-223, actinium-225, and thorium-227, beta emitters such as tritium, strontium-90, cesium-137, carbon-11, nitrogen-13, oxygen-15, fluorine-18, iron-52, cobalt-55, cobalt-60, copper-61, copper-62, copper-64, zinc-62, zinc-63, arsenic-70, arsenic-71, arsenic-74, bromine-76, bromine-79, rubidium-82, yttrium-86, zirconium-89, indium-110, iodine-120, iodine-124, iodine-129, iodine-131, iodine-125, xenon-122, technetium-94m, technetium-94, technetium-99m, and technetium-99, or gamma emitters such as cobalt-60, cesium-137, and technetium-99m. Exemplary chemotherapeutic agents also include antibodies such as Alemtuzumab, Daclizumab, Rituximab (MABTHERA™), Trastuzumab (HERCEPTIN™), Gemtuzumab, Ibritumomab, Edrecolomab, Tositumomab, CeaVac, Epratuzumab, Mitumomab, Bevacizumab, Cetuximab, Edrecolomab, Lintuzumab, MDX-210, IGN-101, MDX-010, MAb, AME, ABX-EGF, EMD 72 000, Apolizumab, Labetuzumab, ior-t1, MDX-220, MRA, H-11 scFv, Oregovomab, huJ591 MAb, BZL, Visilizumab, TriGem, TriAb, R3, MT-201, G-250, unconjugated, ACA-125, Onyvax-105, CDP-860, BrevaRex MAb, AR54, IMC-1C11, GlioMAb-H, ING-1, Anti-LCG MAbs, MT-103, KSB-303, Therex, KW-2871, Anti-HMI.24, Anti-PTHrP, 2C4 antibody, SGN-30, TRAIL-RI MAb, CAT, Prostate cancer antibody, H22xKi-4, ABX-MA1, Imuteran, and Monopharm-C. Exemplary chemotherapeutic agents also include Acivicin; Aclarubicin; Acodazole Hydrochloride; Acronine; Adozelesin; Adriamycin; Aldesleukin; Altretamine; Ambomycin; A. metantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Camptothecin; Caracemide; Carbetimer; Carboplatin; Carmustine; Carubicin Hydrochloride; Carzelesin; Cedefingol; Chlorambucil; Cirolemycin; Cisplatin; Cladribine; Combretestatin A-4; Crisnatol Mesylate; Cyclophosphamide; Cytarabine; Dacarbazine; DACA (N-[2-(Dimethyl-amino) ethyl] acridine-4-carboxamide); Dactinomycin; Daunorubicin Hydrochloride; Daunomycin; Decitabine; Dexormaplatin; Dezaguanine; Dezaguanine Mesylate; Diaziquone; Docetaxel; Dolasatins; Doxorubicin; Doxorubicin Hydrochloride; Droloxifene; Droloxifene Citrate; Dromostanolone Propionate; Duazomycin; Edatrexate; Eflornithine Hydrochloride; Ellipticine; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Ethiodized Oil I 131; Etoposide; Etoposide Phosphate; Etoprine; Fadrozole Hydrochloride; Fazarabine; Fenretinide; Floxuridine; Fludarabine Phosphate; Fluorouracil; 5-FdUMP; Flurocitabine; Fosquidone; Fostriecin Sodium; Gemcitabine; Gemcitabine Hydrochloride; Gold Au 198; Homocamptothecin; Hydroxyurea; Idarubicin Hydrochloride; Ifosfamide; Ilmofosine; Interferon Alfa-2a; Interferon Alfa-2b; Interferon Alfa-nl; Interferon Alfan-3; Interferon Beta-I a; Interferon Gamma-I b; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Nocodazole; Nogalamycin; Ormaplatin; Oxisuran; Paclitaxel; Pegaspargase; Peliomycin; Pentamustine; PeploycinSulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Rhizoxin; Rhizoxin D; Riboprine; Rogletimide; Safingol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Strontium Chloride Sr 89; Sulofenur; Talisomycin; Taxane; Taxoid; Tecogalan Sodium; Tegafur; Teloxantrone Hydrochloride; Temoporfin; Teniposide; Teroxirone; Testolactone; Thiamiprine; Thioguanine; Thiotepa; Thymitaq; Tiazofurin; Tirapazamine; Tomudex; TOP53; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine; Vinblastine Sulfate; Vincristine; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride; 2-Chlorodeoxyadenosine; 2' Deoxyformycin; 9-aminocamptothecin; raltitrexed; N-propargyl-5,8-dideazafolic acid; 2chloro-2'-arabino-fluoro-2'-deoxyadenosine; 2-chloro-2'-deoxyadenosine; anisomycin; trichostatin A; hPRL-G129R; CEP-751; linomide; sulfur mustard; nitrogen mustard (mechlor ethamine); cyclophosphamide; melphalan; chlorambucil; ifosfamide; busulfan; N-methyl-Nnitrosourea (MNU); N, N'-Bis (2-chloroethyl)-N-nitrosourea (BCNU); N-(2-chloroethyl)-N'cyclohexyl-N-nitrosourea (CCNU); N- (2-chloroethyl)-N'-(trans-4-methylcyclohexyl-N-nitrosourea (MeCCNU); N- (2-chloroethyl)-N'-(diethyl)ethylphosphonate-N-nitrosourea (fotemustine); streptozotocin; diacarbazine (DTIC); mitozolomide; temozolomide; thiotepa; mitomycin C; AZQ; adozelesin; Cisplatin; Carboplatin; Ormaplatin; Oxaliplalin; C1-973; DWA 2114R; JM216; JM335; Bis (platinum); tomudex; azacitidine; cytarabine; gemcitabine; 6-Mercaptopurine; 6-Thioguanine; Hypoxanthine; teniposide 9-amino camptothecin; Topotecan; CPT-11; Doxorubicin; Daunomycin; Epirubicin; darubicin; mitoxantrone; losoxantrone; Dactinomycin (Actinomycin D); amsacrine; pyrazoloacridine; all-trans retinol; 14-hydroxy-retro-retinol; all-trans retinoic acid; N-(4- Hydroxyphenyl) retinamide; 13-cis retinoic acid; 3-Methyl TTNEB; 9-cis retinoic acid; fludarabine (2-F-ara-AMP); or 2-chlorodeoxyadenosine (2-Cda).

Other chemotherapeutic agents include, but are not limited to, 20-pi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; argininedeaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bleomycin A2; bleomycin B2; bropirimine; budotitane; buthionine sulfoximine; calcipotriol; calphostin C; camptothecin derivatives (e.g., 10-hydroxy-camptothecin); canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorins; chloroquinoxaline sulfonamide; cicaprost; cis-porphyrin; cladribine; clomifene analogues; clotrimazole; collismycin A ; collismycin B; combretastatin A4; combretastatin analogue; conagenin; crambescidin 816 ; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; 2'deoxycoformycin (DCF); deslorelin; dexifosfamide; dexrazoxane; dexverapamil; diaziquone; didemnin B; didox; diethylnorspermine; dihydro-5-azacytidine; dihydrotaxol, 9- ; dioxamycin; diphenyl spiromustine; discodermolide; docosanol; dolasetron; doxifluridine; droloxifene; dronabinol; duocarmycin SA; ebselen; ecomustine; edelfosine; edrecolomab; eflornithine; elemene; emitefur; epirubicin; epothilones (A, R = H; B, R = Me); epithilones; epristeride; estramustine analogue; estrogen agonists; estrogen antagonists; etanidazole; etoposide; etoposide 4'-phosphate (etopofos); exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; homoharringtonine (HHT); hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; irinotecan; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide + estrogen + progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maytansine; mannostatin A; marimastat; masoprocol; maspin; matrilysin inhibitors; matrix metalloproteinase inhibitors; menogaril; rnerbarone; meterelin; methioninase; metoclopramide; MIF inhibitor; ifepristone; miltefosine; mirimostim; mismatched double stranded RNA; mithracin; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A + myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone + pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; 06-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; podophyllotoxin; porfimer sodium; porfiromycin; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B 1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thalidomide; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene dichloride; topotecan; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer.

To "inhibit growth" as used herein means causing a reduction in cell growth *in vivo* or *in vitro* by, e.g., 10%, 20%, 30%, 40%, 50%, 60%, 70%, 80%, 90%, 95%, or 99% or more, as evident by a reduction in the size or number of cells exposed to a treatment (e.g., exposure to a compound), relative to the size or number of cells in the absence of the treatment. Growth inhibition may be the result of a treatment that induces apoptosis in a cell, induces necrosis in a cell, slows cell cycle progression, disrupts cellular metabolism, induces cell lysis, or induces some other mechanism that reduces the size or number of cells.

"Marker gene" or "biomarker gene" as used herein means a gene in a cell the expression of which correlates to sensitivity or resistance of the cell (and thus the patient from which the cell was obtained) to a treatment (e.g., exposure to a compound).

"Microarray" as used herein means a device employed by any method that quantifies one or more subject oligonucleotides, e.g., DNA or RNA, or analogues thereof, at a time. One exemplary class of microarrays consists of DNA probes attached to a glass or quartz surface. For example, many microarrays, including those made by Affymetrix, use several probes for determining the expression of a single gene. The DNA microarray may contain oligonucleotide probes that may be, e.g., full-length cDNAs complementary to an RNA or cDNA fragments that hybridize to part of an RNA. Exemplary RNAs include mRNA, miRNA, and miRNA precursors. Exemplary microarrays also include a "nucleic acid microarray" having a substrate-bound plurality of nucleic acids, hybridization to each of the plurality of bound nucleic acids being separately detectable. The substrate may be solid or porous, planar or non-planar, unitary or distributed. Exemplary nucleic acid microarrays include all of the devices so called in Schena (ed.), DNA Microarrays: A Practical Approach (Practical Approach Series), Oxford University Press (1999); Nature Genet. 21(1)(suppl.):1-60 (1999); Schena (ed.), Microarray Biochip: Tools and Technology, Eaton Publishing Company/BioTechniques Books Division (2000). Additionally, exemplary nucleic acid microarrays include substrate-bound plurality of nucleic acids in which the plurality of nucleic acids are disposed on a plurality of beads, rather than on a unitary planar substrate, as is described, inter alia, in Brenner et al., Proc. Natl. Acad. Sci. USA 97(4):1665-1670 (2000). Examples of nucleic acid microarrays may be found in U.S. Pat. Nos. 6,391,623, 6,383,754, 6,383,749, 6,380,377, 6,379,897, 6,376,191, 6,372,431, 6,351,712 6,344,316, 6,316,193, 6,312,906, 6,309,828, 6,309,824, 6,306,643, 6,300,063, 6,287,850, 6,284,497, 6,284,465, 6,280,954, 6,262,216, 6,251,601, 6,245,518, 6,263,287, 6,251,601, 6,238,866, 6,228,575, 6,214,587, 6,203,989, 6,171,797, 6,103,474, 6,083,726, 6,054,274, 6,040,138, 6,083,726, 6,004,755, 6,001,309, 5,958,342, 5,952,180, 5,936,731, 5,843,655, 5,814,454, 5,837,196, 5,436,327, 5,412,087, 5,405,783.

Exemplary microarrays may also include "peptide microarrays" or "protein microarrays" having a substrate-bound plurality of polypeptides, the binding of a oligonucleotide, a peptide, or a protein to each of the plurality of bound polypeptides being separately detectable. Alternatively, the peptide microarray, may have a plurality of binders, including but not limited to monoclonal antibodies, polyclonal antibodies, phage display binders, yeast 2 hybrid binders, aptamers, which can specifically detect the binding of specific oligonucleotides, peptides, or proteins. Examples of peptide arrays may be found in WO 02/31463, WO 02/25288, WO 01/94946, WO 01/88162, WO 01/68671, WO 01/57259, WO 00/61806, WO 00/54046, WO 00/47774, WO 99/40434, WO 99/39210, WO 97/42507 and U.S. Pat. Nos. 6,268,210, 5,766,960, 5,143,854.

"Gene expression" as used herein means the amount of a gene product in a cell, tissue, organism, or subject, e.g., amounts of DNA, RNA, or proteins, amounts of modifications of DNA, RNA, or protein, such as splicing, phosphorylation, acetylation, or methylation, or amounts of activity of DNA, RNA, or proteins associated with a given gene.

"NCI60" as used herein means a panel of 60 cancer cell lines from lung, colon, breast, ovarian, leukemia, renal, melanoma, prostate and brain cancers including the following cancer cell lines: NSCLC_NCIH23, NSCLC_NCIH522, NSCLC_A549ATCC, NSCLC_EKVX, NSCLC_NCIH226, NSCLC_NCIH332M, NSCLC_H460, NSCLC_HOP62, NSCLC_HOP92, COLON_HT29, COLON_HCC-2998, COLON_HCT116, COLON_SW620, COLON_COLO205, COLON_HCT15, COLON_KM12, BREAST_MCF7, BREAST_MCF7ADRr, BREAST_MDAMB231, BREAST_HS578T, BREAST_MDAMB435, BREAST_MDN, BREAST_BT549, BREAST_T47D, OVAR_OVCAR3, OVAR_OVCAR4, OVAR_OVCAR5, OVAR_OVCAR8, OVAR_IGROV1, OVAR_SKOV3, LEUK_CCRFCEM, LEUK_K562, LEUK_MOLT4, LEUK_IIL60, LEUK_RPMI8266, LEUK_SR, RENAL_UO31, RBNAL_SN12C, RENAL_A498, RENAL_CAKI1, RENAL_RXF393, RENAL_7860, RENAL_ACHN, RENAL_TK10, MELAN_LOXIMVI, MELAN_MALME3M, MELAN_SKMEL2, MELAN_SKMEL5, MELAN_SKMEL28, MELAN_M14, MELAN_UACC62, MELAN_UACC257, PROSTATE_PC3, PROSTATE_DU145, CNS_SNB19, CNS_SNB75, CNS_U251, CNS_SF268, CNS_SF295, and CNS_SF539.

"Treatment" or "medical treatment" means administering to a subject or living organism or exposing to a cell or tumor a compound (e.g., a drug, a protein, an antibody, an oligonucleotide, a chemotherapeutic agent, and a radioactive agent) or some other form of medical intervention used to treat or prevent cancer or the symptoms of cancer (e.g., cryotherapy and radiation therapy). Radiation therapy includes the administration to a patient of radiation generated from sources such as particle accelerators and related medical devices that emit X-radiation, gamma radiation, or electron (Beta radiation) beams. A treatment may further include surgery, e.g., to remove a tumor from a subject or living organism.

## BRIEF DESCRIPTION OF THE DRAWINGS

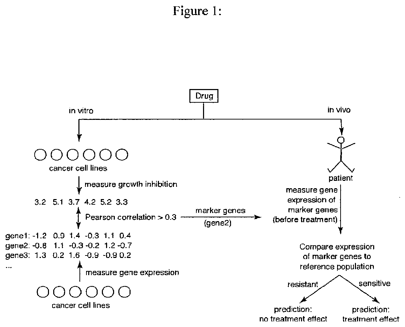

Figure 1 depicts an illustration of the method of identifying biomarkers and predicting patient sensitivity to a medical treatment. The method has an *in vitro* component where the growth inhibition of a compound or medical treatment is measured on cell lines (6 of the 60 cell lines tested are shown). The gene expression is measured on the same cell lines without compound treatment Those genes that have a correlation above a certain cutoff (e.g., a preffered cutoff of 0.3, in which a correlation coefficient equal to or greater than the cutoff of 0.3 is deemed statistcally significant by, e.g., cross-validation) to the growth inhibition are termed marker genes and the expression of those genes *in vivo*, e.g., may predict the sensitivity or resistance of a patient's cancer to a compound or other medical treatment. The *in vivo* component is applied to a patient to determine whether or not the treatment will be effective in treating disease in the patient. Here, the gene expression in cells of a sample of the suspected disease tissue (e.g., a tumor) in the patient is measured before or after treatment. The activity of the marker genes in the sample is compared to a reference population of patients known to be sensitive or resistant to the treatment. The expression of marker genes in the cells of the patient known to be expressed in the cells of reference patients sensitive to the treatment indicates that the patient to be treated is sensitive to the treatment and vice versa. Based on this comparison the patient is predicted to be sensitive or resistant to treatment with the compound.

## DETAILED DESCRIPTION

The present invention relates to the use of a device for predicting the growth inhibition of cancer cells of a cancer patient by at least one treatment for cancer, wherein said device comprises at least one single-stranded nucleic acid molecule that is complementary or identical to at least 5 consecutive nucleotides of hsa-miR-766_st, wherein said at least one single stranded nucleic acid molecule is capable of specifically hybridizing with hsa-miR-766_st or its complement, said device allowing determination of a level of expression of hsa-miR-766_st in a biological sample from said patient,

wherein said use further comprises assaying a level of expression of hsa-miR-766_st in a sample from said patient using said device, and wherein:

said patient is predicted to exhibit growth inhibition of cancer cells by said at least one treatment if said level of expression of hsa-miR-766_st is substantially similar to the level of expression of hsa-miR-766_st in a cell or tissue known to exhibit growth inhibition of cancer cells by said at least one treatment,

wherein said at least one treatment for cancer is selected from the group consisting of etoposide, adriamycin, paclitaxel, dexamethasone, methylprednisolone, belinostat, 5-Aza-2'-deoxycytidine (decitabine), melphalan, suberoylanilide hydroxamic acid (SAHA, vorinostat), leukeran, fludarabine, busulfan, dacarbazine, hydroxyurea, daunorubicin, mechlorethamine, carmustine, lomustine, mercaptopurine, teniposide, dactinomycin, ifosfamide, irinotecan, and thioguanine.

Disclosed are methods for identifying and using one or more biomarkers of treatment sensitivity or resistance, e.g., sensitivity or resistance to one or more treatments for cancer (e.g., a chemothereutic agent), to predict treatment efficacy in a patient (e.g., a patient diagnosed with cancer), devices that include nucleic acid molecules that can detect the level of expression of the biomarkers, and kits that include the devices and instructions for use. The kits include an array (e.g., a microarray) with one or more single-stranded oligonucleotide probes that have at least 85% (e.g., at least 90%, 95%, 99%, or 100%) sequence identity to a target nucleic acid molecule having a sequence that is complementary or identical to the nucleic acid sequence of one or more biomarkers of sensitivity or resistance to treatment (e.g., treatment with a chemotherapeutic agent) described herein. For example, the probes can be used to detect one or more (e.g., two, three, four, five, ten, twenty, thirty, fifty, or all) of the biomarkers listed in one or more of Tables 1-65 and/or one or more of Tables 66-129, such as hsa-miR-766_st. The oligonucleotide probes of the array can be used detect nucleic acid molecules present in sample applied to the array (e.g., nucleic acid molecules from a sample obtained, derived, or prepared from a subject (e.g., a cell, tissue, or organ sample from a subject)). The kits can also include instructions for using the device to predict the sensitivity or resistance of the subject to the treatment.

A beneficial aspect of the disclosure is that the methods, devices, and kits can be used to determine the sensitivity and/or resistance of a patient to one or more treatments for cancer (e.g., two, three, four, five, ten, twenty, thirty, or more treatments for cancer, and even all of the treatments for cancer described herein) at the same time by assaying for the level of expression of one or more biomarkers, the expression of which has been correlated with a patient's sensitivity or resistance to a specific treatment for cancer. For example, the methods, devices, and kits can utilize oligonucleotide probes capable of detecting the level of expression of at least one cancer therapy-sensitive biomarker associated with each of the cancer therapies described herein (e.g., the methods, devices, and kits can utilize oligonucleotide probes to detect at least one (e.g., two, three, or more, e.g. all) of the cancer therapy-specific sensitivity biomarkers listed in one or more of Tables 1-65, or a subset thereof). Alternatively, or in addition to the cancer therapy-sensitive biomarkers, the methods, devices, and kits can utilize oligonucleotide probes capable of detecting the level of expression of at least one cancer therapy-resistant biomarker associated with each of the cancer therapies described herein (e.g., the methods, devices, and kits can utilize oligonucleotide probes to detect at least one (e.g., two, three, or more, e.g. all) of the cancer therapy-resistant sensitivity biomarkers listed in one or more of Tables 66-129, or a subset thereof).

In other examples, the methods, devices, and kits can utilize oligonucleotide probes capable of detecting the level of expression of the cancer therapy-sensitive biomarkers of one or more of Tables 1-65 (or a subset thereof) having the first and/or second and/or third highest mean score correlation coefficient (positive correlation). Alternatively, or in addition to the cancer therapy-sensitive biomarkers, the methods, devices, and kits can utilize oligonucleotide probes capable of detecting the level of expression of the cancer therapy-resistant biomarkers of one or more of Tables 66-129 (or a subset thereof) having the first and/or second and/or third highest mean score correlation coefficient (negative correleation).

Disclosed are methods of using the microarrays to determine whether a subject, e.g., a cancer patient, will be sensitive or resistant to treatment with, e.g., a chemotherapy agent. Also disclosed are methods of identifying biomarkers of sensitivity or resistance to a medical treatment based on the correlation of biomarker expression to treatment efficacy, e.g., the growth inhibition of cancer cells. Biomarkers that identify subjects as sensitive or resistant to a treatment may also be identified within patient populations already thought to be sensitive or resistant to that treatment. Thus, the methods, devices, and kits can be used to identify patient subpopulations that are responsive to one or more treatments thought to be ineffective for treating disease (e.g., cancer) in the general population. More generally, cancer patient sensitivity to one or more compounds or other medical treatments may be predicted using biomarker expression regardless of prior knowledge about patient responsiveness to treatment. The method can be implemented using software that is run on an apparatus for measuring gene expression in connection with a microarray. Devices (e.g., a microarray, such as a DNA and/or RNA microarray) can be included in a kit for processing a tumor sample from a subject (e.g., a cell, tissue, or organ sample containing a tumor or a cell thereof), and the apparatus for reading the device and turning the result into a chemosensitivity profile for the subject may be used to implement the methods.

**Devices Containing Oligonucleotide Probes for Use of the Invention**

The devices (e.g. microarrays) for use of the invention can include one or more (e.g., two, three, four, five, ten, twenty, thirty, fifty, or all) oligonucleotide probes that have nucleotide sequences that are identical (or share at least 85%, 90%, 95%, or 99% identity) to or complementary to, e.g., at least 5, 8, 12, 20, 30, 40, 60, 80, 100, 150, or 200 consecutive nucleotides (or nucleotide analogues) of one or more of the biomarkers listed in one or more of Tables 1-65 and/or one or more of Tables 66-129,
and hsa-miR-766_st. The oligonucleotide probes may be, e.g., 5-20, 25, 5-50, 50-100, or over 100 nucleotides long. The oligonucleotide probes may be deoxyribonucleic acids (DNA) or ribonucleic acids (RNA). Consecutive nucleotides within the oligonucleotide probes (e.g., 5-20, 25, 5-50, 50-100, or over 100 consecutive nucleotides), which are used as biomarkers of chemosensitivity, may also appear as consecutive nucleotides in one or more of the genes and/or RNAs (e.g., miRNA(s)) described herein beginning at or near, e.g., the first, tenth, twentieth, thirtieth, fortieth, fiftieth, sixtieth, seventieth, eightieth, ninetieth, hundredth, hundred-fiftieth, two-hundredth, five-hundredth, or one-thousandth nucleotide of one or more of the biomarkers listed in one or more of Tables 1-65 and/or one or more of Tables 66-129 below.

Probes that may be employed on devices (e.g., microarrays) for use of the invention include oligonucleotide probes having sequences complementary to or identical to (or sharing at least 85%, 90%, 95%, or 99% identity to) any of the target biomarker sequences described herein. Additionally, probes employed on devices (e.g., microarrays) for use of the invention may also include proteins, peptides, or antibodies that selectively bind any of the oligonucleotide probe sequences or their complementary sequences. Exemplary probes of treatment sensitivity are listed in Tables 1-65 and exemplary probes of treatment resistance are listed in Tables 66-129, wherein for each treatment listed, the biomarkers indicative of treatment sensitivity or resistance, respectively, the correlation of biomarker expression to growth inhibition and the sequence of an exemplary probe are shown.

**Identification of Biomarkers**

The gene expression measurements of the NCI60 cancer cell lines were obtained from the National Cancer Institute and the Massachusetts Institute of Technology (MIT). Each dataset was normalized so that sample expression measured by different chips could be compared. The preferred method of normalization is the logit transformation, which is performed for each gene $y$ on each chip:

$$\text{logit}(y) = \log\left[(y\text{-}background) / (saturation - y)\right],$$

where *background* is calculated as the minimum intensity measured on the chip minus 0.1% of the signal intensity range: min-0.001 *(max-min), and *saturation* is calculated as the maximum intensity measured on the chip plus 0.1% of the signal intensity range: max+0.001*(max-min). The resulting logit transformed data is then z-transformed to mean zero and standard deviation 1.

Next, gene expression is correlated to cancer cell growth inhibition. Growth inhibition data (GI50) of the NCI60 cell lines in the presence of any one of thousands of tested compounds was obtained from the NCI. The correlation between the logit-transformed expression level of each gene in each cell line and the logarithm of GI50 (the concentration of a given compound that results in a 50% inhibition of growth) can be calculated, e.g., using the Pearson correlation coefficient or the Spearman Rank-Order correlation coefficient. Instead of using GI50s, any other measure of patient sensitivity to a given compound may be correlated to the patient's gene expression. Since a plurality of measurements may be available for a single gene, the most accurate determination of correlation coefficient was found to be the median of the correlation coefficients calculated for all probes measuring expression of the same gene.

The median correlation coefficient of gene expression measured on a probe to growth inhibition or patient sensitivity is calculated for all genes, and genes that have a median correlation above 0.3, 0.4, 0.5, 0.6, 0.7, 0.8, 0.9, 0.95, or 0.99 are retained as biomarker genes. Preferably, the correlation coefficient of biomarker genes will exceed 0.3. This is repeated for all the compounds to be tested. The result is a list of marker genes that correlates to sensitivity for each compound tested.

**Predicting Patient Sensitivity or Resistance to Medical Treatments**

For a given medical treatment (e.g., a compound or drug, such as a chemotherapy agent), the expression of one or more biomarkers has been shown to correlate to chemosensitivity and/or chemoresistance. This correlation can be used to classify a patient, e.g., a cancer patient, or a subpopulation of patients, as sensitive or resistant to one or more medical treatments, e.g., one or more of the chemotherapeutic agents or radiation therapies described herein. Using a tumor sample or a blood sample (e.g., in case of leukemia or lymphoma) from a patient, expression of the biomarkers in the cells of the patient in the presence of the treatment agent is determined (using, for example, an RNA extraction kit, a DNA microarray and a DNA microarray scanner). Measurements of biomarker expression are then logit transformed as described above. The sum of the expression measurements of the biomarkers is then compared to the median of the sums derived from a training set population of patients having the same tumor. If the sum of biomarker expression in the patient is closest to the median of the sums of expression in the surviving members of the training set, the patient is predicted to be sensitive to the compound or other medical treatment. If the sum of biomarker expression in the patient is closest to the median of the sums of expression in the non-surviving members of the training set, the patient is predicted to be resistant to the compound.

It is disclosed that machine learning techniques such as Neural Networks, Support Vector Machines, K Nearest Neighbor, and Nearest Centroids may also be employed to develop models that discriminate patients sensitive to treatment from those resistant to treatment using biomarker expression as model variables which assign each patient a classification as resistant or sensitive. Machine learning techniques used to classify patients using various measurements are described in U.S. Patent No. 5,822,715; U.S. Patent Application Publication Nos. 2003/0073083, 2005/0227266, 2005/0208512, 2005/0123945, 2003/0129629, and 2002/0006613; and in Vapnik V N. Statistical Learning Theory, John Wiley & Sons, New York, 1998; Hastie et al., 2001, The Elements of Statistical Learning: Data Mining, Inference, and Prediction, Springer, N.Y.; Agresti, 1996, An Introduction to Categorical Data Analysis, John Wiley & Sons, New York; V. Tresp et al., "Neural Network Modeling of Physiological Processes", in Hanson S. J. et al. (Eds.), Computational Learning Theory and Natural Learning Systems 2, MIT Press, 1994.

A more compact microarray may be designed using only the oligonucleotide biomarker probes having measurements yielding the median correlation coefficients with cancer cell growth inhibition. Thus, in this embodiment, only one probe needs to be used to measure expression of each biomarker. For example, a microarray

includes one, two, or three (or more, e.g., all) of the biomarker probes provided in Tables 1-65 having the highest correlation coefficients (positive correlation) from among those biomarkers listed and/or one, two, or three (or more, e.g., all) of the biomarker probes provided in Tables 66-129 having the highest correlation coefficients (negative correlation) from among those biomarkers listed. The device for use of the invention can also include one or more probes directed to hsa-miR-766_st along with one or more probes directed to one or more of the biomarker probes provided in one or more of Tables 1-65 and/or one or more of Tables 66-129.

As an example, Table 2 provides exemplary biomarker probes for determining a patient's sensitivity to cisplatin. A disclosed device can be prepared for assaying a patient's sensitivity to cisplatin by incorporating one or more probes directed to the biomarker hsa-miR-342-3p_st, which has the highest correlation coefficient of 0.43 for this group. In other disclosure , a device can be prepared for assaying a patient's sensitivity to cisplatin by incorporating one or more probes directed to the two biomarkers associated with cisplatin having the highest correlation coefficients (e.g., hsa-miR-342-3p_st (0.43) and hsa-miR-124_st (0.35)). Other devices can include one or more biomarker probes directed to those biomarkers having the first, second, and/or third, etc., highest correlation coefficients (positive correlatin) for each therapy agent of one or more of Tables 1-65 and/or one or more biomarker probes directed to those biomarkers having the first, second, and/or third, etc., highest correlation coefficients (negative correlation) for each therapy agent of one or more of Tables 66-129.

**Identifying a Subpopulation of Patients Sensitive to One or More Treatments for Cancer**

The disclosure may also be used to identify a subpopulation of patients, e.g., cancer patients, that are sensitive to a compound or other medical treatment previously thought to be ineffective for the treatment of cancer. To this end, biomarkers, the expression of which correlates to sensitivity to a compound or other treatment, may be identified so that patients sensitive to a compound or other treatment may be identified. To identify such biomarkers, expression within cell lines may be correlated to the growth of those cell lines in the presence of the same compound or other treatment. Preferably, biomarkers (such as genes or RNAs) whose expression correlates to cell growth with a correlation coefficient exceeding 0.3 may be considered possible biomarkers.

Alternatively, genes or RNAs (e.g., miRNAs) may be identified as biomarkers according to their ability to discriminate patients known to be sensitive to a treatment from those known to be resistant. The significance of the differences in expression of biomarkers between the sensitive and resistant patients may be measured using, e.g., t-tests. Alternatively, naïve Baycsian classifiers may be used to identify biomarkers that discriminate sensitive and resistant patient subpopulations given the biomarker expressions of the sensitive and resistant subpopulations within a treated patient population.

The patient subpopulations considered may be further divided into patients predicted to survive without treatment, patients predicted to die without treatment, and patients predicted to have symptoms without treatment. The above methodology may be similarly applied to any of these further defined patient subpopulations to identify biomarkers able to predict a subject's sensitivity to compounds or other treatments for the treatment of cancer.

Patients with elevated expression of biomarkers correlated to sensitivity to a compound or other medical treatment would be predicted to be sensitive to that compound or other medical treatment.

The disclosure is particularly useful for recovering compounds or other treatments that failed in clinical trials by identifying sensitive patient subpopulations using the gene expression methodology disclosed herein to identify gene or NRA (e.g., miRNA) biomarkers that can be used to predict clinical outcome.

### Kit, Apparatus, and Software for Clinical Use

The disclosure may also be used to predict patients who are resistant or sensitive to a particular treatment by using a kit that includes one or more of the following: a kit for RNA extraction from tumors (e.g., mirVana miRNA isoltaion kit from Ambion Inc), a kit for RNA labeling (e.g., FlashTag from Genisphere Inc), a microarray for measuring biomarker expression (e.g., miRNA-1.0 from Affymetrix Inc), a microarray hybridization station and scanner (e.g., GeneChip System 3000Dx from Affymetrix Inc), and/or software for analyzing the expression of biomarker genes or RNAs (e.g., miRNAs) as described in herein (e.g., implemented in R from R-Project or S-Plus from Insightful Corp.). The predicted sensitivity is either given by the meanscore or diffscore as defined in Example 2 below.

### Methodology of the *In Vitro* Cancer Growth Inhibition Screen

The human tumor cell lines of the cancer screening panel are grown in RPMI 1640 medium containing 5% fetal bovine serum and 2 mM L-glutamine. Cells are inoculated into 96 well microtiter plates in 100 $\mu$L at plating densities ranging from 5,000 to 40,000 cells/well depending on the doubling time of individual cell lines. After cell inoculation, the microtiter plates are incubated at 37°C, 5% CO2, 95% air and 100% relative humidity for 24 hours prior to addition of experimental compounds.

After 24 hours, two plates of each cell line are fixed in situ with TCA, to represent a measurement of the cell population for each cell line at the time of compound addition (Tz). Experimental compounds are solubilized in dimethyl sulfoxide at 400-fold the desired final maximum test concentration and stored frozen prior to use. At the time of compound addition, an aliquot of frozen concentrate is thawed and diluted to twice the desired final maximum test concentration with complete medium containing 50 $\mu$g/ml Gentamicin. Additional four, 10-fold or ½ log serial dilutions are made to provide a total of five compound concentrations plus control. Aliquots of 100 $\mu$l of these different compound dilutions are added to the appropriate microtiter wells already containing 100 $\mu$l of medium, resulting in the required final compound concentrations.

Following compound addition, the plates are incubated for an additional 48 h at 37°C, 5% CO2, 95% air, and 100% relative humidity. For adherent cells, the assay is terminated by the addition of cold TCA. Cells are fixed in situ by the gentle addition of 50 $\mu$l of cold 50% (w/v) TCA (final concentration, 10% TCA) and incubated for 60 minutes at 4°C. The supernatant is discarded, and the plates are washed five times with tap water and air-dried. Sulforhodamine B (SRB) solution (100 $\mu$l) at 0.4% (w/v) in 1% acetic acid is added to each well, and plates are incubated for 10 minutes at room temperature. After staining, unbound dye is removed by washing five times with 1% acetic acid and the plates are air-dried. Bound stain is subsequently solubilized with 10 mM trizma base, and the absorbance is read on an automated plate reader at a wavelength of 515 nm. For suspension cells, the methodology is the same except that the assay is terminated by fixing settled cells at the bottom of the wells by gently adding 50 $\mu$l of 80% TCA (final concentration, 16 % TCA). Using the seven absorbance measurements [time zero, (Tz), control growth, (C), and test growth in the presence of compound at the five concentration levels (Ti)], the percentage growth is calculated at each of the compound concentrations levels. Percentage growth inhibition is calculated as:

$$[(Ti\text{-}Tz)/(C\text{-}Tz)] \times 100 \text{ for concentrations for which } Ti >/= Tz$$

$$[(Ti\text{-}Tz)/Tz] \times 100 \text{ for concentrations for which } Ti < Tz$$

Three dose response parameters are calculated for each experimental agent. Growth inhibition of 50% (GI50) is calculated from [(Ti-Tz)/(C-Tz)] x 100 = 50, which is the compound concentration resulting in a 50% reduction in the net protein increase (as measured by SRB staining) in control cells during the compound incubation. The compound concentration resulting in total growth inhibition (TGI) is calculated from Ti = Tz. The LC50 (concentration of compound resulting in a 50% reduction in the measured protein at the end of the compound treatment as compared to that at the beginning) indicating a net loss of cells following treatment is calculated from [(Ti-Tz)/Tz] x 100 = -50. Values are calculated for each of these three parameters if the level of activity is reached; however, if the effect is not reached or is exceeded, the value for that parameter is expressed as greater or less than the maximum or minimum concentration tested.

## RNA Extraction and Biomarker Expression Measurement

Cell/tissue samples are snap frozen in liquid nitrogen until processing. RNA is extracted using e.g. Trizol Reagent from Invitrogen following manufacturer's instructions. RNA is amplified using, e.g., MessageAmp kit from Ambion following manufacturer's instructions. Amplified RNA is quantified using, e.g., HG-U133A GeneChip from Affymetrix Inc and compatible apparatus e.g. GCS3000Dx from Affymetrix, using manufacturer's instructions.

The resulting biomarker expression measurements are further processed as described in this document The procedures described can be implemented using R software available from R-Project and supplemented with packages available from Bioconductor.

For many drugs, 1-30 (e.g., 5-30 or 10-30) biomarkers are sufficient to give an adequate response. Thus, given the relatively small number of biomarkers required, procedures, such as quantitative reverse transcriptase polymerase chain reaction (qRT-PCR), may be performed to measure, with greater precision, the amount of one or more biomarker genes or RNAs (e.g., miRNAs) expressed in a sample. This will provide an alternative to or a complement to microarrays so that a single companion test, perhaps more quantitative than microarrays alone, employing one or more of the biomarkers of the invention (e.g., hsa-miRNA-766 alone or in combination with one or more can be used to predict sensitivity to a new drug. qRT-PCR may be performed alone or in combination with a microarray described herein. Procedures for performing qRT-PCR are described in, e.g., U.S. Patent No. 7,101,663 and U.S. Patent Application Nos. 2006/0177837 and 2006/0088856. The methods disclosed herein are readily applicable to newly discovered drugs as well as drugs described herein.

## EXAMPLES

### Example 1: Identification of gene biomarkers for chemosensitivity to common chemotherapy drugs.

DNA chip measurements of the 60 cancer cell lines of the NCI60 data set were performed using Affymetrix miRNA 1.0 arrays and logit normalized. Growth inhibition data of thousands of compounds against the same cell lines were downloaded from the National Cancer Institute. Compounds where the difference concentration to achieve 50% in growth inhibition (GI50) was less than 1 log were deemed uninformative and rejected. Each gene's expression in each cell line was correlated to its growth (-log(GI50)) in those cell lines in the presence of a given compound. The median Pearson correlation coefficient was used when multiple expression measurements were available for a given gene, and genes having a median correlation coefficient greater than 0.3 were identified as biomarkers for a given compound. Table 1-65 lists biomarkers for a number of drugs used in treating cancer.

They can be used to predict the response of a human tumor sample to treatment by the drug in question by following the procedure described in "Kit, Apparatus, and Software for Clinical Use."

### Example 2: Difference between positive and negative correlated genes.

In Example 1 a procedure for identifying microRNA biomarkers with a positive correlation to drug sensitivity is described. Sensitivity to a drug can then be predicted based on the mean expression of those microRNA biomarkers. This is called mean score. But there is also information in those biomakers that correlate negatively with drug sensitivity. They could be called biomarkers of resistance.

Tables 66-129 lists the negatively correlated biomarkers for a number of drugs used to treat cancer. An even better accuracy of predicted response to a drug can be obtained when both negatively and positively correlated markers are used. One way of combining them is to subtract the mean expression of the negatively correlated biomarkers from the mean expression of the positively correlated biomarkers. This is called a difference score, or diffscore.

Table 130 shows how mean score and difference score compare in their ability to predict the GI50 values of cell lines treated with a drug. As the method would be applied to a human tumor sample in the same manner as it is here applied to a human cell line sample, the diffscore can also be used (e.g., as an alternative to the mean score, which is based on the correleation coefficient alone) to predict the response of human tumors (and thus, the patient) to treatment with one or more drugs (e.g., one or more of the chemotherapy drugs or radiation described herein).

**Table 130:** comparison of diff score and mean score in predicting human cell line response to a number of drugs (correlation coefficient between prediction and measurement is shown, a higher CC means more accurate prediction)

| Drug | Diff score CC | Mean score CC |
|---|---|---|
| Vincristine | 0.56 | 0.35 |
| Herceptin | 0.50 | 0.41 |
| Iressa | 0.60 | 0.53 |
| Fulvestrant | 0.76 | 0.74 |
| Erlotinib (tarceva) | 0.62 | 0.59 |
| Belinostat (PXD101) | 0.61 | 0.60 |
| Cisplatin | 0.66 | 0.67 |

**Example 3: Exemplary Arrays**

Exemplary arrays can be prepared with one or more oligonucleotide probes that are capable of detecting the level of expression of one or more of the biomarkers identified herein (e.g., one or more of the biomarkers of sensitivity described in Tables 1-65 and/or one of more of the biomarkers of resistance described in Tables 66-129). Examples of such arrays are described in the Table 131 below. The arrays include probes capable of detecting the level of expression of the indicated biomarkers. Probes for the indicated biomarkers are selected based on the median score correlation coefficient for the biomarker. Array 1 includes one or more probes for the representative biomarker(s) selected from Tables 1-65 for each specified therapeutic. Array 2 includes one or more probes for the representative biomarkers selected from Tables 1-65 for each specific therapeutic. Array 3 includes one or more probes for the representative biomarkers from one or more of Tables 1-65 for the indicated therapeutic. Other combinations of biomarker probes can also be used to produce one or more additional arrays.

The arrays include arrays for use of the invention containing biomarker hsa-miR-766_st.

**Table 131**: Examples of Arrays with Biomarkers of Sensitivity

| Therapeutic | Array 1 Probes | Array 2 Probes | Array 3 Probes |
|---|---|---|---|
| Vincristine | hsa-miR-106b-star_st | hsa-miR-106b-star_st<br>hsa-miR-25-star_st | |
| Cisplatin | hsa-miR-342-3p_st | hsa-miR-342-3p_st<br>hsa-miR-124_st | |
| Etoposide | hsa-miR-92b_st | hsa-miR-92b _st<br>hsa-miR-140-3p_ st | hsa-mik-92b_st<br>hsa-miR-140-3p_st<br>hsa-miR-766_ st |
| Azaguanine | hsa-miR-140-3p_st | hsa-miR-140-3p_st<br>hsa-miR-146a_ st | |
| Carboplatin | hsa-miR-342-3p_st | hsa-miR-342-3p_st<br>hsa-miR-124_ st | |
| Adriamycin | hsa-miR-106b-star_st | hsa-miR-106b-star st<br>hsa-miR-106b_st | hsa-miR-106b-star_ st<br>hsa-miR-106b_st<br>hsa-miR-766_ st |
| Aclarubicin | hsa-miR-1275_st | hsa-miR-1275 st<br>ACA10_s_st | |
| Mitoxantrone | hsa-miR-181a-star_st | hsa-miR-181a-star_st<br>hsa-miR-432_st | |

(continued)

| Therapeutic | Array 1 Probes | Array 2 Probes | Array 3 Probes |
|---|---|---|---|
| Mitomycin | hsa-miR-181b_st | hsa-miR-18 1b_st<br>hsa-miR-124_ st | |
| Paclitaxel | hsa-miR-652_st | hsa-miR-652_st<br>hsa-miR-532-5p_st | hsa-miR-652 _st<br>hsa-miR-532-5p_st<br>hsa-miR-766_ st |
| Gemcitabine | hsa-miR-155_st | hsa-miR-155_st<br>hsa-miR-342-3p_ st | |
| Taxotere | hsa-miR-769-5p_st | hsa-miR-769-5p_st<br>hsa-miR-769-3p_ st | |
| Dexamethasone | U49B_s_st | U49B_s_st<br>hsa-miR-424-star_st | U49B_s_st<br>hsa-miR-424-star_st<br>hsa-miR-766_ st |
| Ara-C | hsa-mir-181a-star_st | hsa-miR-181a-star_st<br>HBII-202_st | |
| Methylprednisolone | hsa-miR-1207-5p_st | hsa-miR-1207-5p_st<br>hsa-miR-20b-star_st | hsa-miR-1207-5p_st<br>hsa-miR-20b-star_st<br>hsa-miR-766_ st |
| Methotrexate | hsa-miR-663b_st | hsa-miR-663b_st<br>U104_st | |
| Bleomycin | 14qII-26_st | 14qII-26_st<br>14qII-14_st<br>hsa-miR-127-3p_st<br>hsa-miR-455-3p_st | |
| Methyl-GAG | U26_st | U26_st<br>ENSG00000200879_st<br>ENSG00000202252_st<br>U25_st<br>hsa-miR-181a-star_st | |
| Belinostat | hsa-miR-19b_st | hsa-miR-19b_st<br>hsa-miR-18a_st | hsa-miR-19b_st<br>hsa-miR-18a_st<br>hsa-miR-766_st |
| Fluorouracil | U74_x_st | U74_x_st<br>U104_ st | |
| Radiation | hsa-let-7i-star_st | hsa-let-7i-star_st<br>hsa-miR-34a_ st | |
| Aza-2'-deoxycytidine | hsa-miR-324-3p_st | hsa-miR-324-3p_st<br>hsa-miR-766_ st | hsa-miR-324-3p_st<br>hsa-miR-195-star_st<br>hsa-miR-766_ st |
| Idarubicin | hsa-miR-124_ st | hsa-miR-124_st<br>U164_st | |
| Melphalan | hsa-miR-181a-star_st | hsa-miR-181a-star_st<br>hsa-miR-297_st | hsa-miR-181a-star_st<br>hsa-miR-297_st<br>hsa-miR-766_st |

(continued)

| Therapeutic | Array 1 Probes | Array 2 Probes | Array 3 Probes |
|---|---|---|---|
| 1L4-PR38 fusion protein | hsa-miR-150_st<br>hsa-miR-339-3p_st | hsa-miR-150_st<br>hsa-miR-339-3p_st<br>hsa-miR-768-3p_st | |
| Valproic acid | hsa-miR-34a_st | hsa-miR-34a_ st<br>hsa-miR-1308 _st<br>hsa-miR-34a-star_ st | |
| All-trans retinoic acid | hsa-miR-449b_st | hsa-miR-449b_ st<br>hsa-miR-449a_ st | |
| Cytoxan | hsa-miR-449a_st<br><br>hsa-miR-449b_ st | hsa-miR-449a_st<br>hsa-miR-449b _st<br>hsa-miR-196a_st | |
| Topotecan | hsa-miR-342-3p_st | hsa-miR-342-3p_st<br>hsa-miR-342-5p_ st | |
| Suberoylanilide hydroxamic acid | hsa-miR-20b_st | hsa-miR-20b_st<br>hsa-miR-18b_st | hsa-miR-20b_st<br>hsa-miR-18b_ st<br>hsa-miR-766_ st |
| Depsipeptide | hsa-miR-652_st | hsa-miR-652_st<br>hsa-miR-185_st<br>hsa-miR-532-5p_st | |
| Bortezomib | hsa-miR-106b_st | hsa-miR-1 06b_st<br>hsa-miR-93_ st | |
| Leukeran | hsa-miR-181a-star_st | hsa-miR-181a-star_st<br>hsa-miR-181b_st | hsa-miR-181a-star_ st<br>hsa-miR-181b_ st<br>hsa-miR-766_ st |
| Fludarabine | HBII-438A_ s_st | HBII-438A_s_st<br>hsa-miR-181b_st<br>hsa-miR-34c-3p_st | HBII-438A_s_st<br>hsa-miR-181b_ st<br>hsa-miR-34c-3p_st<br>hsa-miR-766_ st |
| Vinblastine | hsa-miR-652_st | hsa-miR-652_st<br>hsa-miR-500_st<br>hsa-miR-532-5p_st | |
| Busulfan | hsa-miR-1281_st | hsa-miR-1281_st<br>hsa-miR-181a-star_st | hsa-miR-1281_st<br>hsa-miR-181a-star_st<br>hsa-miR-766_st |
| Dacarbazine | hsa-miR-223_st<br>hsa-miR-92a-1-star_st | hsa-miR-223_ st<br>hsa-miR-92a-1 -star_st<br>U52_st | hsa-miR-223_st<br>hsa-miR-92a-1-star_st<br>U52_st<br>hsa-miR-766_st |
| Oxaliplatin | U104_st<br>hsa-miR-17 st | U104_st<br>hsa-miR-17_ st<br>hsa-miR-19b_ st | |
| Hydroxyurea | hsa-miR-223_st | hsa-miR-223_st<br>hsa-miR-297_st | hsa-miR-223_st<br>hsa-miR-297_st<br>hsa-miR-766_ st |

(continued)

| Therapeutic | Array 1 Probes | Array 2 Probes | Array 3 Probes |
|---|---|---|---|
| Tegafur | U74_x_st | U74_x_st<br>hsa-miR-34b_ st | |
| Daunorubicin | U55_x_st | U55_x_st<br>U55_st | U55_x_st<br>U55_st<br>hsa-miR-766_ st |
| Bleomycin | 14qII-26_st | 14qII-26_st<br>14qII-14_st<br>hsa-miR-127-3p_st<br>hsa-miR-455-3p_s | |
| Estramustine | hsa-miR-378_st | hsa-miR-378_st<br>hsa-miR-586 st | |
| Mechlorethamine | U104_st | U104_st<br>hsa-miR-768-3p_ st<br>hsa-miR-768-5p st | U104_st<br><br>hsa-miR-766_st |
| Streptozocin | hsa-miR-296-3p_st | hsa-miR-296-3p_ st<br>hsa-miR-923_ st | |
| Carmustine | hsa-miR-146b-3p_st | hsa-miR-146b-3p_st<br>hsa-miR-18a-star_ st | hsa-miR-146b-3p_st<br>hsa-miR-18a-star_st<br>hsa-miR-766_ st |
| Lomustine | hsa-miR-766 st | hsa-miR-766_st<br>hsa-miR-1183_st | hsa-miR-92a-2-star_st<br>hsa-miR-1183_ st<br>hsa-miR-766_ st |
| Mercaptopurine | hsa-miR-92a-1-star_st | hsa-miR-92a-1-star_ st<br>U55_st<br>hsa-miR-17_st | hsa-miR-92a-1-star_st<br>U55_st<br>hsa-miR-17_st<br>hsa-miR-766_st |
| Teniposide | hsa-miR-181a-star_st<br><br>hsa-miR-768-3p st | hsa-miR-181a-star_st<br>hsa-miR-768-3p_st<br>hsa-miR-1299_st<br>hsa-miR-34b_st | hsa-miR-18 1a-star_st<br>hsa-miR-768-3p_ st<br>hsa-miR-1299_st<br>hsa-miR-34b_st<br>hsa-miR-766_st |
| Dactinomycin | hsa-miR-652_st | hsa-miR-652_st<br>hsa-miR-106b_ st | hsa-miR-652_st<br>hsa-miR-106b_st<br>hsa-miR-766_st |
| Tretinoin | hsa-miR-449b_st | hsa-miR-449b_st<br>hsa-miR-449a_st | |
| Ifosfamide | hsa-miR-766 st | hsa-miR-766_st<br>hsa-miR-181a-star_st | hsa-miR-181a-star_st<br>hsa-miR-20b-star_st<br>hsa-miR-766_ st |
| Tamoxifen | U17b_st | U17b_st<br>HBII-180A_x_st | |
| Irinotecan | hsa-miR-181a-star_st | hsa-miR-181a-star_st<br>hsa-miR-874_st | hsa-miR-181a-star_st<br>hsa-miR-874_st<br>hsa-miR-766_ st |

(continued)

| Therapeutic | Array 1 Probes | Array 2 Probes | Array 3 Probes |
|---|---|---|---|
| Floxuridine | HBII-202_st | HBII-202_st<br>U13_st | |
| Thioguanine | hsa-miR-17_st | hsa-miR-17_st<br>U52_st<br>hsa-miR-106a_st<br>hsa-miR-17-star_st | hsa-miR-17_st<br>U52_st<br>hsa-miR-106a_st<br>hsa-miR-17-star_st<br>hsa-miR-766_st |
| PSC 833 | hsa-miR-378-star_st | hsa-miR-378-star_st<br>hsa-miR-422a_ st | |
| Erlotinib | hsa-miR-30c_st | hsa-miR-30c_ st<br>hsa-miR-30c-2-star_ st | |
| Herceptin | hsa-miR-34c-3p_st | hsa-miR-34c-3p_st<br>hsa-miR-4c-5p_ st | |
| Celecoxib | hsa-miR-768-3p_st | hsa-miR-768-3p_st<br>HBII-142_ x_st | |
| Fulvestrant | hsa-miR-489_st | hsa-miR-489_st<br>hsa-miR-425_ st | |
| Iressa | hsa-miR-934_st | hsa-miR-934 _st<br>hsa-miR-30c_ st | |
| Anastrozole | hsa-miR-517a_ st | hsa-miR-517a_st<br>hsa-miR-512-3p_st<br>hsa-miR-551a_st | |
| Letrozole | hsa-mik-551a_st | hsa-miR-551a _st<br>hsa-miR-517a_ st | |
| Cetuximab | hsa-miR-34c-3p_st | hsa-miR-34c-3p_st<br>hsa-miR-34c-5p_ st | |

Table 132: Examples of Arrays of the Disclosure with Biomarkers of Resistance

| Therapeutic | Array 4 Probes | Array 5 Probes |
|---|---|---|
| Vincristine | hsa-miR-449a_st | hsa-miR-449a_st<br>hsa-miR-130a_ st |
| Cisplatin | hsa-miR-192_st<br>hsa-miR-194_st<br>hsa-miR-30b_st | hsa-miR-192_st<br>hsa-miR-194_st<br>hsa-miR-30b _st<br>hsa-miR-200b_ st |
| Etoposide | hsa-miR-151-5p_st | hsa-miR-151-5p_st<br>hsa-miR-200a-star_st |
| Azaguanine | hsa-miR-200c_st | hsa-miR-200c_st<br>hsa-miR-1244_st<br>hsa-miR-331-3p_ st |
| Carboplatin | hsa-miR-29b_st | hsa-miR-29b_st<br>hsa-miR-10a_st<br>hsa-miR-7_ st |

(continued)

| Therapeutic | Array 4 Probes | Array 5 Probes |
|---|---|---|
| Adriamycin | hsa-miR-516a-5p_st | hsa-miR-516a-5p_st<br>hsa-miR-518d-5p_ st |
| Aclarubicin | hsa-miR-24-2-star_st | hsa-miR-24-2-star_st<br>hsa-let-7f_ st |
| Mitoxantrone | hsa-miR-30d_st | hsa-miR-30d_ st<br>hsa-miR-513c_ st |
| Mitomycin | hsa-miR-516a-5p_st | hsa-miR-516a-5p_st<br>hsa-miR-526a_st |
| Paclitaxel | hsa-miR-30a_st | hsa-miR-30a_st<br>hsa-miR-34a_ st |
| Gemcitabine | hsa-miR-141_st | hsa-miR-141_st<br>hsa-miR-584_st<br>hsa-miR-934_st |
| Taxotere | HBII-85-29_ st | HBII-85-29_st<br>hsa-miR-184_st<br>hsa-miR-424-star_st |
| Dexamethasone | hsa-miR-34a_st | hsa-miR-34a_st<br>hsa-miR-151-5p_ st |
| Ara-C | hsa-miR-23b_st | hsa-miR-23b_st<br>hsa-miR-151-5p_ st |
| Methylprednisolone | hsa-miR-24_st | hsa-miR-24_ st<br>hsa-miR-339-3p_ st |
| Methotrexate | hsa-miR-30a_ st | hsa-miR-30a_st<br>hsa-miR-22_st<br>hsa-miR-34c-5p_st |
| Bleomycin | hsa-miR-25_st | hsa-miR-25_st<br>hsa-miR-203_ st<br>hsa-miR-215_ st |
| Methyl-GAG | hsa-miR-10b_st | hsa-miR-10b_ st<br>hsa-miR-10a_ st |
| Belinostat | hsa-let-7e_ st | hsa-let-7e_st<br>hsa-miR-370_st<br>hsa-miR-452_st<br>hsa-miR-495_st |
| Fluorouracil | hsa-miR-130a_ st | hsa-miR-130a_st<br>hsa-miR-100_st |
| Radiation | hsa-miR-766_ st | hsa-miR-766_st<br>hsa-miR-1207-5p_st<br>hsa-miR-625_ st |
| Aza-2'-deoxycytidine | hsa-miR-30a_ st | hsa-miR-30a_st<br>hsa-miR-22_st<br>hsa-miR-99b_st |
| Idarubicin | hsa-miR-151-5p_ st | hsa-miR-151-5p_st<br>hsa-miR-526a_ st |

(continued)

| Therapeutic | Array 4 Probes | Array 5 Probes |
|---|---|---|
| Melphalan | hsa-miR-151-5p_st | hsa-miR-151-5p_ st<br>hsa-miR-29b_ st |
| IL4-PR38 fusion protein | hsa-miR-100_ st | hsa-miR-100_st<br>hsa-let-7a_st<br>hsa-miR-146a_st |
| Valproic acid | hsa-miR-744_st | hsa-miR-744_ st<br>hsa-miR-625_ st |
| All-trans retinoic acid | hsa-miR-221_ st | hsa-miR-221_st<br>hsa-miR-10a_st |
| Cytoxan | hsa-miR-222_st | hsa-miR-222_st<br>hsa-let-7b_st<br>hsa-miR-625_st |
| Topotecan | hsa-miR-584_ st | hsa-miR-584_ st<br>HBII-289_st |
| Suberoylanilide hydroxamic acid | hsa-miR-210_ st | hsa-miR-210_st<br>hsa-miR-28-3p_st<br>hsa-miR-455-3p_st<br>hsa-miR-758_st |
| Depsipeptide | hsa-miR-516a-5p_st | hsa-miR-516a-5p_st<br>HBII-85-23_x_st<br>HBII-85-29_x_st<br>hsa-miR-200a-star_st<br>hsa-miR-203_st<br>hsa-miR-519a_st<br>hsa-miR-523-star_st |
| Bortezomib | hsa-miR-9-star_ st | hsa-miR-9-star_st<br>hsa-miR-98_ st |
| Leukeran | hsa-miR-151-5p_st | hsa-miR-151-5p_ st<br>hsa-miR-125a-3p_ st |
| Fludarabine | hsa-miR-23b_st | hsa-miR-23b_st<br>hsa-miR-203_st<br>hsa-miR-339-5p_st<br>hsa-miR-487a_st |
| Vinblastine | hsa-miR-30c_st | hsa-miR-30c_st<br>hsa-miR-34c-3p_st |
| Busulfan | hsa-miR-125a-5p _st | hsa-miR-125a-5p_ st<br>hsa-miR-24-1-star_ st |
| Dacarbazine | hsa-miR-151-5p_ st | hsa-miR-151-5p_st<br>hsa-miR-125a-3p_st<br>hsa-miR-182_st<br>hsa-miR-215_st<br>hsa-miR-30a_st<br>hsa-miR-34a-star_st<br>hsa-miR-935_st |

(continued)

| Therapeutic | Array 4 Probes | Array 5 Probes |
|---|---|---|
| Oxaliplatin | hsa-miR-28-3p_st hsa-miR-28-5p_st | hsa-miR-28-3p_st<br>hsa-miR-28-5p_st<br>hsa-miR-145_ st |
| Hydroxyurea | hsa-miR-151-5p_st | hsa-miR-151-5p_st<br>hsa-miR-34a_ st |
| Tegafur | hsa-miR-30a-star_st | hsa-miR-30a-star_st<br>hsa-miR-495_ st |
| Daunorubicin | hsa-miR-151-5p_st | hsa-miR-151-5p_ st<br>hsa-miR-146a_ st<br>hsa-miR-200b-star_st<br>hsa-miR-221-star_ st<br>hsa-miR-523-star_ st |
| Bleomycin | hsa-miR-25_st | hsa-miR-25_st<br>HBII-180C_x_st<br>hsa-miR-18a-star_st<br>hsa-miR-192_st<br>hsa-miR-20a_st<br>hsa-miR-320d_st<br>hsa-miR-93-star_st<br>hsa-miR-934_st |
| Estramustine | hsa-miR-552_ st | hsa-miR-552_st<br>hsa-miR-194-star_st<br>hsa-miR-21-star_st |
| Mechlorethamine | hsa-miR-151-5p_ st | hsa-miR-151-5p_st<br>hsa-miR-519b-5p_st |
| Streptozocin | hsa-miR-26a_ st | hsa-miR-26a_ st<br>hsa-miR-526a_ st |
| Carmustine | hsa-let-7b st | hsa-let-7b_st<br>hsa-let-7f_ st<br>hsa-miR-130a_ st<br>hsa-miR-331-3p_ st |
| Lomustine | hsa-miR-99b_st | hsa-miR-99b_ st<br>hsa-miR-200c_st<br>hsa-miR-331-3p_ st |
| Mercaptopurine | hsa-miR-22_st | hsa-miR-22_ st<br>hsa-miR-23a_ st |
| Teniposide | hsa-miR-151-5p_st | hsa-miR-151-5p_ st<br>hsa-miR-30c-2-star_ st<br>hsa-miR-518e-star_ st |
| Dactinomycin | hsa-miR-30a_st | hsa-miR-30a_st<br>hsa-miR-27a-star_st<br>hsa-miR-372_st |
| Tretinoin | hsa-miR-221_ st | hsa-miR-221_st<br>hsa-miR-125a-5p_ st |

(continued)

| Therapeutic | Array 4 Probes | Array 5 Probes |
|---|---|---|
| Ifosfamide | hsa-miR-24_st | hsa-miR-24_st<br>hsa-miR-27b_ st |
| Tamoxifen | hsa-miR-30a-star_ st | hsa-miR-30a-star_st<br>hsa-let-7a_st<br>hsa-miR-23b_st<br>hsa-miR-30e-star_st |
| Irinotecan | hsa-miR-125a-5p_st | hsa-miR-125a-5p_st<br>hsa-miR-320d_ st<br>hsa-miR-584_ st |
| Floxuridine | hsa-miR-30d_st | hsa-miR-30d_st<br>hsa-miR-30b_st -0.38<br>hsa-miR-584_st |
| Thioguanine | hsa-miR-27b_st | hsa-miR-27b_st<br>hsa-let-7b_st<br>hsa-miR-30a_st |
| PSC 833 | hsa-miR-30a-star_ st hsa-miR-30a_ st | hsa-miR-30a-star_st<br>hsa-miR-30a_st<br>hsa-miR-193b-star_st<br>hsa-miR-22-star_st |
| Erlotinib | hsa-miR-671-5p_st | hsa-miR-671-5p_st<br>hsa-miR-500_ st<br>hsa-miR-532-3p_ st |
| Herceptin | hsa-miR-107_st | hsa-miR-107_st<br>hsa-let-7d_st<br>hsa-miR-103_st |
| Celecoxib | hsa-miR-28-5p_st | hsa-miR-28-5p_ st<br>hsa-miR-151-3p_ st<br>hsa-miR-31-star_ st |
| Fulvestrant | hsa-miR-222_ st | hsa-miR-222_st<br>hsa-miR-28-5p_st |
| Iressa | hsa-miR-505-star_st | hsa-miR-505-star_st<br>hsa-miR-671-5p_ st |
| Letrozole | U27_st | U27_st<br>U29 st |
| Cetuximab | hsa-miR-491-5p_st | hsa-miR-491-5p_st<br>hsa-miR-191_st |

It is disclosed that the probes of Arrays 4 and/or 5 can be included on one or more of Arrays 1,2, and/or 3 to produce a single array that can be used to detect biomarkers of sensitivity and biomarkers of resistance.

The arrays described above and arrays for use of the invention can also include one or more probes that bind to one or more conrol gene products, e.g., the products of housekeeping gene (e.g., beta actin).

Table 1 Vincristine microRNA biomarkers.

|  | Medianprobe | Corr | Sequence |
|---|---|---|---|
| [1,] | hsa-miR-106b-star_st | 0.35 | GCAGCAAGTACCCACAGTGCGG |
| [2,] | hsa-miR-25-star_st | 0.3 | CAATTGCCCAAGTCTCCGCCT |

Table 2 Cisplatin microRNA biomarkers.

| | Medianprobe | Corr | Sequence |
|---|---|---|---|
| [1,] | HBII-85-2_x_st | 0.31 | GAATGTTTTTTTTGGGGACTCATCA |
| [2,] | U48_st | 0.32 | ATGGCATCAGCGACACACTCAAGAG |
| [3,] | U55_x_st | 0.32 | GCACTCGGGAGTATGCAGCATTACC |
| [4,] | hsa-miR-124_st | 0.35 | GGCATTCACCGCGTGCCTTA |
| [5,] | hsa-miR-1281_st | 0.32 | GGGAGAGGAGGAGGCGA |
| [6,] | hsa-miR-181a-star_st | 0.34 | GGTACAATCAACGGTCGATGGT |
| [7,] | hsa-miR-181b_st | 0.33 | ACCCACCGACAGCAATGAATGTT |
| [8,] | hsa-miR-342-3p_st | 0.43 | ACGGGTGCGATTTCTGTGTGAGA |
| [9,] | hsa-miR-432_st | 0.33 | CCACCCAATGACCTACTCCAAGA |

Table 3 Etoposide microRNA biomarkers.

| | Medianprobe | Corr | Sequence |
|---|---|---|---|
| [1,] | hsa-miR-124_st | 0.33 | GGCATTCACCGCGTGCCTTA |
| [2,] | hsa-miR-1281_st | 0.31 | GGGAGAGGAGGAGGCGA |
| [3,] | hsa-miR-140-3p_st | 0.36 | CCGTGGTTCTACCCTGTGGTA |
| [4,] | hsa-miR-181a-star_st | 0.3 | GGTACAATCAACGGTCGATGGT |
| [5,] | hsa-miR-181b_st | 0.32 | ACCCACCGACAGCAATGAATGTT |
| [6,] | hsa-miR-195-star_st | 0.34 | GGAGCAGCACAGCCAATATTGG |
| [7,] | hsa-miR-342-3p_st | 0.33 | ACGGGTGCGATTTCTGTGTGAGA |
| [8,] | hsa-miR-766_st | 0.35 | GCTGAGGCTGTGGGGCTGGAGT |
| [9,] | hsa-miR-92b_st | 0.43 | GGAGGCCGGGACGAGTGCAATA |
| [10,] | hsa-miR-938_st | 0.33 | ACTGGGTTCACCTTTAAGGGCA |

Table 4 Azaguanine microRNA biomarkers.

| | Medianprobe | Corr | Sequence |
|---|---|---|---|
| [1,] | hsa-miR-100_st | 0.37 | CACAAGTTCGGATCTACGGGTT |
| [2,] | hsa-miR-140-3p_st | 0.45 | CCGTGGTTCTACCCTGTGGTA |
| [3,] | hsa-miR-140-5p_st | 0.4 | CTACCATAGGGTAAAACCACTG |
| [4,] | hsa-miR-146a_st | 0.45 | AACCCATGGAATTCAGTTCTCA |
| [5,] | hsa-miR-155_st | 0.37 | ACCCCTATCACGATTAGCATTAA |
| [6,] | hsa-miR-506_st | 0.35 | TCTACTCAGAAGGGTGCCTTA |
| [7,] | hsa-miR-508-5p_st | 0.31 | CATGAGTGACGCCCTCTGGAGTA |
| [8,] | hsa-miR-509-3-5p_st | 0.31 | CATGATTGCCACGTCTGCAGTA |
| [9,] | hsa-miR-509-3p_st | 0.31 | CTACCCACAGACGTACCAATCA |
| [10,] | hsa-miR-510_st | 0.32 | GTGATTGCCACTCTCCTGAGTA |
| [11,] | hsa-miR-513a-5p_st | 0.31 | ATGACACCTCCCTGTGAA |
| [12,] | hsa-miR-513b_st | 0.34 | ATAAATGACACCTCCTTGTGAA |
| [13,] | hsa-miR-663_st | 0.36 | GCGGTCCCGCGGCGCCCCGCCT |
| [14,] | hsa-miR-923_st | 0.34 | AGTTTCTTTTCCTCCGCTGAC |

Table 5 Carboplatin microRNA biomarkers.

| | Medianprobe | Corr | Sequence |
|---|---|---|---|
| [1,] | U55_x_st | 0.32 | GCACTCGGGAGTATGCAGCATTACC |
| [2,] | hsa-miR-124_st | 0.39 | GGCATTCACCGCGTGCCTTA |
| [3,] | hsa-miR-1271_st | 0.33 | TGAGTGCTTGCTAGGTGCCAAG |
| [4,] | hsa-miR-143_st | 0.32 | GAGCTACAGTGCTTCATCTCA |

(continued)

| | Medianprobe | Corr | Sequence |
|---|---|---|---|
| [5,] | hsa-miR-342-3p_st | 0.46 | ACGGGTGCGATTTCTGTGTGAGA |
| [6,] | hsa-miR-370_st | 0.32 | ACCAGGTTCCACCCCAGCAGGC |
| [7,] | hsa-miR-433_st | 0.31 | ACACCGAGGAGCCCATCATGAT |
| [8,] | hsa-miR-654-3p_st | 0.32 | AAGGTGATGGTCAGCAGACATA |
| [9,] | hsa-miR-758_st | 0.3 | GGTTAGTGGACCAGGTCACAAA |

Table 6 Adriamycin microRNA biomarkers.

| | Medianprobe | Corr | Sequence |
|---|---|---|---|
| [1,] | U55_st | 0.34 | TGGCTTCCCCACCGCGCACTCGGGA |
| [2,] | U55_x_st | 0.34 | GCACTCGGGAGTATGCAGCATTACC |
| [3,] | hsa-miR-106b-star_st | 0.36 | GCAGCAAGTACCCACAGTGCGG |
| [4,] | hsa-miR-106b_st | 0.36 | ATCTGCACTGTCAGCACTTTA |
| [5,] | hsa-miR-124_st | 0.31 | GGCATTCACCGCGTGCCTTA |
| [6,] | hsa-miR-1299_st | 0.31 | TCCCTCACACAGAATTCCAGAA |
| [7,] | hsa-miR-29b-2-star_st | 0.33 | CTAAGCCACCATGTGAAACCAG |
| [8,] | hsa-miR-33b-star_st | 0.33 | GGGCTGCACTGCCGAGGCACTG |
| [9,] | hsa-miR-629-star_st | 0.33 | GCTGGGCTTACGTTGGGAGAAC |
| [10,] | hsa-miR-652_st | 0.35 | CACAACCCTAGTGGCGCCATT |
| [11,] | hsa-miR-671-5p_st | 0.34 | CTCCAGCCCCTCCAGGGCTTCCT |
| [12,] | hsa-miR-766_st | 0.31 | GCTGAGGCTGTGGGGCTGGAGT |
| [13,] | hsa-miR-768-3p_st | 0.33 | GTCAGCAGTTTGAGTGTCAGCATTG |
| [14,] | hsa-miR-93-star_st | 0.32 | CGGGAAGTGCTAGCTCAGCAGT |
| [15,] | hsa-miR-93_st | 0.34 | CTACCTGCACGAACAGCACTTTG |

Table 7 Aclarubicin microRNA biomarkers.

| | Medianprobe | Corr | Sequence |
|---|---|---|---|
| [1,] | ACA10_s_st | 0.4 | TAGGAACAGAGAGGCCATTCTGGGC |
| [2,] | ACA18_x_st | 0.36 | GCTACAGGAAAAGCCCCATCGGGAT |
| [3,] | ACA44_st | 0.3 | TGGAGATCCCATGGCTATGACCAGC |
| [4,] | ACA51_x_st | 0.34 | TACCTCCTCTTTCTATACAGTCAGT |
| [5,] | ACA61_st | 0.39 | TAGGCCAGCTTCACTATTACTTTTC |
| [6,] | ENSG00000200394_st | 0.32 | TTGATCTTGAGCCTGCGGAGAGCAA |
| [7,] | ENSG00000202252_st | 0.31 | GGTGTTGCCATCATTAGCCAAGCTT |
| [8,] | HBII-180A_x_st | 0.31 | GGCACCGTGTCCTCAGTGGCAGTCG |
| [9,] | HBII-429_st | 0.32 | ATCAGAAGGGTGACATGGCAGTTTC |
| [10,] | U104_st | 0.38 | AGGCTCAGACTCCAGTTCGCATCAC |
| [11,] | U13_st | 0.33 | GTTCAAGGGTGGCACATCTCACACA |
| [12,] | U17b_st | 0.34 | GAGGCCCAGCTTCATCTTCAACGTT |
| [13,] | U17b_x_st | 0.31 | ACGAGGCCCAGCTTCATCTTCAACG |
| [14,] | U26_st | 0.35 | GTTCAGTTCGTAAAATCATCCCCGT |
| [15,] | U3-2_s_st | 0.31 | CATCAATGGCTGACGGCAGTTGCAG |
| [16,] | U35A_st | 0.3 | GACATCCGCAGACCATCGTGAGATA |
| [17,] | U49A_st | 0.31 | AGGAGTAGTCTTCGTCAGTTATCGC |
| [18,] | U49A_x_st | 0.31 | ACAGGAGTAGTCTTCGTCAGTTATC |
| [19,] | U49B_s_st | 0.36 | GTCAGTTATCGCTTCTGACGGCACT |
| [20,] | U67_st | 0.33 | ATCCCAGTTCCCCAAAGGCCTTAGG |
| [21,] | U68_st | 0.31 | CGACAAGATCCGCTTGCTGTTTGCA |
| [22,] | U68_x_st | 0.31 | AAGGCGACAAGATCCGCTTGCTGTT |

(continued)

| | Medianprobe | Corr | Sequence |
|---|---|---|---|
| [23,] | U74_x_st | 0.38 | CGGTTGGCATTCATCATTACTCTCA |
| [24,] | hsa-miR-1275_st | 0.45 | GACAGCCTCTCCCCCAC |
| [25,] | hsa-miR-1281_st | 0.3 | GGGAGAGGAGGAGGCGA |
| [26,] | hsa-miR-18a-star_st | 0.36 | CCAGAAGGAGCACTTAGGGCAGT |
| [27,] | hsa-miR-18a_st | 0.33 | CTATCTGCACTAGATGCACCTTA |
| [28,] | hsa-miR-25-star_st | 0.35 | CAATTGCCCAAGTCTCCGCCT |
| [29,] | hsa-miR-33b-star_st | 0.33 | GGGCTGCACTGCCGAGGCACTG |

Table 8 Mitoxantrone microRNA biomarkers.

| | Medianprobe | Corr | Sequence |
|---|---|---|---|
| [1,] | 14qII-14_st | 0.33 | CCAACACTCATACGCCGGCAGTTGT |
| [2,] | 14qII-1_st | 0.31 | TGGACCTCAGACTTCCAGACCTGTA |
| [3,] | 14qII-26_st | 0.33 | TCATCGTATGTGACTCATACTCCAC |
| [4,] | 14qII-26_x_st | 0.3 | GTGACTCATACTCCACCAGTGCTCA |
| [5,] | hsa-miR-127-3p_st | 0.36 | AGCCAAGCTCAGACGGATCCGA |
| [6,] | hsa-miR-181a-star_st | 0.38 | GGTACAATCAACGGTCGATGGT |
| [7,] | hsa-miR-181a_st | 0.31 | ACTCACCGACAGCGTTGAATGTT |
| [8,] | hsa-miR-181b_st | 0.35 | ACCCACCGACAGCAATGAATGTT |
| [9,] | hsa-miR-181c_st | 0.31 | ACTCACCGACAGGTTGAATGTT |
| [10,] | hsa-miR-342-3p_st | 0.36 | ACGGGTGCGATTTCTGTGTGAGA |
| [11,] | hsa-miR-342-5p_st | 0.31 | TCAATCACAGATAGCACCCCT |
| [12,] | hsa-miR-409-3p_st | 0.34 | AGGGGTTCACCGAGCAACATTC |
| [13,] | hsa-miR-432_st | 0.37 | CCACCCAATGACCTACTCCAAGA |
| [14,] | hsa-miR-487b_st | 0.31 | AACTGGATGACCCTGTACGATT |
| [15,] | hsa-miR-654-3p_st | 0.3 | AAGGTGATGGTCAGCAGACATA |
| [16,] | hsa-miR-768-5p_st | 0.31 | ATCACTCCGTACTTTCATCCTCCAA |
| [17,] | hsa-miR-92b_st | 0.32 | GGAGGCCGGGACGAGTGCAATA |

Table 9 Mitomycin microRNA biomarkers.

| | Medianprobe | Corr | Sequence |
|---|---|---|---|
| [1,] | hsa-miR-124_st | 0.31 | GGCATTCACCGCGTGCCTTA |
| [2,] | hsa-miR-181a-star_st | 0.31 | GGTACAATCAACGGTCGATGGT |
| [3,] | hsa-miR-181b_st | 0.33 | ACCCACCGACAGCAATGAATGTT |

Table 10 Paclitaxel (Taxol) microRNA biomarkers.

| | Medianprobe | Corr | Sequence |
|---|---|---|---|
| [1,] | hsa-miR-106b-star_st | 0.31 | GCAGCAAGTACCCACAGTGCGG |
| [2,] | hsa-miR-1228_st | 0.31 | GGGGGGCGAGGCAGGTGTGA |
| [3,] | hsa-miR-185_st | 0.33 | TCAGGAACTGCCTTTCTCTCCA |
| [4,] | hsa-miR-188-5p_st | 0.31 | CCCTCCACCATGCAAGGGATG |
| [5,] | hsa-miR-18b_st | 0.34 | CTAACTGCACTAGATGCACCTTA |
| [6,] | hsa-miR-20b_st | 0.32 | CTACCTGCACTATGAGCACTTTG |
| [7,] | hsa-miR-25_st | 0.32 | TCAGACCGAGACAAGTGCAATG |
| [8,] | hsa-miR-320c_st | 0.32 | ACCCTCTCAACCCAGCTTTT |
| [9,] | hsa-miR-320d_st | 0.34 | TCCTCTCAACCCAGCTTTT |
| [10,] | hsa-miR-362-5p_st | 0.4 | ACTCACACCTAGGTTCCAAGGATT |

(continued)

| | Medianprobe | Corr | Sequence |
|---|---|---|---|
| [11,] | hsa-miR-500-starst | 0.34 | CAGAATCCTTGCCCAGGTGCAT |
| [12,] | hsa-miR-500_st | 0.34 | TCTCACCCAGGTAGCAAGGATTA |
| [13,] | hsa-miR-501-3p_st | 0.33 | AGAATCCTTGCCCGGGTGCATT |
| [14,] | hsa-miR-502-3p_st | 0.34 | TGAATCCTTGCCCAGGTGCATT |
| [15,] | hsa-miR-532-3p_st | 0.35 | TGCAAGCCTTGGGTGTGGGAGG |
| [16,] | hsa-miR-532-5p_st | 0.41 | ACGGTCCTACACTCAAGGCATG |
| [17,] | hsa-miR-652_st | 0.47 | CACAACCCTAGTGGCGCCATT |
| [18,] | hsa-miR-766_st | 0.31 | GCTGAGGCTGTGGGGCTGGAGT |

Table 11 Gemcitabine (Gemzar) microRNA biomarkers.

| | Medianprobe | Corr | Sequence |
|---|---|---|---|
| [1,] | HBII-438A_s_st | 0.33 | ATCTGGAATGAGTCCCTCAGCATCC |
| [2,] | hsa-miR-155_st | 0.37 | ACCCCTATCACGATTAGCATTAA |
| [3,] | hsa-miR-181a-star_st | 0.33 | GGTACAATCAACGGTCGATGGT |
| [4,] | hsa-miR-181b_st | 0.31 | ACCCACCGACAGCAATGAATGTT |
| [5,] | hsa-miR-342-3p_st | 0.36 | ACGGGTGCGATTTCTGTGTGAGA |
| [6,] | hsa-miR-424-star_st | 0.35 | ATAGCAGCGCCTCACGTTTTG |
| [7,] | hsa-miR-503_st | 0.31 | CTGCAGAACTGTTCCCGCTGCTA |

Table 12 Taxotere (docetaxel) microRNA biomarkers.

| | Medianprobe | Corr | Sequence |
|---|---|---|---|
| [1,] | hsa-miR-1307_st | 0.31 | CACGACCGACGCCACGCCGAGT |
| [2,] | hsa-miR-505_st | 0.31 | AGGAAACCAGCAAGTGTTGACG |
| [3,] | hsa-miR-769-3p_st | 0.35 | AACCAAGACCCCGGAGATCCCAG |
| [4,] | hsa-miR-769-5p_st | 0.36 | AGCTCAGAACCCAGAGGTCTCA |

Table 13 Dexamethasone microRNA biomarkers.

| | Medianprobe | Corr | Sequence |
|---|---|---|---|
| [1,] | U49A_st | 0.38 | ACAGGAGTAGTCTTCGTCAGTTATC |
| [2,] | U49A_x_st | 0.41 | TCAGTTATCGCTTCTGACGGCACTT |
| [3,] | U49B_s_st | 0.42 | GTCAGTTATCGCTTCTGACGGCACT |
| [4,] | U49B_x_st | 0.35 | CGTCAGTTATCGCTTCTGACGGCAC |
| [5,] | hsa-miR-10a-star_st | 0.31 | TATTCCCCTACATACGAATTTG |
| [6,] | hsa-miR-1207-5p_st | 0.37 | CCCCTCCCAGCCTCCCTGCCA |
| [7,] | hsa-miR-128_st | 0.34 | AAAGAGACCGGTTCACTGTGA |
| [8,] | hsa-miR-150_st | 0.33 | CACTGGTACAAGGGTTGGGAGA |
| [9,] | hsa-miR-424-star_st | 0.42 | ATAGCAGCGCCTCACGTTTTG |
| [10,] | _ hsa-miR-424_st | 0.38 | TTCAAAACATGAATTGCTGCTG |
| [11,] | hsa-miR-503_st | 0.41 | CTGCAGAACTGTTCCCGCTGCTA |
| [12,] | hsa-miR-766_st | 0.33 | GCTGAGGCTGTGGGGCTGGAGT |
| [13,] | hsa-miR-768-5p_st | 0.31 | ATCACTCCGTACTTTCATCCTCCAA |

Table 14 Ara-C (Cytarabine hydrochloride) microRNA biomarkers.

| | Medianprobe | Corr | Sequence |
|---|---|---|---|
| [1,] | HBII-202_st | 0.4 | TTCATCAAGGCCGTACAGCGATTCC |

(continued)

| | Medianprobe | Corr | Sequence |
|---|---|---|---|
| [2,] | HBII-438A_s_st | 0.33 | AGCATCCTCAGACAATTATTCTCAT |
| [3,] | HBII-85-11_st | 0.3 | TGTTCAACTTTCCAAGGAACCCACG |
| [4,] | U104_st | 0.36 | GGCAGGCTCAGACTCCAGTTCCCAT |
| [5,] | U17b_st | 0.31 | CGAGGCCCAGCTTCATCTTCAACGT |
| [6,] | U17b_x_st | 0.3 | ACGAGGCCCAGCTTCATCTTCAACG |
| [7,] | U48_st | 0.31 | GGTGATGGCATCAGCGACACACTCA |
| [8,] | U78_s_st | 0.34 | CATGCTCATTTCAGGTCAGACATTT |
| [9,] | U78_x_st | 0.31 | TTTGTCTACATGCTCATTTCAGGTC |
| [10,] | hsa-miR-181a-star_st | 0.42 | GGTACAATCAACGGTCGATGGT |
| [11,] | hsa-miR-181b_st | 0.32 | ACCCACCGACAGCAATGAATGTT |
| [12,] | hsa-miR-342-3p_st | 0.34 | ACGGGTGCGATTTCTGTGTGAGA |
| [13,] | hsa-miR-424-star_st | 0.3 | ATAGCAGCGCCTCACGTTTTG |

Table 15 Methylprednisolone microRNA biomarkers.

| | Medianprobe | Corr | Sequence |
|---|---|---|---|
| [1,] | ACA23_st | 0.31 | GCCAGTGGTAGATGTGTCCAGAGAC |
| [2,] | ACA24_x_st | 0.37 | CAAGGATATGCTCTTCCATGGCTAG |
| [3,] | ACA54_st | 0.31 | GTCATGTGTCGCTGGAAATGCTATT |
| [4,] | U31_st | 0.3 | AATACCTTTCAGTCACACATTGATC |
| [5,] | hsa-let-7d-star_st | 0.34 | AGAAAGGCAGCAGGTCGTATAG |
| [6,] | hsa-miR-106b-star_st | 0.36 | GCAGCAAGTACCCACAGTGCGG |
| [7,] | hsa-miR-1183_st | 0.41 | TGCCCACTCTCACCATCACCTACAG |
| [8,] | hsa-miR-1207-5p_st | 0.5 | CCCCTCCCAGCCTCCCTGCCA |
| [9,] | hsa-miR-1268_st | 0.34 | CCCCCACCACCACGCCCG |
| [10,] | hsa-miR-1281_st | 0.41 | GGGAGAGGAGGAGGCGA |
| [11,] | hsa-miR-128_st | 0.45 | AAAGAGACCGGTTCACTGTGA |
| [12,] | hsa-miR-150_st | 0.33 | CACTGGTACAAGGGTTGGGAGA |
| [13,] | hsa-miR-15a_st | 0.38 | CACAAACCATTATGTGCTGCTA |
| [14,] | hsa-miR-181a-star_st | 0.38 | GGTACAATCAACGGTCGATGGT |
| [15,] | hsa-miR-181c_st | 0.37 | ACTCACCGACAGGTTGAATGTT |
| [16,] | hsa-miR-18b_st | 0.31 | CTAACTGCACTAGATGCACCTTA |
| [17,] | hsa-miR-198_st | 0.31 | GAACCTATCTCCCCTCTGGACC |
| [18,] | hsa-miR-20b-star_st | 0.49 | CTGGAAGTGCCCATACTACAGT |
| [19,] | hsa-miR-223_st | 0.38 | TGGGGTATTTGACAAACTGACA |
| [20,] | hsa-miR-297_st | 0.34 | CATGCACATGCACACATACAT |
| [21,] | hsa-miR-342-5p_st | 0.44 | TCAATCACAGATAGCACCCCT |
| [22,] | hsa-miR-363_st | 0.38 | TACAGATGGATACCGTGCAATT |
| [23,] | hsa-miR-588_st | 0.31 | GTTCTAACCCATTGTGGCCAA |
| [24,] | hsa-miR-631_st | 0.41 | GCTGAGGTCTGGGCCAGGTCT |
| [25,] | hsa-miR-766_st | 0.49 | GCTGAGGCTGTGGGGCTGGAGT |
| [26,] | hsa-miR-768-5p_st | 0.32 | ATCACTCCGTACTTTCATCCTCCAA |
| [27,] | hsa-miR-92a-2-star_st | 0.44 | GTAATGCAACAAATCCCCACCC |
| [28,] | hsa-miR-940_st | 0.34 | GGGGAGCGGGGGCCCTGCCTT |

Table 16 Methotrexate microRNA biomarkers.

| | Medianprobe | Corr | Sequence |
|---|---|---|---|
| [1,] | ACA10_s_st | 0.3 | CACTCCTAGGAACAGAGAGGCCATT |
| [2,] | ACA18_x_st | 0.31 | GCGTTTCCAACGATGTGCAGGCTAC |

(continued)

| | Medianprobe | Corr | Sequence |
|---|---|---|---|
| [3,] | HBII-202_st | 0.35 | TTCATCAAGGCCGTACAGCGATTCC |
| [4,] | U104_st | 0.37 | TTTAATTGGAATGTCATCACAGCAG |
| [5,] | U17b_st | 0.34 | CGAGGCCCAGCTTCATCTTCAACGT |
| [6,] | U30_st | 0.34 | GTCATCAGCCGAACGAGATTCCATG |
| [7,] | U31_x_st | 0.31 | GAAAATACCTTTCAGTCACACATTG |
| [8,] | U49A_st | 0.31 | TTCCTATTAGTGATTTCATCAGAGC |
| [9,] | U49A_x_st | 0.32 | GGAGTAGTCTTCGTCAGTTATCGCT |
| [10,] | U49B_s_st | 0.33 | GTTATCGCTTCTGACGGCACTTCCT |
| [11,] | U55_st | 0.34 | GAGTATGCAGCATTACCGAGTTGTC |
| [12,] | U55_x_st | 0.33 | GAGTATGCAGCATTACCGAGTTGTC |
| [13,] | U56_st | 0.31 | GAGTCTCAACACTCACTAGGTGAAC |
| [14,] | U67_st | 0.36 | TGAGAGGCACTGATGTCCCCTTGGA |
| [15,] | U67_x_st | 0.3 | CAGTTCCCCAAAGGCCTTAGGCATG |
| [16,] | hsa-miR-106a_st | 0.33 | CTACCTGCACTGTAAGCACTTTT |
| [17,] | hsa-miR-1254_st | 0.31 | ACTGCAGGCTCCAGCTTCCAGGCT |
| [18,] | hsa-miR-1275_st | 0.31 | GACAGCCTCTCCCCCAC |
| [19,] | hsa-miR-17_st | 0.35 | CTACCTGCACTGTAAGCACTTTG |
| [20,] | hsa-miR-18a-star_st | 0.36 | CCAGAAGGAGCACTTAGGGCAGT |
| [21,] | hsa-miR-18a_st | 0.35 | CTATCTGCACTAGATGCACCTTA |
| [22,] | hsa-miR-19b_st | 0.32 | TCAGTTTTGCATGGATTTGCACA |
| [23,] | hsa-miR-25-star_st | 0.3 | CAATTGCCCAAGTCTCCGCCT |
| [24,] | hsa-miR-297_st | 0.31 | CATGCACATGCACACATACAT |
| [25,] | hsa-miR-34b_st | 0.31 | ATGGCAGTGGAGTTAGTGATTG |
| [26,] | hsa-miR-663b_st | 0.38 | CCTCAGGCACGGCCGGGCCACC |
| [27,] | hsa-miR-92a_st | 0.33 | ACAGGCCGGGACAAGTGCAATA |

Table 17 Bleomycin microRNA biomarkers.

| | Medianprobe | Corr | Sequence |
|---|---|---|---|
| [1,] | 14qII-14_st | 0.41 | ACCCAACACTCATACGCCGGCAGTT |
| [2,] | 14qII-14_x_st | 0.4 | ATACGCCGGCAGTTGTCATCATTGG |
| [3,] | 14qII-1_st | 0.38 | TCAGACTTCCAGACCTGTATTCACC |
| [4,] | 14qII-1_x_st | 0.32 | TGGACCTCAGACTTCCAGACCTGTA |
| [5,] | 14qII-26_st | 0.43 | TGTGACTCATACTCCACCAGTGCTC |
| [6,] | 14qII-26_x_st | 0.4 | ATCGTATGTGACTCATACTCCACCA |
| [7,] | 14qII-3_st | 0.33 | CAGACACGTAGTATTCATCGTCCAT |
| [8,] | hsa-miR-125b-1-star_st | 0.32 | AGCTCCCAAGAGCCTAACCCGT |
| [9,] | hsa-miR-127-3p_st | 0.41 | AGCCAAGCTCAGACGGATCCGA |
| [10,] | hsa-miR-1271_st | 0.32 | TGAGTGCTTGCTAGGTGCCAAG |
| [11,] | hsa-miR-134_st | 0.34 | CCCCTCTGGTCAACCAGTCACA |
| [12,] | hsa-miR-155_st | 0.37 | ACCCCTATCACGATTAGCATTAA |
| [13,] | hsa-miR-193a-5p_st | 0.32 | TCATCTCGCCCGCAAAGACCCA |
| [14,] | hsa-miR-21-star_st | 0.39 | ACAGCCCATCGACTGGTGTTG |
| [15,] | hsa-miR-22_st | 0.31 | ACAGTTCTTCAACTGGCAGCTT |
| [16,] | hsa-miR-299-3p_st | 0.34 | AAGCGGTTTACCATCCCACATA |
| [17,] | hsa-miR-337-5p_st | 0.3 | AACTCCTGTATGAAGCCGTTC |
| [18,] | hsa-miR-370_st | 0.35 | ACCAGGTTCCACCCCAGCAGGC |
| [19,] | hsa-miR-376c_st | 0.3 | ACGTGGAATTTCCTCTATGTT |
| [20,] | hsa-miR-377-star_st | 0.37 | GAATTCACCAAGGGCAACCTCT |
| [21,] | hsa-miR-379_st | 0.36 | CCTACGTTCCATAGTCTACCA |

(continued)

| | Medianprobe | Corr | Sequence |
|---|---|---|---|
| [22,] | hsa-miR-381_st | 0.4 | ACAGAGAGCTTGCCCTTGTATA |
| [23,] | hsa-miR-382_st | 0.32 | CGAATCCACCACGAACAACTTC |
| [24,] | hsa-miR-409-3p_st | 0.36 | AGGGGTTCACCGAGCAACATTC |
| [25,] | hsa-miR-409-5p_st | 0.35 | ATGCAAAGTTGCTCGGGTAACCT |
| [26,] | hsa-miR-411_st | 0.39 | CGTACGCTATACGGTCTACTA |
| [27,] | hsa-miR-431_st | 0.32 | TGCATGACGGCCTGCAAGACA |
| [28,] | hsa-miR-455-3p_st | 0.41 | GTGTATATGCCCATGGACTGC |
| [29,] | hsa-miR-485-5p_st | 0.34 | GAATTCATCACGGCCAGCCTCT |
| [30,] | hsa-miR-487b_st | 0.37 | AAGTGGATGACCCTGTACGATT |
| [31,] | hsa-miR-493_st | 0.34 | CCTGGCACACAGTAGACCTTCA |
| [32,] | hsa-miR-494_st | 0.39 | GAGGTTTCCCGTGTATGTTTCA |
| [33,] | hsa-miR-543_st | 0.33 | AAGAAGTGCACCGCGAATGTTT |
| [34,] | hsa-miR-663_st | 0.3 | GCGGTCCCGCGGCGCCCCGCCT |
| [35,] | hsa-miR-671-5p_st | 0.32 | CTCCAGCCCCTCCAGGGCTTCCT |
| [36,] | hsa-miR-758_st | 0.33 | GGTTAGTGGACCAGGTCACAAA |

Table 18 Methyl-GAG (methyl glyoxal bis amidinohydrazone dihydrochloride) microRNA biomarkers.

| | Medianprobe | Corr | Sequence |
|---|---|---|---|
| [1,] | ENSG00000200879_ st | 0.34 | GCGAATGTTTTGGACCATTCATCAT |
| [2,] | ENSG00000202252_st | 0.34 | AAGGAAGGTGTTGCCATCATTAGCC |
| [3,] | HBII-202 _ st | 0.31 | AGGCCGTACAGCGATTCCGGAGAAT |
| [4,] | U25_st | 0.34 | GTCTGTGAAAAGGTCCTCATCATAG |
| [5,] | U26_st | 0.35 | TCCACTAATCCATCAGAAAGAGAGA |
| [6,] | U28_x_st | 0.3 | ACTTTTGTACCTCACAGAACATCAG |
| [7,] | U29_st | 0.32 | TTCTCAGGTGTTCATGTATTTTCAC |
| [8,] | U30_st | 0.31 | GTCATCAGCCGAACGAGATTCCATG |
| [9,] | U31_x_ st | 0.3 | CAGCTCAGAAAATACCTTTCAGTCA |
| [10,] | U74 4_x_st | 0.3 | TTCATCATTACTCTCAGATGTCCCT |
| [11,] | hsa-miR-181a-star_st | 0.34 | GGTACAATCAACGGTCGATGGT |
| [12,] | hsa-miR-297_ st | 0.31 | CATGCACATGCACACATACAT |
| [13,] | hsa-miR-34b_ st | 0.32 | ATGGCAGTGGAGTTAGTGATTG |
| [14,] | hsa-miR-92a-1-star_st | 0.31 | AGCATTGCAACCGATCCCAACCT |

Table 19 Belinostat (PXD101) microRNA biomarkers.

| | Medianprobe | Corr | Sequence |
|---|---|---|---|
| [1,] | ACA18_x_st | 0.32 | TCTTGTAATTCCTTCCCACAGATCT |
| [2,] | ACA51_x_st | 0.32 | TGGGGTAGGTTTACTCTACCTCCTC |
| [3,] | ACA9_st | 0.33 | AATGCATAGGGTCACCGCAGACCCA |
| [4,] | ACA9_x_st | 0.33 | ATAGGGTCACCGCAGACCCAAGCAC |
| [5,] | HBII-180A_x_st | 0.32 | ACCGTGTCCTCAGTGGCAGTCGGAG |
| [6,] | HBII-202_st | 0.33 | ACAGCGATTCCGGAGAATGTCATCA |
| [7,] | HBII-336_st | 0.37 | GTAAATCCTTTAATCCATCACAGCA |
| [8,] | HBII-55_st | 0.37 | GTGATTGCACTCAGGGGATTGACAG |
| [9,] | U104_st | 0.4 | GCGTCAGCAGTCTAACACGTGCTTT |
| [10,] | U27_st | 0.36 | TGTCATTTTGTGTTCATCATGGAGT |
| [11,] | U29_st | 0.34 | GAGCTAGTTTGATTCATCATAGAAA |
| [12,] | U30_st | 0.32 | CAGCCGAACGAGATTCCATGTAAGT |
| [13,] | U31_st | 0.32 | GCTCAGAAAATACCTTTCAGTCACA |

(continued)

| | Medianprobe | Corr | Sequence |
|---|---|---|---|
| [14,] | U31_x_st | 0.31 | CCTTTCAGTCACACATTGATCAGAC |
| [15,] | U38A_st | 0.33 | TCTCTCTCCCTTCAGTAGCAGAACT |
| [16,] | U55_st | 0.35 | TTCCCCACCGCGCACTCGGGAGTAT |
| [17,] | U55_x_st | 0.36 | CCACCGCGCACTCGGGAGTATGCAG |
| [18,] | U56_st | 0.32 | ACCCAGAGTCTCAACACTCACTAGG |
| [19,] | U56_x_st | 0.37 | CACTAGGTGAACTGCTGTTGACGAA |
| [20,] | U57_st | 0.33 | TTCTACAAGGTCAGGCTCAGACAGT |
| [21,] | U60_st | 0.33 | TACGAGGTGTCAGAAGTCAAAGCAA |
| [22,] | U74_x_st | 0.35 | TTCATCATTACTCTCAGATGTCCCT |
| [23,] | hsa-miR-106a_st | 0.48 | CTACCTGCACTGTAAGCACTTTT |
| [24,] | hsa-miR-1183_st | 0.32 | TGCCCACTCTCACCATCACCTACAG |
| [25,] | hsa-miR-1207-5p_st | 0.34 | CCCCTCCCAGCCTCCCTGCCA |
| [26,] | hsa-miR-1246_st | 0.38 | CCTGCTCCAAAAATCCATT |
| [27,] | hsa-miR-1268_st | 0.33 | CCCCCACCACCACGCCCG |
| [28,] | hsa-miR-1299_st | 0.39 | TCCCTCACACAGAATTCCAGAA |
| [29,] | hsa-miR-1307_st | 0.3 | CACGACCGACGCCACGCCGAGT |
| [30,] | hsa-miR-142-5p_st | 0.31 | AGTAGTGCTTTCTACTTTATG |
| [31,] | hsa-miR-17-star_st | 0.43 | CTACAAGTGCCTTCACTGCAGT |
| [32,] | hsa-miR-17_st | 0.49 | CTACCTGCACTGTAAGCACTTTG |
| [33,] | hsa-miR-18a-star_st | 0.45 | CCAGAAGGAGCACTTAGGGCAGT |
| [34,] | hsa-miR-18a_st | 0.55 | CTATCTGCACTAGATGCACCTTA |
| [35,] | hsa-miR-18b_st | 0.48 | CTAACTGCACTAGATGCACCTTA |
| [36,] | hsa-miR-195-star_st | 0.32 | GGAGCAGCACAGCCAATATTGG |
| [37,] | hsa-miR-19a_st | 0.49 | TCAGTTTTGCATAGATTTGCACA |
| [38,] | hsa-miR-19b_st | 0.56 | TCAGTTTTGCATGGATTTGCACA |
| [39,] | hsa-miR-20a_st | 0.46 | CTACCTGCACTATAAGCACTTTA |
| [40,] | hsa-miR-20b_st | 0.31 | CTACCTGCACTATGAGCACTTTG |
| [41,] | hsa-miR-297_st | 0.38 | CATGCACATGCACACATACAT |
| [42,] | hsa-miR-330-3p_st | 0.37 | TCTCTGCAGGCCGTGTGCTTTGC |
| [43,] | hsa-miR-346_st | 0.33 | AGAGGCAGGCATGCGGGCAGACA |
| [44,] | hsa-miR-34b_st | 0.41 | ATGGCAGTGGAGTTAGTGATTG |
| [45,] | hsa-miR-595_st | 0.31 | AGACACACCACGGCACACTTC |
| [46,] | hsa-miR-629-star_st | 0.32 | GCTGGGCTTACGTTGGGAGAAC |
| [47,] | hsa-miR-647_st | 0.31 | GAAGGAAGTGAGTGCAGCCAC |
| [48,] | hsa-miR-766_st | 0.41 | GCTGAGGCTGTGGGGCTGGAGT |
| [49,] | hsa-miR-768-5p_st | 0.32 | ATCACTCCGTACTTTCATCCTCCAA |
| [50,] | hsa-miR-92a_st | 0.48 | ACAGGCCGGGACAAGTGCAATA |

Table 20 5-Fluorouracil microRNA biomarkers.

| | Medianprobe | Corr | Sequence |
|---|---|---|---|
| [1,] | ACA10_s_st | 0.34 | TTCTGGGCGGGTCTGTCGTGCATTA |
| [2,] | ENSG00000199411_s_st | 0.31 | TCACTGTCCTCTTCATCTCCCTGCT |
| [3,] | U104_st | 0.4 | GCGTCAGCAGTCTAACACGTGCTTT |
| [4,] | U13_st | 0.34 | AGACGGGTAATGTGCCCACGTCGTA |
| [5,] | U36A_x_st | 0.31 | CGCACTTCAAGGTTGAATTCAGTGA |
| [6,] | U36C_st | 0.31 | GCCTCAGATGCAATGCTGACCACAT |
| [7,] | U74_x_st | 0.41 | CGGTTGGCATTCATCATTACTCTCA |
| [8,] | U78_x_st | 0.31 | ACCTTTGTCTACATGCTCATTTCAG |
| [9,] | U8_x_st | 0.34 | CTAATCTGCCCTCCGGAGGAGGAAC |

(continued)

| | Medianprobe | Corr | Sequence |
|---|---|---|---|
| [10,] | hsa-miR-1246_st | 0.3 | CCTGCTCCAAAAATCCATT |
| [11,] | hsa-miR-194-star_st | 0.32 | CAGATAACAGCAGCCCCACTGG |
| [12,] | hsa-miR-200c-star_st | 0.33 | CCAAACACTGCTGGGTAAGACG |
| [13,] | hsa-miR-34b_st | 0.32 | ATGGCAGTGGAGTTAGTGATTG |
| [14,] | hsa-miR-768-3p_st | 0.32 | GTCAGCAGTTTGAGTGTCAGCATTG |

Table 21 Radiation microRNA biomarkers.

| | Medianprobe | Corr | Sequence |
|---|---|---|---|
| [1,] | hsa-let-7i-star_st | 0.4 | AGCAAGGCAGTAGCTTGCGCAG |
| [2,] | hsa-miR-338-3p_st | 0.31 | CAACAAAATCACTGATGCTGGA |
| [3,] | hsa-miR-34a_st | 0.37 | ACAACCAGCTAAGACACTGCCA |

Table 22 5-Aza-2'-deoxycytidine(decitabine) microRNA biomarkers.

| | Medianprobe | Corr | Sequence |
|---|---|---|---|
| [1,] | hsa-miR-106a_st | 0.31 | CTACCTGCACTGTAAGCACTTTT |
| [2,] | hsa-miR-106b-star_st | 0.32 | GCAGCAAGTACCCACAGTGCGG |
| [3,] | hsa-miR-1183_st | 0.32 | TGCCCACTCTCACCATCACCTACAG |
| [4,] | hsa-miR-195-star_st | 0.36 | GGAGCAGCACAGCCAATATTGG |
| [5,] | hsa-miR-297_st | 0.31 | CATGCACATGCACACATACAT |
| [6,] | hsa-miR-324-3p_st | 0.41 | CCAGCAGCACCTGGGGCAGT |
| [7,] | hsa-miR-34b_st | 0.34 | ATGGCAGTGGAGTTAGTGATTG |
| [8,] | hsa-miR-371-5p_st | 0.33 | AGTGCCCCCACAGTTTGAGT |
| [9,] | hsa-miR-766_st | 0.37 | GCTGAGGCTGTGGGGCTGGAGT |
| [10,] | hsa-miR-92a_st | 0.33 | ACAGGCCGGGACAAGTGCAATA |
| [11,] | hsa-miR-93-star_st | 0.31 | CGGGAAGTGCTAGCTCAGCAGT |

Table 23 Idarubicin microRNA biomarkers.

| | Medianprobe | Corr | Sequence |
|---|---|---|---|
| [1,] | ACA41_x_st | 0.34 | ATACCCACTTTCTGTTTTGCTAAGA |
| [2,] | ACA48_x_st | 0.35 | GGGCACGGATCAAAAGCCAAGCTGG |
| [3,] | HBII-202_st | 0.32 | GCGATTCCGGAGAATGTCATCACGC |
| [4,] | HBII-429_st | 0.3 | GAGGGAGCCAGTTGTCATCATGTAC |
| [5,] | U104_st | 0.39 | TTTAATTGGAATGTCATCACAGCAG |
| [6,] | U49A_st | 0.3 | AGGAGTAGTCTTCGTCAGTTATCGC |
| [7,] | U49B_s_st | 0.32 | GTTATCGCTTCTGACGGCACTTCCT |
| [8,] | U55_st | 0.38 | GCACTCGGGAGTATGCAGCATTACC |
| [9,] | U55_x_st | 0.38 | CGGGAGTATGCAGCATTACCGAGTT |
| [10,] | U74_x_st | 0.33 | ACCATCAGAGCGGTTGGCATTCATC |
| [11,] | hsa-miR-124_st | 0.44 | GGCATTCACCGCGTGCCTTA |
| [12,] | hsa-miR-1299_st | 0.3 | TCCCTCACACAGAATTCCAGAA |
| [13,] | hsa-miR-181a-star_st | 0.35 | GGTACAATCAACGGTCGATGGT |
| [14,] | hsa-miR-297_st | 0.31 | CATGCACATGCACACATACAT |
| [15,] | hsa-miR-342-5p_st | 0.37 | TCAATCACAGATAGCACCCCT |
| [16,] | hsa-miR-34b_st | 0.32 | ATGGCAGTGGAGTTAGTGATTG |
| [17,] | hsa-miR-631_st | 0.3 | GCTGAGGTCTGGGCCAGGTCT |
| [18,] | hsa-miR-768-3p_st | 0.34 | GTCAGCAGTTTGAGTGTCAGCATTG |

(continued)

| | Medianprobe | Corr | Sequence |
|---|---|---|---|
| [19,] | hsa-miR-768-5p_st | 0.33 | ATCACTCCGTACTTTCATCCTCCAA |

Table 24 Melphalan microRNA biomarkers.

| | Medianprobe | Corr | Sequence |
|---|---|---|---|
| [1,] | ACA7_s_st | 0.43 | ATGGGTGCCTCTGCCAGGCTTGCTT |
| [2,] | ENSG00000202252_st | 0.35 | ACATCCAAGGAAGGTGTTGCCATCA |
| [3,] | ENSG00000207002_x_st | 0.3 | TGCCTACACAGGCAGGGCAGGCACC |
| [4,] | HBII-202_st | 0.35 | TTCATCAAGGCCGTACAGCGATTCC |
| [5,] | HBII-429_st | 0.36 | GTTTCCTCATGGCAGTTCAGTAGAG |
| [6,] | HBII-438A_s_st | 0.36 | ATCCTCAGACAATTATTCTCATCAT |
| [7,] | HBII-55_st | 0.31 | TGCACTCAGGGGATTGACAGATTTG |
| [8,] | HBII-85-11_st | 0.34 | TCCAAGGAACCCACGTATGGAAGTC |
| [9,] | HBII-85-23_x_st | 0.33 | AAGGAATGCATGTATTGAGGTCATC |
| [10,] | HBII-85-26_st | 0.31 | TCCATTTTTTTTATAGTCATCATCG |
| [11,] | HBII-85-2_x_st | 0.34 | ACCTCAGTTCCGATGAGAATGACGG |
| [12,] | HBII-85-6_x_st | 0.31 | TGTTTTTTTTGGAGGACTCATCATC |
| [13,] | U104_st | 0.41 | TTTAATTGGAATGTCATCACAGCAG |
| [14,] | U13_st | 0.31 | AGACGGGTAATGTGCCCACGTCGTA |
| [15,] | U17b_st | 0.38 | GGCCCAGCTTCATCTTCAACGTTGT |
| [16,] | U17b_x_st | 0.35 | ACGAGGCCCAGCTTCATCTTCAACG |
| [17,] | U33_st | 0.3 | GTCATCTCATGGTCGGGAAACTCGA |
| [18,] | U34_st | 0.33 | TAGGTAGTTGCGGAACATCATGGAC |
| [19,] | U41_st | 0.36 | ATCAGTTCCACATCAACAGTCACAG |
| [20,] | U52_st | 0.38 | GTTTTGACATCATGACCAGCATCGG |
| [21,] | U55_st | 0.37 | TATGCAGCATTACCGAGTTGTCATC |
| [22,] | U55_x_st | 0.39 | GCTCAGCTCTCCAAGGTTGGCTTCC |
| [23,] | U56_st | 0.33 | AGACCCAGAGTCTCAACACTCACTA |
| [24,] | U57_st | 0.37 | TTTTGCCTCCATTCTACAAGGTCAG |
| [25,] | U68_st | 0.3 | GTTGTGGAACCTCCAAATTCACTTT |
| [26,] | U74_x_st | 0.38 | AGAGCGGTTGGCATTCATCATTACT |
| [27,] | U78_s_st | 0.31 | ATGCTCATTTCAGGTCAGACATTTG |
| [28,] | U78_x_st | 0.31 | CTTCAGTGTTACCTTTGTCTACATG |
| [29,] | U83_st | 0.31 | TGAGGTGCTCCTGTTTCAAATAAAC |
| [30,] | U95_st | 0.33 | AGCCTCTGGATTTCAGCACCGACAC |
| [31,] | hsa-miR-1183_st | 0.35 | TGCCCACTCTCACCATCACCTACAG |
| [32,] | hsa-miR-1207-5p_st | 0.38 | CCCCTCCCAGCCTCCCTGCCA |
| [33,] | hsa-miR-1228_st | 0.32 | GGGGGGCGAGGCAGGTGTGA |
| [34,] | hsa-miR-124_st | 0.38 | GGCATTCACCGCGTGCCTTA |
| [35,] | hsa-miR-1281 st | 0.44 | GGGAGAGGAGGAGGCGA |
| [36,] | hsa-miR-1299_st | 0.45 | TCCCTCACACAGAATTCCAGAA |
| [37,] | hsa-miR-140-3p_st | 0.3 | CCGTGGTTCTACCCTGTGGTA |
| [38,] | hsa-miR-142-5p_st | 0.4 | AGTAGTGCTTTCTACTTTATG |
| [39,] | hsa-miR-181a-star_st | 0.53 | GGTACAATCAACGGTCGATGGT |
| [40,] | hsa-miR-181a_st | 0.37 | ACTCACCGACAGCGTTGAATGTT |
| [41,] | hsa-miR-181b_st | 0.45 | ACCCACCGACAGCAATGAATGTT |
| [42,] | hsa-miR-181c_st | 0.42 | ACTCACCGACAGGTTGAATGTT |
| [43,] | hsa-miR-195-star_st | 0.45 | GGAGCAGCACAGCCAATATTGG |
| [44,] | hsa-miR-223_st | 0.39 | TGGGGTATTTGACAAACTGACA |
| [45,] | hsa-miR-297_st | 0.49 | CATGCACATGCACACATACAT |

(continued)

| Medianprobe | | Corr | Sequence |
|---|---|---|---|
| [46,] | hsa-miR-29b-2-star_st | 0.32 | CTAAGCCACCATGTGAAACCAG |
| [47,] | hsa-miR-342-3p_st | 0.45 | ACGGGTGCGATTTCTGTGTGAGA |
| [48,] | hsa-miR-342-5p_st | 0.38 | TCAATCACAGATAGCACCCCT |
| [49,] | hsa-miR-34b_st | 0.48 | ATGGCAGTGGAGTTAGTGATTG |
| [50,] | hsa-miR-541-star_st | 0.35 | AGTGGGACCGACAGCAGAATCCTTT |
| [51,] | hsa-miR-610_st | 0.31 | TCCCAGCACACATTTAGCTCA |
| [52,] | hsa-miR-647_st | 0.32 | GAAGGAAGTGAGTGCAGCCAC |
| [53,] | hsa-miR-766_st | 0.46 | GCTGAGGCTGTGGGGCTGGAGT |
| [54,] | hsa-miR-768-3p_st | 0.36 | GTCAGCAGTTTGAGTGTCAGCATTG |
| [55,] | hsa-miR-768-5p_st | 0.35 | ATCACTCCGTACTTTCATCCTCCAA |
| [56,] | hsa-miR-885-5p_st | 0.31 | AGAGGCAGGGTAGTGTAATGGA |

Table 25 IL4-PR38 fusion protein microRNA biomarkers.

| Medianprobe | | Corr | Sequence |
|---|---|---|---|
| [1,] | hsa-miR-1246_st | 0.33 | CCTGCTCCAAAAATCCATT |
| [2,] | hsa-miR-150_st | 0.38 | CACTGGTACAAGGGTTGGGAGA |
| [3,] | hsa-miR-339-3p_st | 0.38 | CGGCTCTGTCGTCGAGGCGCTCA |
| [4,] | hsa-miR-339-5p_st | 0.3 | CGTGAGCTCCTGGAGGACAGGGA |
| [5,] | hsa-miR-768-3p_st | 0.35 | GTCAGCAGTTTGAGTGTCAGCATTG |

Table 26 Valproic acid (VPA) microRNA biomarkers.

| Medianprobe | | Corr | Sequence |
|---|---|---|---|
| [1,] | hsa-miR-1308_st | 0.26 | CCACTGAACCACCCATGC |
| [2,] | hsa-miR-148a_st | 0.25 | ACAAAGTTCTGTAGTGCACTGA |
| [3,] | hsa-miR-152_st | 0.25 | CCAAGTTCTGTCATGCACTGA |
| [4,] | hsa-miR-34a-star_st | 0.26 | AGGGCAGTATACTTGCTGATTG |
| [5,] | hsa-miR-34a_st | 0.27 | ACAACCAGCTAAGACACTGCCA |

Table 27 All-trans retinoic acid (ATRA) microRNA biomarkers.

| Medianprobe | | Corr | Sequence |
|---|---|---|---|
| [1,] | HBII-142_st | 0.37 | CATGGTGTCTCAGTGGCCCAGACAC |
| [2,] | HBII-142_x_st | 0.35 | AGTTCCATCATGGTGTCTCAGTGGC |
| [3,] | U55_st | 0.3 | GCACTCGGGAGTATGCAGCATTACC |
| [4,] | U55_x_st | 0.3 | GTATGCAGCATTACCGAGTTGTCAT |
| [5,] | hsa-miR-1202_st | 0.4 | CTCCCCCACTGCAGCTGGCAC |
| [6,] | hsa-miR-124_st | 0.34 | GGCATTCACCGCGTGCCTTA |
| [7,] | hsa-miR-148a-star _ st | 0.31 | AGTCGGAGTGTCTCAGAACTTT |
| [8,] | hsa-miR-148a_st | 0.35 | ACAAAGTTCTGTAGTGCACTGA |
| [9,] | hsa-miR-184_st | 0.36 | ACCCTTATCAGTTCTCCGTCCA |
| [10,] | hsa-miR-191_st | 0.32 | CAGCTGCTTTTGGGATTCCGTTG |
| [11,] | hsa-miR-195-star_st | 0.31 | GGAGCAGCACAGCCAATATTGG |
| [12,] | hsa-miR-29b-2-star_st | 0.37 | CTAAGCCACCATGTGAAACCAG |
| [13,] | hsa-miR-425-star_st | 0.32 | GGGCGGACACGACATTCCCGAT |
| [14,] | hsa-miR-425_st | 0.37 | TCAACGGGAGTGATCGTGTCATT |
| [15,] | hsa-miR-449a_st | 0.43 | ACCAGCTAACAATACACTGCCA |
| [16,] | hsa-miR-449b_st | 0.5 | GCCAGCTAACAATACACTGCCT |

(continued)

| | Medianprobe | Corr | Sequence |
|---|---|---|---|
| [17,] | hsa-miR-551b_st | 0.31 | CTGAAACCAAGTATGGGTCGC |
| [18,] | hsa-miR-593-star_st | 0.31 | GCTGAGCAATGCCTGGCTGGTGCCT |
| [19,] | hsa-miR-768-3p_st | 0.38 | GTCAGCAGTTTGAGTGTCAGCATTG |
| [20,] | hsa-miR-768-5p_st | 0.31 | ATCACTCCGTACTTTCATCCTCCAA |
| [21,] | hsa-miR-877_st | 0.3 | CCCTGCGCCATCTCCTCTAC |

Table 28 Cytoxan microRNA biomarkers.

| | Medianprobe | Corr | Sequence |
|---|---|---|---|
| [1,] | ENSG00000200879_st | 0.33 | ATCCAAGGAAGGTAGTTGCCAACAC |
| [2,] | ENSG00000202252_st | 0.34 | ACATCCAAGGAAGGTGTTGCCATCA |
| [3,] | HBII-142_st | 0.3 | GTGTCTCAGTGGCCCAGACACGTGG |
| [4,] | hsa-miR-1202_st | 0.32 | CTCCCCCACTGCAGCTGGCAC |
| [5,] | hsa-miR-184_st | 0.37 | ACCCTTATCAGTTCTCCGTCCA |
| [6,] | hsa-miR-196a_st | 0.39 | CCCAACAACATGAAACTACCTA |
| [7,] | hsa-miR-205_st | 0.32 | CAGACTCCGGTGGAATGAAGGA |
| [8,] | hsa-miR-29b-2-star_st | 0.32 | CTAAGCCACCATGTGAAACCAG |
| [9,] | hsa-miR-29c-star_st | 0.31 | GAACACCAGGAGAAATCGGTCA |
| [10,] | hsa-miR-375_st | 0.34 | TCACGCGAGCCGAACGAACAAA |
| [11,] | hsa-miR-449a_st | 0.73 | ACCAGCTAACAATACACTGCCA |
| [12,] | hsa-miR-449b_st | 0.73 | GCCAGCTAACAATACACTGCCT |
| [13,] | hsa-miR-489_st | 0.32 | GCTGCCGTATATGTGATGTCAC |
| [14,] | hsa-miR-768-3p_st | 0.33 | GTCAGCAGTTTGAGTGTCAGCATTG |

Table 29 Topotecan (Hycamtin) microRNA biomarkers.

| | Medianprobe | Corr | Sequence |
|---|---|---|---|
| [1,] | hsa-miR-155_st | 0.31 | ACCCCTATCACGATTAGCATTAA |
| [2,] | hsa-miR-181b_st | 0.32 | ACCCACCGACAGCAATGAATGTT |
| [3,] | hsa-miR-342-3p_st | 0.4 | ACGGGTGCGATTTCTGTGTGAGA |
| [4,] | hsa-miR-342-5p_st | 0.39 | TCAATCACAGATAGCACCCCT |
| [5,] | hsa-miR-424-star_st | 0.34 | ATAGCAGCGCCTCACGTTTTG |
| [6,] | hsa-miR-503_st | 0.31 | CTGCAGAACTGTTCCCGCTGCTA |

Table 30 Suberoylanilide hydroxamic acid (SAHA, vorinostat, Zolinza) microRNA biomarkers.

| | Medianprobe | Corr | Sequence |
|---|---|---|---|
| [1,] | U56_x_st | 0.3 | CACTAGGTGAACTGCTGTTGACGAA |
| [2,] | hsa-miR-106a_st | 0.41 | CTACCTGCACTGTAAGCACTTTT |
| [3,] | hsa-miR-126_st | 0.34 | CGCATTATTACTCACGGTACGA |
| [4,] | hsa-miR-128_st | 0.4 | AAAGAGACCGGTTCACTGTGA |
| [5,] | hsa-miR-148a-star_st | 0.3 | AGTCGGAGTGTCTCAGAACTTT |
| [6,] | hsa-miR-148a_st | 0.43 | ACAAAGTTCTGTAGTGCACTGA |
| [7,] | hsa-miR-153_st | 0.35 | GATCACTTTTGTGACTATGCAA |
| [8,] | hsa-miR-17_st | 0.44 | CTACCTGCACTGTAAGCACTTTG |
| [9,] | hsa-miR-18a-star_st | 0.31 | CCAGAAGGAGCACTTAGGGCAGT |
| [10,] | hsa-miR-18a_st | 0.34 | CTATCTGCACTAGATGCACCTTA |
| [11,] | hsa-miR-18b_st | 0.49 | CTAACTGCACTAGATGCACCTTA |
| [12,] | hsa-miR-19a_st | 0.34 | TCAGTTTTGCATAGATTTGCACA |

(continued)

| | Medianprobe | Corr | Sequence |
|---|---|---|---|
| [13,] | hsa-miR-19b_st | 0.48 | TCAGTTTTGCATGGATTTGCACA |
| [14,] | hsa-miR-20a_st | 0.41 | CTACCTGCACTATAAGCACTTTA |
| [15,] | hsa-miR-20b_st | 0.56 | CTACCTGCACTATGAGCACTTTG |
| [16,] | hsa-miR-25_st | 0.35 | TCAGACCGAGACAAGTGCAATG |
| [17,] | hsa-miR-30c_st | 0.31 | GCTGAGAGTGTAGGATGTTTACA |
| [18,] | hsa-miR-30e_st | 0.35 | CTTCCAGTCAAGGATGTTTACA |
| [19,] | hsa-miR-363-star_st | 0.31 | AAATTGCATCGTGATCCACCCG |
| [20,] | hsa-miR-363_st | 0.4 | TACAGATGGATACCGTGCAATT |
| [21,] | hsa-miR-766_st | 0.31 | GCTGAGGCTGTGGGGCTGGAGT |
| [22,] | hsa-miR-92a_st | 0.43 | ACAGGCCGGGACAAGTGCAATA |

Table 31 Depsipeptide (FR901228) microRNA biomarkers.

| | Medianprobe | Corr | Sequence |
|---|---|---|---|
| [1,] | hsa-miR-185_st | 0.39 | TCAGGAACTGCCTTTCTCTCCA |
| [2,] | hsa-miR-188-5p_st | 0.31 | CCCTCCACCATGCAAGGGATG |
| [3,] | hsa-miR-362-5p_st | 0.34 | ACTCACACCTAGGTTCCAAGGATT |
| [4,] | hsa-miR-500-star_st | 0.35 | CAGAATCCTTGCCCAGGTGCAT |
| [5,] | hsa-miR-500_st | 0.36 | TCTCACCCAGGTAGCAAGGATTA |
| [6,] | hsa-miR-501-3p_st | 0.31 | AGAATCCTTGCCCGGGTGCATT |
| [7,] | hsa-miR-501-5p_st | 0.33 | TCTCACCCAGGGACAAAGGATT |
| [8,] | hsa-miR-502-3p_st | 0.34 | TGAATCCTTGCCCAGGTGCATT |
| [9,] | hsa-miR-532-3p_st | 0.37 | TGCAAGCCTTGGGTGTGGGAGG |
| [10,] | hsa-miR-532-5p_st | 0.39 | ACGGTCCTACACTCAAGGCATG |
| [11,] | hsa-miR-652_st | 0.46 | CACAACCCTAGTGGCGCCATT |
| [12,] | hsa-miR-660_st | 0.32 | CAACTCCGATATGCAATGGGTA |
| [13,] | hsa-miR-93_st | 0.31 | CTACCTGCACGAACAGCACTTTG |

Table 32 Bortezomib microRNA biomarkers.

| | Medianprobe | Corr | Sequence |
|---|---|---|---|
| [1,] | hsa-miR-106b-star_st | 0.39 | GCAGCAAGTACCCACAGTGCGG |
| [2,] | hsa-miR-106b_st | 0.52 | ATCTGCACTGTCAGCACTTTA |
| [3,] | hsa-miR-1307_st | 0.33 | CACGACCGACGCCACGCCGAGT |
| [4,] | hsa-miR-188-5p_st | 0.37 | CCCTCCACCATGCAAGGGATG |
| [5,] | hsa-miR-25-star_st | 0.31 | CAATTGCCCAAGTCTCCGCCT |
| [6,] | hsa-miR-25_st | 0.33 | TCAGACCGAGACAAGTGCAATG |
| [7,] | hsa-miR-320c_st | 0.31 | ACCCTCTCAACCCAGCTTTT |
| [8,] | hsa-miR-324-3p_st | 0.32 | CCAGCAGCACCTGGGGCAGT |
| [9,] | hsa-miR-500_st | 0.33 | TCTCACCCAGGTAGCAAGGATTA |
| [10,] | hsa-miR-501-5p_st | 0.3 | TCTCACCCAGGGACAAAGGATT |
| [11,] | hsa-miR-638_st | 0.3 | AGGCCGCCACCCGCCCGCGATCCCT |
| [12,] | hsa-miR-652_st | 0.3 | CACAACCCTAGTGGCGCCATT |
| [13,] | hsa-miR-93-star_st | 0.34 | CGGGAAGTGCTAGCTCAGCAGT |
| [14,] | hsa-miR-93_st | 0.5 | CTACCTGCACGAACAGCACTTTG |

Table 33 Leukeran microRNA biomarkers.

| | Medianprobe | Corr | Sequence |
|---|---|---|---|
| [1,] | HBII-85-2_x_st | 0.3 | ACCTCAGTTCCGATGAGAATGACGG |
| [2,] | U104_st | 0.33 | GCGTCAGCAGTCTAACACGTGCTTT |
| [3,] | U41_st | 0.34 | AGGATCAGCCAGTACGAATACGCGA |
| [4,] | U52_st | 0.34 | GTTTTGACATCATGACCAGCATCGG |
| [5,] | U55_st | 0.36 | GCACTCGGGAGTATGCAGCATTACC |
| [6,] | U55_x_st | 0.36 | CGCGCACTCGGGAGTATGCAGCATT |
| [7,] | U57_st | 0.31 | TTTTGCCTCCATTCTACAAGGTCAG |
| [8,] | U74_x_st | 0.32 | ACCAACACAGGCTTCATCAGAGGCA |
| [9,] | hsa-miR-124_st | 0.35 | GGCATTCACCGCGTGCCTTA |
| [10,] | hsa-miR-1281_st | 0.37 | GGGAGAGGAGGAGGCGA |
| [11,] | hsa-miR-1299_st | 0.32 | TCCCTCACACAGAATTCCAGAA |
| [12,] | hsa-miR-140-3p_st | 0.3 | CCGTGGTTCTACCCTGTGGTA |
| [13,] | hsa-miR-142-5p_st | 0.33 | AGTAGTGCTTTCTACTTTATG |
| [14,] | hsa-miR-181a-star_st | 0.52 | GGTACAATCAACGGTCGATGGT |
| [15,] | hsa-miR-181a_st | 0.39 | ACTCACCGACAGCGTTGAATGTT |
| [16,] | hsa-miR-181b_st | 0.49 | ACCCACCGACAGCAATGAATGTT |
| [17,] | hsa-miR-181c_st | 0.41 | ACTCACCGACAGGTTGAATGTT |
| [18,] | hsa-miR-195-star_st | 0.34 | GGAGCAGCACAGCCAATATTGG |
| [19,] | hsa-miR-223_st | 0.34 | TGGGGTATTTGACAAACTGACA |
| [20,] | hsa-miR-297_st | 0.39 | CATGCACATGCACACATACAT |
| [21,] | hsa-miR-342-3p_st | 0.42 | ACGGGTGCGATTTCTGTGTGAGA |
| [22,] | hsa-miR-342-5p_st | 0.37 | TCAATCACAGATAGCACCCCT |
| [23,] | hsa-miR-34b_st | 0.36 | ATGGCAGTGGAGTTAGTGATTG |
| [24,] | hsa-miR-766_st | 0.43 | GCTGAGGCTGTGGGGCTGGAGT |
| [25,] | hsa-miR-768-5p_st | 0.35 | ATCACTCCGTACTTTCATCCTCCAA |
| [26,] | hsa-miR-874_st | 0.3 | TCGGTCCCTCGGGCCAGGGCAG |

Table 34 Fludarabine microRNA biomarkers.

| | Medianprobe | Corr | Sequence |
|---|---|---|---|
| [1,] | HBII-276_st | 0.3 | GGCAAAAAGTAATTTAAGTCATCAT |
| [2,] | HBII-438A_s_st | 0.44 | ATCCTCAGACAATTATTCTCATCAT |
| [3,] | HBII-52-32_x_st | 0.3 | AGGGCAATATCAGGTTCTCATCATT |
| [4,] | HBII-85-11_st | 0.39 | TGTTCAACTTTCCAAGGAACCCACG |
| [5,] | hsa-miR-130a_st | 0.32 | ATGCCCTTTTAACATTGCACTG |
| [6,] | hsa-miR-155_st | 0.32 | ACCCCTATCACGATTAGCATTAA |
| [7,] | hsa-miR-181a-star_st | 0.35 | GGTACAATCAACGGTCGATGGT |
| [8,] | hsa-miR-181a_st | 0.32 | ACTCACCGACAGCGTTGAATGTT |
| [9,] | hsa-miR-181b_st | 0.4 | ACCCACCGACAGCAATGAATGTT |
| [10,] | hsa-miR-20b-star_st | 0.33 | CTGGAAGTGCCCATACTACAGT |
| [11,] | hsa-miR-20b_st | 0.3 | CTACCTGCACTATGAGCACTTTG |
| [12,] | hsa-miR-34c-3p_st | 0.4 | CCTGGCCGTGTGGTTAGTGATT |
| [13,] | hsa-miR-363_st | 0.33 | TACAGATGGATACCGTGCAATT |
| [14,] | hsa-miR-424-star_st | 0.38 | ATAGCAGCGCCTCACGTTTTG |
| [15,] | hsa-miR-503_st | 0.31 | CTGCAGAACTGTTCCCGCTGCTA |
| [16,] | hsa-miR-554_st | 0.3 | ACTGGCTGAGTCAGGACTAGC |
| [17,] | hsa-miR-766_st | 0.31 | GCTGAGGCTGTGGGGCTGGAGT |

Table 35 Vinblastine microRNA biomarkers.

| | Medianprobe | Corr | Sequence |
|---|---|---|---|
| [1,] | hsa-miR-106b-star_st | 0.32 | GCAGCAAGTACCCACAGTGCGG |
| [2,] | hsa-miR-25-star_st | 0.31 | CAATTGCCCAAGTCTCCGCCT |
| [3,] | hsa-miR-362-5p_st | 0.33 | ACTCACACCTAGGTTCCAAGGATT |
| [4,] | hsa-miR-500-star_st | 0.34 | CAGAATCCTTGCCCAGGTGCAT |
| [5,] | hsa-miR-500_st | 0.36 | TCTCACCCAGGTAGCAAGGATTA |
| [6,] | hsa-miR-502-3p_st | 0.35 | TGAATCCTTGCCCAGGTGCATT |
| [7,] | hsa-miR-532-5p_st | 0.36 | ACGGTCCTACACTCAAGGCATG |
| [8,] | hsa-miR-652_st | 0.49 | CACAACCCTAGTGGCGCCATT |
| [9,] | hsa-miR-671-5p_st | 0.33 | CTCCAGCCCCTCCAGGGCTTCCT |

Table 36 Busulfan microRNA biomarkers.

| | Medianprobe | Corr | Sequence |
|---|---|---|---|
| [1,] | U55_st | 0.34 | CGCGCACTCGGGAGTATGCAGCATT |
| [2,] | U55_x_st | 0.31 | GCACTCGGGAGTATGCAGCATTACC |
| [3,] | hsa-miR-1207-5p_st | 0.3 | CCCCTCCCAGCCTCCCTGCCA |
| [4,] | hsa-miR-1246_ st | 0.34 | CCTGCTCCAAAAATCCATT |
| [5,] | hsa-miR-1281_st | 0.41 | GGGAGAGGAGGAGGCGA |
| [6,] | hsa-miR-181a-star_ st | 0.4 | GGTACAATCAACGGTCGATGGT |
| [7,] | hsa-miR-181b_st | 0.31 | ACCCACCGACAGCAATGAATGTT |
| [8,] | hsa-miR-181c_st | 0.37 | ACTCACCGACAGGTTGAATGTT |
| [9,] | hsa-miR-297_st | 0.35 | CATGCACATGCACACATACAT |
| [10,] | hsa-miR-766_ st | 0.31 | GCTGAGGCTGTGGGGCTGGAGT |
| [11,] | hsa-miR-923_st | 0.31 | AGTTTCTTTTCCTCCGCTGAC |

Table 37 Dacarbazine microRNA biomarkers.

| | Medianprobe | Corr | Sequence |
|---|---|---|---|
| [1,] | U13_st | 0.33 | AACAAGGTTCAAGGGTGGCACATCT |
| [2,] | U17a_st | 0.31 | AGGCCCAGCTTTATTTCCAACGTTG |
| [3,] | U25_st | 0.31 | TGTGAAAAGGTCCTCATCATAGGAA |
| [4,] | U29_st | 0.3 | GAGCTAGTTTGATTCATCATAGAAA |
| [5,] | U3-2_s_st | 0.34 | GCAGCCAAGCAACGCCAGAAAGCCG |
| [6,] | U41_st | 0.35 | AGGATCAGCCAGTACGAATACGCGA |
| [7,] | U52_st | 0.37 | ATCATGACCAGCATCGGAGACTCTA |
| [8,] | U55_st | 0.33 | CGGGAGTATGCAGCATTACCGAGTT |
| [9,] | hsa-miR-1207-5p_st | 0.31 | CCCCTCCCAGCCTCCCTGCCA |
| [10,] | hsa-miR-1299_st | 0.32 | TCCCTCACACAGAATTCCAGAA |
| [11,] | hsa-miR-140-3p_st | 0.33 | CCGTGGTTCTACCCTGTGGTA |
| [12,] | hsa-miR-140-5p_st | 0.31 | CTACCATAGGGTAAAACCACTG |
| [13,] | hsa-miR-17-star_st | 0.36 | CTACAAGTGCCTTCACTGCAGT |
| [14,] | hsa-miR-181d_st | 0.31 | ACCCACCGACAACAATGAATGTT |
| [15,] | hsa-miR-223_st | 0.44 | TGGGGTATTTGACAAACTGACA |
| [16,] | hsa-miR-297_st | 0.34 | CATGCACATGCACACATACAT |
| [17,] | hsa-miR-34b_st | 0.34 | ATGGCAGTGGAGTTAGTGATTG |
| [18,] | hsa-miR-766_st | 0.33 | GCTGAGGCTGTGGGGCTGGAGT |
| [19,] | hsa-miR-92a-1-star_st | 0.44 | AGCATTGCAACCGATCCCAACCT |
| [20,] | hsa-miR-92a_st | 0.33 | ACAGGCCGGGACAAGTGCAATA |

Table 38 Oxaliplatin microRNA biomarkers.

| | Medianprobe | Corr | Sequence |
|---|---|---|---|
| [1,] | ACA10_s_st | 0.36 | CACTCCTAGGAACAGAGAGCCCATT |
| [2,] | ACA15_s_st | 0.32 | GTCTGGCTGTGTAAACTACTGATAA |
| [3,] | ACA18_x_st | 0.34 | GCGTTTCCAACGATGTGCAGGCTAC |
| [4,] | ACA21_st | 0.33 | AAAAGCGATGTTTTCACTCTCCCCT |
| [5,] | ACA51_x_st | 0.32 | GAACACAGCCTGTGGTAAGCACCAG |
| [6,] | HBII-180A_x_st | 0.4 | GGCACCGTGTCCTCAGTGGCAGTCG |
| [7,] | HBII-202_st | 0.34 | TTCATCAAGGCCGTACAGCGATTCC |
| [8,] | HBII-429_st | 0.32 | ATCAGAAGGGTGACATGGCAGTTTC |
| [9,] | HBII-55_st | 0.35 | TGCACTCAGGGGATTGACAGATTTG |
| [10,] | HBII-99_st | 0.37 | CCAATGCATCAGACAAAACTGGCCA |
| [11,] | U104_st | 0.45 | CACGTGCTTTAATTGGAATGTCATC |
| [12,] | U17b_st | 0.35 | GCCCAGCTTCATCTTCAACGTTGTG |
| [13,] | U17b_x_st | 0.33 | CAGCTTCATCTTCAACGTTGTGGAA |
| [14,] | U25_st | 0.32 | AGTACAGGTCTGTGAAAAGGTCCTC |
| [15,] | U27_st | 0.38 | GATGACATCACTTGAAAGTTCAGCC |
| [16,] | U29_st | 0.4 | GTTTCTCAGGTGTTCATGTATTTTC |
| [17,] | U30_st | 0.36 | GCCGAACGAGATTCCATGTAAGTCA |
| [18,] | U31_st | 0.34 | AATACCTTTCAGTCACACATTGATC |
| [19,] | U31_x_st | 0.31 | GGCGGTATTCAACTCATCACTGGTG |
| [20,] | U33_st | 0.32 | TGGAGTCATCTCATGGTCGGGAAAC |
| [21,] | U36C_st | 0.3 | TCAGATGCAATGCTGACCACATGGT |
| [22,] | U50B_st | 0.37 | AGCCGAATCCGTACTTATTTTTCTT |
| [23,] | U55_st | 0.34 | CGGGAGTATGCAGCATTACCGAGTT |
| [24,] | U55_x_st | 0.32 | GCTCAGCTCTCCAAGGTTGGCTTCC |
| [25,] | U56_st | 0.39 | TCAGACCCAGAGTCTCAACACTCAC |
| [26,] | U56_x_st | 0.35 | CTCAACACTCACTAGGTGAACTGCT |
| [27,] | U57_st | 0.31 | AAGGTCAGGCTCAGACAGTTCATCA |
| [28,] | U71d_x_st | 0.31 | CGCGATTTCTTTCCCTGCACTATCA |
| [29,] | U73a_st | 0.33 | TGGCCATCATCTGGGACCGAAACTT |
| [30,] | U74_x_st | 0.42 | CGGTTGGCATTCATCATTACTCTCA |
| [31,] | U78_s_st | 0.36 | ATGCTCATTTCAGGTCAGACATTTG |
| [32,] | U78_x_st | 0.36 | CTTCAGTGTTACCTTTGTCTACATG |
| [33,] | U95_st | 0.34 | AGCCTCTGGATTTCAGCACCGACAC |
| [34,] | hsa-miR-106a_st | 0.38 | CTACCTGCACTGTAAGCACTTTT |
| [35,] | hsa-miR-106b_st | 0.36 | ATCTGCACTGTCAGCACTTTA |
| [36,] | hsa-miR-1246_st | 0.35 | CCTGCTCCAAAAATCCATT |
| [37,] | hsa-miR-1275_st | 0.31 | GACAGCCTCTCCCCCAC |
| [38,] | hsa-miR-1307_st | 0.38 | CACGACCGACGCCACGCCGAGT |
| [39,] | hsa-miR-142-5p_st | 0.32 | AGTAGTGCTTTCTACTTTATG |
| [40,] | hsa-miR-148a-star_st | 0.33 | AGTCGGAGTGTCTCAGAACTTT |
| [41,] | hsa-miR-153_st | 0.38 | GATCACTTTTGTGACTATGCAA |
| [42,] | hsa-miR-17-star_st | 0.35 | CTACAAGTGCCTTCACTGCAGT |
| [43,] | hsa-miR-17_st | 0.45 | CTACCTGCACTGTAAGCACTTTG |
| [44,] | hsa-miR-18a-star_st | 0.32 | CCAGAAGGAGCACTTAGGGCAGT |
| [45,] | hsa-miR-18a_st | 0.38 | CTATCTGCACTAGATGCACCTTA |
| [46,] | hsa-miR-18b_st | 0.34 | CTAACTGCACTAGATGCACCTTA |
| [47,] | hsa-miR-19a_st | 0.37 | TCAGTTTTGCATAGATTTGCACA |
| [48,] | hsa-miR-19b_st | 0.43 | TCAGTTTTGCATGGATTTGCACA |
| [49,] | hsa-miR-20a_st | 0.34 | CTACCTGCACTATAAGCACTTTA |

(continued)

| | Medianprobe | Corr | Sequence |
|---|---|---|---|
| [50,] | hsa-miR-297_st | 0.32 | CATGCACATGCACACATACAT |
| [51,] | hsa-miR-301a_st | 0.32 | GCTTTGACAATACTATTGCACTG |
| [52,] | hsa-miR-34b_st | 0.32 | ATGGCAGTGGAGTTAGTGATTG |

Table 39 Hydroxyurea microRNA biomarkers.

| | Medianprobe | Corr | Sequence |
|---|---|---|---|
| [1,] | ACA7_s_st | 0.35 | ATGGGTGCCTCTGCCAGGCTTGCTT |
| [2,] | ENSG00000199282_st | 0.31 | GCTCCACTATTTCAATACTGTTCTG |
| [3,] | ENSG00000201859_x_st | 0.4 | TCTTATCTGGAATGGCATCTAGCTT |
| [4,] | HBII-202_st | 0.39 | GCGATTCCGGAGAATGTCATCACGC |
| [5,] | HBII-336_st | 0.36 | GCCTCAGGTAAATCCTTTAATCCAT |
| [6,] | HBII-429_st | 0.31 | GTTTCCTCATGGCAGTTCAGTAGAG |
| [7,] | U104_st | 0.38 | TTTAATTGGAATGTCATCACAGCAG |
| [8,] | U25_st | 0.3 | AGTACAGGTCTGTGAAAAGGTCCTC |
| [9,] | U33_st | 0.33 | GCATGTGGAGTCATCTCATGGTCGG |
| [10,] | U34_st | 0.33 | AGGTAGTTGCGGAACATCATGGACG |
| [11,] | U38A_st | 0.3 | GGCTCTCATCTCTCTCCCTTCAGTA |
| [12,] | U41_st | 0.32 | AGGATCAGCCAGTACGAATACGCGA |
| [13,] | U49B_s_st | 0.3 | CGTCAGTTATCGCTTCTGACGGCAC |
| [14,] | U52_st | 0.4 | GACCAGCATCGGAGACTCTAGTCTG |
| [15,] | U55_st | 0.43 | GCACTCGGGAGTATGCAGCATTACC |
| [16,] | U55_x_st | 0.43 | GCTCAGCTCTCCAAGGTTGGCTTCC |
| [17,] | U74_x_st | 0.32 | GAGCGGTTGGCATTCATCATTACTC |
| [18,] | hsa-miR-1207-5p_st | 0.33 | CCCCTCCCAGCCTCCCTGCCA |
| [19,] | hsa-miR-124_st | 0.45 | GGCATTCACCGCGTGCCTTA |
| [20,] | hsa-miR-1281_st | 0.33 | GGGAGAGGAGGAGGCGA |
| [21,] | hsa-miR-1299_st | 0.4 | TCCCTCACACAGAATTCCAGAA |
| [22,] | hsa-miR-140-3p_st | 0.37 | CCGTGGTTCTACCCTGTGGTA |
| [23,] | hsa-miR-155_st | 0.35 | ACCCCTATCACGATTAGCATTAA |
| [24,] | hsa-miR-181a-star_st | 0.43 | GGTACAATCAACGGTCGATGGT |
| [25,] | hsa-miR-181b_st | 0.35 | ACCCACCGACAGCAATGAATGTT |
| [26,] | hsa-miR-181c_st | 0.3 | ACTCACCGACAGGTTGAATGTT |
| [27,] | hsa-miR-195-star_st | 0.41 | GGAGCAGCACAGCCAATATTGG |
| [28,] | hsa-miR-223_st | 0.49 | TGGGGTATTTGACAAACTGACA |
| [29,] | hsa-miR-297_st | 0.47 | CATGCACATGCACACATACAT |
| [30,] | hsa-miR-34b_st | 0.44 | ATGGCAGTGGAGTTAGTGATTG |
| [31,] | hsa-miR-491-3p_st | 0.37 | GTAGAAGGGAATCTTGCATAAG |
| [32,] | hsa-miR-595_st | 0.32 | AGACACACCACGGCACACTTC |
| [33,] | hsa-miR-631_st | 0.39 | GCTGAGGTCTGGGCCAGGTCT |
| [34,] | hsa-miR-766_st | 0.37 | GCTGAGGCTGTGGGGCTGGAGT |
| [35,] | hsa-miR-768-5p_st | 0.35 | ATCACTCCGTACTTTCATCCTCCAA |

Table 40 Tegafur microRNA biomarkers.

| | Medianprobe | Corr | Sequence |
|---|---|---|---|
| [1,] | HBII-180A_x_ st | 0.34 | TGTCCTCAGTGGCAGTCGGAGCCCA |
| [2,] | U104_st | 0.34 | AGTTCGCATCACCCGCGTCAGCAGT |
| [3,] | U33_st | 0.33 | TCGAATGTGAGTGGGAGAAGTTCTC |
| [4,] | U34_st | 0.32 | GCTGCTTTCATGAGGATCAAACAAT |

(continued)

| | Medianprobe | Corr | Sequence |
|---|---|---|---|
| [5,] | U43_x_st | 0.31 | AATCAGCACACAGTTTCTGTCCGCC |
| [6,] | U51_st | 0.31 | AAAGCAAATCCATCACGAACTCAGC |
| [7,] | U55_x_st | 0.34 | TGGCTTCCCCACCGCGCACTCGGGA |
| [8,] | U74_x_st | 0.44 | TTCATCATTACTCTCAGATGTCCCT |
| [9,] | U78_s_st | 0.33 | CATGCTCATTTCAGGTCAGACATTT |
| [10,] | U78_x_st | 0.32 | ACCTTTGTCTACATGCTCATTTCAG |
| [11,] | hsa-miR-1228-star_st | 0.33 | CACACACCTGCCCCCGCCCAC |
| [12,] | hsa-miR-1246_st | 0.38 | CCTGCTCCAAAAATCCATT |
| [13,] | hsa-miR-124_st | 0.35 | GGCATTCACCGCGTGCCTTA |
| [14,] | hsa-miR-1299_st | 0.32 | TCCCTCACACAGAATTCCAGAA |
| [15,] | hsa-miR-1307_st | 0.4 | CACGACCGACGCCACGCCGAGT |
| [16,] | hsa-miR-149-star_st | 0.34 | GCACAGCCCCCGTCCCTCCCT |
| [17,] | hsa-miR-16_st | 0.32 | CGCCAATATTTACGTGCTGCTA |
| [18,] | hsa-miR-18a_st | 0.32 | CTATCTGCACTAGATGCACCTTA |
| [19,] | hsa-miR-195-star_st | 0.4 | GGAGCAGCACAGCCAATATTGG |
| [20,] | hsa-miR-297_st | 0.37 | CATGCACATGCACACATACAT |
| [21,] | hsa-miR-330-3p_st | 0.4 | TCTCTGCAGGCCGTGTGCTTTGC |
| [22,] | hsa-miR-346_st | 0.33 | AGAGGCAGGCATGCGGGCAGACA |
| [23,] | hsa-miR-34b_st | 0.42 | ATGGCAGTGGAGTTAGTGATTG |
| [24,] | hsa-miR-638_st | 0.33 | AGGCCGCCACCCGCCCGCGATCCCT |
| [25,] | hsa-miR-923_st | 0.35 | AGTTTCTTTTCCTCCGCTGAC |
| [26,] | hsa-miR-92a_st | 0.33 | ACAGGCCGGGACAAGTGCAATA |

Table 41 Daunorubicin microRNA biomarkers.

| | Medianprobe | Corr | Sequence |
|---|---|---|---|
| [1,] | ACA48_x_st | 0.3 | GGGCACGGATCAAAAGCCAAGCTGG |
| [2,] | HBII-85-26_st | 0.31 | ATCCATTTTTTTTATAGTCATCATC |
| [3,] | HBII-85-6_x_st | 0.3 | TTTTTTTTGGAGGACTCATCATCGA |
| [4,] | U104_st | 0.37 | TTTAATTGGAATGTCATCACAGCAG |
| [5,] | U55_st | 0.41 | GTATGCAGCATTACCGAGTTGTCAT |
| [6,] | U55_x_st | 0.42 | CGCGCACTCGGGAGTATGCAGCATT |
| [7,] | U74_x_st | 0.32 | AGAGCGGTTGGCATTCATCATTACT |
| [8,] | hsa-miR-106a-star_st | 0.32 | GTAAGAAGTGCTTACATTGCAG |
| [9,] | hsa-miR-106b-star_st | 0.4 | GCAGCAAGTACCCACAGTGCGG |
| [10,] | hsa-miR-106b_st | 0.34 | ATCTGCACTGTCAGCACTTTA |
| [11,] | hsa-miR-124_st | 0.38 | GGCATTCACCGCGTGCCTTA |
| [12,] | hsa-miR-1281_st | 0.34 | GGGAGAGGAGGAGGCGA |
| [13,] | hsa-miR-1299_st | 0.35 | TCCCTCACACAGAATTCCAGAA |
| [14,] | hsa-miR-181a-star_st | 0.3 | GGTACAATCAACGGTCGATGGT |
| [15,] | hsa-miR-195-star_st | 0.3 | GGAGCAGCACAGCCAATATTGG |
| [16,] | hsa-miR-297_st | 0.33 | CATGCACATGCACACATACAT |
| [17,] | hsa-miR-29b-2-star_st | 0.32 | CTAAGCCACCATGTGAAACCAG |
| [18,] | hsa-miR-33b-star_st | 0.39 | GGGCTGCACTGCCGAGGCACTG |
| [19,] | hsa-miR-342-5p_st | 0.31 | TCAATCACAGATAGCACCCCT |
| [20,] | hsa-miR-34b_st | 0.35 | ATGGCAGTGGAGTTAGTGATTG |
| [21,] | hsa-miR-629-star_st | 0.36 | GCTGGGCTTACGTTGGGAGAAC |
| [22,] | hsa-miR-652_st | 0.34 | CACAACCCTAGTGGCGCCATT |
| [23,] | hsa-miR-671-5p_st | 0.36 | CTCCAGCCCCTCCAGGGCTTCCT |
| [24,] | hsa-miR-766_st | 0.38 | GCTGAGGCTGTGGGGCTGGAGT |

(continued)

| | Medianprobe | Corr | Sequence |
|---|---|---|---|
| [25,] | hsa-miR-768-3p_st | 0.36 | GTCAGCAGTTTGAGTGTCAGCATTG |
| [26,] | hsa-miR-768-5p_st | 0.33 | ATCACTCCGTACTTTCATCCTCCAA |
| [27,] | hsa-miR-877-star_st | 0.34 | CTGGGAGGAGGGAGAAGAGGA |
| [28,] | hsa-miR-93-star_st | 0.38 | CGGGAAGTGCTAGCTCAGCAGT |
| [29,] | hsa-miR-93_st | 0.31 | CTACCTGCACGAACAGCACTTTG |

### Table 42 Bleomycin microRNA biomarkers.

| | Medianprobe | Corr | Sequence |
|---|---|---|---|
| [1,] | 14qII-14_st | 0.41 | ACCCAACACTCATACGCCGGCAGTT |
| [2,] | 14qII-14_x_st | 0.4 | ATACGCCGGCAGTTGTCATCATTGG |
| [3,] | 14qII-1_st | 0.38 | TCAGACTTCCAGACCTGTATTCACC |
| [4,] | 14qII-1_x_st | 0.32 | TGGACCTCAGACTTCCAGACCTGTA |
| [5,] | 14qII-26_st | 0.43 | TGTGACTCATACTCCACCAGTGCTC |
| [6,] | 14qII-26_x_st | 0.4 | ATCGTATGTGACTCATACTCCACCA |
| [7,] | 14qII-3_st | 0.33 | CAGACACGTAGTATTCATCGTCCAT |
| [8,] | hsa-miR-125b-1-star_st | 0.32 | AGCTCCCAAGAGCCTAACCCGT |
| [9,] | hsa-miR-127-3p_st | 0.41 | AGCCAAGCTCAGACGGATCCGA |
| [10,] | hsa-miR-1271_st | 0.32 | TGAGTGCTTGCTAGGTGCCAAG |
| [11,] | hsa-miR-134_st | 0.34 | CCCCTCTGGTCAACCAGTCACA |
| [12,] | hsa-miR-155_st | 0.37 | ACCCCTATCACGATTAGCATTAA |
| [13,] | hsa-miR-193a-5p_st | 0.32 | TCATCTCGCCCGCAAAGACCCA |
| [14,] | hsa-miR-21-star_st | 0.39 | ACAGCCCATCGACTGGTGTTG |
| [15,] | hsa-miR-22_st | 0.31 | ACAGTTCTTCAACTGGCAGCTT |
| [16,] | hsa-miR-299-3p_st | 0.34 | AAGCGGTTTACCATCCCACATA |
| [17,] | hsa-miR-337-5p_st | 0.3 | AACTCCTGTATGAAGCCGTTC |
| [18,] | hsa-miR-370_st | 0.35 | ACCAGGTTCCACCCCAGCAGGC |
| [19,] | hsa-miR-376c_st | 0.3 | ACGTGGAATTTCCTCTATGTT |
| [20,] | hsa-miR-377-star_st | 0.37 | GAATTCACCAAGGGCAACCTCT |
| [21,] | hsa-miR-379_st | 0.36 | CCTACGTTCCATAGTCTACCA |
| [22,] | hsa-miR-381_st | 0.4 | ACAGAGAGCTTGCCCTTGTATA |
| [23,] | hsa-miR-382_st | 0.32 | CGAATCCACCACGAACAACTTC |
| [24,] | hsa-miR-409-3p_st | 0.36 | AGGGGTTCACCGAGCAACATTC |
| [25,] | hsa-miR-409-5p_st | 0.35 | ATGCAAAGTTGCTCGGGTAACCT |
| [26,] | hsa-miR-411_st | 0.39 | CGTACGCTATACGGTCTACTA |
| [27,] | hsa-miR-431_st | 0.32 | TGCATGACGGCCTGCAAGACA |
| [28,] | hsa-miR-455-3p_st | 0.41 | GTGTATATGCCCATGGACTGC |
| [29,] | hsa-miR-485-5p_st | 0.34 | GAATTCATCACGGCCAGCCTCT |
| [30,] | hsa-miR-487b_st | 0.37 | AAGTGGATGACCCTGTACGATT |
| [31,] | hsa-miR-493_st | 0.34 | CCTGGCACACAGTAGACCTTCA |
| [32,] | hsa-miR-494_st | 0.39 | GAGGTTTCCCGTGTATGTTTCA |
| [33,] | hsa-miR-543_st | 0.33 | AAGAAGTGCACCGCGAATGTTT |
| [34,] | hsa-miR-663_st | 0.3 | GCGGTCCCGCGGCGCCCCGCCT |
| [35,] | hsa-miR-671-5p_st | 0.32 | CTCCAGCCCCTCCAGGGCTTCCT |
| [36,] | hsa-miR-758_st | 0.33 | GGTTAGTGGACCAGGTCACAAA |

### Table 43 Estramustine microRNA biomarkers.

| | Medianprobe | Corr | Sequence |
|---|---|---|---|
| [1,] | hsa-miR-1226_st | 0.25 | CTAGGGAACACAGGGCTGGTGA |

(continued)

| | Medianprobe | Corr | Sequence |
|---|---|---|---|
| [2,] | hsa-miR-193a-3p _st | 0.26 | ACTGGGACTTTGTAGGCCAGTT |
| [3,] | hsa-miR-193a-5p _st | 0.26 | TCATCTCGCCCGCAAAGACCCA |
| [4,] | hsa-miR-330-3p_st | 0.25 | TCTCTGCAGGCCGTGTGCTTTGC |
| [5,] | hsa-miR-330-5p_st | 0.25 | GCCTAAGACACAGGCCCAGAGA |
| [6,] | hsa-miR-378 _st | 0.34 | CCTTCTGACTCCAAGTCCAGT |
| [7,] | hsa-miR-586_ st | 0.28 | GGACCTAAAAATACAATGCATA |

Table 44 Mechlorethamine microRNA biomarkers.

| | Medianprobe | Corr | Sequence |
|---|---|---|---|
| [1,] | ACA13_st | 0.3 | GCCTTTTTTGACACCACATTCACTA |
| [2,] | ACA21_st | 0.33 | AAAAGCGATGTTTTCACTCTCCCCT |
| [3,] | ACA48_x_st | 0.32 | ATGGGCATCACCAACCAGATGCCAC |
| [4,] | ACA7_s_st | 0.33 | GAATGGGTGCCTCTGCCAGGCTTGC |
| [5,] | ACA9_st | 0.31 | CTGTTCTAGCAAGCACTGAAGGAAT |
| [6,] | ENSG00000202252_st | 0.31 | AAGGAAGGTGTTGCCATCATTAGCC |
| [7,] | HBII-202_st | 0.35 | ACAGCGATTCCGGAGAATGTCATCA |
| [8,] | HBTT-239_st | 0.41 | TGTCAGCAGTTTGAGTGTCAGCATT |
| [9,] | HBII-336_st | 0.35 | GCCTCAGGTAAATCCTTTAATCCAT |
| [10,] | HBII-429_st | 0.37 | GAGGGAGCCAGTTGTCATCATGTAC |
| [11,] | U104_st | 0.49 | TTTAATTGGAATGTCATCACAGCAG |
| [12,] | U33_st | 0.35 | TGAGTGGGAGAAGTTCTCATCACCG |
| [13,] | U34_st | 0.32 | AGTGCTGCTTTCATGAGGATCAAAC |
| [14,] | U52_st | 0.32 | GTTTTGACATCATGACCAGCATCGG |
| [15,] | U55_st | 0.34 | CGCGCACTCGGGAGTATGCAGCATT |
| [16,] | U55_x_st | 0.36 | GCTCAGCTCTCCAAGGTTGGCTTCC |
| [17,] | U74_x_st | 0.44 | CAGAGCGGTTGGCATTCATCATTAC |
| [18,] | U75_st | 0.37 | TTCTGTCCACTACTCTTAAAGCATC |
| [19,] | U78_s_st | 0.32 | ATGCTCATTTCAGGTCAGACATTTG |
| [20,] | U78_x_st | 0.33 | TTTGTCTACATGCTCATTTCAGGTC |
| [21,] | U95_st | 0.32 | CGACACTCAGATGGCATGTTGGGGT |
| [22,] | hsa-miR-124_st | 0.43 | GGCATTCACCGCGTGCCTTA |
| [23,] | hsa-miR-1281_st | 0.34 | GGGAGAGGAGGAGGCGA |
| [24,] | hsa-miR-1299 st | 0.37 | TCCCTCACACAGAATTCCAGAA |
| [25,] | hsa-miR-142-5p_st | 0.33 | AGTAGTGCTTTCTACTTTATG |
| [26,] | hsa-miR-181a-star_st | 0.37 | GGTACAATCAACGGTCGATGGT |
| [27,] | hsa-miR-181a_st | 0.31 | ACTCACCGACAGCGTTGAATGTT |
| [28,] | hsa-miR-181b_st | 0.32 | ACCCACCGACAGCAATGAATGTT |
| [29,] | hsa-miR-195-star_st | 0.35 | GGAGCAGCACAGCCAATATTGG |
| [30,] | hsa-miR-297_st | 0.44 | CATGCACATGCACACATACAT |
| [31,] | hsa-miR-29b-2-star_st | 0.35 | CTAAGCCACCATGTGAAACCAG |
| [32,] | hsa-miR-331-5p_st | 0.32 | GGATCCCTGGGACCATACCTAG |
| [33,] | hsa-miR-34b_st | 0.42 | ATGGCAGTGGAGTTAGTGATTG |
| [34,] | hsa-miR-425_st | 0.31 | TCAACGGGAGTGATCGTGTCATT |
| [35,] | hsa-miR-766_st | 0.36 | GCTGAGGCTGTGGGGCTGGAGT |
| [36,] | hsa-miR-768-3p_st | 0.46 | GTCAGCAGTTTGAGTGTCAGCATTG |
| [37,] | hsa-miR-768-5p_st | 0.46 | ATCACTCCGTACTTTCATCCTCCAA |

Table 45 Streptozocin microRNA biomarkers.

| | Medianprobe | Corr | Sequence |
|---|---|---|---|
| [1,] | hsa-miR-296-3p_st | 0.3 | GGAGAGCCTCCACCCAACCCTC |
| [2,] | hsa-miR-923_st | 0.27 | AGTTTCTTTTCCTCCGCTGAC |

Table 46 Carmustine microRNA biomarkers.

| | Medianprobe | Corr | Sequence |
|---|---|---|---|
| [1,] | ACA10_s_st | 0.34 | TGAAAACACGCCAAGCACACACTGG |
| [2,] | ACA44_st | 0.43 | CAGGTTGCTCTTGCATGCAGTTGGA |
| [3,] | ACA52_st | 0.32 | GGAGCCCTGCTCACTCCTCGGGTGA |
| [4,] | ACA61_st | 0.37 | ACCAAGACCAGTGTTCAGATCCGAT |
| [5,] | HBII-142_st | 0.3 | CATGGTGTCTCAGTGGCCCAGACAC |
| [6,] | HBII-180A_x_st | 0.3 | AGTGCTGGACATCATGGAGGCCCCG |
| [7,] | HBII-382_s_st | 0.33 | AATCGCCTCGATAATCAGTGGCCGG |
| [8,] | HBII-429_st | 0.34 | GGAGCCAGTTGTCATCATGTACAGC |
| [9,] | U13_st | 0.31 | GGTCAGACGGGTAATGTGCCCACGT |
| [10,] | U17a_st | 0.49 | ACCTCCCGGGTGTATCCACGTTGGA |
| [11,] | U17a_x_st | 0.33 | AGGCGCAGACACGAGGCCCAGCTTT |
| [12,] | U17b_st | 0.4 | GAGGCCCAGCTTCATCTTCAACGTT |
| [13,] | U17b_x_st | 0.41 | AGCTTCATCTTCAACGTTGTGGAAA |
| [14,] | U38A_st | 0.36 | TCCTCAGCCTAAAAGGCTCTCATCT |
| [15,] | U41_st | 0.35 | GGATCAGCCAGTACGAATACGCGAT |
| [16,] | U48_st | 0.31 | GGTCAGAGCGCTGCGGTGATGGCAT |
| [17,] | U52_st | 0.36 | CATGACCAGCATCGGAGACTCTAGT |
| [18,] | U55_st | 0.42 | GAGTATGCAGCATTACCGAGTTGTC |
| [19,] | U55_x_st | 0.36 | GTATGCAGCATTACCGAGTTGTCAT |
| [20,] | U67_st | 0.38 | ATCCCAGTTCCCCAAAGGCCTTAGG |
| [21,] | U67_x_st | 0.31 | TTCCTGAGAGGCACTGATGTCCCCT |
| [22,] | U70_x_st | 0.36 | ACCCATACAACCAACAGGCTGCGTA |
| [23,] | U74_x_st | 0.33 | TTACTCTCAGATGTCCCTACCAACA |
| [24,] | U83B_st | 0.32 | ACATTCCAGGCCTCATCACTGAACA |
| [25,] | hsa-miR-106a_st | 0.32 | CTACCTGCACTGTAAGCACTTTT |
| [26,] | hsa-miR-1274a_st | 0.35 | TGGCGCCTGAACAGGGAC |
| [27,] | hsa-miR-1275_st | 0.41 | GACAGCCTCTCCCCCAC |
| [28,] | hsa-miR-1280_st | 0.31 | GGGTGGCAGCGGTGGGA |
| [29,] | hsa-miR-130b_st | 0.45 | ATGCCCTTTCATCATTGCACTG |
| [30,] | hsa-miR-146b-3p_st | 0.63 | CCAGAACTGAGTCCACAGGGCA |
| [31,] | hsa-miR-146b-5p_st | 0.54 | AGCCTATGGAATTCAGTTCTCA |
| [32,] | hsa-miR-17-star_st | 0.43 | CTACAAGTGCCTTCACTGCAGT |
| [33,] | hsa-miR-185-star_st | 0.31 | GACCAGAGGAAAGCCAGCCCCT |
| [34,] | hsa-miR-18a-star_st | 0.61 | CCAGAAGGAGCACTTAGGGCAGT |
| [35,] | hsa-miR-18a_st | 0.44 | CTATCTGCACTAGATGCACCTTA |
| [36,] | hsa-miR-18b_st | 0.45 | CTAACTGCACTAGATGCACCTTA |
| [37,] | hsa-miR-19a_st | 0.4 | TCAGTTTTGCATAGATTTGCACA |
| [38,] | hsa-miR-19b_st | 0.33 | TCAGTTTTGCATGGATTTGCACA |
| [39,] | hsa-miR-202_st | 0.35 | TTCCCATGCCCTATACCTCT |
| [40,] | hsa-miR-20a_st | 0.33 | CTACCTGCACTATAAGCACTTTA |
| [41,] | hsa-miR-20b_st | 0.3 | CTACCTGCACTATGAGCACTTTG |
| [42,] | hsa-miR-223_st | 0.51 | TGGGGTATTTGACAAACTGACA |
| [43,] | hsa-miR-25-star_st | 0.54 | CAATTGCCCAAGTCTCCGCCT |
| [44,] | hsa-miR-373_st | 0.31 | ACACCCCAAAATCGAAGCACTTC |

(continued)

| | Medianprobe | Corr | Sequence |
|---|---|---|---|
| [45,] | hsa-miR-378-star_st | 0.34 | ACACAGGACCTGGAGTCAGGAG |
| [46,] | hsa-miR-422a_st | 0.32 | GCCTTCTGACCCTAAGTCCAGT |
| [47,] | hsa-miR-423-5p_st | 0.32 | AAAGTCTCGCTCTCTGCCCCTCA |
| [48,] | hsa-miR-451_st | 0.44 | AACTCAGTAATGGTAACGGTTT |
| [49,] | hsa-miR-486-3p_st | 0.55 | ATCCTGTACTGAGCTGCCCCG |
| [50,] | hsa-miR-486-5p_st | 0.39 | CTCGGGGCAGCTCAGTACAGGA |
| [51,] | hsa-miR-504_st | 0.31 | GATAGAGTGCAGACCAGGGTCT |
| [52,] | hsa-miR-550_st | 0.43 | GGGCTCTTACTCCCTCAGGCACT |
| [53,] | hsa-miR-616_st | 0.38 | CTGCTCAAACCCTCCAATGACT |
| [54,] | hsa-miR-671-3p_st | 0.34 | GGTGGAGCCCTGAGAACCGGA |
| [55,] | hsa-miR-671-5p_st | 0.37 | CTCCAGCCCCTCCAGGGCTTCCT |
| [56,] | hsa-miR-766_st | 0.38 | GCTGAGGCTGTGGGGCTGGAGT |
| [57,] | hsa-miR-92a-1-star_st | 0.51 | AGCATTGCAACCGATCCCAACCT |
| [58,] | hsa-miR-92a_st | 0.39 | ACAGGCCGGGACAAGTGCAATA |
| [59,] | hsa-miR-93-star_st | 0.32 | CGGGAAGTGCTAGCTCAGCAGT |

Table 47 Lomustine microRNA biomarkers.

| | Medianprobe | Corr | Sequence |
|---|---|---|---|
| [1,] | U41_st | 0.31 | GGATCAGCCAGTACGAATACGCGAT |
| [2,] | hsa-miR-106b-star_st | 0.33 | GCAGCAAGTACCCACAGTGCGG |
| [3,] | hsa-miR-1183_st | 0.34 | TGCCCACTCTCACCATCACCTACAG |
| [4,] | hsa-miR-1207-5p_st | 0.31 | CCCCTCCCAGCCTCCCTGCCA |
| [5,] | hsa-miR-1281_st | 0.31 | GGGAGAGGAGGAGGCGA |
| [6,] | hsa-miR-18b_st | 0.3 | CTAACTGCACTAGATGCACCTTA |
| [7,] | hsa-miR-195-star_st | 0.33 | GGAGCAGCACAGCCAATATTGG |
| [8,] | hsa-miR-615-3p_st | 0.32 | AAGAGGGAGACCCAGGCTCGGA |
| [9,] | hsa-miR-631_st | 0.3 | GCTGAGGTCTGGGCCAGGTCT |
| [10,] | hsa-miR-766_st | 0.46 | GCTGAGGCTGTGGGGCTGGAGT |
| [11,] | hsa-miR-92a-2-star_st | 0.41 | GTAATGCAACAAATCCCCACCC |

Table 48 Mercaptopurine microRNA biomarkers.

| | Medianprobe | Corr | Sequence |
|---|---|---|---|
| [1,] | ACA43_st | 0.31 | AGGCCATAAACCATTCTCAGTGCCC |
| [2,] | ACA57_st | 0.32 | AAGAGCCAGCCCTATTCTTAGGACG |
| [3,] | ENSG00000199411s_st | 0.34 | TTCATCTCCCTGCTATTGAGTCCAC |
| [4,] | ENSG00000200879_st | 0.31 | TGTTTTGGACCATTCATCATTGTGA |
| [5,] | ENSG00000202252_st | 0.43 | GCCAAGCTTTTGGTGGAAACTACGA |
| [6,] | HBII-142_st | 0.38 | GTGTCTCAGTGGCCCAGACACGTGG |
| [7,] | HBII-142_x_st | 0.35 | TCAGATCCTCAGTTCCATCATGGTG |
| [8,] | HBII-202_st | 0.43 | TGTGCTTTCATCAAGGCCGTACAGC |
| [9,] | HBII-429_st | 0.34 | CTCATGGCAGTTCAGTAGAGGGAGC |
| [10,] | U13_st | 0.35 | GTGCCCACGTCGTAACAAGGTTCAA |
| [11,] | U25_st | 0.34 | TCCTCAGAGTTATTTATCCTCACGG |
| [12,] | U27_st | 0.34 | GATGACATCACTTGAAAGTTCAGCC |
| [13,] | U29_st | 0.34 | ATGTATTTTCACTGTCGGTCATAGT |
| [14,] | U30_st | 0.36 | GATTTCAGAGTCTCAACAGCAAGTC |
| [15,] | U31_x_st | 0.35 | CAGCTCAGAAAATACCTTTCAGTCA |
| [16,] | U36C_st | 0.33 | TCAGATGCAATGCTGACCACATGGT |

(continued)

| | Medianprobe | Corr | Sequence |
|---|---|---|---|
| [17,] | U38A_st | 0.37 | CTCTCTCCCTTCAGTAGCAGAACTG |
| [18,] | U52_st | 0.4 | GACCAGCATCGGAGACTCTAGTCTG |
| [19,] | U55_st | 0.45 | CCGCGCACTCGGGAGTATGCAGCAT |
| [20,] | U55_x_st | 0.39 | GAGTATGCAGCATTACCGAGTTGTC |
| [21,] | U59B_st | 0.35 | GTCAGAACGTACTCATCAGTGAGGA |
| [22,] | U74_x_st | 0.42 | CAGAGCGGTTGGCATTCATCATTAC |
| [23,] | U83B_st | 0.37 | ACATTCCAGGCCTCATCACTGAACA |
| [24,] | U83_st | 0.35 | AGAGTCGTCCTTGCACTGAGGTGCT |
| [25,] | hsa-miR-106a_st | 0.44 | CTACCTGCACTGTAAGCACTTTT |
| [26,] | hsa-miR-1275_st | 0.3 | GACAGCCTCTCCCCCAC |
| [27,] | hsa-miR-17-star_st | 0.42 | CTACAAGTGCCTTCACTGCAGT |
| [28,] | hsa-miR-17_st | 0.45 | CTACCTGCACTGTAAGCACTTTG |
| [29,] | hsa-miR-18a-star_st | 0.4 | CCAGAAGGAGCACTTAGGGCAGT |
| [30,] | hsa-miR-18a_st | 0.42 | CTATCTGCACTAGATGCACCTTA |
| [31,] | hsa-miR-18b_st | 0.39 | CTAACTGCACTAGATGCACCTTA |
| [32,] | hsa-miR-19a_st | 0.35 | TCAGTTTTGCATAGATTTGCACA |
| [33,] | hsa-miR-19b_st | 0.4 | TCAGTTTTGCATGGATTTGCACA |
| [34,] | hsa-miR-20a_st | 0.41 | CTACCTGCACTATAAGCACTTTA |
| [35,] | hsa-miR-20b_st | 0.36 | CTACCTGCACTATGAGCACTTTG |
| [36,] | hsa-miR-25-star_st | 0.37 | CAATTGCCCAAGTCTCCGCCT |
| [37,] | hsa-miR-378-star_st | 0.32 | ACACAGGACCTGGAGTCAGGAG |
| [38,] | hsa-miR-378_st | 0.36 | CCTTCTGACTCCAAGTCCAGT |
| [39,] | hsa-miR-422a_st | 0.33 | GCCTTCTGACCCTAAGTCCAGT |
| [40,] | hsa-miR-423-5p_st | 0.34 | AAAGTCTCGCTCTCTGCCCCTCA |
| [41,] | hsa-miR-663bst | 0.33 | CCTCAGGCACGGCCGGGCCACC |
| [42,] | hsa-miR-766_st | 0.32 | GCTGAGGCTGTGGGGCTGGAGT |
| [43,] | hsa-miR-92a-1-star_st | 0.47 | AGCATTGCAACCGATCCCAACCT |
| [44,] | hsa-miR-92a_st | 0.44 | ACAGGCCGGGACAAGTGCAATA |

Table 49 Teniposide microRNA biomarkers.

| | Medianprobe | Corr | Sequence |
|---|---|---|---|
| [1,] | ACA48_x_st | 0.33 | GAGTTGCTTTGTCCCTGGCTGATAT |
| [2,] | ACA7_s_st | 0.31 | AATGGGTGCCTCTGCCAGGCTTGCT |
| [3,] | HBII-239_st | 0.31 | CCGTACTTTCATCCTCCAACACACA |
| [4,] | HBII-429_st | 0.31 | ATCAGAAGGGTGACATGGCAGTTTC |
| [5,] | HBII-85-26_st | 0.32 | AGATCCATTTTTTTTATAGTCATCA |
| [6,] | U104_st | 0.39 | TTTAATTGGAATGTCATCACAGCAG |
| [7,] | U17b_st | 0.31 | GAGGCCCAGCTTCATCTTCAACGTT |
| [8,] | U17b_x_st | 0.31 | CAGCTTCATCTTCAACGTTGTGGAA |
| [9,] | U55_st | 0.34 | CGCGCACTCGGGAGTATGCAGCATT |
| [10,] | U55_x_st | 0.37 | GTATGCAGCATTACCGAGTTGTCAT |
| [11,] | hsa-miR-106b-star_st | 0.31 | GCAGCAAGTACCCACAGTGCGG |
| [12,] | hsa-miR-124_st | 0.36 | GGCATTCACCGCGTGCCTTA |
| [13,] | hsa-miR-1281_st | 0.33 | GGGAGAGGAGGAGGCGA |
| [14,] | hsa-miR-1299_st | 0.41 | TCCCTCACACAGAATTCCAGAA |
| [15,] | hsa-miR-140-3p_st | 0.34 | CCGTGGTTCTACCCTGTGGTA |
| [16,] | hsa-miR-142-5p_st | 0.31 | AGTAGTGCTTTCTACTTTATG |
| [17,] | hsa-miR-181a-star_st | 0.43 | GGTACAATCAACGGTCGATGGT |
| [18,] | hsa-miR-181a_st | 0.33 | ACTCACCGACAGCGTTGAATGTT |

(continued)

| | Medianprobe | Corr | Sequence |
|---|---|---|---|
| [19,] | hsa-miR-181b_st | 0.35 | ACCCACCGACAGCAATGAATGTT |
| [20,] | hsa-miR-181c_st | 0.35 | ACTCACCGACAGGTTGAATGTT |
| [21,] | hsa-miR-195-star_st | 0.4 | GGAGCAGCACAGCCAATATTGG |
| [22,] | hsa-miR-297_st | 0.4 | CATGCACATGCACACATACAT |
| [23,] | hsa-miR-29b-2-star_st | 0.37 | CTAAGCCACCATGTGAAACCAG |
| [24,] | hsa-miR-33b-star_st | 0.31 | GGGCTGCACTGCCGAGGCACTG |
| [25,] | hsa-miR-34b_st | 0.41 | ATGGCAGTGGAGTTAGTGATTG |
| [26,] | hsa-miR-629-star_st | 0.3 | GCTGGGCTTACGTTGGGAGAAC |
| [27,] | hsa-miR-766_st | 0.32 | GCTGAGGCTGTGGGGCTGGAGT |
| [28,] | hsa-miR-768-3p_st | 0.43 | GTCAGCAGTTTGAGTGTCAGCATTG |
| [29,] | hsa-miR-768-5p_st | 0.38 | ATCACTCCGTACTTTCATCCTCCAA |
| [30,] | hsa-miR-92b_st | 0.31 | GGAGGCCGGGACGAGTGCAATA |

Table 50 Dactinomycin microRNA biomarkers.

| | Medianprobe | Corr | Sequence |
|---|---|---|---|
| [1,] | hsa-miR-106b-star_st | 0.36 | GCAGCAAGTACCCACAGTGCGG |
| [2,] | hsa-miR-106b_st | 0.43 | ATCTGCACTGTCAGCACTTTA |
| [3,] | hsa-miR-1275_st | 0.31 | GACAGCCTCTCCCCCAC |
| [4,] | hsa-miR-25-star_st | 0.34 | CAATTGCCCAAGTCTCCGCCT |
| [5,] | hsa-miR-25_st | 0.39 | TCAGACCGAGACAAGTGCAATG |
| [6,] | hsa-miR-33b-star_st | 0.36 | GGGCTGCACTGCCGAGGCACTG |
| [7,] | hsa-miR-362-5p_st | 0.33 | ACTCACACCTAGGTTCCAAGGATT |
| [8,] | hsa-miR-500-star_st | 0.31 | CAGAATCCTTGCCCAGGTGCAT |
| [9,] | hsa-miR-500_st | 0.36 | TCTCACCCAGGTAGCAAGGATTA |
| [10,] | hsa-miR-501-5p_st | 0.35 | TCTCACCCAGGGACAAAGGATT |
| [11,] | hsa-miR-502-3p_st | 0.31 | TGAATCCTTGCCCAGGTGCATT |
| [12,] | hsa-miR-532-5p_st | 0.36 | ACGGTCCTACACTCAAGGCATG |
| [13,] | hsa-miR-629-star_st | 0.41 | GCTGGGCTTACGTTGGGAGAAC |
| [14,] | hsa-miR-629_st | 0.35 | AGTTCTCCCAACGTAAACCCA |
| [15,] | hsa-miR-652_st | 0.51 | CACAACCCTAGTGGCGCCATT |
| [16,] | hsa-miR-671-5p_st | 0.34 | CTCCAGCCCCTCCAGGGCTTCCT |
| [17,] | hsa-miR-766_st | 0.35 | GCTGAGGCTGTGGGGCTGGAGT |
| [18,] | hsa-miR-93-star_st | 0.31 | CGGGAAGTGCTAGCTCAGCAGT |
| [19,] | hsa-miR-93_st | 0.41 | CTACCTGCACGAACAGCACTTTG |

Table 51 Tretinoin microRNA biomarkers.

| | Medianprobe | Corr | Sequence |
|---|---|---|---|
| [1,] | HBII-142_st | 0.37 | CATGGTGTCTCAGTGGCCCAGACAC |
| [2,] | HBII-142x_st | 0.35 | AGTTCCATCATGGTGTCTCAGTGGC |
| [3,] | U55_st | 0.3 | GCACTCGGGAGTATGCAGCATTACC |
| [4,] | U55_x_st | 0.3 | GTATGCAGCATTACCGAGTTGTCAT |
| [5,] | hsa-miR-1202_st | 0.4 | CTCCCCCACTGCAGCTGGCAC |
| [6,] | hsa-miR-124_st | 0.34 | GGCATTCACCGCGTGCCTTA |
| [7,] | hsa-miR-148a-star_st | 0.31 | AGTCGGAGTGTCTCAGAACTTT |
| [8,] | hsa-miR-148a_st | 0.35 | ACAAAGTTCTGTAGTGCACTGA |
| [9,] | hsa-miR-184_st | 0.36 | ACCCTTATCAGTTCTCCGTCCA |
| [10,] | hsa-miR-191_st | 0.32 | CAGCTGCTTTTGGGATTCCGTTG |
| [11,] | hsa-miR-195-star_st | 0.31 | GGAGCAGCACAGCCAATATTGG |

(continued)

| | Medianprobe | Corr | Sequence |
|---|---|---|---|
| [12,] | hsa-miR-29b-2-star_st | 0.37 | CTAAGCCACCATGTGAAACCAG |
| [13,] | hsa-miR-425-star_st | 0.32 | GGGCGGACACGACATTCCCGAT |
| [14,] | hsa-miR-425_st | 0.37 | TCAACGGGAGTGATCGTGTCATT |
| [15,] | hsa-miR-449a_st | 0.43 | ACCAGCTAACAATACACTGCCA |
| [16,] | hsa-miR-449b_st | 0.5 | GCCAGCTAACAATACACTGCCT |
| [17,] | hsa-miR-551b_st | 0.31 | CTGAAACCAAGTATGGGTCGC |
| [18,] | hsa-miR-593-star_st | 0.31 | GCTGAGCAATGCCTGGCTGGTGCCT |
| [19,] | hsa-miR-768-3p_st | 0.38 | GTCAGCAGTTTGAGTGTCAGCATTG |
| [20,] | hsa-miR-768-5p_st | 0.31 | ATCACTCCGTACTTTCATCCTCCAA |
| [21,] | hsa-miR-877_st | 0.3 | CCCTGCGCCATCTCCTCTAC |

Table 52 Ifosfamide microRNA biomarkers.

| | Medianprobe | Corr | Sequence |
|---|---|---|---|
| [1,] | U17b_st | 0.3 | GGCCCAGCTTCATCTTCAACGTTGT |
| [2,] | U48_st | 0.33 | CTGCGGTGATGGCATCAGCGACACA |
| [3,] | U49B_s_st | 0.31 | AGTTATCGCTTCTGACGGCACTTCC |
| [4,] | U55_st | 0.32 | CCACCGCGCACTCGGGAGTATGCAG |
| [5,] | hsa-miR-1207-5p_st | 0.32 | CCCCTCCCAGCCTCCCTGCCA |
| [6,] | hsa-miR-128_st | 0.33 | AAAGAGACCGGTTCACTGTGA |
| [7,] | hsa-miR-181a-star_st | 0.38 | GGTACAATCAACGGTCGATGGT |
| [8,] | hsa-miR-181c-star_st | 0.33 | GTCCACTCAACGGTCGATGGTT |
| [9,] | hsa-miR-181d_st | 0.34 | ACCCACCGACAACAATGAATGTT |
| [10,] | hsa-miR-20b-star_st | 0.36 | CTGGAAGTGCCCATACTACAGT |
| [11,] | hsa-miR-20b_st | 0.32 | CTACCTGCACTATGAGCACTTTG |
| [12,] | hsa-miR-223_st | 0.33 | TGGGGTATTTGACAAACTGACA |
| [13,] | hsa-miR-363_st | 0.33 | TACAGATGGATACCGTGCAATT |
| [14,] | hsa-miR-766_st | 0.4 | GCTGAGGCTGTGGGGCTGGAGT |

Table 53 Tamoxifen microRNA biomarkers.

| | Medianprobe | Corr | Sequence |
|---|---|---|---|
| [1,] | ACA10_s_st | 0.33 | TCTGCCTCCCTGAAAACACGCCAAG |
| [2,] | ACA15s_st | 0.37 | CTGGCTGTGTAAACTACTGATAAAA |
| [3,] | ACA43_st | 0.34 | AACTGTCTGTGGTGCCAACAGGTCC |
| [4,] | ACA44_st | 0.34 | TATTGTACTGACCTGCGCTGTCAAA |
| [5,] | ACA51x_st | 0.3 | GAACACAGCCTGTGGTAAGCACCAG |
| [6,] | ACA57_st | 0.41 | AGGGAATTGCTGTGTGTCCTGCCAG |
| [7,] | ENSG00000202252_st | 0.32 | ATCCAAGGAAGGTGTTGCCATCATT |
| [8,] | HBII-142_st | 0.38 | TCCATCATGGTGTCTCAGTGGCCCA |
| [9,] | HBII-142_x_st | 0.33 | TCAGATCCTCAGTTCCATCATGGTG |
| [10,] | HBII-180A_x_st | 0.48 | GGCACCGTGTCCTCAGTGGCAGTCG |
| [11,] | HBII-180C_st | 0.38 | TGCACTGTGTCCTCAGGGGTGATCA |
| [12,] | HBII-180Cx_st | 0.4 | GTCCTGGGGTGCACTGTGTCCTCAG |
| [13,] | HBII-202_st | 0.31 | ACAGCGATTCCGGAGAATGTCATCA |
| [14,] | HBII-429_st | 0.36 | GGAGCCAGTTGTCATCATGTACAGC |
| [15,] | HBII-55_st | 0.39 | GACATGGAGACATCAGTGATTGCAC |
| [16,] | U104_st | 0.38 | AGGCTCAGACTCCAGTTCGCATCAC |
| [17,] | U17a_st | 0.34 | CGAGGCCCAGCTTTATTTCCAACGT |
| [18,] | U17a_x_st | 0.31 | CTACGCCACTTGGACAGAGCCCGGG |

(continued)

| | Medianprobe | Corr | Sequence |
|---|---|---|---|
| [19,] | U17b_st | 0.5 | CCCAGCTTCATCTTCAACGTTGTGG |
| [20,] | U17b_x_st | 0.45 | GACCTCCCAGGGTATCCACGTTGGA |
| [21,] | U32A_x_st | 0.39 | ATGGTGAATGTTGCTCATCACTGAC |
| [22,] | U33_st | 0.44 | TCTCATGGTCGGGAAACTCGAATGT |
| [23,] | U34_st | 0.4 | TAGGTAGTTGCGGAACATCATGGAC |
| [24,] | U35A_st | 0.36 | TCGTGAGATAAGGACATCATCTGCC |
| [25,] | U38A_st | 0.37 | CTGGACAGAGTTTTCATCACGAGAA |
| [26,] | U43_st | 0.31 | ACACAGTTTCTGTCCGCCCGTCAAT |
| [27,] | U43_x_st | 0.31 | ACACAGTTTCTGTCCGCCCGTCAAT |
| [28,] | U46_x_st | 0.32 | GTGGCACACAGGTGACCAAGACGGC |
| [29,] | U52_st | 0.32 | GTTTTGACATCATGACCAGCATCGG |
| [30,] | U55_st | 0.44 | CCACCGCGCACTCGGGAGTATGCAG |
| [31,] | U55_x_st | 0.44 | GAGTATGCAGCATTACCGAGTTGTC |
| [32,] | U56_st | 0.35 | CCAGAGTCTCAACACTCACTAGGTG |
| [33,] | U56_x_st | 0.3 | CACTAGGTGAACTGCTGTTGACGAA |
| [34,] | U59B_st | 0.3 | CGAAAGTCAGAACGTACTCATCAGT |
| [35,] | U68_st | 0.33 | AAAGGCGACAAGATCCGCTTGCTGT |
| [36,] | U70_x_st | 0.38 | ACCCATACAACCAACAGGCTGCGTA |
| [37,] | U71d_st | 0.35 | TTCCGCGATTTCTTTCCCTGCACTA |
| [38,] | U71d_x_st | 0.34 | AAACACGGGGAAGCACTTTCCGCGA |
| [39,] | U74_x_st | 0.43 | TTCATCATTACTCTCAGATGTCCCT |
| [40,] | U83B_st | 0.4 | TGTCTCGGGCGCTGTGCCCAGCGCA |
| [41,] | hsa-miR-106a_st | 0.31 | CTACCTGCACTGTAAGCACTTTT |
| [42,] | hsa-miR-106b-star_st | 0.37 | GCAGCAAGTACCCACAGTGCGG |
| [43,] | hsa-miR-106b_st | 0.31 | ATCTGCACTGTCAGCACTTTA |
| [44,] | hsa-miR-1183_st | 0.33 | TGCCCACTCTCACCATCACCTACAG |
| [45,] | hsa-miR-1228_st | 0.34 | GGGGGGCGAGGCAGGTGTGA |
| [46,] | hsa-miR-1246_st | 0.35 | CCTGCTCCAAAAATCCATT |
| [47,] | hsa-miR-1281_st | 0.38 | GGGAGAGGAGGAGGCGA |
| [48,] | hsa-miR-149-star_st | 0.33 | GCACAGCCCCCGTCCCTCCCT |
| [49,] | hsa-miR-17-star_st | 0.44 | CTACAAGTGCCTTCACTGCAGT |
| [50,] | hsa-miR-17_st | 0.32 | CTACCTGCACTGTAAGCACTTTG |
| [51,] | hsa-miR-18a-star_st | 0.37 | CCAGAAGGAGCACTTAGGGCAGT |
| [52,] | hsa-miR-18a_st | 0.37 | CTATCTGCACTAGATGCACCTTA |
| [53,] | hsa-miR-195-star_st | 0.32 | GGAGCAGCACAGCCAATATTGG |
| [54,] | hsa-miR-19a_st | 0.34 | TCAGTTTTGCATAGATTTGCACA |
| [55,] | hsa-miR-19b_st | 0.41 | TCAGTTTTGCATGGATTTGCACA |
| [56,] | hsa-miR-20a_st | 0.3 | CTACCTGCACTATAAGCACTTTA |
| [57,] | hsa-miR-25-star_st | 0.35 | CAATTGCCCAAGTCTCCGCCT |
| [58,] | hsa-miR-297_st | 0.38 | CATGCACATGCACACATACAT |
| [59,] | hsa-miR-34b_st | 0.38 | ATGGCAGTGGAGTTAGTGATTG |
| [60,] | hsa-miR-593-star_st | 0.31 | GCTGAGCAATGCCTGGCTGGTGCCT |
| [61,] | hsa-miR-629-star_st | 0.3 | GCTGGGCTTACGTTGGGAGAAC |
| [62,] | hsa-miR-652st | 0.32 | CACAACCCTAGTGGCGCCATT |
| [63,] | hsa-miR-671-5p_st | 0.35 | CTCCAGCCCCTCCAGGGCTTCCT |
| [64,] | hsa-miR-769-5p_st | 0.33 | AGCTCAGAACCCAGAGGTCTCA |
| [65,] | hsa-miR-92a_st | 0.38 | ACAGGCCGGGACAAGTGCAATA |
| [66,] | hsa-miR-93-star_st | 0.36 | CGGGAAGTGCTAGCTCAGCAGT |
| [67,] | hsa-miR-93_st | 0.31 | CTACCTGCACGAACAGCACTTTG |

Table 54 Irinotecan microRNA biomarkers.

| | Medianprobe | Corr | Sequence |
|---|---|---|---|
| [1,] | hsa-miR-181a-star_st | 0.39 | GGTACAATCAACGGTCGATGGT |
| [2,] | hsa-miR-297_st | 0.32 | CATGCACATGCACACATACAT |
| [3,] | hsa-miR-432_st | 0.31 | CCACCCAATGACCTACTCCAAGA |
| [4,] | hsa-miR-766_st | 0.34 | GCTGAGGCTGTGGGGCTGGAGT |
| [5,] | hsa-miR-874_st | 0.33 | TCGGTCCCTCGGGCCAGGGCAG |

Table 55 Floxuridine microRNA biomarkers.

| | Medianprobe | Corr | Sequence |
|---|---|---|---|
| [1,] | HBII-202_st | 0.35 | GCGATTCCGGAGAATGTCATCACGC |
| [2,] | HBII-85-11_st | 0.31 | AAGGAACCCACGTATGGAAGTCATC |
| [3,] | U104_st | 0.3 | AATTGGAATGTCATCACAGCAGGCC |
| [4,] | U13_st | 0.32 | GTGGCACATCTCACACAAGCGTATG |

Table 56 Thioguanine microRNA biomarkers.

| | Medianprobe | Corr | Sequence |
|---|---|---|---|
| [1,] | ACA13_st | 0.3 | GGCAGGAAAAGGCTGCAAAGCGTTT |
| [2,] | ENSG00000202093x_st | 0.3 | TTTCATAGTTATTCCAAAGGTGTCC |
| [3,] | ENSG00000202252_st | 0.4 | ACATCCAAGGAAGGTGTTGCCATCA |
| [4,] | HBII-202_st | 0.4 | ACAGCGATTCCGGAGAATGTCATCA |
| [5,] | HBII-429_st | 0.35 | GTGACATGGCAGTTTCCTCATGGCA |
| [6,] | HBII-55_st | 0.32 | TGGAGACATCAGTGATTGCACTCAG |
| [7,] | U104_st | 0.41 | GGCAGGCTCAGACTCCAGTTCGCAT |
| [8,] | U13_st | 0.37 | ACCCAATCATCACGCTCATGAACTA |
| [9,] | U17b_st | 0.35 | CCCAGCTTCATCTTCAACGTTGTGG |
| [10,] | U17b_x_st | 0.32 | ACGAGGCCCAGCTTCATCTTCAACG |
| [11,] | U25_st | 0.35 | TCTCCTCAGAGTTATTTATCCTCAC |
| [12,] | U27_st | 0.33 | GATGACATCACTTGAAAGTTCAGCC |
| [13,] | U29_st | 0.35 | GAGCTAGTTTGATTCATCATAGAAA |
| [14,] | U30_st | 0.37 | TCAACAGCAAGTCATCAGCCGAACG |
| [15,] | U31_st | 0.31 | GAAAATACCTTTCAGTCACACATTG |
| [16,] | U31_x_st | 0.31 | GCTCAGAAAATACCTTTCAGTCACA |
| [17,] | U36A_st | 0.31 | GCTCATGGATTCGCACTTCAAGGTT |
| [18,] | U36C_st | 0.31 | TGACCACATGGTTTATTCAGGTGAA |
| [19,] | U38A_st | 0.37 | GGCTCTCATCTCTCTCCCTTCAGTA |
| [20,] | U52_st | 0.45 | GTTTTGACATCATGACCAGCATCGG |
| [21,] | U54_st | 0.32 | AACACAATAGGTACCTCCTCATCGC |
| [22,] | U55_st | 0.38 | TTCCCCACCGCGCACTCGGGAGTAT |
| [23,] | U55_x_st | 0.38 | GTATGCAGCATTACCGAGTTGTCAT |
| [24,] | U56_x_st | 0.3 | ACCCAGAGTCTCAACACTCACTAGG |
| [25,] | U74_x_st | 0.44 | AGAGCGGTTGGCATTCATCATTACT |
| [26,] | U75_st | 0.32 | TCAGAAATCCCTTCTGTCCACTACT |
| [27,] | U78_s_st | 0.31 | CTACATGCTCATTTCAGGTCAGACA |
| [28,] | U78_x_st | 0.32 | ACCTTTGTCTACATGCTCATTTCAG |
| [29,] | U83B_st | 0.34 | CAAGGAACGGTTCCGCAGTCTGTCT |
| [30,] | U83_st | 0.32 | ATAAGAGTCGTCCTTGCACTGAGGT |
| [31,] | U95_st | 0.32 | TCTGGATTTCAGCACCGACACTCAG |
| [32,] | hsa-miR-106a_st | 0.45 | CTACCTGCACTGTAAGCACTTTT |

(continued)

| | Medianprobe | Corr | Sequence |
|---|---|---|---|
| [33,] | hsa-miR-1183_st | 0.31 | TGCCCACTCTCACCATCACCTACAG |
| [34,] | hsa-miR-1246_st | 0.38 | CCTGCTCCAAAAATCCATT |
| [35,] | hsa-miR-1281_st | 0.32 | GGGAGAGGAGGAGGCGA |
| [36,] | hsa-miR-142-5p_st | 0.31 | AGTAGTGCTTTCTACTTTATG |
| [37,] | hsa-miR-17-star_st | 0.45 | CTACAAGTGCCTTCACTGCAGT |
| [38,] | hsa-miR-17_st | 0.47 | CTACCTGCACTGTAAGCACTTTG |
| [39,] | hsa-miR-18a-star_st | 0.39 | CCAGAAGGAGCACTTAGGGCAGT |
| [40,] | hsa-miR-18a_st | 0.41 | CTATCTGCACTAGATGCACCTTA |
| [41,] | hsa-miR-18b_st | 0.33 | CTAACTGCACTAGATGCACCTTA |
| [42,] | hsa-miR-19a_st | 0.36 | TCAGTTTTGCATAGATTTGCACA |
| [43,] | hsa-miR-19b_st | 0.38 | TCAGTTTTGCATGGATTTGCACA |
| [44,] | hsa-miR-20a_st | 0.43 | CTACCTGCACTATAAGCACTTTA |
| [45,] | hsa-miR-297_st | 0.33 | CATGCACATGCACACATACAT |
| [46,] | hsa-miR-34b_st | 0.34 | ATGGCAGTGGAGTTAGTGATTG |
| [47,] | hsa-miR-378-star_st | 0.32 | ACACAGGACCTGGAGTCAGGAG |
| [48,] | hsa-miR-378_st | 0.36 | CCTTCTGACTCCAAGTCCAGT |
| [49,] | hsa-miR-491-3p_st | 0.32 | GTAGAAGGGAATCTTGCATAAG |
| [50,] | hsa-miR-766_st | 0.36 | GCTGAGGCTGTGGGGCTGGAGT |
| [51,] | hsa-miR-92a-1-star_st | 0.41 | AGCATTGCAACCGATCCCAACCT |
| [52,] | hsa-miR-92a_st | 0.45 | ACAGGCCGGGACAAGTGCAATA |

Table 57 PSC 833 microRNA biomarkers.

| | Medianprobe | Corr | Sequence |
|---|---|---|---|
| [1,] | ACA18_x_st | 0.31 | GCTACAGGAAAAGCCCCATCGGGAT |
| [2,] | ACA9_st | 0.34 | CTGTTCTAGCAAGCACTGAAGGAAT |
| [3,] | ENSG00000202252st | 0.31 | GCCATCATTAGCCAAGCTTTTGGTG |
| [4,] | HBII-180A_x_st | 0.4 | GGCACCGTGTCCTCAGTGGCAGTCG |
| [5,] | HBII-180B_x_st | 0.32 | TGCACCGTGTCCTCAGGGGCAGTCA |
| [6,] | HBII-180C_st | 0.34 | GTCCTCAGGGGTGATCAGAGCCCAG |
| [7,] | HBII-180C_x_st | 0.36 | TCAGGGGTGATCAGAGCCCAGTGCT |
| [8,] | HBII-382s_st | 0.32 | ATCAGAATCGCCTCGATAATCAGTG |
| [9,] | U3-2_s_st | 0.37 | GCAGTTGCAGCCAAGCAACGCCAGA |
| [10,] | U34_st | 0.3 | GCTGCTTTCATGAGGATCAAACAAT |
| [11,] | U38B_st | 0.31 | TTATCTTCACTTACTGTCAGTAGCA |
| [12,] | U50B_x_st | 0.31 | TCGGGATAGGTTTCATCATTGATTA |
| [13,] | U56_st | 0.3 | TCACTCAGACCCAGAGTCTCAACAC |
| [14,] | U59B_st | 0.32 | TCAGAAATTGCATCTGGCTTCAGCA |
| [15,] | U68_x_st | 0.32 | CAAATTCACTTTGAGGGGCACGGCC |
| [16,] | hsa-miR-106a_st | 0.42 | CTACCTGCACTGTAAGCACTTTT |
| [17,] | hsa-miR-1281_st | 0.32 | GGGAGAGGAGGAGGCGA |
| [18,] | hsa-miR-1292_st | 0.35 | CAGCGTCTGCCGGAACCCGTTCCCA |
| [19,] | hsa-miR-1307_st | 0.34 | CACGACCGACGCCACGCCGAGT |
| [20,] | hsa-miR-17-star_st | 0.32 | CTACAAGTGCCTTCACTGCAGT |
| [21,] | hsa-miR-17_st | 0.44 | CTACCTGCACTGTAAGCACTTTG |
| [22,] | hsa-miR-18a-star_st | 0.39 | CCAGAAGGAGCACTTAGGGCAGT |
| [23,] | hsa-miR-18a_st | 0.43 | CTATCTGCACTAGATGCACCTTA |
| [24,] | hsa-miR-18b_st | 0.32 | CTAACTGCACTAGATGCACCTTA |
| [25,] | hsa-miR-19b_st | 0.34 | TCAGTTTTGCATGGATTTGCACA |
| [26,] | hsa-miR-20a_st | 0.37 | CTACCTGCACTATAAGCACTTTA |

(continued)

| | Medianprobe | Corr | Sequence |
|---|---|---|---|
| [27,] | hsa-miR-25-star_st | 0.31 | CAATTGCCCAAGTCTCCGCCT |
| [28,] | hsa-miR-378-star_st | 0.48 | ACACAGGACCTGGAGTCAGGAG |
| [29,] | hsa-miR-378_st | 0.45 | CCTTCTGACTCCAAGTCCAGT |
| [30,] | hsa-miR-422a_st | 0.47 | GCCTTCTGACCCTAAGTCCAGT |
| [31,] | hsa-miR-92a-1-star_st | 0.34 | AGCATTGCAACCGATCCCAACCT |
| [32,] | hsa-miR-92a_st | 0.45 | ACAGGCCGGGACAAGTGCAATA |
| [33,] | hsa-miR-93-star_st | 0.31 | CGGGAAGTGCTAGCTCAGCAGT |

Table 58 Erlotinib (tarceva) microRNA biomarkers.

| | Medianprobe | Corr | Sequence |
|---|---|---|---|
| [1,] | hsa-miR-130a_st | 0.36 | ATGCCCTTTTAACATTGCACTG |
| [2,] | hsa-miR-149_st | 0.34 | GGGAGTGAAGACACGGAGCCAGA |
| [3,] | hsa-miR-193a-3p_st | 0.31 | ACTGGGACTTTGTAGGCCAGTT |
| [4,] | hsa-miR-27a_st | 0.3 | GCGGAACTTAGCCACTGTGAA |
| [5,] | hsa-miR-30a-star_st | 0.53 | GCTGCAAACATCCGACTGAAAG |
| [6,] | hsa-miR-30a_st | 0.51 | CTTCCAGTCGAGGATGTTTACA |
| [7,] | hsa-miR-30c-2-star_st | 0.56 | AGAGTAAACAGCCTTCTCCCAG |
| [8,] | hsa-miR-30c_st | 0.65 | GCTGAGAGTGTAGGATGTTTACA |
| [9,] | hsa-miR-30e-star_st | 0.35 | GCTGTAAACATCCGACTGAAAG |

Table 59 Herceptin microRNA biomarkers.

| | Medianprobe | Corr | Sequence |
|---|---|---|---|
| [1,] | hsa-miR-34c-3p_st | 0.34 | CCTGGCCGTGTGGTTAGTGATT |
| [2,] | hsa-miR-34c-5p_st | 0.32 | GCAATCAGCTAACTACACTGCCT |
| [3,] | hsa-miR-489_st | 0.3 | GCTGCCGTATATGTGATGTCAC |

Table 60 Celecoxib microRNA biomarkers.

| | Medianprobe | Corr | Sequence |
|---|---|---|---|
| [1,] | ACA18_x_st | 0.3 | GCGTTTCCAACGATGTGCAGGCTAC |
| [2,] | ACA51_x_st | 0.3 | CTATGTTCCCCCATTCACAATACAG |
| [3,] | HBII-142_st | 0.32 | GTGTCTCAGTGGCCCAGACACGTGG |
| [4,] | HBII-142x_st | 0.35 | TGGTGTCTCAGTGGCCCAGACACGT |
| [5,] | HBII-180A_x_st | 0.31 | AGTGCTGGACATCATGGAGGCCCCG |
| [6,] | HBII-180Cxst | 0.3 | TGCACTGTGTCCTCAGGGGTGATCA |
| [7,] | U68_x_st | 0.3 | CGACAAGATCCGCTTGCTGTTTGCA |
| [8,] | U70_x_st | 0.3 | GTGCAGCCATCGCACACTGGGTCCC |
| [9,] | hsa-miR-1207-5p_st | 0.33 | CCCCTCCCAGCCTCCCTGCCA |
| [10,] | hsa-miR-1268_st | 0.31 | CCCCCACCACCACGCCCG |
| [11,] | hsa-miR-768-3p_st | 0.37 | GTCAGCAGTTTGAGTGTCAGCATTG |

Table 61 Fulvestrant microRNA biomarkers.

| | Medianprobe | Corr | Sequence |
|---|---|---|---|
| [1,] | U104_st | 0.35 | GGCAGGCTCAGACTCCAGTTCGCAT |
| [2,] | hsa-miR-1202_st | 0.43 | CTCCCCCACTGCAGCTGGCAC |
| [3,] | hsa-miR-1226st | 0.33 | CTAGGGAACACAGGGCTGGTGA |

(continued)

| | Medianprobe | Corr | Sequence |
|---|---|---|---|
| [4,] | _ hsa-miR-15b_st | 0.3 | TGTAAACCATGATGTGCTGCTA |
| [5,] | hsa-miR-191_st | 0.46 | CAGCTGCTTTTGGGATTCCGTTG |
| [6,] | hsa-miR-203_st | 0.32 | CTAGTGGTCCTAAACATTTCAC |
| [7,] | hsa-miR-25_st | 0.33 | TCAGACCGAGACAAGTGCAATG |
| [8,] | hsa-miR-29b-2-star_st | 0.3 | CTAAGCCACCATGTGAAACCAG |
| [9,] | hsa-miR-301a_st | 0.47 | GCTTTGACAATACTATTGCACTG |
| [10,] | hsa-miR-339-5p_st | 0.35 | CGTGAGCTCCTGGAGGACAGGGA |
| [11,] | hsa-miR-342-5p_st | 0.45 | TCAATCACAGATAGCACCCCT |
| [12,] | hsa-miR-421_st | 0.36 | GCGCCCAATTAATGTCTGTTGAT |
| [13,] | hsa-miR-425-star_st | 0.36 | GGGCGGACACGACATTCCCGAT |
| [14,] | hsa-miR-425_st | 0.49 | TCAACGGGAGTGATCGTGTCATT |
| [15,] | hsa-miR-449b_st | 0.35 | GCCAGCTAACAATACACTGCCT |
| [16,] | hsa-miR-489_st | 0.61 | GCTGCCGTATATGTGATGTCAC |
| [17,] | hsa-miR-492_st | 0.36 | AAGAATCTTGTCCCGCAGGTCCT |
| [18,] | hsa-miR-622_st | 0.37 | GCTCCAACCTCAGCAGACTGT |
| [19,] | hsa-miR-768-3p_st | 0.41 | GTCAGCAGTTTGAGTGTCAGCATTG |
| [20,] | hsa-miR-768-5p_st | 0.39 | ATCACTCCGTACTTTCATCCTCCAA |

Table 62 Iressa microRNA biomarkers.

| | Medianprobe | Corr | Sequence |
|---|---|---|---|
| [1,] | hsa-miR-1826_st | 0.34 | ATTGCGTTCGAAGTGTCGATGATCA |
| [2,] | hsa-miR-205_st | 0.3 | CAGACTCCGGTGGAATGAAGGA |
| [3,] | hsa-miR-30c_st | 0.46 | GCTGAGAGTGTAGGATGTTTACA |
| [4,] | hsa-miR-675_st | 0.32 | CACTGTGGGCCCTCTCCGCACCA |
| [5,] | hsa-miR-934_st | 0.48 | CCAGTGTCTCCAGTAGTAGACA |

Table 63 Anastrozole microRNA biomarkers.

| | Medianprobe | Corr | Sequence |
|---|---|---|---|
| [1,] | hsa-miR-1323_st | 0.31 | AGAAAATGCCCCTCAGTTTTGA |
| [2,] | hsa-miR-184_st | 0.34 | ACCCTTATCAGTTCTCCGTCCA |
| [3,] | hsa-miR-187_st | 0.38 | CCGGCTGCAACACAAGACACGA |
| [4,] | hsa-miR-197_st | 0.32 | GCTGGGTGGAGAAGGTGGTGAA |
| [5,] | hsa-miR-498_st | 0.45 | GAAAAACGCCCCCTGGCTTGAAA |
| [6,] | hsa-miR-504_st | 0.42 | GATAGAGTGCAGACCAGGGTCT |
| [7,] | hsa-miR-506_st | 0.38 | TCTACTCAGAAGGGTGCCTTA |
| [8,] | hsa-miR-508-5p_st | 0.42 | CATGAGTGACGCCCTCTGGAGTA |
| [9,] | hsa-miR-509-3-5p_st | 0.35 | CATGATTGCCACGTCTGCAGTA |
| [10,] | hsa-miR-509-3p_st | 0.37 | CTACCCACAGACGTACCAATCA |
| [11,] | hsa-miR-509-5p_st | 0.38 | TGATTGCCACTGTCTGCAGTA |
| [12,] | hsa-miR-510_st | 0.36 | GTGATTGCCACTCTCCTGAGTA |
| [13,] | hsa-miR-512-3p_st | 0.54 | GACCTCAGCTATGACAGCACTT |
| [14,] | hsa-miR-513a-5p_st | 0.39 | ATGACACCTCCCTGTGAA |
| [15,] | hsa-miR-513b_st | 0.31 | ATAAATGACACCTCCTTGTGAA |
| [16,] | hsa-miR-513c_st | 0.39 | ATAAACGACACCTCCTTGAGAA |
| [17,] | hsa-miR-516b_st | 0.53 | AAAGTGCTTCTTACCTCCAGAT |
| [18,] | hsa-miR-517a_st | 0.56 | ACACTCTAAAGGGATGCACGAT |
| [19,] | hsa-miR-517b_st | 0.38 | AACACTCTAAAGGGATGCACGA |
| [20,] | hsa-miR-525-5p_st | 0.52 | AGAAAGTGCATCCCTCTGGAG |

(continued)

| | Medianprobe | Corr | Sequence |
|---|---|---|---|
| [21,] | hsa-miR-526b_st | 0.33 | ACAGAAAGTGCTTCCCTCAAGAG |
| [22,] | hsa-miR-551a_st | 0.54 | TGGAAACCAAGAGTGGGTCGC |
| [23,] | hsa-miR-873_st | 0.31 | AGGAGACTCACAAGTTCCTGC |
| [24,] | hsa-miR-891a_st | 0.36 | TCAGTGGCTCAGGTTCGTTGCA |

Table 64 Letrozole microRNA biomarkers.

| | Medianprobe | Corr | Sequence |
|---|---|---|---|
| [1,] | hsa-miR-1323_st | 0.31 | AGAAAATGCCCCTCAGTTTTGA |
| [2,] | hsa-miR-361-5p_st | 0.35 | GTACCCCTGGAGATTCTGATAA |
| [3,] | hsa-miR-498_st | 0.36 | GAAAAACGCCCCCTGGCTTGAAA |
| [4,] | hsa-miR-512-3p_st | 0.37 | GACCTCAGCTATGACAGCACTT |
| [5,] | hsa-miR-516b_st | 0.48 | AAAGTGCTTCTTACCTCCAGAT |
| [6,] | hsa-miR-517a_st | 0.52 | ACACTCTAAAGGGATGCACGAT |
| [7,] | hsa-miR-525-5p_st | 0.42 | AGAAAGTGCATCCCTCTGGAG |
| [8,] | hsa-miR-551a_st | 0.57 | TGGAAACCAAGAGTGGGTCGC |
| [9,] | hsa-miR-873_st | 0.32 | AGGAGACTCACAAGTTCCTGC |

Table 65 Cetuximab (erbitux) microRNA biomarkers.

| | Medianprobe | Corr | Sequence |
|---|---|---|---|
| [1,] | hsa-miR-1826_st | 0.31 | ATTGCGTTCGAAGTGTCGATGATCA |
| [2,] | hsa-miR-30c-2-star_st | 0.31 | AGAGTAAACAGCCTTCTCCCAG |
| [3,] | hsa-miR-34b-star_st | 0.37 | CAATCAGCTAATGACACTGCCTA |
| [4,] | hsa-miR-34c-3p_st | 0.46 | CCTGGCCGTGTGGTTAGTGATT |
| [5,] | hsa-miR-34c-5p_st | 0.4 | GCAATCAGCTAACTACACTGCCT |
| [6,] | hsa-miR-489_st | 0.3 | GCTGCCGTATATGTGATGTCAC |

Table 66 Vincristine microRNA biomarkers.

| | Medianprobe | Corr | Sequence |
|---|---|---|---|
| [1,] | HBII-85-29_st | -0.34 | TTCAGATTTCCAAGGTTTCCATTTT |
| [2,] | hsa-miR-130ast | -0.3 | ATGCCCTTTTAACATTGCACTG |
| [3,] | hsa-miR-148b_st | -0.33 | ACAAAGTTCTGTGATGCACTGA |
| [4,] | hsa-miR-184_st | -0.4 | ACCCTTATCAGTTCTCCGTCCA |
| [5,] | hsa-miR-26a_st | -0.31 | AGCCTATCCTGGATTACTTGAA |
| [6,] | hsa-miR-30a-star_st | -0.33 | GCTGCAAACATCCGACTGAAAG |
| [7,] | hsa-miR-30a_st | -0.39 | CTTCCAGTCGAGGATGTTTACA |
| [8,] | hsa-miR-30c-2-star_st | -0.33 | AGAGTAAACAGCCTTCTCCCAG |
| [9,] | hsa-miR-30c_st | -0.43 | GCTGAGAGTGTAGGATGTTTACA |
| [10,] | hsa-miR-34a-star_st | -0.33 | AGGGCAGTATACTTGCTGATTG |
| [11,] | hsa-miR-34a_st | -0.32 | ACAACCAGCTAAGACACTGCCA |
| [12,] | hsa-miR-34c-5p_st | -0.38 | GCAATCAGCTAACTACACTGCCT |
| [13,] | hsa-miR-449a_st | -0.5 | ACCAGCTAACAATACACTGCCA |
| [14,] | hsa-miR-449b_st | -0.49 | GCCAGCTAACAATACACTGCCT |

Table 67 Cisplatin microRNA biomarkers.

|      | Medianprobe          | Corr  | Sequence                   |
|------|----------------------|-------|----------------------------|
| [1,] | hsa-miR-10a_st       | -0.34 | CACAAATTCGGATCTACAGGGTA     |
| [2,] | hsa-miR-151-3p_st    | -0.38 | CCTCAAGGAGCTTCAGTCTAG       |
| [3,] | hsa-miR-151-5p_st    | -0.35 | ACTAGACTGTGAGCTCCTCGA       |
| [4,] | hsa-miR-192-star_st  | -0.33 | CTGTGACCTATGGAATTGGCAG      |
| [5,] | hsa-miR-192_st       | -0.4  | GGCTGTCAATTCATAGGTCAG       |
| [6,] | hsa-miR-194_st       | -0.4  | TCCACATGGAGTTGCTGTTACA      |
| [7,] | hsa-miR-200b_st      | -0.32 | TCATCATTACCAGGCAGTATTA      |
| [8,] | hsa-miR-203_st       | -0.36 | CTAGTGGTCCTAAACATTTCAC      |
| [9,] | hsa-miR-29b_st       | -0.36 | AACACTGATTTCAAATGGTGCTA     |
| [10,] | hsa-miR-30b_st      | -0.4  | AGCTGAGTGTAGGATGTTTACA      |
| [11,] | hsa-miR-30d_st      | -0.38 | CTTCCAGTCGGGGATGTTTACA      |
| [12,] | hsa-miR-625_st      | -0.34 | GGACTATAGAACTTTCCCCCT       |

Table 68 Etoposide microRNA biomarkers.

|      | Medianprobe          | Corr  | Sequence                   |
|------|----------------------|-------|----------------------------|
| [1,] | hsa-let-7e_st        | -0.34 | AACTATACAACCTCCTACCTCA      |
| [2,] | hsa-miR-10b_st       | -0.37 | CACAAATTCGGTTCTACAGGGTA     |
| [3,] | hsa-miR-125a-5p_st   | -0.33 | TCACAGGTTAAAGGGTCTCAGGGA    |
| [4,] | hsa-miR-141_st       | -0.37 | CCATCTTTACCAGACAGTGTTA      |
| [5,] | hsa-miR-151-3p_st    | -0.43 | CCTCAAGGAGCTTCAGTCTAG       |
| [6,] | hsa-miR-151-5p_st    | -0.47 | ACTAGACTGTGAGCTCCTCGA       |
| [7,] | hsa-miR-200a-star_st | -0.3  | TCCAGCACTGTCCGGTAAGATG      |
| [8,] | hsa-miR-200a_st      | -0.34 | ACATCGTTACCAGACAGTGTTA      |
| [9,] | hsa-miR-200b-star_st | -0.33 | TCCAATGCTGCCCAGTAAGATG      |
| [10,] | hsa-miR-200b_st     | -0.38 | TCATCATTACCAGGCAGTATTA      |
| [11,] | hsa-miR-200c_st     | -0.35 | TCCATCATTACCCGGCAGTATTA     |
| [12,] | hsa-miR-203_st      | -0.34 | CTAGTGGTCCTAAACATTTCAC      |
| [13,] | hsa-miR-30a_st      | -0.32 | CTTCCAGTCGAGGATGTTTACA      |
| [14,] | hsa-miR-30b_st      | -0.35 | AGCTGAGTGTAGGATGTTTACA      |
| [15,] | hsa-miR-30c_st      | -0.34 | GCTGAGAGTGTAGGATGTTTACA     |
| [16,] | hsa-miR-30d_st      | -0.43 | CTTCCAGTCGGGGATGTTTACA      |
| [17,] | hsa-miR-429_st      | -0.31 | ACGGTTTTACCAGACAGTATTA      |
| [18,] | hsa-miR-516a-5p_st  | -0.37 | GAAAGTGCTTCTTTCCTCGAGAA     |
| [19,] | hsa-miR-934_st      | -0.41 | CCAGTGTCTCCAGTAGTAGACA      |
| [20,] | hsa-miR-99b_st      | -0.33 | CGCAAGGTCGGTTCTACGGGTG      |

Table 69 Azaguanine microRNA biomarkers.

|      | Medianprobe          | Corr  | Sequence                   |
|------|----------------------|-------|----------------------------|
| [1,] | hsa-miR-1244_st      | -0.3  | AACCATCTCATACAAACCAACTACT   |
| [2,] | hsa-miR-1303_st      | -0.32 | AGAGCAAGACCCCGTCTCTAAA      |
| [3,] | hsa-miR-141-star_st  | -0.35 | TCCAACACTGTACTGGAAGATG      |
| [4,] | hsa-miR-141_st       | -0.6  | CCATCTTTACCAGACAGTGTTA      |
| [5,] | hsa-miR-182_st       | -0.38 | AGTGTGAGTTCTACCATTGCCAAA    |
| [6,] | hsa-miR-183-star_st  | -0.38 | TTATGGCCCTTCGGTAATTCAC      |
| [7,] | hsa-miR-183_st       | -0.45 | AGTGAATTCTACCAGTGCCATA      |
| [8,] | hsa-miR-192-star_st  | -0.4  | CTGTGACCTATGGAATTGGCAG      |
| [9,] | hsa-miR-192_st       | -0.46 | GGCTGTCAATTCATAGGTCAG       |
| [10,] | hsa-miR-194_st      | -0.46 | TCCACATGGAGTTGCTGTTACA      |

(continued)

| | Medianprobe | Corr | Sequence |
|---|---|---|---|
| [11,] | hsa-miR-200a-star_st | -0.37 | TCCAGCACTGTCCGGTAAGATG |
| [12,] | hsa-miR-200a_st | -0.43 | ACATCGTTACCAGACAGTGTTA |
| [13,] | hsa-miR-200b-star_st | -0.42 | TCCAATGCTGCCCAGTAAGATG |
| [14,] | hsa-miR-200b_st | -0.42 | TCATCATTACCAGGCAGTATTA |
| [15,] | hsa-miR-200c-star_st | -0.5 | CCAAACACTGCTGGGTAAGACG |
| [16,] | hsa-miR-200c_st | -0.64 | TCCATCATTACCCGGCAGTATTA |
| [17,] | hsa-miR-203_st | -0.51 | CTAGTGGTCCTAAACATTTCAC |
| [18,] | hsa-miR-205_st | -0.38 | CAGACTCCGGTGGAATGAAGGA |
| [19,] | hsa-miR-215_st | -0.36 | GTCTGTCAATTCATAGGTCAT |
| [20,] | hsa-miR-27b_st | -0.3 | GCAGAACTTAGCCACTGTGAA |
| [21,] | hsa-miR-331-3p_st | -0.3 | TTCTAGGATAGGCCCAGGGGC |
| [22,] | hsa-miR-375_st | -0.35 | TCACGCGAGCCGAACGAACAAA |
| [23,] | hsa-miR-429_st | -0.4 | ACGGTTTTACCAGACAGTATTA |
| [24,] | hsa-miR-622_st | -0.43 | GCTCCAACCTCAGCAGACTGT |
| [25,] | hsa-miR-934_st | -0.37 | CCAGTGTCTCCAGTAGTAGACA |
| [26,] | hsa-miR-99b-star_st | -0.31 | CGGACCCACAGACACGAGCTTG |

Table 70 Carboplatin microRNA biomarkers.

| | Medianprobe | Corr | Sequence |
|---|---|---|---|
| [1,] | hsa-miR-10a_st | -0.31 | CACAAATTCGGATCTACAGGGTA |
| [2,] | hsa-miR-183_st | -0.33 | AGTGAATTCTACCAGTGCCATA |
| [3,] | hsa-miR-192-star_st | -0.33 | CTGTGACCTATGGAATTGGCAG |
| [4,] | hsa-miR-192_st | -0.38 | GGCTGTCAATTCATAGGTCAG |
| [5,] | hsa-miR-194_st | -0.4 | TCCACATGGAGTTGCTGTTACA |
| [6,] | hsa-miR-200a-star_st | -0.31 | TCCAGCACTGTCCGGTAAGATG |
| [7,] | hsa-miR-200a_st | -0.35 | ACATCGTTACCAGACAGTGTTA |
| [8,] | hsa-miR-200b-star_st | -0.35 | TCCAATGCTGCCCAGTAAGATG |
| [9,] | hsa-miR-200b_st | -0.37 | TCATCATTACCAGGCAGTATTA |
| [10,] | hsa-miR-200c-star_st | -0.31 | CCAAACACTGCTGGGTAAGACG |
| [11,] | hsa-miR-203_st | -0.39 | CTAGTGGTCCTAAACATTTCAC |
| [12,] | hsa-miR-29b_st | -0.41 | AACACTGATTTCAAATGGTGCTA |
| [13,] | hsa-miR-30b_st | -0.39 | AGCTGAGTGTAGGATGTTTACA |
| [14,] | hsa-miR-30d_st | -0.32 | CTTCCAGTCGGGGATGTTTACA |
| [15,] | hsa-miR-429_st | -0.36 | ACGGTTTTACCAGACAGTATTA |
| [16,] | hsa-miR-625_st | -0.37 | GGACTATAGAACTTTCCCCCT |
| [17,] | hsa-miR-7_st | -0.31 | ACAACAAAATCACTAGTCTTCCA |

Table 71 Adriamycin microRNA biomarkers.

| | Medianprobe | Corr | Sequence |
|---|---|---|---|
| [1,] | hsa-let-7e_st | -0.37 | AACTATACAACCTCCTACCTCA |
| [2,] | hsa-miR-125a-5p_st | -0.36 | TCACAGGTTAAAGGGTCTCAGGGA |
| [3,] | hsa-miR-151-3p_st | -0.41 | CCTCAAGGAGCTTCAGTCTAG |
| [4,] | hsa-miR-151-5p_st | -0.43 | ACTAGACTGTGAGCTCCTCGA |
| [5,] | hsa-miR-203_st | -0.31 | CTAGTGGTCCTAAACATTTCAC |
| [6,] | hsa-miR-23a_st | -0.36 | GGAAATCCCTGGCAATGTGAT |
| [7,] | hsa-miR-29b_st | -0.34 | AACACTGATTTCAAATGGTGCTA |
| [8,] | hsa-miR-30a_st | -0.31 | CTTCCAGTCGAGGATGTTTACA |
| [9,] | hsa-miR-30c_st | -0.31 | GCTGAGAGTGTAGGATGTTTACA |

(continued)

| | Medianprobe | Corr | Sequence |
|---|---|---|---|
| [10,] | hsa-miR-516a-5p_st | -0.53 | GAAAGTGCTTCTTTCCTCGAGAA |
| [11,] | hsa-miR-518d-5p_st | -0.3 | CAGAAAGTGCTTCCCTCTAGAG |
| [12,] | hsa-miR-518e-star_st | -0.35 | CAGAAAGCGCTTCCCTCTAGAG |
| [13,] | hsa-miR-519a-star_st | -0.36 | CAGAAAGCGCTTCCCTCTAGAG |
| [14,] | hsa-miR-519a_st | -0.41 | ACACTCTAAAAGGATGCACTTT |
| [15,] | hsa-miR-519b-5p_st | -0.35 | CAGAAAGCGCTTCCCTCTAGAG |
| [16,] | hsa-miR-519c-5p_st | -0.38 | CAGAAAGCGCTTCCCTCTAGAG |
| [17,] | hsa-miR-522-star_st | -0.34 | CAGAAAGCGCTTCCCTCTAGAG |
| [18,] | hsa-miR-523-star_st | -0.32 | CAGAAAGCGCTTCCCTCTAGAG |
| [19,] | hsa-miR-526a_st | -0.36 | CAGAAAGTGCTTCCCTCTAGAG |
| [20,] | hsa-miR-99b_st | -0.33 | CGCAAGGTCGGTTCTACGGGTG |

Table 72 Aclarubicin microRNA biomarkers.

| | Medianprobe | Corr | Sequence |
|---|---|---|---|
| [1,] | hsa-let-7a_st | -0.33 | AACTATACAACCTACTACCTCA |
| [2,] | hsa-let-7c_st | -0.32 | AACCATACAACCTACTACCTCA |
| [3,] | hsa-let-7e_st | -0.37 | AACTATACAACCTCCTACCTCA |
| [4,] | hsa-let-7f_st | -0.31 | AACTATACAATCTACTACCTCA |
| [5,] | hsa-miR-125a-5p_st | -0.36 | TCACAGGTTAAAGGGTCTCAGGGA |
| [6,] | hsa-miR-193a-5p_st | -0.33 | TCATCTCGCCCGCAAAGACCCA |
| [7,] | hsa-miR-22-star_st | -0.33 | TAAAGCTTGCCACTGAAGAACT |
| [8,] | hsa-miR-22_st | -0.33 | ACAGTTCTTCAACTGGCAGCTT |
| [9,] | hsa-miR-23a_st | -0.35 | GGAAATCCCTGGCAATGTGAT |
| [10,] | hsa-miR-24-2-star_st | -0.43 | CTGTGTTTCAGCTCAGTAGGCA |
| [11,] | hsa-miR-24_st | -0.33 | CTGTTCCTGCTGAACTGAGCCA |
| [12,] | hsa-miR-30a_st | -0.34 | CTTCCAGTCGAGGATGTTTACA |
| [13,] | hsa-miR-99b_st | -0.33 | CGCAAGGTCGGTTCTACGGGTG |

Table 73 Mitoxantrone microRNA biomarkers.

| | Medianprobe | Corr | Sequence |
|---|---|---|---|
| [1,] | hsa-miR-151-5p_st | -0.35 | ACTAGACTGTGAGCTCCTCGA |
| [2,] | hsa-miR-29b_st | -0.31 | AACACTGATTTCAAATGGTGCTA |
| [3,] | hsa-miR-30b_st | -0.43 | AGCTGAGTGTAGGATGTTTACA |
| [4,] | hsa-miR-30d_st | -0.48 | CTTCCAGTCGGGGATGTTTACA |
| [5,] | hsa-miR-506_st | -0.34 | TCTACTCAGAAGGGTGCCTTA |
| [6,] | hsa-miR-508-5p_st | -0.32 | CATGAGTGACGCCCTCTGGAGTA |
| [7,] | hsa-miR-509-3-5p_st | -0.36 | CATGATTGCCACGTCTGCAGTA |
| [8,] | hsa-miR-509-3p_st | -0.34 | CTACCCACAGACGTACCAATCA |
| [9,] | hsa-miR-509-5p_st | -0.32 | TGATTGCCACTGTCTGCAGTA |
| [18,] | hsa-miR-510_st | -0.31 | GTGATTGCCACTCTCCTGAGTA |
| [11,] | hsa-miR-513a-5p_st | -0.34 | ATGACACCTCCCTGTGAA |
| [12,] | hsa-miR-513c_st | -0.3 | ATAAACGACACCTCCTTGAGAA |
| [13,] | hsa-miR-516a-5p_st | -0.33 | GAAAGTGCTTCTTTCCTCGAGAA |
| [14,] | hsa-miR-584_st | -0.41 | CTCAGTCCCAGGCAAACCATAA |
| [15,] | hsa-miR-885-3p_st | -0.32 | TATCCACTACACCCCGCTGCCT |

Table 74 Mitomycin microRNA biomarkers.

| | Medianprobe | Corr | Sequence |
|---|---|---|---|
| [1,] | hsa-miR-516a-5p_st | -0.33 | GAAAGTGCTTCTTTCCTCGAGAA |
| [2,] | hsa-miR-526a_st | -0.35 | CAGAAAGTGCTTCCCTCTAGAG |

Table 75 Paclitaxel (Taxol) microRNA biomarkers.

| | Medianprobe | Corr | Sequence |
|---|---|---|---|
| [1,] | HBII-85-29_st | -0.31 | TTCAGATTTCCAAGGTTTCCATTTT |
| [2,] | hsa-let-7e_st | -0.31 | AACTATACAACCTCCTACCTCA |
| [3,] | hsa-miR-125a-5p_st | -0.32 | TCACAGGTTAAAGGGTCTCAGGGA |
| [4,] | hsa-miR-130a_st | -0.31 | ATGCCCTTTTAACATTGCACTG |
| [5,] | hsa-miR-193b_st | -0.34 | AGCGGGACTTTGAGGGCCAGTT |
| [6,] | hsa-miR-22_st | -0.46 | ACAGTTCTTCAACTGGCAGCTT |
| [7,] | hsa-miR-27a_st | -0.34 | GCGGAACTTAGCCACTGTGAA |
| [8,] | hsa-miR-29a_st | -0.33 | TAACCGATTTCAGATGGTGCTA |
| [9,] | hsa-miR-29b_st | -0.43 | AACACTGATTTCAAATGGTGCTA |
| [10,] | hsa-miR-30a-star_st | -0.42 | GCTGCAAACATCCGACTGAAAG |
| [11,] | hsa-miR-30a_st | -0.49 | CTTCCAGTCGAGGATGTTTACA |
| [12,] | hsa-miR-30c-2-star_st | -0.47 | AGAGTAAACAGCCTTCTCCCAG |
| [13,] | hsa-miR-30c_st | -0.44 | GCTGAGAGTGTAGGATGTTTACA |
| [14,] | hsa-miR-34a_st | -0.3 | ACAACCAGCTAAGACACTGCCA |
| [15,] | hsa-miR-34b-star_st | -0.39 | CAATCAGCTAATGACACTGCCTA |
| [16,] | hsa-miR-34c-3p_st | -0.42 | CCTGGCCGTGTGGTTAGTGATT |
| [17,] | hsa-miR-34c-5p_st | -0.48 | GCAATCAGCTAACTACACTGCCT |

Table 76 Gemcitabine (Gemzar microRNA biomarkers.

| | Medianprobe | Corr | Sequence |
|---|---|---|---|
| [1,] | hsa-miR-141_st | -0.33 | CCATCTTTACCAGACAGTGTTA |
| [2,] | hsa-miR-584_st | -0.31 | CTCAGTCCCAGGCAAACCATAA |
| [3,] | hsa-miR-934_st | -0.31 | CCAGTGTCTCCAGTAGTAGACA |

Table 77 Taxotere (docetaxel) microRNA biomarkers.

| | Medianprobe | Corr | Sequence |
|---|---|---|---|
| [1,] | HBII-438A_s_st | -0.42 | TGACATCTGGAATGAGTCCCTCAGC |
| [2,] | HBII-85-11_st | -0.42 | TCCAAGGAACCCACGTATGGAAGTC |
| [3,] | HBII-85-15x_st | -0.33 | TGTATATATGGAAGTCATCATCGAT |
| [4,] | HBII-85-23_x_st | -0.42 | CATGTATTGAGGTCATCATCGATCC |
| [5,] | HBII-85-29_st | -0.47 | GTTCAGATTTCCAAGGTTTCCATTT |
| [6,] | HBII-85-29x_st | -0.38 | GCTCACAGAAGTGTCTTGGTCACTC |
| [7,] | hsa-miR-184_st | -0.3 | ACCCTTATCAGTTCTCCGTCCA |
| [8,] | hsa-miR-29ast | -0.33 | TAACCGATTTCAGATGGTGCTA |
| [9,] | hsa-miR-29b_st | -0.34 | AACACTGATTTCAAATGGTGCTA |
| [10,] | hsa-miR-34ast | -0.33 | ACAACCAGCTAAGACACTGCCA |
| [11,] | hsa-miR-34c-3p_st | -0.46 | CCTGGCCGTGTGGTTAGTGATT |
| [12,] | hsa-miR-34c-5p_st | -0.41 | GCAATCAGCTAACTACACTGCCT |
| [13,] | hsa-miR-424-star_st | -0.3 | ATAGCAGCGCCTCACGTTTTG |

Table 78 Dexamethasone microRNA biomarkers.

| | Medianprobe | Corr | Sequence |
|---|---|---|---|
| [1,] | hsa-1et-7e_st | -0.31 | AACTATACAACCTCCTACCTCA |
| [2,] | hsa-miR-151-5p_st | -0.3 | ACTAGACTGTGAGCTCCTCGA |
| [3,] | hsa-miR-182_st | -0.31 | AGTGTGAGTTCTACCATTGCCAAA |
| [4,] | hsa-miR-23b_st | -0.31 | GGTAATCCCTGGCAATGTGAT |
| [5,] | hsa-miR-24_st | -0.35 | CTGTTCCTGCTGAACTGAGCCA |
| [6,] | hsa-miR-34a_st | -0.36 | ACAACCAGCTAAGACACTGCCA |
| [7,] | hsa-miR-99b_st | -0.32 | CGCAAGGTCGGTTCTACGGGTG |

Table 79 Ara-C (Cytarabine hydrochloride) microRNA biomarkers.

| | Medianprobe | Corr | Sequence |
|---|---|---|---|
| [1,] | hsa-miR-151-5p_st | -0.3 | ACTAGACTGTGAGCTCCTCGA |
| [2,] | hsa-miR-23b-star_st | -0.31 | AAATCAGCATGCCAGGAACCCA |
| [3,] | hsa-miR-23b_st | -0.38 | GGTAATCCCTGGCAATGTGAT |
| [4,] | hsa-miR-27b_st | -0.36 | GCAGAACTTAGCCACTGTGAA |
| [5,] | hsa-miR-99b_st | -0.31 | CGCAAGGTCGGTTCTACGGGTG |

Table 80 Methylprednisolone microRNA biomarkers.

| | Medianprobe | Corr | Sequence |
|---|---|---|---|
| [1,] | hsa-let-7e_st | -0.43 | AACTATACAACCTCCTACCTCA |
| [2,] | hsa-miR-125a-5p_st | -0.41 | TCACAGGTTAAAGGGTCTCAGGGA |
| [3,] | hsa-miR-1308_st | -0.39 | CCACTGAACCACCCATGC |
| [4,] | hsa-miR-151-3p_st | -0.37 | CCTCAAGGAGCTTCAGTCTAG |
| [5,] | hsa-miR-151-5p_st | -0.42 | ACTAGACTGTGAGCTCCTCGA |
| [6,] | hsa-miR-182_st | -0.39 | AGTGTGAGTTCTACCATTGCCAAA |
| [7,] | hsa-miR-193b_st | -0.33 | AGCGGGACTTTGAGGGCCAGTT |
| [8,] | hsa-miR-21-star_st | -0.31 | ACAGCCCATCGACTGGTGTTG |
| [9,] | hsa-miR-21_st | -0.34 | TCAACATCAGTCTGATAAGCTA |
| [10,] | hsa-miR-22_st | -0.37 | ACAGTTCTTCAACTGGCAGCTT |
| [11,] | hsa-miR-23a_st | -0.5 | GGAAATCCCTGGCAATGTGAT |
| [12,] | hsa-miR-23b_st | -0.4 | GGTAATCCCTGGCAATGTGAT |
| [13,] | hsa-miR-24-2-star_st | -0.36 | CTGTGTTTCAGCTCAGTAGGCA |
| [14,] | hsa-miR-24_st | -0.57 | CTGTTCCTGCTGAACTGAGCCA |
| [15,] | hsa-miR-27a-star_st | -0.42 | TGCTCACAAGCAGCTAAGCCCT |
| [16,] | hsa-miR-27a_st | -0.4 | GCGGAACTTAGCCACTGTGAA |
| [17,] | hsa-miR-27b_st | -0.34 | GCAGAACTTAGCCACTGTGAA |
| [18,] | hsa-miR-339-3p_st | -0.3 | CGGCTCTGTCGTCGAGGCGCTCA |
| [19,] | hsa-miR-34a_st | -0.32 | ACAACCAGCTAAGACACTGCCA |
| [20,] | hsa-miR-99b_st | -0.43 | CGCAAGGTCGGTTCTACGGGTG |

Table 81 Methotrexate microRNA biomarkers.

| | Medianprobe | Corr | Sequence |
|---|---|---|---|
| [1,] | hsa-let-7e_st | -0.31 | AACTATACAACCTCCTACCTCA |
| [2,] | hsa-miR-125a-5p_st | -0.32 | TCACAGGTTAAAGGGTCTCAGGGA |
| [3,] | hsa-miR-22_st | -0.3 | ACAGTTCTTCAACTGGCAGCTT |
| [4,] | hsa-miR-23b_st | -0.35 | GGTAATCCCTGGCAATGTGAT |
| [5,] | hsa-miR-27b_st | -0.31 | GCAGAACTTAGCCACTGTGAA |

(continued)

| | Medianprobe | Corr | Sequence |
|---|---|---|---|
| [6,] | hsa-miR-28-5p_st | -0.35 | CTCAATAGACTGTGAGCTCCTT |
| [7,] | hsa-miR-30a-star_st | -0.32 | GCTGCAAACATCCGACTGAAAG |
| [8,] | hsa-miR-30a_st | -0.37 | CTTCCAGTCGAGGATGTTTACA |
| [9,] | hsa-miR-34c-5p_st | -0.3 | GCAATCAGCTAACTACACTGCCT |
| [10,] | hsa-miR-516b_st | -0.31 | AAAGTGCTTCTTACCTCCAGAT |
| [11,] | hsa-miR-517a_st | -0.32 | ACACTCTAAAGGGATGCACGAT |
| [12,] | hsa-miR-525-5p_st | -0.32 | AGAAAGTGCATCCCTCTGGAG |

Table 82 Bleomycin microRNA biomarkers.

| | Medianprobe | Corr | Sequence |
|---|---|---|---|
| [1,] | hsa-miR-1244_st | -0.31 | AACCATCTCATACAAACCAACTACT |
| [2,] | hsa-miR-128_st | -0.32 | AAAGAGACCGGTTCACTGTGA |
| [3,] | hsa-miR-1292_st | -0.32 | CAGCGTCTGCCGGAACCCGTTCCCA |
| [4,] | hsa-miR-141_st | -0.34 | CCATCTTTACCAGACAGTGTTA |
| [5,] | hsa-miR-15b_st | -0.33 | TGTAAACCATGATGTGCTGCTA |
| [6,] | hsa-miR-185-star_st | -0.32 | GACCAGAGGAAAGCCAGCCCCT |
| [7,] | hsa-miR-188-5p_st | -0.32 | CCCTCCACCATGCAAGGGATG |
| [8,] | hsa-miR-18b_st | -0.37 | CTAACTGCACTAGATGCACCTTA |
| [9,] | hsa-miR-200c_st | -0.33 | TCCATCATTACCCGGCAGTATTA |
| [10,] | hsa-miR-203_st | -0.3 | CTAGTGGTCCTAAACATTTCAC |
| [11,] | hsa-miR-20b_st | -0.31 | CTACCTGCACTATGAGCACTTTG |
| [12,] | hsa-miR-215_st | -0.3 | GTCTGTCAATTCATAGGTCAT |
| [13,] | hsa-miR-25_st | -0.39 | TCAGACCGAGACAAGTGCAATG |
| [14,] | hsa-miR-30b_st | -0.32 | AGCTGAGTGTAGGATGTTTACA |
| [15,] | hsa-miR-30d_st | -0.36 | CTTCCAGTCGGGGATGTTTACA |
| [16,] | hsa-miR-362-5p_st | -0.36 | ACTCACACCTAGGTTCCAAGGATT |
| [17,] | hsa-miR-378-star_st | -0.31 | ACACAGGACCTGGAGTCAGGAG |
| [18,] | hsa-miR-421_st | -0.32 | GCGCCCAATTAATGTCTGTTGAT |
| [19,] | hsa-miR-425_st | -0.35 | TCAACGGGAGTGATCGTGTCATT |
| [20,] | hsa-miR-532-5p_st | -0.33 | ACGGTCCTACACTCAAGGCATG |
| [21,] | hsa-miR-93_st | -0.34 | CTACCTGCACGAACAGCACTTTG |
| [22,] | hsa-miR-941_st | -0.34 | GCACATGTGCACACAGCCGGGTG |

Table 83 Methyl-GAG (methyl glyoxal bis amidinohydrazone dihydrochloride) microRNA biomarkers.

| | Medianprobe | Corr | Sequence |
|---|---|---|---|
| [1,] | hsa-let-7e_st | -0.39 | AACTATACAACCTCCTACCTCA |
| [2,] | hsa-let-7i_st | -0.39 | AACAGCACAAACTACTACCTCA |
| [3,] | hsa-miR-10a_st | -0.3 | CACAAATTCGGATCTACAGGGTA |
| [4,] | hsa-miR-10b_st | -0.41 | CACAAATTCGGTTCTACAGGGTA |
| [5,] | hsa-miR-125a-5p_st | -0.37 | TCACAGGTTAAAGGGTCTCAGGGA |
| [6,] | hsa-miR-1301_st | -0.35 | GAAGTCACTCCCAGGCAGCTGCAA |
| [7,] | hsa-miR-140-5p_st | -0.32 | CTACCATAGGGTAAAACCACTG |
| [8,] | hsa-miR-99b_st | -0.34 | CGCAAGGTCGGTTCTACGGGTG |

Table 84 Belinostat (PXD101) microRNA biomarkers.

|  | Medianprobe | Corr | Sequence |
|---|---|---|---|
| [1,] | 14qII-14_st | -0.32 | TCACCCAACACTCATACGCCGGCAG |
| [2,] | 14qII-1_x_st | -0.32 | GGACCTCAGACTTCCAGACCTGTAT |
| [3,] | hsa-let-7b_st | -0.35 | AACCACACAACCTACTACCTCA |
| [4,] | hsa-let-7c_st | -0.4 | AACCATACAACCTACTACCTCA |
| [5,] | hsa-let-7e_st | -0.45 | AACTATACAACCTCCTACCTCA |
| [6,] | hsa-miR-10a_st | -0.4 | CACAAATTCGGATCTACAGGGTA |
| [7,] | hsa-miR-10b_st | -0.31 | CACAAATTCGGTTCTACAGGGTA |
| [8,] | hsa-miR-125a-3p_st | -0.38 | GGCTCCCAAGAACCTCACCTGT |
| [9,] | hsa-miR-125a-5p_st | -0.43 | TCACAGGTTAAAGGGTCTCAGGGA |
| [10,] | hsa-miR-125b-1-star_st | -0.33 | AGCTCCCAAGAGCCTAACCCGT |
| [11,] | hsa-miR-125b_st | -0.31 | TCACAAGTTAGGGTCTCAGGGA |
| [12,] | hsa-miR-1287_st | -0.31 | GACTCGAACCACTGATCCAGCA |
| [13,] | hsa-miR-134_st | -0.34 | CCCCTCTGGTCAACCAGTCACA |
| [14,] | hsa-miR-151-3p_st | -0.37 | CCTCAAGGAGCTTCAGTCTAG |
| [15,] | hsa-miR-151-5p_st | -0.42 | ACTAGACTGTGAGCTCCTCGA |
| [16,] | hsa-miR-22_st | -0.36 | ACAGTTCTTCAACTGGCAGCTT |
| [17,] | hsa-miR-23a-star_st | -0.33 | AAATCCCATCCCCAGGAACCCC |
| [18,] | hsa-miR-23a_st | -0.34 | GGAAATCCCTGGCAATGTGAT |
| [19,] | hsa-miR-23b_st | -0.31 | GGTAATCCCTGGCAATGTGAT |
| [20,] | hsa-miR-24-2-star_st | -0.35 | CTGTGTTTCAGCTCAGTAGGCA |
| [21,] | hsa-miR-24_st | -0.37 | CTGTTCCTGCTGAACTGAGCCA |
| [22,] | hsa-miR-27a_st | -0.31 | GCGGAACTTAGCCACTGTGAA |
| [23,] | hsa-miR-27b_st | -0.33 | GCAGAACTTAGCCACTGTGAA |
| [24,] | hsa-miR-28-3p_st | -0.39 | TCCAGGAGCTCACAATCTAGTG |
| [25,] | hsa-miR-28-5p_st | -0.35 | CTCAATAGACTGTGAGCTCCTT |
| [26,] | hsa-miR-299-5p_st | -0.32 | ATGTATGTGGGACGGTAAACCA |
| [27,] | hsa-miR-337-5p_st | -0.36 | AACTCCTGTATGAAGCCGTTC |
| [28,] | hsa-miR-370_st | -0.3 | ACCAGGTTCCACCCCAGCAGGC |
| [29,] | hsa-miR-376a_st | -0.38 | ACGTGGATTTTCCTCTATGAT |
| [30,] | hsa-miR-376c_st | -0.37 | ACGTGGAATTTCCTCTATGTT |
| [31,] | hsa-miR-379_st | -0.38 | CCTACGTTCCATAGTCTACCA |
| [32,] | hsa-miR-381_st | -0.35 | ACAGAGAGCTTGCCCTTGTATA |
| [33,] | hsa-miR-382_st | -0.37 | CGAATCCACCACGAACAACTTC |
| [34,] | hsa-miR-409-3pst | -0.38 | AGGGGTTCACCGAGCAACATTC |
| [35,] | hsa-miR-409-5p_st | -0.32 | ATGCAAAGTTGCTCGGGTAACCT |
| [36,] | hsa-miR-411_st | -0.31 | CGTACGCTATACGGTCTACTA |
| [37,] | hsa-miR-431_st | -0.31 | TGCATGACGGCCTGCAAGACA |
| [38,] | hsa-miR-452_st | -0.3 | TCAGTTCCTCTGCAAACAGTT |
| [39,] | hsa-miR-485-5p_st | -0.31 | GAATTCATCACGGCCAGCCTCT |
| [40,] | hsa-miR-487a_st | -0.32 | AACTGGATGTCCCTGTATGATT |
| [41,] | hsa-miR-487b_st | -0.32 | AAGTGGATGACCCTGTACGATT |
| [42,] | hsa-miR-494_st | -0.37 | GAGGTTTCCCGTGTATGTTTCA |
| [43,] | hsa-miR-495_st | -0.3 | AAGAAGTGCACCATGTTTGTTT |
| [44,] | hsa-miR-543_st | -0.34 | AAGAAGTGCACCGCGAATGTTT |
| [45,] | hsa-miR-654-5p_st | -0.31 | GCACATGTTCTGCGGCCCACCA |
| [46,] | hsa-miR-99b-star_st | -0.37 | CGGACCCACAGACACGAGCTTG |
| [47,] | hsa-miR-99b_st | -0.4 | CGCAACGTCGGTTCTACGGGTG |

### Table 85 5-Fluorouracil microRNA biomarkers.

|  | Medianprobe | Corr | Sequence |
|---|---|---|---|
| [1,] | hsa-miR-100_st | -0.31 | CACAAGTTCGGATCTACGGGTT |
| [2,] | hsa-miR-125b_st | -0.34 | TCACAAGTTAGGGTCTCAGGGA |
| [3,] | hsa-miR-130a_st | -0.39 | ATGCCCTTTTAACATTGCACTG |
| [4,] | hsa-miR-181a-2-star_st | -0.33 | GGTACAGTCAACGGTCAGTGGT |
| [5,] | hsa-miR-184_st | -0.34 | ACCCTTATCAGTTCTCCGTCCA |
| [6,] | hsa-miR-197_st | -0.34 | GCTGGGTGGAGAAGGTGGTGAA |
| [7,] | hsa-miR-221-star_st | -0.34 | AAATCTACATTGTATGCCAGGT |
| [8,] | hsa-miR-30a-star_st | -0.36 | GCTGCAAACATCCGACTGAAAG |
| [9,] | hsa-miR-30a_st | -0.34 | CTTCCAGTCGAGGATGTTTACA |
| [10,] | hsa-miR-30c-2-star_st | -0.33 | AGAGTAAACAGCCTTCTCCCAG |
| [11,] | hsa-miR-525-5p_st | -0.32 | AGAAAGTGCATCCCTCTGGAG |
| [12,] | hsa-miR-584_st | -0.32 | CTCAGTCCCAGGCAAACCATAA |

### Table 86 Radiation microRNA biomarkers.

|  | Medianprobe | Corr | Sequence |
|---|---|---|---|
| [1,] | hsa-miR-1207-5p_st | -0.31 | CCCCTCCCAGCCTCCCTGCCA |
| [2,] | hsa-miR-625_st | -0.31 | GGACTATAGAACTTTCCCCCT |
| [3,] | hsa-miR-766_st | -0.33 | GCTGAGGCTGTGGGGCTGGAGT |

### Table 87 5-Aza-2'-deoxycytidine(decitabine) microRNA biomarkers.

|  | Medianprobe | Corr | Sequence |
|---|---|---|---|
| [1,] | hsa-let-7e_st | -0.34 | AACTATACAACCTCCTACCTCA |
| [2,] | hsa-miR-151-3p_st | -0.34 | CCTCAAGGAGCTTCAGTCTAG |
| [3,] | hsa-miR-151-5p_st | -0.35 | ACTAGACTGTGAGCTCCTCGA |
| [4,] | hsa-miR-21_st | -0.39 | TCAACATCAGTCTGATAAGCTA |
| [5,] | hsa-miR-22_st | -0.31 | ACAGTTCTTCAACTGGCAGCTT |
| [6,] | hsa-miR-30a-star_st | -0.36 | GCTGCAAACATCCGACTGAAAG |
| [7,] | hsa-miR-30a_st | -0.42 | CTTCCAGTCGAGGATGTTTACA |
| [8,] | hsa-miR-30c-2-star_st | -0.32 | AGAGTAAACAGCCTTCTCCCAG |
| [9,] | hsa-miR-99b_st | -0.31 | CGCAAGGTCGGTTCTACGGGTG |

### Table 88 Idarubicin microRNA biomarkers.

|  | Medianprobe | Corr | Sequence |
|---|---|---|---|
| [1,] | hsa-let-7e_st | -0.31 | AACTATACAACCTCCTACCTCA |
| [2,] | hsa-miR-125a-5p_st | -0.31 | TCACAGGTTAAAGGGTCTCAGGGA |
| [3,] | hsa-miR-151-3p_st | -0.4 | CCTCAAGGAGCTTCAGTCTAG |
| [4,] | hsa-miR-151-5p_st | -0.44 | ACTAGACTGTGAGCTCCTCGA |
| [5,] | hsa-miR-221-star_st | -0.35 | AAATCTACATTGTATGCCAGGT |
| [6,] | hsa-miR-23a_st | -0.4 | GGAAATCCCTGGCAATGTGAT |
| [7,] | hsa-miR-24-2-star_st | -0.35 | CTGTGTTTCAGCTCAGTAGGCA |
| [8,] | hsa-miR-24_st | -0.35 | CTGTTCCTGCTGAACTGAGCCA |
| [9,] | hsa-miR-30b_st | -0.31 | AGCTGAGTGTAGGATGTTTACA |
| [10,] | hsa-miR-30d_st | -0.38 | CTTCCAGTCGGGGATGTTTACA |
| [11,] | hsa-miR-526a_st | -0.3 | CAGAAAGTGCTTCCCTCTAGAG |
| [12,] | hsa-miR-584_st | -0.43 | CTCAGTCCCAGGCAAACCATAA |
| [13,] | hsa-miR-99b_st | -0.33 | CGCAAGGTCGGTTCTACGGGTG |

Table 89 Melphalan microRNA biomarkers.

| | Medianprobe | Corr | Sequence |
|---|---|---|---|
| [1,] | hsa-let-7e_st | -0.5 | AACTATACAACCTCCTACCTCA |
| [2,] | hsa-miR-10a_st | -0.35 | CACAAATTCGGATCTACAGGGTA |
| [3,] | hsa-miR-125a-3p_st | -0.33 | GGCTCCCAAGAACCTCACCTGT |
| [4,] | hsa-miR-125a-5p_st | -0.5 | TCACAGGTTAAAGGGTCTCAGGGA |
| [5,] | hsa-miR-151-3p_st | -0.58 | CCTCAAGGAGCTTCAGTCTAG |
| [6,] | hsa-miR-151-5p_st | -0.65 | ACTAGACTGTGAGCTCCTCGA |
| [7,] | hsa-miR-182_st | -0.34 | AGTGTGAGTTCTACCATTGCCAAA |
| [8,] | hsa-miR-183-star_st | -0.32 | TTATGGCCCTTCGGTAATTCAC |
| [9,] | hsa-miR-183_st | -0.35 | AGTGAATTCTACCAGTGCCATA |
| [10,] | hsa-miR-193b_st | -0.45 | AGCGGGACTTTGAGGGCCAGTT |
| [11,] | hsa-miR-200b-star_st | -0.31 | TCCAATGCTGCCCAGTAAGATG |
| [12,] | hsa-miR-200b_st | -0.31 | TCATCATTACCAGGCAGTATTA |
| [13,] | hsa-miR-21_st | -0.33 | TCAACATCAGTCTGATAAGCTA |
| [14,] | hsa-miR-221-star_st | -0.35 | AAATCTACATTGTATGCCAGGT |
| [15,] | hsa-miR-22_st | -0.39 | ACAGTTCTTCAACTGGCAGCTT |
| [16,] | hsa-miR-23a_st | -0.4 | GGAAATCCCTGGCAATGTGAT |
| [17,] | hsa-miR-23bst | -0.38 | GGTAATCCCTGGCAATGTGAT |
| [18,] | hsa-miR-24_st | -0.41 | CTGTTCCTGCTGAACTGAGCCA |
| [19,] | hsa-miR-27b_st | -0.31 | GCAGAACTTAGCCACTGTGAA |
| [20,J | hsa-miR-29b_st | -0.3 | AACACTGATTTCAAATGGTGCTA |
| [21,] | hsa-miR-30a_st | -0.31 | CTTCCAGTCGAGGATGTTTACA |
| [22,] | hsa-miR-30d_st | -0.37 | CTTCCAGTCGGGGATGTTTACA |
| [23,] | hsa-miR-31-star_st | -0.34 | ATGGCAATATGTTGGCATAGCA |
| [24,] | hsa-miR-31_st | -0.34 | AGCTATGCCAGCATCTTGCCT |
| [25,] | hsa-miR-320d_st | -0.31 | TCCTCTCAACCCAGCTTTT |
| [26,] | hsa-miR-584_st | -0.33 | CTCAGTCCCAGGCAAACCATAA |
| [27,] | hsa-miR-99b-star_st | -0.38 | CGGACCCACAGACACGAGCTTG |
| [28,] | hsa-miR-99b_st | -0.5 | CGCAAGGTCGGTTCTACGGGTG |

Table 90 IL4-PR38 fusion protein microRNA biomarkers.

| | Medianprobe | Corr | Sequence |
|---|---|---|---|
| [1,] | hsa-let-7a_st | -0.31 | AACTATACAACCTACTACCTCA |
| [2,] | hsa-miR-100_st | -0.38 | CACAAGTTCGGATCTACGGGTT |
| [3,] | hsa-miR-146a_st | -0.3 | AACCCATGGAATTCAGTTCTCA |

Table 91 Valproic acid (VPA) microRNA biomarkers.

| | Medianprobe | Corr | Sequence |
|---|---|---|---|
| [1,] | HBII-85-26 _st | -0.3 | AGATCCATTTTTTTTATAGTCATCA |
| [2,] | HBII-85-6_x_st | -0.28 | GTTTTTTTTGGAGGACTCATCATCG |
| [3,] | hsa-miR-491-5p_st | -0.28 | CCTCATGGAAGGGTTCCCCACT |
| [4,] | hsa-miR-589-star_ st | -0.32 | TCTGGGAACCGGCATTTGTTCTGA |
| [5,] | hsa-miR-625_ st | -0.27 | GGACTATAGAACTTTCCCCCT |
| [6,] | hsa-miR-744_ st | -0.33 | TGCTGTTAGCCCTAGCCCCGCA |

Table 92 All-trans retinoic acid (ATRA) microRNA biomarkers.

| | Medianprobe | Corr | Sequence |
|---|---|---|---|
| [1,] | hsa-let-7b_st | -0.3 | AACCACACAACCTACTACCTCA |
| [2,] | hsa-let-7e_st | -0.34 | AACTATACAACCTCCTACCTCA |
| [3,] | hsa-let-7i_st | -0.32 | AACAGCACAAACTACTACCTCA |
| [4,] | hsa-miR-10a_st | -0.25 | CACAAATTCGGATCTACAGGGTA |
| [5,] | hsa-miR-10b_st | -0.28 | CACAAATTCGGTTCTACAGGGTA |
| [6,] | hsa-miR-125a-3p_st | -0.26 | GGCTCCCAAGAACCTCACCTGT |
| [7,] | hsa-miR-125a-5p_st | -0.31 | TCACAGGTTAAAGGGTCTCAGGGA |
| [8,] | hsa-miR-151-3p_st | -0.37 | CCTCAAGGAGCTTCAGTCTAG |
| [9,] | hsa-miR-151-5p_st | -0.39 | ACTAGACTGTGAGCTCCTCGA |
| [10,] | hsa-miR-181a-2-star_st | -0.33 | GGTACAGTCAACGGTCAGTGGT |
| [11,] | hsa-miR-21-star_st | -0.33 | ACAGCCCATCGACTGGTGTTG |
| [12,] | hsa-miR-21_st | -0.38 | TCAACATCAGTCTGATAAGCTA |
| [13,] | hsa-miR-221_st | -0.6 | GAAACCCAGCAGACAATGTAGCT |
| [14,] | hsa-miR-222_st | -0.56 | ACCCAGTAGCCAGATGTAGCT |
| [15,] | hsa-miR-22_st | -0.37 | ACAGTTCTTCAACTGGCAGCTT |
| [16,] | hsa-miR-28-3p_st | -0.46 | TCCAGGAGCTCACAATCTAGTG |
| [17,] | hsa-miR-28-5p_st | -0.43 | CTCAATAGACTGTGAGCTCCTT |
| [18,] | hsa-miR-29b-1-star_st | -0.27 | TCTAAACCACCATATGAAACCAGC |
| [19,] | hsa-miR-30a-star_st | -0.32 | GCTGCAAACATCCGACTGAAAG |
| [20,] | hsa-miR-30a_st | -0.36 | CTTCCAGTCGAGGATGTTTACA |
| [21,] | hsa-miR-30c-2-star_st | -0.33 | AGAGTAAACAGCCTTCTCCCAG |
| [22,] | hsa-miR-455-3p_st | -0.32 | GTGTATATGCCCATGGACTGC |
| [23,] | hsa-miR-99b-star_st | -0.32 | CGGACCCACAGACACGAGCTTG |
| [24,] | hsa-miR-99b_st | -0.3 | CGCAAGGTCGGTTCTACGGGTG |

Table 93 Cytoxan microRNA biomarkers.

| | Medianprobe | Corr | Sequence |
|---|---|---|---|
| [1,] | hsa-let-7b_st | -0.26 | AACCACACAACCTACTACCTCA |
| [2,] | hsa-let-7d_st | -0.36 | AACTATGCAACCTACTACCTCT |
| [3,] | hsa-let-7g_st | -0.29 | AACTGTACAAACTACTACCTCA |
| [4,] | hsa-let-7i_st | -0.46 | AACAGCACAAACTACTACCTCA |
| [5,] | hsa-miR-100_st | -0.29 | CACAAGTTCGGATCTACGGGTT |
| [6,] | hsa-miR-138_st | -0.31 | CGGCCTGATTCACAACACCAGCT |
| [7,] | hsa-miR-221_st | -0.62 | GAAACCCAGCAGACAATGTAGCT |
| [8,] | hsa-miR-222_st | -0.65 | ACCCAGTAGCCAGATGTAGCT |
| [9,] | hsa-miR-27a-star_st | -0.28 | TGCTCACAAGCAGCTAAGCCCT |
| [10,] | hsa-miR-29a_st | -0.34 | TAACCGATTTCAGATGGTGCTA |
| [11,] | hsa-miR-31_st | -0.29 | AGCTATGCCAGCATCTTGCCT |
| [12,] | hsa-miR-503_st | -0.29 | CTGCAGAACTGTTCCCGCTGCTA |
| [13,] | hsa-miR-625_st | -0.26 | GGACTATAGAACTTTCCCCCT |

Table 94 Topotecan (Hycamtin) microRNA biomarkers.

| | Medianprobe | Corr | Sequence |
|---|---|---|---|
| [1,] | HBII-289_st | -0.27 | AGGTCAGACTAGTGGTTCGCTTCAG |
| [2,] | hsa-miR-141_st | -0.29 | CCATCTTTACCAGACAGTGTTA |
| [3,] | hsa-miR-373_st | -0.28 | ACACCCCAAAATCGAAGCACTTC |
| [4,] | hsa-miR-512-3p_st | -0.32 | GACCTCAGCTATGACAGCACTT |
| [5,] | hsa-miR-517a_st | -0.32 | ACACTCTAAAGGGATGCACGAT |

(continued)

| | Medianprobe | Corr | Sequence |
|---|---|---|---|
| [6,] | hsa-miR-525-5p_st | -0.29 | AGAAAGTGCATCCCTCTGGAG |
| [7,] | hsa-miR-584_st | -0.33 | CTCAGTCCCAGGCAAACCATAA |
| [8,] | hsa-miR-934_st | -0.31 | CCAGTGTCTCCAGTAGTAGACA |

Table 95 Suberoylanilide hydroxamic acid (SAHA, vorinostat, Zolinza) microRNA biomarkers.

| | Medianprobe | Corr | Sequence |
|---|---|---|---|
| [1,] | 14qII-14_st | -0.32 | ATCACCCAACACTCATACGCCGGCA |
| [2,] | 14qII-14_x_st | -0.29 | CCAACACTCATACCCCGGCAGTTGT |
| [3,] | 14qII-1_x_st | -0.28 | TTCACCGTCAATGACTCATACGCCA |
| [4,] | 14qII-26_st | -0.35 | ATGTGACTCATACTCCACCAGTGCT |
| [5,] | 14qII-26_x_st | -0.28 | ATACTCCACCAGTGCTCATCATCGA |
| [6,] | hsa-let-7e_st | -0.3 | AACTATACAACCTCCTACCTCA |
| [7,] | hsa-miR-125a-3p_st | -0.34 | GGCTCCCAAGAACCTCACCTGT |
| [8,] | hsa-miR-125a-5p_st | -0.29 | TCACAGGTTAAAGGGTCTCAGGGA |
| [9,] | hsa-miR-125b-1-star_st | -0.33 | AGCTCCCAAGAGCCTAACCCGT |
| [10,] | hsa-miR-127-3p_st | -0.31 | AGCCAAGCTCAGACGGATCCGA |
| [11,] | hsa-miR-127-5p_st | -0.3 | ATCAGAGCCCTCTGAGCTTCAG |
| [12,] | hsa-miR-134_st | -0.35 | CCCCTCTGGTCAACCAGTCACA |
| [13,] | hsa-miR-143-star_st | -0.26 | ACCAGAGATGCAGCACTGCACC |
| [14,] | hsa-miR-143_st | -0.32 | GAGCTACAGTGCTTCATCTCA |
| [15,] | hsa-miR-193a-3p_st | -0.27 | ACTGGGACTTTGTAGGCCAGTT |
| [16,] | hsa-miR-193a-5p_st | -0.35 | TCATCTCGCCCGCAAAGACCCA |
| [17,] | hsa-miR-193b-star_st | -0.26 | TCATCTCGCCCTCAAAACCCCG |
| [18,] | hsa-miR-199a-5p_st | -0.29 | GAACAGGTAGTCTGAACACTGGG |
| [19,] | hsa-miR-21-star_st | -0.29 | ACAGCCCATCGACTGGTGTTG |
| [20,] | hsa-miR-210_st | -0.46 | TCAGCCGCTGTCACACGCACAG |
| [21,] | hsa-miR-214_st | -0.3 | ACTGCCTGTCTGTGCCTGCTGT |
| [22,] | hsa-miR-22_st | -0.39 | ACAGTTCTTCAACTGGCAGCTT |
| [23,] | hsa-miR-23a-star_st | -0.41 | AAATCCCATCCCCAGGAACCCC |
| [24,] | hsa-miR-23b-star_st | -0.3 | AAATCAGCATGCCAGGAACCCA |
| [25,] | hsa-miR-23b_st | -0.32 | GGTAATCCCTGGCAATGTGAT |
| [26,] | hsa-miR-24-2-star_st | -0.26 | CTGTGTTTCAGCTCAGTAGGCA |
| [27,] | hsa-miR-24_st | -0.33 | CTGTTCCTGCTGAACTGAGCCA |
| [28,] | hsa-miR-27a-star_st | -0.39 | TGCTCACAAGCAGCTAAGCCCT |
| [29,] | hsa-miR-27a_st | -0.37 | GCGGAACTTAGCCACTGTGAA |
| [30,] | hsa-miR-27b_st | -0.31 | GCAGAACTTAGCCACTGTGAA |
| [31,] | hsa-miR-28-3p_st | -0.25 | TCCAGGAGCTCACAATCTAGTG |
| [32,] | hsa-miR-299-3p_st | -0.32 | AAGCGGTTTACCATCCCACATA |
| [33,] | hsa-miR-337-5p_st | -0.32 | AACTCCTGTATGAAGCCGTTC |
| [34,] | hsa-miR-339-3p_st | -0.36 | CGGCTCTGTCGTCGAGGCGCTCA |
| [35,] | hsa-miR-339-5p_st | -0.34 | CGTGAGCTCCTGGAGGACAGGGA |
| [36,] | hsa-miR-370_st | -0.3 | ACCAGGTTCCACCCCAGCAGGC |
| [37,] | hsa-miR-376c_st | -0.28 | ACGTGGAATTTCCTCTATGTT |
| [38,] | hsa-miR-377-star_st | -0.3 | GAATTCACCAAGGGCAACCTCT |
| [39,] | hsa-miR-379_st | -0.29 | CCTACGTTCCATAGTCTACCA |
| [40,] | hsa-miR-381_st | -0.3 | ACAGAGAGCTTGCCCTTGTATA |
| [41,] | hsa-miR-409-3p_st | -0.32 | AGGGGTTCACCGAGCAACATTC |
| [42,] | hsa-miR-409-5p_st | -0.3 | ATGCAAAGTTGCTCGGGTAACCT |
| [43,] | hsa-miR-410_st | -0.26 | ACAGGCCATCTGTGTTATATT |

(continued)

| | Medianprobe | Corr | Sequence |
|---|---|---|---|
| [44,] | hsa-miR-411_st | -0.3 | CGTACGCTATACGGTCTACTA |
| [45,] | hsa-miR-431_st | -0.26 | TGCATGACGGCCTGCAAGACA |
| [46,] | hsa-miR-455-3p_st | -0.25 | GTGTATATGCCCATGGACTGC |
| [47,] | hsa-miR-485-5p_st | -0.28 | GAATTCATCACGGCCAGCCTCT |
| [48,] | hsa-miR-487a_st | -0.29 | AACTGGATGTCCCTGTATGATT |
| [49,] | hsa-miR-487b_st | -0.31 | AAGTGGATGACCCTGTACGATT |
| [50,] | hsa-miR-491-5p_st | -0.34 | CCTCATGGAAGGGTTCCCCACT |
| [51,] | hsa-miR-493_st | -0.28 | CCTGGCACACAGTAGACCTTCA |
| [52,] | hsa-miR-494_st | -0.38 | GAGGTTTCCCGTGTATGTTTCA |
| [53,] | hsa-miR-542-5p_st | -0.27 | TCTCGTGACATGATGATCCCCGA |
| [54,] | hsa-miR-744_st | -0.39 | TGCTGTTAGCCCTAGCCCCGCA |
| [55,] | hsa-miR-758_st | -0.25 | GGTTAGTGGACCAGGTCACAAA |
| [56,] | hsa-miR-935_st | -0.26 | GCGGTAGCGGAAGCGGTAACTGG |
| [57,] | hsa-miR-99b-star_st | -0.41 | CGGACCCACAGACACGAGCTTG |
| [58,] | hsa-miR-99b_st | -0.34 | CGCAAGGTCGGTTCTACGGGTG |

Table 96 Depsipeptide (FR901228) microRNA biomarkers.

| | Medianprobe | Corr | Sequence |
|---|---|---|---|
| [1,] | HBII-85-11_st | -0.26 | AAGGAACCCACGTATGGAAGTCATC |
| [2,] | HBII-85-1_x_st | -0.27 | AGCTTTTCCAAGGAATGTTTTTATA |
| [3,] | HBII-85-23_x_st | -0.25 | ATGCATGTATTGAGGTCATCATCGA |
| [4,] | HBII-85-29_st | -0.33 | TTGTTCAGATTTCCAAGGTTTCCAT |
| [5,] | HBTT-85-29x_st | -0.25 | TTGTTCAGATTTCCAAGGTTTCCAT |
| [6,] | U28_st | -0.27 | TGCCATCAGAACTCTAACATGCTAT |
| [7,] | hsa-miR-155_st | -0.29 | ACCCCTATCACGATTAGCATTAA |
| [8,] | hsa-miR-196b_st | -0.36 | CCCAACAACAGGAAACTACCTA |
| [9,] | hsa-miR-200a-star_st | -0.25 | TCCAGCACTGTCCGGTAAGATG |
| [10,] | hsa-miR-200a_st | -0.31 | ACATCGTTACCAGACAGTGTTA |
| [11,] | hsa-miR-200b-star_st | -0.3 | TCCAATGCTGCCCAGTAAGATG |
| [12,] | hsa-miR-200b_st | -0.26 | TCATCATTACCAGGCAGTATTA |
| [13,] | hsa-miR-203_st | -0.25 | CTAGTGGTCCTAAACATTTCAC |
| [14,] | hsa-miR-29b_st | -0.35 | AACACTGATTTCAAATGGTGCTA |
| [15,] | hsa-miR-30c_st | -0.31 | GCTGAGAGTGTAGGATGTTTACA |
| [16,] | hsa-miR-371-3p_st | -0.31 | ACACTCAAAAGATGGCGGCACTT |
| [17,] | hsa-miR-371-5p_st | -0.38 | AGTGCCCCCACAGTTTGAGT |
| [18,] | hsa-miR-372_st | -0.39 | ACGCTCAAATGTCGCAGCACTTT |
| [19,] | hsa-miR-373_st | -0.32 | ACACCCCAAAATCGAAGCACTTC |
| [20,] | hsa-miR-516a-5p_st | -0.46 | GAAAGTGCTTCTTTCCTCGAGAA |
| [21,] | hsa-miR-518d-5p_st | -0.27 | CAGAAAGTGCTTCCCTCTAGAG |
| [22,] | hsa-miR-518e-star_st | -0.28 | CAGAAAGCGCTTCCCTCTAGAG |
| [23,] | hsa-miR-519a-star_st | -0.3 | CAGAAAGCGCTTCCCTCTAGAG |
| [24,] | hsa-miR-519a_st | -0.25 | ACACTCTAAAAGGATGCACTTT |
| [25,] | hsa-miR-519b-5p_st | -0.27 | CAGAAAGCGCTTCCCTCTAGAG |
| [26,] | hsa-miR-519c-5p_st | -0.27 | CAGAAAGCGCTTCCCTCTAGAG |
| [27,] | hsa-miR-523-star_st | -0.25 | CAGAAAGCGCTTCCCTCTAGAG |
| [28,] | hsa-miR-886-3p_st | -0.26 | AAGGGTCAGTAAGCACCCGCG |

Table 97 Bortezomib microRNA biomarkers.

| | Medianprobe | Corr | Sequence |
|---|---|---|---|
| [1,] | HBII-438A_s_st | -0.28 | GAATGAGTCCCTCAGCATCCTCAGA |
| [2,] | HBII-52-32_x_st | -0.39 | GGGCAATATCAGGTTCTCATCATTG |
| [3,] | HBII-85-1_x_st | -0.26 | AGCTTTTCCAAGGAATGTTTTTATA |
| [4,] | HBII-85-23_x_st | -0.28 | AATGCATGTATTGAGGTCATCATCG |
| [5,] | hsa-let-7e_st | -0.3 | AACTATACAACCTCCTACCTCA |
| [6,] | hsa-miR-125a-3p_st | -0.29 | GGCTCCCAAGAACCTCACCTGT |
| [7,] | hsa-miR-125a-5p_st | -0.31 | TCACAGGTTAAAGGGTCTCAGGGA |
| [8,] | hsa-miR-149_st | -0.28 | GGGAGTGAAGACACGGAGCCAGA |
| [9,] | hsa-miR-151-3p_st | -0.29 | CCTCAAGGAGCTTCAGTCTAG |
| [10,] | hsa-miR-151-5p_st | -0.27 | ACTAGACTGTGAGCTCCTCGA |
| [11,] | hsa-miR-193b_st | -0.28 | AGCGGGACTTTGAGGGCCAGTT |
| [12,] | hsa-miR-21-star_st | -0.29 | ACAGCCCATCGACTGGTGTTG |
| [13,] | hsa-miR-21_st | -0.35 | TCAACATCAGTCTGATAAGCTA |
| [14,] | hsa-miR-22_st | -0.26 | ACAGTTCTTCAACTGGCAGCTT |
| [15,] | hsa-miR-23a-star_st | -0.4 | AAATCCCATCCCCAGGAACCCC |
| [16,] | hsa-miR-23a_st | -0.3 | GGAAATCCCTGGCAATGTGAT |
| [17,] | hsa-miR-24-2-star_st | -0.3 | CTGTGTTTCAGCTCAGTAGGCA |
| [18,] | hsa-miR-24_st | -0.29 | CTGTTCCTGCTGAACTGAGCCA |
| [19,] | hsa-miR-27a-star_st | -0.36 | TGCTCACAAGCAGCTAAGCCCT |
| [20,] | hsa-miR-27a_st | -0.26 | GCGGAACTTAGCCACTGTGAA |
| [21,] | hsa-miR-30a-star_st | -0.35 | GCTGCAAACATCCGACTGAAAG |
| [22,] | hsa-miR-30a_st | -0.34 | CTTCCAGTCGAGGATGTTTACA |
| [23,] | hsa-miR-30c-2-star_st | -0.3 | AGAGTAAACAGCCTTCTCCCAG |
| [24,] | hsa-miR-30c_st | -0.28 | GCTGAGAGTGTAGGATGTTTACA |
| [25,] | hsa-miR-31-star_st | -0.27 | ATGGCAATATGTTGGCATAGCA |
| [26,] | hsa-miR-516a-5p_st | -0.35 | GAAAGTGCTTCTTTCCTCGAGAA |
| [27,] | hsa-miR-518d-5p_st | -0.27 | CAGAAAGTGCTTCCCTCTAGAG |
| [28,] | hsa-miR-518e-star_st | -0.26 | CAGAAAGCGCTTCCCTCTAGAG |
| [29,] | hsa-miR-519a-star_st | -0.28 | CAGAAAGCGCTTCCCTCTAGAG |
| [30,] | hsa-miR-519c-5p_st | -0.32 | CAGAAAGCGCTTCCCTCTAGAG |
| [31,] | hsa-miR-522-starst | -0.29 | CAGAAAGCGCTTCCCTCTAGAG |
| [32,] | hsa-miR-9-star_st | -0.41 | ACTTTCGGTTATCTAGCTTTAT |
| [33,] | hsa-miR-935_st | -0.3 | GCGGTAGCGGAAGCGGTAACTGG |
| [34,] | hsa-miR-98_st | -0.25 | AACAATACAACTTACTACCTCA |
| [35,] | hsa-miR-99b-star_st | -0.26 | CGGACCCACAGACACGAGCTTG |
| [36,] | hsa-miR-99b_st | -0.27 | CGCAAGGTCGGTTCTACGGGTG |

Table 98 Leukeran microRNA biomarkers.

| | Medianprobe | Corr | Sequence |
|---|---|---|---|
| [1,] | hsa-let-7e_st | -0.42 | AACTATACAACCTCCTACCTCA |
| [2,] | hsa-miR-10a_st | -0.36 | CACAAATTCGGATCTACAGGGTA |
| [3,] | hsa-miR-125a-3p_st | -0.25 | GGCTCCCAAGAACCTCACCTGT |
| [4,] | hsa-miR-125a-5p_st | -0.43 | TCACAGGTTAAAGGGTCTCAGGGA |
| [5,] | hsa-miR-1287_st | -0.26 | GACTCGAACCACTGATCCAGCA |
| [6,] | hsa-miR-141_st | -0.31 | CCATCTTTACCAGACAGTGTTA |
| [7,] | hsa-miR-151-3p_st | -0.53 | CCTCAAGGAGCTTCAGTCTAG |
| [8,] | hsa-miR-151-5pst | -0.58 | ACTAGACTGTGAGCTCCTCGA |
| [9,] | hsa-miR-182_st | -0.26 | AGTGTGAGTTCTACCATTGCCAAA |
| [10,] | hsa-miR-183-star_st | -0.27 | TTATGGCCCTTCGGTAATTCAC |

(continued)

| | Medianprobe | Corr | Sequence |
|---|---|---|---|
| [11,] | hsa-miR-183_st | -0.31 | AGTGAATTCTACCAGTGCCATA |
| [12,] | hsa-miR-192_st | -0.29 | GGCTGTCAATTCATAGGTCAG |
| [13,] | hsa-miR-193b_st | -0.37 | AGCGGGACTTTGAGGGCCAGTT |
| [14,] | hsa-miR-194_st | -0.29 | TCCACATGGAGTTGCTGTTACA |
| [15,] | hsa-miR-200b-star_st | -0.27 | TCCAATGCTGCCCAGTAAGATG |
| [16,] | hsa-miR-200b_st | -0.29 | TCATCATTACCAGGCAGTATTA |
| [17,] | hsa-miR-200c_st | -0.29 | TCCATCATTACCCGGCAGTATTA |
| [18,] | hsa-miR-203_st | -0.3 | CTAGTGGTCCTAAACATTTCAC |
| [19,] | hsa-miR-21_st | -0.28 | TCAACATCAGTCTGATAAGCTA |
| [20,] | hsa-miR-221-star_st | -0.29 | AAATCTACATTGTATGCCAGGT |
| [21,] | hsa-miR-22_st | -0.32 | ACAGTTCTTCAACTGGCAGCTT |
| [22,] | hsa-miR-23a_st | -0.36 | GGAAATCCCTGGCAATGTGAT |
| [23,] | hsa-miR-23b-star_st | -0.27 | AAATCAGCATGCCAGGAACCCA |
| [24,] | hsa-miR-23b_st | -0.36 | GGTAATCCCTGGCAATGTGAT |
| [25,] | hsa-miR-24_st | -0.36 | CTGTTCCTGCTGAACTGAGCCA |
| [26,] | hsa-miR-27b_st | -0.32 | GCAGAACTTAGCCACTGTGAA |
| [27,] | hsa-miR-29b_st | -0.28 | AACACTGATTTCAAATGGTGCTA |
| [28,] | hsa-miR-30b_st | -0.33 | AGCTGAGTGTAGGATGTTTACA |
| [29,] | hsa-miR-30d_st | -0.42 | CTTCCAGTCGGGGATGTTTACA |
| [30,] | hsa-miR-320d_st | -0.33 | TCCTCTCAACCCAGCTTTT |
| [31,] | hsa-miR-584_st | -0.38 | CTCAGTCCCAGGCAAACCATAA |
| [32,] | hsa-miR-99b-star_st | -0.29 | CGGACCCACAGACACGAGCTTG |
| [33,] | hsa-miR-99b_st | -0.42 | CGCAAGGTCGGTTCTACGGGTG |

Table 99 Fludarabine microRNA biomarkers.

| | Medianprobe | Corr | Sequence |
|---|---|---|---|
| [1,] | U91_s_st | -0.26 | ATACTCCATCACCTGGTTCCCAGAA |
| [2,] | hsa-miR-10a_st | -0.27 | CACAAATTCGGATCTACAGGGTA |
| [3,] | hsa-miR-125a-5p_st | -0.26 | TCACAGGTTAAAGGGTCTCAGGGA |
| [4,] | hsa-miR-134_st | -0.27 | CCCCTCTGGTCAACCAGTCACA |
| [5,] | hsa-miR-141-star_st | -0.27 | TCCAACACTGTACTGGAAGATG |
| [6,] | hsa-miR-141_st | -0.39 | CCATCTTTACCAGACAGTGTTA |
| [7,] | hsa-miR-151-5p_st | -0.31 | ACTAGACTGTGAGCTCCTCGA |
| [8,] | hsa-miR-192_st | -0.28 | GGCTGTCAATTCATAGGTCAG |
| [9,] | hsa-miR-193b_st | -0.26 | AGCGGGACTTTGAGGGCCAGTT |
| [10,] | hsa-miR-194_st | -0.28 | TCCACATGGAGTTGCTGTTACA |
| [11,] | hsa-miR-200a-star_st | -0.28 | TCCAGCACTGTCCGGTAAGATG |
| [12,] | hsa-miR-200a_st | -0.32 | ACATCGTTACCAGACAGTGTTA |
| [13,] | hsa-miR-200b-star_st | -0.28 | TCCAATGCTGCCCAGTAAGATG |
| [14,] | hsa-miR-200b_st | -0.28 | TCATCATTACCAGGCAGTATTA |
| [15,] | hsa-miR-200c_st | -0.35 | TCCATCATTACCCGGCAGTATTA |
| [16,] | hsa-miR-203_st | -0.25 | CTAGTGGTCCTAAACATTTCAC |
| [17,] | hsa-miR-205_st | -0.27 | CAGACTCCGGTGGAATGAAGGA |
| [18,] | hsa-miR-210_st | -0.4 | TCAGCCGCTGTCACACGCACAG |
| [19,] | hsa-miR-215_st | -0.3 | GTCTGTCAATTCATAGGTCAT |
| [20,] | hsa-miR-23b-star_st | -0.38 | AAATCAGCATGCCAGGAACCCA |
| [21,] | hsa-miR-23b_st | -0.44 | GGTAATCCCTGGCAATGTGAT |
| [22,] | hsa-miR-24_st | -0.3 | CTGTTCCTGCTGAACTGAGCCA |
| [23,] | hsa-miR-27b-star_st | -0.35 | GTTCACCAATCAGCTAAGCTCT |

(continued)

| | Medianprobe | Corr | Sequence |
|---|---|---|---|
| [24,] | hsa-miR-27b_st | -0.43 | GCAGAACTTAGCCACTGTGAA |
| [25,] | hsa-miR-299-5p_st | -0.26 | ATGTATGTGGGACGGTAAACCA |
| [26,] | hsa-miR-320a_st | -0.3 | TCGCCCTCTCAACCCAGCTTTT |
| [27,] | hsa-miR-320b_st | -0.3 | TTGCCCTCTCAACCCAGCTTTT |
| [28,] | hsa-miR-320cst | -0.28 | ACCCTCTCAACCCAGCTTTT |
| [29,] | hsa-miR-320d_st | -0.33 | TCCTCTCAACCCAGCTTTT |
| [30,] | hsa-miR-337-5p_st | -0.26 | AACTCCTGTATGAAGCCGTTC |
| [31,] | hsa-miR-339-3p_st | -0.31 | CGGCTCTGTCGTCGAGGCGCTCA |
| [32,] | hsa-miR-339-5p_st | -0.25 | CGTGAGCTCCTGGAGGACAGGGA |
| [33,] | hsa-miR-370_st | -0.26 | ACCAGGTTCCACCCCAGCAGGC |
| [34,] | hsa-miR-375_st | -0.26 | TCACGCGAGCCGAACGAACAAA |
| [35,] | hsa-miR-382_st | -0.29 | CGAATCCACCACGAACAACTTC |
| [36,] | hsa-miR-429_st | -0.28 | ACGGTTTTACCAGACAGTATTA |
| [37,] | hsa-miR-485-5p_st | -0.28 | GAATTCATCACGGCCAGCCTCT |
| [38,] | hsa-miR-487a_st | -0.25 | AACTGGATGTCCCTGTATGATT |
| [39,] | hsa-miR-494_st | -0.34 | GAGGTTTCCCGTGTATGTTTCA |
| [40,] | hsa-miR-99b-star_st | -0.3 | CGGACCCACAGACACGAGCTTG |
| [41,] | hsa-miR-99b_st | -0.32 | CGCAAGGTCGGTTCTACGGGTG |

Table 100 Vinblastine microRNA biomarkers.

| | Medianprobe | Corr | Sequence |
|---|---|---|---|
| [1,] | HBII-85-1_x_st | -0.26 | ACCTCAGTTCCGATGAGAATGACGT |
| [2,] | HBII-85-29_st | -0.26 | TTCAGATTTCCAAGGTTTCCATTTT |
| [3,] | hsa-miR-125a-5p_st | -0.28 | TCACAGGTTAAAGGGTCTCAGGGA |
| [4,] | hsa-miR-130a_st | -0.31 | ATGCCCTTTTAACATTGCACTG |
| [5,] | hsa-miR-139-5p_st | -0.36 | CTGGAGACACGTGCACTGTAGA |
| [6,] | hsa-miR-149_st | -0.29 | GGGAGTGAAGACACGGAGCCAGA |
| [7,] | hsa-miR-16_st | -0.27 | CGCCAATATTTACGTGCTGCTA |
| [8,] | hsa-miR-193b_st | -0.34 | AGCGGGACTTTGAGGGCCAGTT |
| [9,] | hsa-miR-196b_st | -0.27 | CCCAACAACAGGAAACTACCTA |
| [10,] | hsa-miR-200a_st | -0.28 | ACATCGTTACCAGACAGTGTTA |
| [11,] | hsa-miR-200b-star_st | -0.28 | TCCAATGCTGCCCAGTAAGATG |
| [12,] | hsa-miR-200b_st | -0.28 | TCATCATTACCAGGCAGTATTA |
| [13,] | hsa-miR-203_st | -0.33 | CTAGTGGTCCTAAACATTTCAC |
| [14,] | hsa-miR-22_st | -0.27 | ACAGTTCTTCAACTGGCAGCTT |
| [15,] | hsa-miR-26a_st | -0.27 | AGCCTATCCTGGATTACTTGAA |
| [16,] | hsa-miR-29b_st | -0.34 | AACACTGATTTCAAATGGTGCTA |
| [17,] | hsa-miR-30a-star_st | -0.38 | GCTGCAAACATCCGACTGAAAG |
| [18,] | hsa-miR-30a_st | -0.41 | CTTCCAGTCGAGGATGTTTACA |
| [19,] | hsa-miR-30c-2-star_st | -0.38 | AGAGTAAACAGCCTTCTCCCAG |
| [20,] | hsa-miR-30c_st | -0.5 | GCTGAGAGTGTAGGATGTTTACA |
| [21,] | hsa-miR-331-3p_st | -0.27 | TTCTAGGATAGGCCCAGGGGC |
| [22,] | hsa-miR-34a_st | -0.26 | ACAACCAGCTAAGACACTGCCA |
| [23,] | hsa-miR-34b-star_st | -0.29 | CAATCAGCTAATGACACTGCCTA |
| [24,] | hsa-miR-34c-3p_st | -0.25 | CCTGGCCGTGTGGTTAGTGATT |
| [25,] | hsa-miR-34c-5p_st | -0.34 | GCAATCAGCTAACTACACTGCCT |
| [26,] | hsa-miR-449a_st | -0.43 | ACCAGCTAACAATACACTGCCA |
| [27,] | hsa-miR-449b_st | -0.38 | GCCAGCTAACAATACACTGCCT |
| [28,] | hsa-miR-516a-5p_st | -0.28 | GAAAGTGCTTCTTTCCTCGAGAA |

(continued)

| | Medianprobe | Corr | Sequence |
|---|---|---|---|
| [29,] | hsa-miR-675_st | -0.26 | CACTGTGGGCCCTCTCCGCACCA |

Table 101 Busulfan microRNA biomarkers.

| | Medianprobe | Corr | Sequence |
|---|---|---|---|
| [1,] | hsa-let-7b_st | -0.27 | AACCACACAACCTACTACCTCA |
| [2,] | hsa-let-7e_st | -0.66 | AACTATACAACCTCCTACCTCA |
| [3,] | hsa-miR-10a_st | -0.26 | CACAAATTCGGATCTACAGGGTA |
| [4,] | hsa-miR-125a-3p_st | -0.49 | GGCTCCCAAGAACCTCACCTGT |
| [5,] | hsa-miR-125a-5p_st | -0.69 | TCACAGGTTAAAGGGTCTCAGGGA |
| [6,] | hsa-miR-149_st | -0.36 | GGGAGTGAAGACACGGAGCCAGA |
| [7,] | hsa-miR-151-3p_st | -0.44 | CCTCAAGGAGCTTCAGTCTAG |
| [8,] | hsa-miR-151-5p_st | -0.48 | ACTAGACTGTGAGCTCCTCGA |
| [9,] | hsa-miR-182_st | -0.27 | AGTGTGAGTTCTACCATTGCCAAA |
| [10,] | hsa-miR-183-star_st | -0.35 | TTATGGCCCTTCGGTAATTCAC |
| [11,] | hsa-miR-183_st | -0.31 | AGTGAATTCTACCAGTGCCATA |
| [12,] | hsa-miR-193b_st | -0.41 | AGCGGGACTTTGAGGGCCAGTT |
| [13,] | hsa-miR-203_st | -0.27 | CTAGTGGTCCTAAACATTTCAC |
| [14,] | hsa-miR-22_st | -0.31 | ACAGTTCTTCAACTGGCAGCTT |
| [15,] | hsa-miR-23a_st | -0.26 | GGAAATCCCTGGCAATGTGAT |
| [16,] | hsa-miR-23b_st | -0.38 | GGTAATCCCTGGCAATGTGAT |
| [17,] | hsa-miR-24-1-star_st | -0.25 | ACTGATATCAGCTCAGTAGGCA |
| [18,] | hsa-miR-24_st | -0.26 | CTGTTCCTGCTGAACTGAGCCA |
| [19,] | hsa-miR-27b_st | -0.36 | GCAGAACTTAGCCACTGTGAA |
| [20,] | hsa-miR-30b_st | -0.34 | AGCTGAGTGTAGGATGTTTACA |
| [21,] | hsa-miR-30d_st | -0.33 | CTTCCAGTCGGGGATGTTTACA |
| [22,] | hsa-miR-320d_st | -0.26 | TCCTCTCAACCCAGCTTTT |
| [23,] | hsa-miR-532-3p_st | -0.26 | TGCAAGCCTTGGGTGTGGGAGG |
| [24,] | hsa-miR-99b-star_st | -0.52 | CGGACCCACAGACACGAGCTTG |
| [25,] | hsa-miR-99b_st | -0.66 | CGCAAGGTCGGTTCTACGGGTG |

Table 102 Dacarbazinc microRNA biomarkers.

| | Medianprobe | Corr | Sequence |
|---|---|---|---|
| [1,] | hsa-let-7est | -0.34 | AACTATACAACCTCCTACCTCA |
| [2,] | hsa-miR-103_st | -0.3 | TCATAGCCCTGTACAATGCTGCT |
| [3,] | hsa-miR-107_st | -0.31 | TGATAGCCCTGTACAATGCTGCT |
| [4,] | hsa-miR-1180_st | -0.27 | ACACACCCACGCGAGCCGGAAA |
| [5,] | hsa-miR-1231_st | -0.27 | GCAGCTGTCCGCCCAGACAC |
| [6,] | hsa-miR-125a-3p_st | -0.26 | GGCTCCCAAGAACCTCACCTGT |
| [7,] | hsa-miR-125a-5p_st | -0.35 | TCACAGGTTAAAGGGTCTCAGGGA |
| [8,] | hsa-miR-1301_st | -0.3 | GAAGTCACTCCCAGGCAGCTGCAA |
| [9,] | hsa-miR-1303_st | -0.29 | AGAGCAAGACCCCGTCTCTAAA |
| [10,] | hsa-miR-132_st | -0.31 | CGACCATGGCTGTAGACTGTTA |
| [11,] | hsa-miR-141_st | -0.3 | CCATCTTTACCAGACAGTGTTA |
| [12,] | hsa-miR-151-3p_st | -0.44 | CCTCAAGGAGCTTCAGTCTAG |
| [13,] | hsa-miR-151-5p_st | -0.49 | ACTAGACTGTGAGCTCCTCGA |
| [14,] | hsa-miR-182_st | -0.26 | AGTGTGAGTTCTACCATTGCCAAA |
| [15,] | hsa-miR-183_st | -0.29 | AGTGAATTCTACCAGTGCCATA |
| [16,] | hsa-miR-192_st | -0.28 | GGCTGTCAATTCATAGGTCAG |

(continued)

| Medianprobe | Corr | Sequence |
|---|---|---|
| [17,] hsa-miR-194_st | -0.27 | TCCACATGGAGTTGCTGTTACA |
| [18,] hsa-miR-200b_st | -0.27 | TCATCATTACCAGGCAGTATTA |
| [19,] hsa-miR-200c_st | -0.32 | TCCATCATTACCCGGCAGTATTA |
| [20,] hsa-miR-215_st | -0.26 | GTCTGTCAATTCATAGGTCAT |
| [21,] hsa-miR-22_st | -0.4 | ACAGTTCTTCAACTGGCAGCTT |
| [22,] hsa-miR-24_st | -0.27 | CTGTTCCTGCTGAACTGAGCCA |
| [23,] hsa-miR-28-3p_st | -0.38 | TCCAGGAGCTCACAATCTAGTG |
| [24,] hsa-miR-28-5p_st | -0.4 | CTCAATAGACTGTGAGCTCCTT |
| [25,] hsa-miR-30a_st | -0.26 | CTTCCAGTCGAGGATGTTTACA |
| [26,] hsa-miR-30b_st | -0.38 | AGCTGAGTGTAGGATGTTTACA |
| [27,] hsa-miR-30d_st | -0.39 | CTTCCAGTCGGGGATGTTTACA |
| [28,] hsa-miR-324-5p_st | -0.34 | ACACCAATGCCCTAGGGGATGCG |
| [29,] hsa-miR-339-3p_st | -0.3 | CGGCTCTGTCGTCGAGGCGCTCA |
| [30,] hsa-miR-34a-star_st | -0.26 | AGGGCAGTATACTTGCTGATTG |
| [31,] hsa-miR-34a_st | -0.42 | ACAACCAGCTAAGACACTGCCA |
| [32,] hsa-miR-429_st | -0.31 | ACGGTTTTACCAGACAGTATTA |
| [33,] hsa-miR-589_st | -0.27 | CTCAGAGCAGACGTGGTTCTCA |
| [34,] hsa-miR-934_st | -0.3 | CCAGTGTCTCCAGTAGTAGACA |
| [35,] hsa-miR-935_st | -0.26 | GCGGTAGCGGAAGCGGTAACTGG |
| [36,] hsa-miR-99b-star_st | -0.39 | CGGACCCACAGACACGAGCTTG |
| [37,] hsa-miR-99b_st | -0.4 | CGCAAGGTCGGTTCTACGGGTG |

Table 103 Oxaliplatin microRNA biomarkers.

| Medianprobe | Corr | Sequence |
|---|---|---|
| [1,] hsa-let-7e_st | -0.35 | AACTATACAACCTCCTACCTCA |
| [2,] hsa-miR-100_st | -0.36 | CACAAGTTCGGATCTACGGGTT |
| [3,] hsa-miR-125a-5p_st | -0.35 | TCACAGGTTAAAGGGTCTCAGGGA |
| [4,] hsa-miR-125b-1-star_st | -0.41 | AGCTCCCAAGAGCCTAACCCGT |
| [5,] hsa-miR-125b_st | -0.39 | TCACAAGTTAGGGTCTCAGGGA |
| [6,] hsa-miR-1269_st | -0.36 | CCAGTAGCACGGCTCAGTCCAG |
| [7,] hsa-miR-1270_st | -0.31 | ACACAGCTCTTCCATATCTCCAG |
| [8,] hsa-miR-130a_st | -0.33 | ATGCCCTTTTAACATTGCACTG |
| [9,] hsa-miR-134_st | -0.26 | CCCCTCTGGTCAACCAGTCACA |
| [10,] hsa-miR-143_st | -0.3 | GAGCTACAGTGCTTCATCTCA |
| [11,] hsa-miR-145_st | -0.25 | AGGGATTCCTGGGAAAACTGGAC |
| [12,] hsa-miR-151-3p_st | -0.31 | CCTCAAGGAGCTTCAGTCTAG |
| [13,] hsa-miR-151-5p_st | -0.31 | ACTAGACTGTGAGCTCCTCGA |
| [14,] hsa-miR-181a-2-star_st | -0.35 | GGTACAGTCAACGGTCAGTGGT |
| [15,] hsa-miR-193a-3p_st | -0.26 | ACTGGGACTTTGTAGGCCAGTT |
| [16,] hsa-miR-193a-5p_st | -0.39 | TCATCTCGCCCGCAAAGACCCA |
| [17,] hsa-miR-217_st | -0.32 | TCCAATCAGTTCCTGATGCAGTA |
| [18,] hsa-miR-21_st | -0.26 | TCAACATCAGTCTGATAAGCTA |
| [19,] hsa-miR-221-star_st | -0.29 | AAATCTACATTGTATGCCAGGT |
| [20,] hsa-miR-22_st | -0.38 | ACAGTTCTTCAACTGGCAGCTT |
| [21,] hsa-miR-23a-star_st | -0.34 | AAATCCCATCCCCAGGAACCCC |
| [22,] hsa-miR-23a_st | -0.37 | GGAAATCCCTGGCAATGTGAT |
| [23,] hsa-miR-23b-star_st | -0.3 | AAATCAGCATGCCAGGAACCCA |
| [24,] hsa-miR-23b_st | -0.39 | GGTAATCCCTGGCAATGTGAT |
| [25,] hsa-miR-24-2-star_st | -0.37 | CTGTGTTTCAGCTCAGTAGGCA |

(continued)

|  | Medianprobe | Corr | Sequence |
|---|---|---|---|
| [26,] | hsa-miR-24_st | -0.39 | CTGTTCCTGCTGAACTGAGCCA |
| [27,] | hsa-miR-27a-star_st | -0.29 | TGCTCACAAGCAGCTAAGCCCT |
| [28,] | hsa-miR-27a_st | -0.27 | GCGGAACTTAGCCACTGTGAA |
| [29,] | hsa-miR-27b-star_st | -0.37 | GTTCACCAATCAGCTAAGCTCT |
| [30,] | hsa-miR-27b_st | -0.35 | GCAGAACTTAGCCACTGTGAA |
| [31,] | hsa-miR-28-3p_st | -0.42 | TCCAGGAGCTCACAATCTAGTG |
| [32,] | hsa-miR-28-5p_st | -0.42 | CTCAATAGACTGTGAGCTCCTT |
| [33,] | hsa-miR-30a-starst | -0.39 | GCTGCAAACATCCGACTGAAAG |
| [34,] | hsa-miR-30a_st | -0.38 | CTTCCAGTCGAGGATGTTTACA |
| [35,] | hsa-miR-30c-2-starst | -0.39 | AGAGTAAACAGCCTTCTCCCAG |
| [36,] | hsa-miR-34c-5p_st | -0.28 | GCAATCAGCTAACTACACTGCCT |
| [37,] | hsa-miR-376a_st | -0.28 | ACGTGGATTTTCCTCTATGAT |
| [38,] | hsa-miR-455-3p_st | -0.27 | GTGTATATGCCCATGGACTGC |
| [39,] | hsa-miR-543_st | -0.26 | AAGAAGTGCACCGCGAATGTTT |
| [40,] | hsa-miR-574-3p_st | -0.4 | TGTGGGTGTGTGCATGAGCGTG |
| [41,] | hsa-miR-99b-star_st | -0.27 | CGGACCCACAGACACGAGCTTG |
| [42,] | hsa-miR-99b_st | -0.31 | CGCAAGGTCGGTTCTACGGGTG |

Table 104 Hydroxyurea microRNA biomarkers.

|  | Medianprobe | Corr | Sequence |
|---|---|---|---|
| [1,] | hsa-let-7e_st | -0.43 | AACTATACAACCTCCTACCTCA |
| [2,] | hsa-miR-103_st | -0.26 | TCATAGCCCTGTACAATGCTGCT |
| [3,] | hsa-miR-107_st | -0.27 | TGATAGCCCTGTACAATGCTGCT |
| [4,] | hsa-miR-10a_st | -0.35 | CACAAATTCGGATCTACAGGGTA |
| [5,] | hsa-miR-1180_st | -0.26 | ACACACCCACGCGAGCCGGAAA |
| [6,] | hsa-miR-125a-3p_st | -0.29 | GGCTCCCAAGAACCTCACCTGT |
| [7,] | hsa-miR-125a-5p_st | -0.44 | TCACAGGTTAAAGGGTCTCAGGGA |
| [8,] | hsa-miR-1287_st | -0.31 | GACTCGAACCACTGATCCAGCA |
| [9,] | hsa-miR-132_st | -0.39 | CGACCATGGCTGTAGACTGTTA |
| [10,] | hsa-miR-149_st | -0.28 | GGGAGTGAAGACACGGAGCCAGA |
| [11,] | hsa-miR-151-3p_st | -0.57 | CCTCAAGGAGCTTCAGTCTAG |
| [12,] | hsa-miR-151-5p_st | -0.64 | ACTAGACTGTGAGCTCCTCGA |
| [13,] | hsa-miR-182_st | -0.35 | AGTGTGAGTTCTACCATTGCCAAA |
| [14,] | hsa-miR-183_st | -0.35 | AGTGAATTCTACCAGTGCCATA |
| [15,] | hsa-miR-193b_st | -0.33 | AGCGGGACTTTGAGGGCCAGTT |
| [16,] | hsa-miR-195_st | -0.27 | GCCAATATTTCTGTGCTGCTA |
| [17,] | hsa-miR-21_st | -0.28 | TCAACATCAGTCTGATAAGCTA |
| [18,] | hsa-miR-221-star_st | -0.31 | AAATCTACATTGTATGCCAGGT |
| [19,] | hsa-miR-22_st | -0.41 | ACAGTTCTTCAACTGGCAGCTT |
| [20,] | hsa-miR-23a_st | -0.29 | GGAAATCCCTGGCAATGTGAT |
| [21,] | hsa-miR-23b_st | -0.36 | GGTAATCCCTGGCAATGTGAT |
| [22,] | hsa-miR-24_st | -0.36 | CTGTTCCTGCTGAACTGAGCCA |
| [23,] | hsa-miR-26a_st | -0.3 | AGCCTATCCTGGATTACTTGAA |
| [24,] | hsa-miR-27b_st | -0.29 | GCAGAACTTAGCCACTGTGAA |
| [25,] | hsa-miR-28-3p_st | -0.29 | TCCAGGAGCTCACAATCTAGTG |
| [26,] | hsa-miR-28-5p_st | -0.32 | CTCAATAGACTGTGAGCTCCTT |
| [27,] | hsa-miR-30a-star_st | -0.26 | GCTGCAAACATCCGACTGAAAG |
| [28,] | hsa-miR-30a_st | -0.34 | CTTCCAGTCGAGGATGTTTACA |
| [29,] | hsa-miR-30b-star_st | -0.27 | GAAGTAAACATCCACCTCCCAG |

(continued)

| | Medianprobe | Corr | Sequence |
|---|---|---|---|
| [30,] | hsa-miR-30b_st | -0.29 | AGCTGAGTGTAGGATGTTTACA |
| [31,] | hsa-miR-30d_st | -0.4 | CTTCCAGTCGGGGATGTTTACA |
| [32,] | hsa-miR-320d_st | -0.33 | TCCTCTCAACCCAGCTTTT |
| [33,] | hsa-miR-34a_st | -0.25 | ACAACCAGCTAAGACACTGCCA |
| [34,] | hsa-miR-501-3p_st | -0.26 | AGAATCCTTGCCCGGGTGCATT |
| [35,] | hsa-miR-532-3p_st | -0.27 | TGCAAGCCTTGGGTGTGGGAGG |
| [36,] | hsa-miR-584_st | -0.35 | CTCAGTCCCAGGCAAACCATAA |
| [37,] | hsa-miR-99b-star_st | -0.35 | CGGACCCACAGACACGAGCTTG |
| [38,] | hsa-miR-99b_st | -0.46 | CGCAAGGTCGGTTCTACGGGTG |

Table 105 Tegafur microRNA bicmarkers.

| | Medianprobe | Corr | Sequence |
|---|---|---|---|
| [1,] | 14qII-14_st | -0.26 | ACACTCATACGCCGGCAGTTGTCAT |
| [2,] | 14qII-14_x_st | -0.26 | ATCACCCAACACTCATACGCCGGCA |
| [3,] | 14qII-1_st | -0.26 | ACTTCCAGACCTGTATTCACCGTCA |
| [4,] | 14qII-1_x_st | -0.26 | GGACCTCAGACTTCCAGACCTGTAT |
| [5,] | 14qII-26_st | -0.26 | CGTATGTGACTCATACTCCACCAGT |
| [6,] | hsa-let-7a_st | -0.27 | AACTATACAACCTACTACCTCA |
| [7,] | hsa-let-7e_st | -0.44 | AACTATACAACCTCCTACCTCA |
| [8,] | hsa-let-7f_st | -0.32 | AACTATACAATCTACTACCTCA |
| [9,] | hsa-miR-100_st | -0.3 | CACAAGTTCGGATCTACGGGTT |
| [10,] | hsa-miR-10b_st | -0.26 | CACAAATTCGGTTCTACAGGGTA |
| [11,] | hsa-miR-125a-3p_st | -0.26 | GGCTCCCAAGAACCTCACCTGT |
| [12,] | hsa-miR-125a-5p_st | -0.4 | TCACAGGTTAAAGGGTCTCAGGGA |
| [13,] | hsa-miR-125b-1-star_st | -0.36 | AGCTCCCAAGAGCCTAACCCGT |
| [14,] | hsa-miR-125b_st | -0.33 | TCACAAGTTAGGGTCTCAGGGA |
| [15,] | hsa-miR-130a_st | -0.35 | ATGCCCTTTTAACATTGCACTG |
| [16,] | hsa-miR-134_st | -0.3 | CCCCTCTGGTCAACCAGTCACA |
| [17,] | hsa-miR-149_st | -0.41 | GGGAGTGAAGACACGGAGCCAGA |
| [18,] | hsa-miR-151-3p_st | -0.33 | CCTCAAGGAGCTTCAGTCTAG |
| [19,] | hsa-miR-151-5p_st | -0.33 | ACTAGACTGTGAGCTCCTCGA |
| [20,] | hsa-miR-181a-2-star_st | -0.35 | GGTACAGTCAACGGTCAGTGGT |
| [21,] | hsa-miR-217_st | -0.27 | TCCAATCAGTTCCTGATGCAGTA |
| [22,] | hsa-miR-21_st | -0.31 | TCAACATCAGTCTGATAAGCTA |
| [23,] | hsa-miR-22-star_st | -0.26 | TAAAGCTTGCCACTGAAGAACT |
| [24,] | hsa-miR-221-star_st | -0.37 | AAATCTACATTGTATGCCAGGT |
| [25,] | hsa-miR-221_st | -0.3 | GAAACCCAGCAGACAATGTAGCT |
| [26,] | hsa-miR-222-star_st | -0.29 | AGGATCTACACTGGCTACTGAG |
| [27,] | hsa-miR-27b_st | -0.31 | GCAGAACTTAGCCACTGTGAA |
| [28,] | hsa-miR-299-3p_st | -0.27 | AAGCGGTTTACCATCCCACATA |
| [29,] | hsa-miR-299-5p_st | -0.28 | ATGTATGTGGGACGGTAAACCA |
| [30,] | hsa-miR-30a-star_st | -0.47 | GCTGCAAACATCCGACTGAAAG |
| [31,] | hsa-miR-30a_st | -0.42 | CTTCCAGTCGAGGATGTTTACA |
| [32,] | hsa-miR-30c-2-star_st | -0.45 | AGAGTAAACAGCCTTCTCCCAG |
| [33,] | hsa-miR-30c_st | -0.37 | GCTGAGAGTGTAGGATGTTTACA |
| [34,] | hsa-miR-30e-star_st | -0.28 | GCTGTAAACATCCGACTGAAAG |
| [35,] | hsa-miR-337-5p_st | -0.36 | AACTCCTGTATGAAGCCGTTC |
| [36,] | hsa-miR-376a_st | -0.32 | ACGTGGATTTTCCTCTATGAT |
| [37,] | hsa-miR-376c_st | -0.36 | ACGTGGAATTTCCTCTATGTT |

(continued)

| | Medianprobe | Corr | Sequence |
|---|---|---|---|
| [38,] | hsa-miR-377-star_st | -0.27 | GAATTCACCAAGGGCAACCTCT |
| [39,] | hsa-miR-379_st | -0.32 | CCTACGTTCCATAGTCTACCA |
| [40,] | hsa-miR-382_st | -0.33 | CGAATCCACCACGAACAACTTC |
| [41,] | hsa-miR-409-3p_st | -0.28 | AGGGGTTCACCGAGCAACATTC |
| [42,] | hsa-miR-409-5p_st | -0.3 | ATGCAAAGTTGCTCGGGTAACCT |
| [43,] | hsa-miR-411_st | -0.34 | CGTACGCTATACGGTCTACTA |
| [44,] | hsa-miR-485-5p_st | -0.27 | GAATTCATCACGGCCAGCCTCT |
| [45,] | hsa-miR-487a_st | -0.26 | AACTGGATGTCCCTGTATGATT |
| [46,] | hsa-miR-487b_st | -0.28 | AAGTGGATGACCCTGTACGATT |
| [47,] | hsa-miR-495_st | -0.25 | AAGAAGTGCACCATGTTTGTTT |
| [48,] | hsa-miR-543_st | -0.35 | AAGAAGTGCACCGCGAATGTTT |
| [49,] | hsa-miR-654-5p_st | -0.31 | GCACATGTTCTGCGGCCCACCA |
| [50,] | hsa-miR-758_st | -0.28 | GGTTAGTGGACCAGGTCACAAA |
| [51,] | hsa-miR-9-star_st | -0.34 | ACTTTCGGTTATCTAGCTTTAT |
| [52,] | hsa-miR-98_st | -0.34 | AACAATACAACTTACTACCTCA |
| [53,] | hsa-miR-99b-star_st | -0.29 | CGGACCCACAGACACGAGCTTG |
| [54,] | hsa-miR-99b_st | -0.37 | CGCAAGGTCGGTTCTACGGGTG |

Table 106 Daunorubicin microRNA biomarkers.

| | Medianprobe | Corr | Sequence |
|---|---|---|---|
| [1,] | hsa-let-7e_st | -0.42 | AACTATACAACCTCCTACCTCA |
| [2,] | hsa-miR-10a_st | -0.28 | CACAAATTCGGATCTACAGGGTA |
| [3,] | hsa-miR-125a-5pst | -0.41 | TCACAGGTTAAAGGGTCTCAGGGA |
| [4,] | hsa-miR-146a_st | -0.26 | AACCCATGGAATTCAGTTCTCA |
| [5,] | hsa-miR-151-3p_st | -0.43 | CCTCAAGGAGCTTCAGTCTAG |
| [6,] | hsa-miR-151-5p_st | -0.49 | ACTAGACTGTGAGCTCCTCGA |
| [7,] | hsa-miR-193b_st | -0.36 | AGCGGGACTTTGAGGGCCAGTT |
| [8,] | hsa-miR-200b-star_st | -0.26 | TCCAATGCTGCCCAGTAAGATG |
| [9,] | hsa-miR-200b_st | -0.27 | TCATCATTACCAGGCAGTATTA |
| [10,] | hsa-miR-203_st | -0.29 | CTAGTGGTCCTAAACATTTCAC |
| [11,] | hsa-miR-221-star_st | -0.26 | AAATCTACATTGTATGCCAGGT |
| [12,] | hsa-miR-22_st | -0.35 | ACAGTTCTTCAACTGGCAGCTT |
| [13,] | hsa-miR-23a_st | -0.41 | GGAAATCCCTGGCAATGTGAT |
| [14,] | hsa-miR-24-2-star_st | -0.31 | CTGTGTTTCAGCTCAGTAGGCA |
| [15,] | hsa-miR-24_st | -0.34 | CTGTTCCTGCTGAACTGAGCCA |
| [16,] | hsa-miR-27a_st | -0.27 | GCGGAACTTAGCCACTGTGAA |
| [17,] | hsa-miR-29a_st | -0.28 | TAACCGATTTCAGATGGTGCTA |
| [18,] | hsa-miR-29b-1-star_st | -0.28 | TCTAAACCACCATATGAAACCAGC |
| [19,] | hsa-miR-29b_st | -0.34 | AACACTGATTTCAAATGGTGCTA |
| [20,] | hsa-miR-30a-star_st | -0.35 | GCTGCAAACATCCGACTGAAAG |
| [21,] | hsa-miR-30a_st | -0.4 | CTTCCAGTCGAGGATGTTTACA |
| [22,] | hsa-miR-30b_st | -0.32 | AGCTGAGTGTAGGATGTTTACA |
| [23,] | hsa-miR-30c-2-star_st | -0.31 | AGAGTAAACAGCCTTCTCCCAG |
| [24,] | hsa-miR-30c_st | -0.32 | GCTGAGAGTGTAGGATGTTTACA |
| [25,] | hsa-miR-30d_st | -0.36 | CTTCCAGTCGGGGATGTTTACA |
| [26,] | hsa-miR-30e-star_st | -0.27 | GCTGTAAACATCCGACTGAAAG |
| [27,] | hsa-miR-372_st | -0.29 | ACGCTCAAATGTCGCAGCACTTT |
| [28,] | hsa-miR-373_st | -0.28 | ACACCCCAAAATCGAAGCACTTC |
| [29,] | hsa-miR-516a-5p_st | -0.44 | GAAAGTGCTTCTTTCCTCGAGAA |

(continued)

| | Medianprobe | Corr | Sequence |
|---|---|---|---|
| [30,] | hsa-miR-518e-star_st | -0.27 | CAGAAAGCGCTTCCCTCTAGAG |
| [31,] | hsa-miR-519a-star_st | -0.31 | CAGAAAGCGCTTCCCTCTAGAG |
| [32,] | hsa-miR-519a_st | -0.33 | ACACTCTAAAAGGATGCACTTT |
| [33,] | hsa-miR-519b-5p_st | -0.29 | CAGAAAGCGCTTCCCTCTAGAG |
| [34,] | hsa-miR-519c-5p_st | -0.31 | CAGAAAGCGCTTCCCTCTAGAG |
| [35,] | hsa-miR-522-star_st | -0.28 | CAGAAAGCGCTTCCCTCTAGAG |
| [36,] | hsa-miR-523-star_st | -0.26 | CAGAAAGCGCTTCCCTCTAGAG |
| [37,] | hsa-miR-526a_st | -0.3 | CAGAAAGTGCTTCCCTCTAGAG |
| [38,] | hsa-miR-584_st | -0.31 | CTCAGTCCCAGGCAAACCATAA |
| [39,] | hsa-miR-675_st | -0.28 | CACTGTGGGCCCTCTCCGCACCA |
| [40,] | hsa-miR-99b_st | -0.37 | CGCAAGGTCGGTTCTACGGGTG |

Table 107 Bleomycin microRNA biomarkers.

| | Medianprobe | Corr | Sequence |
|---|---|---|---|
| [1,] | HBII-180C_st | -0.29 | GTGCACTGTGTCCTCAGGGGTGATC |
| [2,] | HBII-180Cx_st | -0.25 | TCAGGGGTGATCAGAGCCCAGTGCT |
| [3,] | hsa-miR-106a_st | -0.29 | CTACCTGCACTGTAAGCACTTTT |
| [4,] | hsa-miR-106b-star_st | -0.26 | GCAGCAAGTACCCACAGTGCGG |
| [5,] | hsa-miR-106b_st | -0.29 | ATCTGCACTGTCAGCACTTTA |
| [6,] | hsa-miR-107_st | -0.3 | TGATAGCCCTGTACAATGCTGCT |
| [7,] | hsa-miR-1207-5p_st | -0.29 | CCCCTCCCAGCCTCCCTGCCA |
| [8,] | hsa-miR-1244_st | -0.31 | AACCATCTCATACAAACCAACTACT |
| [9,] | hsa-miR-128_st | -0.32 | AAAGAGACCGGTTCACTGTGA |
| [10,] | hsa-miR-1292_st | -0.32 | CAGCGTCTGCCGGAACCCGTTCCCA |
| [11,] | hsa-miR-1306_st | -0.28 | CACCACCAGAGCCAACGT |
| [12,] | hsa-miR-1307_st | -0.29 | CACGACCGACGCCACGCCGAGT |
| [13,] | hsa-miR-130b_st | -0.27 | ATGCCCTTTCATCATTGCACTG |
| [14,] | hsa-miR-141_st | -0.34 | CCATCTTTACCAGACAGTGTTA |
| [15,] | hsa-miR-15b_st | -0.33 | TGTAAACCATGATGTGCTGCTA |
| [16,] | hsa-miR-17_st | -0.26 | CTACCTGCACTGTAAGCACTTTG |
| [17,] | hsa-miR-183_st | -0.26 | AGTGAATTCTACCAGTGCCATA |
| [18,] | hsa-miR-185-star_st | -0.32 | GACCAGAGGAAAGCCAGCCCCT |
| [19,] | hsa-miR-185_st | -0.27 | TCAGGAACTGCCTTTCTCTCCA |
| [20,] | hsa-miR-188-5p_st | -0.32 | CCCTCCACCATGCAAGGGATG |
| [21,] | hsa-miR-18a-star_st | -0.25 | CCAGAAGGAGCACTTAGGGCAGT |
| [22,] | hsa-miR-18a_st | -0.26 | CTATCTGCACTAGATGCACCTTA |
| [23,] | hsa-miR-18b_st | -0.37 | CTAACTGCACTAGATGCACCTTA |
| [24,] | hsa-miR-192_st | -0.25 | GGCTGTCAATTCATAGGTCAG |
| [25,] | hsa-miR-19b_st | -0.27 | TCAGTTTTGCATGGATTTGCACA |
| [26,] | hsa-miR-200c-star_st | -0.28 | CCAAACACTGCTGGGTAAGACG |
| [27,] | hsa-miR-200c_st | -0.33 | TCCATCATTACCCGGCAGTATTA |
| [28,] | hsa-miR-203_st | -0.3 | CTAGTGGTCCTAAACATTTCAC |
| [29,] | _ hsa-miR-205_st | -0.27 | CAGACTCCGGTGGAATGAAGGA |
| [30,] | hsa-miR-20a_st | -0.25 | CTACCTGCACTATAAGCACTTTA |
| [31,] | hsa-miR-20b_st | -0.31 | CTACCTGCACTATGAGCACTTTG |
| [32,] | hsa-miR-215_st | -0.3 | GTCTGTCAATTCATAGGTCAT |
| [33,] | hsa-miR-25-star_st | -0.25 | CAATTGCCCAAGTCTCCGCCT |
| [34,] | hsa-miR-25_st | -0.39 | TCACACCGAGACAAGTGCAATG |
| [35,] | hsa-miR-30b_st | -0.32 | AGCTGAGTGTAGGATGTTTACA |

(continued)

| | Medianprobe | Corr | Sequence |
|---|---|---|---|
| [36,] | hsa-miR-30d_st | -0.36 | CTTCCAGTCGGGGATGTTTACA |
| [37,] | hsa-miR-320d_st | -0.25 | TCCTCTCAACCCAGCTTTT |
| [38,] | hsa-miR-324-5p_st | -0.26 | ACACCAATGCCCTAGGGGATGCG |
| [39,] | hsa-miR-362-5p_st | -0.36 | ACTCACACCTAGGTTCCAAGGATT |
| [40,] | hsa-miR-378-star_st | -0.31 | ACACAGGACCTGGAGTCAGGAG |
| [41,] | hsa-miR-421_st | -0.32 | GCGCCCAATTAATGTCTGTTGAT |
| [42,] | hsa-iR-422a_st | -0.27 | GCCTTCTGACCCTAAGTCCAGT |
| [43,] | hsa-miR-425a_st | -0.35 | TCAACGGGAGTGATCGTGTCATT |
| [44,] | hsa-miR-500-star_st | -0.28 | CAGAATCCTTGCCCAGGTGCAT |
| [45,] | hsa-miR-500_st | -0.26 | TCTCACCCAGGTAGCAAGGATTA |
| [46,] | hsa-miR-501-3p_st | -0.26 | AGAATCCTTGCCCGGGTGCATT |
| [47,] | hsa-miR-532-3p_st | -0.27 | TGCAAGCCTTGGGTGTGGGAGG |
| [48,] | hsa-miR-532-5p_st | -0.33 | ACGGTCCTACACTCAAGGCATG |
| [49,] | hsa-miR-584_st | -0.26 | CTCAGTCCCAGGCAAACCATAA |
| [50,] | hsa-miR-625_st | -0.3 | GGACTATAGAACTTTCCCCCT |
| [51,] | hsa-miR-660_st | -0.3 | CAACTCCGATATGCAATGGGTA |
| [52,] | hsa-miR-720_st | -0.27 | TGGAGGCCCCAGCGAGA |
| [53,] | hsa-miR-93-star_st | -0.25 | CGGGAAGTGCTAGCTCAGCAGT |
| [54,] | hsa-miR-934_st | -0.25 | CCAGTGTCTCCAGTAGTAGACA |
| [55,] | hsa-miR-93_st | -0.34 | CTACCTGCACGAACAGCACTTTG |
| [56,] | hsa-miR-941_st | -0.34 | GCACATGTGCACACAGCCGGGTG |

Table 108 Estramustine microRNA biomarkers.

| | Medianprobe | Corr | Sequence |
|---|---|---|---|
| [1,] | hsa-miR-135b-star_st | -0.38 | CCCATGGCTTTTAGCCCTACAT |
| [2,] | hsa-miR-192-star_st | -0.27 | CTGTGACCTATGGAATTGGCAG |
| [3,] | hsa-miR-194-star_st | -0.25 | CAGATAACAGCAGCCCCACTGG |
| [4,] | hsa-miR-21-star_st | -0.25 | ACAGCCCATCGACTGGTGTTG |
| [5,] | hsa-miR-23b_st | -0.26 | GGTAATCCCTGGCAATGTGAT |
| [6,] | hsa-miR-27b-star_st | -0.27 | GTTCACCAATCAGCTAAGCTCT |
| [7,] | hsa-miR-552_st | -0.52 | TTGTCTAACCAGTCACCTGTT |
| [8,] | hsa-miR-592_st | -0.46 | ACATCATCGCATATTGACACAA |
| [9,] | hsa-miR-7_st | -0.26 | ACAACAAAATCACTAGTCTTCCA |
| [10,] | hsa-miR-874_st | -0.36 | TCGGTCCCTCGGGCCAGGGCAG |

Table 109 Mechlorethamine microRNA biomarkers.

| | Medianprobe | Corr | Sequence |
|---|---|---|---|
| [1,] | hsa-let-7e_st | -0.44 | AACTATACAACCTCCTACCTCA |
| [2,] | hsa-miR-100_st | -0.33 | CACAAGTTCGGATCTACGGGTT |
| [3,] | hsa-miR-125a-5p_st | -0.42 | TCACAGGTTAAAGGGTCTCAGGGA |
| [4,] | hsa-miR-151-3p_st | -0.5 | CCTCAAGGAGCTTCAGTCTAG |
| [5,] | hsa-miR-151-5p_st | -0.53 | ACTAGACTGTGAGCTCCTCGA |
| [6,] | hsa-miR-217_st | -0.32 | TCCAATCAGTTCCTGATGCAGTA |
| [7,] | hsa-miR-221-star_st | -0.31 | AAATCTACATTGTATGCCAGGT |
| [8,] | hsa-miR-22_st | -0.31 | ACAGTTCTTCAACTGGCAGCTT |
| [9,] | hsa-miR-23a_st | -0.35 | GGAAATCCCTGGCAATGTGAT |
| [10,] | hsa-miR-23b_st | -0.32 | GGTAATCCCTGGCAATGTGAT |
| [11,] | hsa-miR-24-2-star_st | -0.33 | CTGTGTTTCAGCTCAGTAGGCA |

(continued)

| | Medianprobe | Corr | Sequence |
|---|---|---|---|
| [12,] | hsa-miR-24_st | -0.32 | CTGTTCCTGCTGAACTGAGCCA |
| [13,] | hsa-miR-30d_st | -0.35 | CTTCCAGTCGGGGATGTTTACA |
| [14,] | hsa-miR-320d_st | -0.35 | TCCTCTCAACCCAGCTTTT |
| [15,] | hsa-miR-512-3p_st | -0.32 | GACCTCAGCTATGACAGCACTT |
| [16,] | hsa-miR-519b-5p_st | -0.3 | CAGAAAGCGCTTCCCTCTAGAG |
| [17,] | hsa-miR-519c-5p_st | -0.31 | CAGAAAGCGCTTCCCTCTAGAG |
| [18,] | hsa-miR-526a_st | -0.36 | CAGAAAGTGCTTCCCTCTAGAG |
| [19,] | hsa-miR-584_st | -0.39 | CTCAGTCCCAGGCAAACCATAA |
| [20,] | hsa-miR-99b-star_st | -0.31 | CGGACCCACAGACACGAGCTTG |
| [21,] | hsa-miR-99b_st | -0.39 | CGCAAGGTCGGTTCTACGGGTG |

Table 110 Streptozocin microRNA biomarkers.

| | Medianprobe | Corr | Sequence |
|---|---|---|---|
| [1,] | hsa-miR-26a_st | -0.3 | AGCCTATCCTGGATTACTTGAA |
| [2,] | hsa-miR-34b-star_st | -0.27 | CAATCAGCTAATGACACTGCCTA |
| [3,] | hsa-miR-34c-5p_st | -0.29 | GCAATCAGCTAACTACACTGCCT |
| [4,] | hsa-miR-516a-5p_st | -0.27 | GAAAGTGCTTCTTTCCTCGAGAA |
| [5,] | hsa-miR-518e-star_st | -0.29 | CAGAAAGCGCTTCCCTCTAGAG |
| [6,] | hsa-miR-519a_st | -0.27 | ACACTCTAAAAGGATGCACTTT |
| [7,] | hsa-miR-519c-5p_st | -0.28 | CAGAAAGCGCTTCCCTCTAGAG |
| [8,] | hsa-miR-526a_st | -0.26 | CAGAAAGTGCTTCCCTCTAGAG |

Table 111 Carmustine microRNA biomarkers.

| | Medianprobe | Corr | Sequence |
|---|---|---|---|
| [1,] | hsa-let-7b st | -0.59 | AACCACACAACCTACTACCTCA |
| [2,] | hsa-let-7c_ st | -0.5 | AACCATACAACCTACTACCTCA |
| [3,] | hsa-let-7d_ st | -0.57 | AACTATGCAACCTACTACCTCT |
| [4,] | hsa-let-7f_ st | -0.3 | AACTATACAATCTACTACCTCA |
| [5,] | hsa-let-7g_st | -0.38 | AACTGTACAAACTACTACCTCA |
| [6,] | hsa-let-7i_ st | -0.55 | AACAGCACAAACTACTACCTCA |
| [7,] | hsa-miR-130a st | -0.3 | ATGCCCTTTTAACATTGCACTG |
| [8,] | hsa-miR-181a_ st | -0.46 | ACTCACCGACAGCGTTGAATGTT |
| [9,] | hsa-miR-193a-3p_st | -0.37 | ACTGGGACTTTGTAGGCCAGTT |
| [10,] | hsa-miR-24-2-star_ st | -0.41 | CTGTGTTTCAGCTCAGTAGGCA |
| [11,] | hsa-miR-24_ st | -0.32 | CTGTTCCTGCTGAACTGAGCCA |
| [12,] | hsa-miR-28-3p_ st | -0.48 | TCCAGGAGCTCACAATCTAGTG |
| [13,] | hsa-miR-28-5p_ st | -0.53 | CTCAATAGACTGTGAGCTCCTT |
| [14,] | hsa-miR-29b_ st | -0.32 | AACACTGATTTCAAATGGTGCTA |
| [15,] | hsa-miR-30a_ st | -0.37 | CTTCCAGTCGAGGATGTTTACA |
| [16,] | hsa-miR-331-3p_ st | -0.3 | TTCTAGGATAGGCCCAGGGGC |
| [17,] | hsa-miR-98_ st | -0.31 | AACAATACAACTTACTACCTCA |

Table 112 Lomustine microRNA biomarkers.

| | Medianprobe | Corr | Sequence |
|---|---|---|---|
| [1,] | hsa-let-7e_st | -0.45 | AACTATACAACCTCCTACCTCA |
| [2,] | hsa-miR-125a-3p_st | -0.35 | GGCTCCCAAGAACCTCACCTGT |

(continued)

| | Medianprobe | Corr | Sequence |
|---|---|---|---|
| [3,] | hsa-miR-125a-5p_st | -0.47 | TCACAGGTTAAAGGGTCTCAGGGA |
| [4,] | hsa-miR-151-3p_st | -0.37 | CCTCAAGGAGCTTCAGTCTAG |
| [5,] | hsa-miR-151-5p_st | -0.43 | ACTAGACTGTGAGCTCCTCGA |
| [6,] | hsa-miR-193b_st | -0.41 | AGCGGGACTTTGAGGGCCAGTT |
| [7,] | hsa-miR-200a_st | -0.33 | ACATCGTTACCAGACAGTGTTA |
| [8,] | hsa-miR-200b-star_st | -0.33 | TCCAATGCTGCCCAGTAAGATG |
| [9,] | hsa-miR-200b_st | -0.32 | TCATCATTACCAGGCAGTATTA |
| [10,] | hsa-miR-200c_st | -0.31 | TCCATCATTACCCGGCAGTATTA |
| [11,] | hsa-miR-21_st | -0.43 | TCAACATCAGTCTGATAAGCTA |
| [12,] | hsa-miR-22_st | -0.38 | ACAGTTCTTCAACTGGCAGCTT |
| [13,] | hsa-miR-30a_st | -0.38 | CTTCCAGTCGAGGATGTTTACA |
| [14,] | hsa-miR-31-star_st | -0.32 | ATGGCAATATGTTGGCATAGCA |
| [15,] | hsa-miR-331-3p_st | -0.31 | TTCTAGGATAGGCCCAGGGGC |
| [16,] | hsa-miR-34a_st | -0.35 | ACAACCAGCTAAGACACTGCCA |
| [17,] | hsa-miR-34c-5p_st | -0.33 | GCAATCAGCTAACTACACTGCCT |
| [18,] | hsa-miR-935_st | -0.33 | GCGGTAGCGGAAGCGGTAACTGG |
| [19,] | hsa-miR-99b-star_st | -0.36 | CGGACCCACAGACACGAGCTTG |
| [20,] | hsa-miR-99b_st | -0.48 | CGCAAGGTCGGTTCTACGGGTG |

Table 113 Mercaptopurine microRNA biomarkers.

| | Medianprobe | Corr | Sequence |
|---|---|---|---|
| [1,] | hsa-let-7e_st | -0.37 | AACTATACAACCTCCTACCTCA |
| [2,] | hsa-let-7i_st | -0.35 | AACAGCACAAACTACTACCTCA |
| [3,] | hsa-miR-125a-5p_st | -0.38 | TCACAGGTTAAAGGGTCTCAGGGA |
| [4,] | hsa-miR-22-star_st | -0.3 | TAAAGCTTGCCACTGAAGAACT |
| [5,] | hsa-miR-22_st | -0.43 | ACAGTTCTTCAACTGGCAGCTT |
| [6,] | hsa-miR-23a_st | -0.3 | GGAAATCCCTGGCAATGTGAT |
| [7,] | hsa-miR-23b_st | -0.39 | GGTAATCCCTGGCAATGTGAT |
| [8,] | hsa-miR-27b_st | -0.38 | GCAGAACTTAGCCACTGTGAA |
| [9,] | hsa-miR-28-3p_st | -0.32 | TCCAGGAGCTCACAATCTAGTG |
| [10,] | hsa-miR-28-5p_st | -0.32 | CTCAATAGACTGTGAGCTCCTT |
| [11,] | hsa-miR-494_st | -0.31 | GAGGTTTCCCGTGTATGTTTCA |
| [12,] | hsa-miR-99b_st | -0.34 | CGCAAGGTCGGTTCTACGGGTG |

Table 114 Teniposide microRNA biomarkers.

| | Medianprobe | Corr | Sequence |
|---|---|---|---|
| [1,] | hsa-let-7e_st | -0.44 | AACTATACAACCTCCTACCTCA |
| [2,] | hsa-miR-10a_st | -0.32 | CACAAATTCGGATCTACAGGGTA |
| [3,] | hsa-miR-125a-5pst | -0.42 | TCACAGGTTAAAGGGTCTCAGGGA |
| [4,] | hsa-miR-151-3p_st | -0.55 | CCTCAAGGAGCTTCAGTCTAG |
| [5,] | hsa-miR-151-5p_st | -0.58 | ACTAGACTGTGAGCTCCTCGA |
| [6,] | hsa-miR-193b_st | -0.31 | AGCGGGACTTTGAGGGCCAGTT |
| [7,] | hsa-miR-221-star_st | -0.33 | AAATCTACATTGTATGCCAGGT |
| [8,] | hsa-miR-23a_st | -0.35 | GGAAATCCCTGGCAATGTGAT |
| [9,] | hsa-miR-30a-star_st | -0.33 | GCTGCAAACATCCGACTGAAAG |
| [10,] | hsa-miR-30a_st | -0.36 | CTTCCAGTCGAGGATGTTTACA |
| [11,] | hsa-miR-30b_st | -0.32 | AGCTGAGTGTAGGATGTTTACA |
| [12,] | hsa-miR-30c-2-star_st | -0.3 | AGAGTAAACAGCCTTCTCCCAG |

(continued)

| | Medianprobe | Corr | Sequence |
|---|---|---|---|
| [13,] | hsa-miR-30c_st | -0.31 | GCTGAGAGTGTAGGATGTTTACA |
| [14,] | hsa-miR-30d_st | -0.4 | CTTCCAGTCGGGGATGTTTACA |
| [15,] | hsa-miR-516a-5p_st | -0.44 | GAAAGTGCTTCTTTCCTCGAGAA |
| [16,] | hsa-miR-518e-star_st | -0.3 | CAGAAAGCGCTTCCCTCTAGAG |
| [17,] | hsa-miR-519a_st | -0.35 | ACACTCTAAAAGGATGCACTTT |
| [18,] | hsa-miR-519c-5p_st | -0.31 | CAGAAAGCGCTTCCCTCTAGAG |
| [19,] | hsa-miR-526a_st | -0.31 | CAGAAAGTGCTTCCCTCTAGAG |
| [20,] | hsa-miR-584_st | -0.35 | CTCAGTCCCAGGCAAACCATAA |
| [21,] | hsa-miR-99b-star_st | -0.32 | CGGACCCACAGACACGAGCTTG |
| [22,] | hsa-miR-99b_st | -0.42 | CGCAAGGTCGGTTCTACGGGTG |

### Table 115 Dactinomycin microRNA biomarkers.

| | Medianprobe | Corr | Sequence |
|---|---|---|---|
| [1,] | hsa-let-7e_st | -0.37 | AACTATACAACCTCCTACCTCA |
| [2,] | hsa-miR-125a-5p_st | -0.37 | TCACAGGTTAAAGGGTCTCAGGGA |
| [3,] | hsa-miR-130a_st | -0.31 | ATGCCCTTTTAACATTGCACTG |
| [4,] | hsa-miR-193b_st | -0.31 | AGCGGGACTTTGAGGGCCAGTT |
| [5,] | hsa-miR-22_st | -0.36 | ACAGTTCTTCAACTGGCAGCTT |
| [6,] | hsa-miR-23a_st | -0.33 | GGAAATCCCTGGCAATGTGAT |
| [7,] | hsa-miR-24-2-star_st | -0.32 | CTGTGTTTCAGCTCAGTAGGCA |
| [8,] | hsa-miR-27a-star_st | -0.3 | TGCTCACAAGCAGCTAAGCCCT |
| [9,] | hsa-miR-27a_st | -0.36 | GCGGAACTTAGCCACTGTGAA |
| [10,] | hsa-miR-29b_st | -0.34 | AACACTGATTTCAAATGGTGCTA |
| [11,] | hsa-miR-30a-star_st | -0.45 | GCTGCAAACATCCGACTGAAAG |
| [12,] | hsa-miR-30a_st | -0.5 | CTTCCAGTCGAGGATGTTTACA |
| [13,] | hsa-miR-30c-2-star_st | -0.46 | AGAGTAAACAGCCTTCTCCCAG |
| [14,] | hsa-miR-30c_st | -0.44 | GCTGAGAGTGTAGGATGTTTACA |
| [15,] | hsa-miR-34c-5p_st | -0.33 | GCAATCAGCTAACTACACTGCCT |
| [16,] | hsa-miR-372_st | -0.3 | ACGCTCAAATGTCGCAGCACTTT |
| [17,] | hsa-miR-516a-5p_st | -0.43 | GAAAGTGCTTCTTTCCTCGAGAA |
| [18,] | hsa-miR-99b_st | -0.32 | CGCAAGGTCGGTTCTACGGGTG |

### Table 116 Tretinoin microRNA biomarkers.

| | Medianprobe | Corr | Sequence |
|---|---|---|---|
| [1,] | hsa-let-7e_st | -0.34 | AACTATACAACCTCCTACCTCA |
| [2,] | hsa-let-7i_st | -0.32 | AACAGCACAAACTACTACCTCA |
| [3,] | hsa-miR-125a-5p_st | -0.31 | TCACAGGTTAAAGGGTCTCAGGGA |
| [4,] | hsa-miR-151-3p_st | -0.37 | CCTCAAGGAGCTTCAGTCTAG |
| [5,] | hsa-miR-151-5p_st | -0.39 | ACTAGACTGTGAGCTCCTCGA |
| [6,] | hsa-miR-181a-2-star_st | -0.33 | GGTACAGTCAACGGTCAGTGGT |
| [7,] | hsa-miR-21-star_st | -0.33 | ACAGCCCATCGACTGGTGTTG |
| [8,] | hsa-miR-21_st | -0.38 | TCAACATCAGTCTGATAAGCTA |
| [9,] | hsa-miR-221_st | -0.6 | GAAACCCAGCAGACAATGTAGCT |
| [10,] | hsa-miR-222_st | -0.56 | ACCCAGTAGCCAGATGTAGCT |
| [11,] | hsa-miR-22_st | -0.37 | ACAGTTCTTCAACTGGCAGCTT |
| [12,] | hsa-miR-28-3p_st | -0.46 | TCCAGGAGCTCACAATCTAGTG |
| [13,] | hsa-miR-28-5p_st | -0.43 | CTCAATAGACTGTGAGCTCCTT |
| [14,] | hsa-miR-30a-star_st | -0.32 | GCTGCAAACATCCGACTGAAAG |

(continued)

| | Medianprobe | Corr | Sequence |
|---|---|---|---|
| [15,] | hsa-miR-30a_st | -0.36 | CTTCCAGTCGAGGATGTTTACA |
| [16,] | hsa-miR-30c-2-star_st | -0.33 | AGAGTAAACAGCCTTCTCCCAG |
| [17,] | hsa-miR-455-3p_st | -0.32 | GTGTATATGCCCATGGACTGC |
| [18,] | hsa-miR-99b-star_st | -0.32 | CGGACCCACAGACACGAGCTTG |

Table 117 Ifosfamide microRNA biomarkers.

| | Medianprobe | Corr | Sequence |
|---|---|---|---|
| [1,] | hsa-let-7e_st | -0.34 | AACTATACAACCTCCTACCTCA |
| [2,] | hsa-miR-125a-5p_st | -0.35 | TCACAGGTTAAAGGGTCTCAGGGA |
| [3,] | hsa-miR-151-3p_st | -0.39 | CCTCAAGGAGCTTCAGTCTAG |
| [4,] | hsa-miR-151-5p_st | -0.39 | ACTAGACTGTGAGCTCCTCGA |
| [5,] | hsa-miR-182_st | -0.36 | AGTGTGAGTTCTACCATTGCCAAA |
| [6,] | hsa-miR-183_st | -0.35 | AGTGAATTCTACCAGTGCCATA |
| [7,] | hsa-miR-193b_st | -0.42 | AGCGGGACTTTGAGGGCCAGTT |
| [8,] | hsa-miR-195_st | -0.39 | GCCAATATTTCTGTGCTGCTA |
| [9,] | hsa-miR-22_st | -0.41 | ACAGTTCTTCAACTGGCAGCTT |
| [10,] | hsa-miR-23a_st | -0.52 | GGAAATCCCTGGCAATGTGAT |
| [11,] | hsa-miR-23b_st | -0.38 | GGTAATCCCTGGCAATGTGAT |
| [12,] | hsa-miR-24_st | -0.53 | CTGTTCCTGCTGAACTGAGCCA |
| [13,] | hsa-miR-27a-star_st | -0.34 | TGCTCACAAGCAGCTAAGCCCT |
| [14,] | hsa-miR-27a_st | -0.4 | GCGGAACTTAGCCACTGTGAA |
| [15,] | hsa-miR-27b_st | -0.3 | GCAGAACTTAGCCACTGTGAA |
| [16,] | hsa-miR-34b-star_st | -0.37 | CAATCAGCTAATGACACTGCCTA |
| [17,] | hsa-miR-497_st | -0.36 | ACAAACCACAGTGTGCTGCTG |
| [18,] | hsa-miR-99b_st | -0.33 | CGCAAGGTCGGTTCTACGGGTG |

Table 118 Tamoxifen microRNA biomarkers.

| | Medianprobe | Corr | Sequence |
|---|---|---|---|
| [1,] | hsa-let-7a_st | -0.3 | AACTATACAACCTACTACCTCA |
| [2,] | hsa-let-7b_st | -0.35 | AACCACACAACCTACTACCTCA |
| [3,] | hsa-let-7c_st | -0.37 | AACCATACAACCTACTACCTCA |
| [4,] | hsa-let-7e_st | -0.47 | AACTATACAACCTCCTACCTCA |
| [5,] | hsa-let-7g_st | -0.31 | AACTGTACAAACTACTACCTCA |
| [6,] | hsa-let-7i_st | -0.34 | AACAGCACAAACTACTACCTCA |
| [7,] | hsa-miR-100_st | -0.44 | CACAAGTTCGGATCTACGGGTT |
| [8,] | hsa-miR-10a_st | -0.32 | CACAAATTCGGATCTACAGGGTA |
| [9,] | hsa-miR-125a-5p_st | -0.42 | TCACAGGTTAAAGGGTCTCAGGGA |
| [10,] | hsa-miR-125b_st | -0.45 | TCACAAGTTAGGGTCTCAGGGA |
| [11,] | hsa-miR-130a_st | -0.52 | ATGCCCTTTTAACATTGCACTG |
| [12,] | hsa-miR-138_st | -0.34 | CGGCCTGATTCACAACACCAGCT |
| [13,] | hsa-miR-149_st | -0.45 | GGGAGTGAAGACACGGAGCCAGA |
| [14,] | hsa-miR-151-3p_st | -0.45 | CCTCAAGGAGCTTCAGTCTAG |
| [15,] | hsa-miR-151-5p_st | -0.4 | ACTAGACTGTGAGCTCCTCGA |
| [16,] | hsa-miR-181a-2-star_st | -0.33 | GGTACAGTCAACGGTCAGTGGT |
| [17,] | hsa-miR-193b-star_st | -0.34 | TCATCTCGCCCTCAAAACCCCG |
| [18,] | hsa-miR-193b_st | -0.35 | AGCGGGACTTTGAGGGCCAGTT |
| [19,] | hsa-miR-21_st | -0.37 | TCAACATCAGTCTGATAAGCTA |
| [20,] | hsa-miR-22-star_st | -0.33 | TAAAGCTTGCCACTGAAGAACT |

(continued)

| | Medianprobe | Corr | Sequence |
|---|---|---|---|
| [21,] | hsa-miR-221_st | -0.34 | GAAACCCAGCAGACAATGTAGCT |
| [22,] | hsa-miR-22_st | -0.37 | ACAGTTCTTCAACTGGCAGCTT |
| [23,] | hsa-miR-23a_st | -0.35 | GGAAATCCCTGGCAATGTGAT |
| [24,] | hsa-miR-23b_st | -0.3 | GGTAATCCCTGGCAATGTGAT |
| [25,] | hsa-miR-24-2-star_st | -0.4 | CTGTGTTTCAGCTCAGTAGGCA |
| [26,] | hsa-miR-24_st | -0.34 | CTGTTCCTGCTGAACTGAGCCA |
| [27,] | hsa-miR-28-3p_st | -0.37 | TCCAGGAGCTCACAATCTAGTG |
| [28,] | hsa-miR-28-5p_st | -0.36 | CTCAATAGACTGTGAGCTCCTT |
| [29,] | hsa-miR-30a-star_st | -0.64 | GCTGCAAACATCCGACTGAAAG |
| [30,] | hsa-miR-30a_st | -0.6 | CTTCCAGTCGAGGATGTTTACA |
| [31,] | hsa-miR-30c-2-star_st | -0.63 | AGAGTAAACAGCCTTCTCCCAG |
| [32,] | hsa-miR-30c_st | -0.49 | GCTGAGAGTGTAGGATGTTTACA |
| [33,] | hsa-miR-30e-star_st | -0.3 | GCTGTAAACATCCGACTGAAAG |
| [34,] | hsa-miR-34c-5p_st | -0.32 | GCAATCAGCTAACTACACTGCCT |
| [35,] | hsa-miR-424-star_st | -0.31 | ATAGCAGCGCCTCACGTTTTG |
| [36,] | hsa-miR-455-3p_st | -0.32 | GTGTATATGCCCATGGACTGC |
| [37,] | hsa-miR-503_st | -0.31 | CTGCAGAACTGTTCCCGCTGCTA |
| [38,] | hsa-miR-935_st | -0.32 | GCGGTAGCGGAAGCGGTAACTGG |
| [39,] | hsa-miR-99b-star_st | -0.34 | CGGACCCACAGACACGAGCTTG |
| [40,] | hsa-miR-99b_st | -0.42 | CGCAAGGTCGGTTCTACGGGTG |

Table 119 Irinotecan microRNA biomarkers.

| | Medianprobe | Corr | Sequence |
|---|---|---|---|
| [1,] | hsa-let-7e_st | -0.44 | AACTATACAACCTCCTACCTCA |
| [2,] | hsa-miR-125a-5p_st | -0.45 | TCACAGGTTAAAGGGTCTCAGGGA |
| [3,] | hsa-miR-151-3p_st | -0.36 | CCTCAAGGAGCTTCAGTCTAG |
| [4,] | hsa-miR-151-5p_st | -0.41 | ACTAGACTGTGAGCTCCTCGA |
| [5,] | hsa-miR-193b_st | -0.33 | AGCGGGACTTTGAGGGCCAGTT |
| [6,] | hsa-miR-30b_st | -0.33 | AGCTGAGTGTAGGATGTTTACA |
| [7,] | hsa-miR-30d_st | -0.44 | CTTCCAGTCGGGGATGTTTACA |
| [8,] | hsa-miR-320d_st | -0.32 | TCCTCTCAACCCAGCTTTT |
| [9,] | hsa-miR-584_st | -0.32 | CTCAGTCCCAGGCAAACCATAA |
| [10,] | hsa-miR-99b_st | -0.42 | CGCAAGGTCGGTTCTACGGGTG |

Table 120 Floxuridine microRNA biomarkers.

| | Medianprobe | Corr | Sequence |
|---|---|---|---|
| [1,] | hsa-miR-30b_st | -0.38 | AGCTGAGTGTAGGATGTTTACA |
| [2,] | hsa-miR-30d_st | -0.4 | CTTCCAGTCGGGGATGTTTACA |
| [3,] | hsa-miR-584_st | -0.3 | CTCAGTCCCAGGCAAACCATAA |

Table 121 Thioguanine microRNA biomarkers.

| | Medianprobe | Corr | Sequence |
|---|---|---|---|
| [1,] | hsa-let-7b_st | -0.3 | AACCACACAACCTACTACCTCA |
| [2,] | hsa-let-7c_st | -0.31 | AACCATACAACCTACTACCTCA |
| [3,] | hsa-let-7e_st | -0.48 | AACTATACAACCTCCTACCTCA |
| [4,] | hsa-miR-125a-5p_st | -0.48 | TCACAGGTTAAAGGGTCTCAGGGA |

(continued)

| | Medianprobe | Corr | Sequence |
|---|---|---|---|
| [5,] | hsa-miR-151-3p_st | -0.33 | CCTCAAGGAGCTTCAGTCTAG |
| [6,] | hsa-miR-151-5p_st | -0.37 | ACTAGACTGTGAGCTCCTCGA |
| [7,] | hsa-miR-217_st | -0.4 | TCCAATCAGTTCCTGATGCAGTA |
| [8,] | hsa-miR-21_st | -0.31 | TCAACATCAGTCTGATAAGCTA |
| [9,] | hsa-miR-22_st | -0.4 | ACAGTTCTTCAACTGGCAGCTT |
| [10,] | hsa-miR-23a_st | -0.36 | GGAAATCCCTGGCAATGTGAT |
| [11,] | hsa-miR-23b-star_st | -0.4 | AAATCAGCATGCCAGGAACCCA |
| [12,] | hsa-miR-23b_st | -0.54 | GGTAATCCCTGGCAATGTGAT |
| [13,] | hsa-miR-24_st | -0.39 | CTGTTCCTGCTGAACTGAGCCA |
| [14,] | hsa-miR-27b-star_st | -0.34 | GTTCACCAATCAGCTAAGCTCT |
| [15,] | hsa-miR-27b_st | -0.57 | GCAGAACTTAGCCACTGTGAA |
| [16,] | hsa-miR-30a_st | -0.3 | CTTCCAGTCGAGGATGTTTACA |
| [17,] | hsa-miR-494_st | -0.33 | GAGGTTTCCCGTGTATGTTTCA |
| [18,] | hsa-miR-9-star_st | -0.42 | ACTTTCGGTTATCTAGCTTTAT |
| [19,] | hsa-miR-99b-star_st | -0.37 | CGGACCCACAGACACGAGCTTG |
| [20,] | hsa-miR-99b_st | -0.44 | CGCAAGGTCGGTTCTACGGGTG |
| [21,] | hsa-miR-9_st | -0.45 | TCATACAGCTAGATAACCAAAGA |

### Table 122 PSC 833 microRNA biomarkers.

| | Medianprobe | Corr | Sequence |
|---|---|---|---|
| [1,] | hsa-let-7e_st | -0.31 | AACTATACAACCTCCTACCTCA |
| [2,] | hsa-miR-125a-5p_st | -0.32 | TCACAGGTTAAAGGGTCTCAGGGA |
| [3,] | hsa-miR-138_st | -0.42 | CGGCCTGATTCACAACACCAGCT |
| [4,] | hsa-miR-149_st | -0.31 | GGGAGTGAAGACACGGAGCCAGA |
| [5,] | hsa-miR-193b-star_st | -0.3 | TCATCTCGCCCTCAAAACCCCG |
| [6,] | hsa-miR-193b_st | -0.31 | AGCGGGACTTTGAGGGCCAGTT |
| [7,] | hsa-miR-21_st | -0.39 | TCAACATCAGTCTGATAAGCTA |
| [8,] | hsa-miR-22-star_st | -0.3 | TAAAGCTTGCCACTGAAGAACT |
| [9,] | hsa-miR-22_st | -0.41 | ACAGTTCTTCAACTGGCAGCTT |
| [10,] | hsa-miR-23a_st | -0.32 | GGAAATCCCTGGCAATGTGAT |
| [11,] | hsa-miR-23b_st | -0.36 | GGTAATCCCTGGCAATGTGAT |
| [12,] | hsa-miR-24-2-star_st | -0.35 | CTGTGTTTCAGCTCAGTAGGCA |
| [13,] | hsa-miR-24_st | -0.32 | CTGTTCCTGCTGAACTGAGCCA |
| [14,] | hsa-miR-27a_st | -0.36 | GCGGAACTTAGCCACTGTGAA |
| [15,] | hsa-milt-27b_st | -0.35 | GCAGAACTTAGCCACTGTGAA |
| [16,] | hsa-miR-30a-star_st | -0.46 | GCTGCAAACATCCGACTGAAAG |
| [17,] | hsa-miR-30a_st | -0.46 | CTTCCAGTCGAGGATGTTTACA |
| [18,] | hsa-miR-30c-2-star_st | -0.44 | AGAGTAAACAGCCTTCTCCCAG |
| [19,] | hsa-miR-30c_st | -0.36 | GCTGAGAGTGTAGGATGTTTACA |
| [20,] | hsa-miR-543_st | -0.31 | AAGAAGTGCACCGCGAATGTTT |
| [21,] | hsa-miR-9_st | -0.32 | TCATACAGCTAGATAACCAAAGA |

### Table 123 Erlotinib (tarceva) microRNA biomarkers.

| | Medianprobe | Corr | Sequence |
|---|---|---|---|
| [1,] | hsa-miR-320a_st | -0.32 | TCGCCCTCTCAACCCAGCTTTT |
| [2,] | hsa-miR-320b_st | -0.32 | TTGCCCTCTCAACCCAGCTTTT |
| [3,] | hsa-miR-320c_st | -0.32 | ACCCTCTCAACCCAGCTTTT |
| [4,] | hsa-miR-362_5p_st | -0.32 | ACTCACACCTAGGTTCCAAGGATT |

(continued)

| | Medianprobe | Corr | Sequence |
|---|---|---|---|
| [5,] | hsa-miR-500-star_st | -0.33 | CAGAATCCTTGCCCAGGTGCAT |
| [6,] | hsa-miR-500_st | -0.31 | TCTCACCCAGGTAGCAAGGATTA |
| [7,] | hsa-miR-502-3p_st | -0.34 | TGAATCCTTGCCCAGGTGCATT |
| [8,] | hsa-miR-532-3p_st | -0.31 | TGCAAGCCTTGGGTGTGGGAGG |
| [9,] | hsa-miR-532-5p_st | -0.35 | ACGGTCCTACACTCAAGGCATG |
| [10,] | hsa-miR-652_st | -0.34 | CACAACCCTAGTGGCGCCATT |
| [11,] | hsa-miR-671-5p_st | -0.36 | CTCCAGCCCCTCCAGGGCTTCCT |

Table 124 Herceptin microRNA biomarkers.

| | Medianprobe | Corr | Sequence |
|---|---|---|---|
| [1,] | hsa-let-7d_st | -0.32 | AACTATGCAACCTACTACCTCT |
| [2,] | bsa-miR-103_st | -0.3 | TCATAGCCCTGTACAATGCTGCT |
| [3,] | hsa-miR-107_st | -0.35 | TGATAGCCCTGTACAATGCTGCT |

Table 125 Celecoxib microRNA biomarkers.

| | Medianprobe | Corr | Sequence |
|---|---|---|---|
| [1,] | hsa-miR-1287_st | -0.39 | GACTCGAACCACTGATCCAGCA |
| [2,] | hsa-miR-130a_st | -0.32 | ATGCCCTTTTAACATTGCACTG |
| [3,] | hsa-miR-151-3p_st | -0.3 | CCTCAAGGAGCTTCAGTCTAG |
| [4,] | hsa-miR-28-3p_st | -0.42 | TCCAGGAGCTCACAATCTAGTG |
| [5,] | hsa-miR-28-5p_st | -0.43 | CTCAATAGACTGTGAGCTCCTT |
| [6,] | hsa-miR-31-star_st | -0.3 | ATGGCAATATGTTGGCATAGCA |
| [7,] | hsa-miR-455-3p_st | -0.35 | GTGTATATGCCCATGGACTGC |

Table 126 Fulvestrant microRNA biomarkers.

| | Medianprobe | Corr | Sequence |
|---|---|---|---|
| [1,] | hsa-let-7b_st | -0.31 | AACCACACAACCTACTACCTCA |
| [2,] | hsa-miR-181a-2-star_st | -0.46 | GGTACAGTCAACGGTCAGTGGT |
| [3,] | hsa-miR-221_st | -0.6 | GAAACCCAGCAGACAATGTAGCT |
| [4,] | hsa-miR-222_st | -0.65 | ACCCAGTAGCCAGATGTAGCT |
| [5,] | hsa-miR-28-5p_st | -0.3 | CTCAATAGACTGTGAGCTCCTT |
| [6,] | hsa-miR-29a_st | -0.36 | TAACCGATTTCAGATGGTGCTA |
| [7,] | hsa-miR-31_st | -0.31 | AGCTATGCCAGCATCTTGCCT |

Table 127 Iressa microRNA biomarkers.

| | Medianprobe | Corr | Sequence |
|---|---|---|---|
| [1,] | hsa-miR-505-star_st | -0.34 | ACATCAATACTTCCTGGCTCCC |
| [2,] | hsa-miR-671-5p_st | -0.34 | CTCCAGCCCCTCCAGGGCTTCCT |

Table 128 Letrozole microRNA biomarkers.

| | Medianprobe | Corr | Sequence |
|---|---|---|---|
| [1,] | U27_st | -0.36 | GAAAGTTCAGCCATATGCTTGTCAT |
| [2,] | U29_st | -0.33 | GAGCTAGTTTGATTCATCATAGAAA |

Table 129 Cotuximab (erbitux) microRNA biomarkers.

| | Medianprobe | Corr | Sequence |
|---|---|---|---|
| [1,] | hsa-miR-191st | -0.33 | CAGCTGCTTTTGGGGATTCCGTTG |
| [2,] | hsa-miR-491-5p_st | -0.36 | CCTCATGGAAGGGTCCCCCACT |

**Claims**

1. Use of a device for predicting the growth inhibition of cancer cells of a cancer patient by at least one treatment for cancer,
   wherein said device comprises at least one single-stranded nucleic acid molecule that is complementary or identical to at least 5 consecutive nucleotides of hsa-miR-766_st,
   wherein said at least one single stranded nucleic acid molecule is capable of specifically hybridizing with hsa-miR-766_st or its complement, said device allowing determination of a level of expression of hsa-miR-766_st in a biological sample from said patient,
   wherein said use further comprises assaying a level of expression of hsa-miR-766_st in a sample from said patient using said device, and wherein:

   said patient is predicted to exhibit growth inhibition of cancer cells by said at least one treatment if said level of expression of hsa-miR-766_st is substantially similar to the level of expression of hsa-miR-766_st in a cell or tissue known to exhibit growth inhibition of cancer cells by said at least one treatment,
   wherein said at least one treatment for cancer is selected from the group consisting of etoposide, adriamycin, paclitaxel, dexamethasone, methylprednisolone, belinostat, 5-Aza-2'-deoxycytidine (decitabine), melphalan, suberoylanilide hydroxamic acid (SAHA, vorinostat), leukeran, fludarabine, busulfan, dacarbazine, hydroxyurea, daunorubicin, mechlorethamine, carmustine, lomustine, mercaptopurine, teniposide, dactinomycin, ifosfamide, irinotecan, and thioguanine.

2. The use according to claim 1, wherein said single-stranded nucleic acid molecule is complementary to or identical to at least 22 consecutive nucleotides of hsa-miR-766_st.

3. The use according to claim 1 or 2, wherein said device comprises:

   i) one or more single-stranded nucleic acid molecules having at least 85% identity, preferably 100% identity, to a target nucleic acid molecule having a sequence that is complementary to or identical to at least 5 consecutive nucleotides of one or more biomarkers selected from hsa-miR-124_st, hsa-miR-1281_st, hsa-miR-140-3p_st, hsa-miR-181a-star_st, hsa-miR-181b_st, hsa-miR-195-star_st, hsa-miR-342-3p_st, hsa-miR-766_st, hsa-miR-92b_st, and/or hsa-miR-938_st, said device allowing a determination of growth inhibition of cancer cells of said patient by etoposide; and/or
   ii) one or more single-stranded nucleic acid molecules having at least 85% identity, preferably 100% identity, to a target nucleic acid molecule having a sequence that is complementary to or identical to at least 5 consecutive nucleotides of one or more biomarkers selected from U55_st, U55_x_st, hsa-miR-106b-star_st, hsa-miR-106b_st, hsa-miR-124_st, hsa-miR-1299_st, hsa-miR-29b-2-star_st, hsa-miR-33b-star_st, hsa-miR-629-star_st, hsa-miR-652_st, hsa-miR-671-5p_st, hsa-miR-766_st, hsa-miR-768-3p_st, hsa-miR-93-star_st, and/or hsa-miR-93_st, said device allowing a determination of growth inhibition of cancer cells of said patient by adriamycin; and/or
   iii) one or more single-stranded nucleic acid molecules having at least 85% identity, preferably 100% identity, to a target nucleic acid molecule having a sequence that is complementary to or identical to at least 5 consecutive nucleotides of one or more biomarkers selected from hsa-miR-106b-star_st, hsa-miR-1228_st, hsa-miR-185_st, hsa-miR-188-5p_st, hsa-miR-18b_st, hsa-miR-20b_st, hsa-miR-25_st, hsa-miR-320c_st, hsa-miR-320d_st, hsa-miR-362-5p_st, hsa-miR-500-star_st, hsa-miR-500_st, hsa-miR-501-3p_st, hsa-miR-502-3p_st, hsa-miR-532-3p_st, hsa-miR-532-5p_st, hsa-miR-652_st, and/or hsa-miR-766_st, said device allowing a determination of growth inhibition of cancer cells of said patient by paclitaxel; and/or
   iv) one or more single-stranded nucleic acid molecules having at least 85% identity, preferably 100% identity, to a target nucleic acid molecule having a sequence that is complementary to or identical to at least 5 consecutive nucleotides of one or more biomarkers selected from U49A_st, U49A_x_st, U498_s_st, U49B_x_st, hsa-miR-10a-star_st, hsa-miR-1207-5p_st, hsa-miR-128_st, hsa-miR-150_st, hsa-miR-424-star_st, hsa-miR-424_st, hsa-miR-503_st, hsa-miR-766_st, and/or hsa-miR-768-5p_st, said device allowing a determination of growth inhibition of cancer cells of said patient by dexamethasone; and/or

v) one or more single-stranded nucleic acid molecules having at least 85% identity, preferably 100% identity, to a target nucleic acid molecule having a sequence that is complementary to or identical to at least 5 consecutive nucleotides of one or more biomarkers selected from ACA23_st, ACA24_x_st, ACA54_st, U31_st, hsa-let-7d-star_st, hsa-miR-106b-star_st, hsa-miR-1183_st, hsa-miR-1207-5p_st, hsa-miR-1268_st, hsa-miR-1281_st, hsa-miR-128_st, hsa-miR-150_st, hsa-miR-15a_st, hsa-miR-181a-star_st, hsa-miR-181c_st, hsa-miR-18b_st, hsa-miR-198_st, hsa-miR-20b-star_st, hsa-miR-223_st, hsa-miR-297_st, hsa-miR-342-5p_st, hsa-miR-363_st, hsa-miR-588_st, hsa-miR-631_st, hsa-miR-766_st, hsa-miR-768-5p_st, hsa-miR-92a-2-star_st, and/or hsa-miR-940_st, said device allowing a determination of growth inhibition of cancer cells of said patient by methylprednisolone; and/or

vi) one or more single-stranded nucleic acid molecules having at least 85% identity, preferably 100% identity, to a target nucleic acid molecule having a sequence that is complementary to or identical to at least 5 consecutive nucleotides of one or more biomarkers selected from ACA18_x_st, ACA51_x_st, ACA9_st, ACA9_x_st, HBII-180A_x_st, HBII-202_st, HBII-336_st, HBII-55_st, U104_st, U27_st, U29_st, U30_st, U31_st, U31_x_st, U38A_st, U55_st, U55_x_st, U56_st, U56_x_st, U57_st, U60_st, U74_x_st, hsa-miR-106a_st, hsa-miR-1183_st, hsa-miR-1207-5p_st, hsa-miR-1246_st, hsa-miR-1268_st, hsa-miR-1299_st, hsa-miR-1307_st, hsa-miR-142-5p_st, hsa-miR-17-star_st, hsa-miR-17_st, hsa-miR-18a-star_st, hsa-miR-18a_st, hsa-miR-18b_st, hsa-miR-195-star_st, hsa-miR-19a_st, hsa-miR-19b_st, hsa-miR-20a_st, hsa-miR-20b_st, hsa-miR297_st, hsa-miR-330-3p_st, hsa-miR-346_st, hsa-miR-34b_st, hsa-miR-595_st, hsa-miR-629-star_st, hsa-miR-647_st, hsa-miR-766_st, hsa-miR-768-5p_st, and/or hsa-miR-92a_st, said device allowing a determination of growth inhibition of cancer cells of said patient by belinostat (PXD101); and/or

vii) one or more single-stranded nucleic acid molecules having at least 85% identity, preferably 100% identity, to a target nucleic acid molecule having a sequence that is complementary to or identical to at least 5 consecutive nucleotides of one or more biomarkers selected from hsa-miR-106a_st, hsa-miR-106b-star_st, hsa-miR-1183_st, hsa-miR-195-star_st, hsa-miR-297_st, hsa-miR-324-3p_st, hsa-miR-34b_st, hsa-miR-371-5p_st, hsa-miR-766_st, hsa-miR-92a_st, and/or hsa-miR-93-star_st, said device allowing a determination of growth inhibition of cancer cells of said patient by 5-Aza-2'-deoxycytidine (decitabine); and/or

viii) one or more single-stranded nucleic acid molecules having at least 85% identity, preferably 100% identity, to a target nucleic acid molecule having a sequence that is complementary to or identical to at least 5 consecutive nucleotides of one or more biomarkers selected from ACA7_s_st, ENSG00000202252_st, ENSG00000207002_x_st, HBII-202_st, HBII-429_st, HBII-438A_s_st, HBII-55_st, HBII-85-11_st, HBII-85-23_x_st, HBII-85-26_st, HBII-85-2_x_st, HBII-85-6_x_st, U104_st, U13_st, U17b_st, U17b_x_st, U33_st, U34_st, U41_st, U52_st, U55_st, U55_x_st, U56_st, U57_st, U68_st, U74_x_st, U78_s_st, U78_x_st, U83_st, U95_st, hsa-miR-1183_st, hsa-miR-1207-5p_st, hsa-miR-1228_st, hsa-miR-124_st, hsa-miR-1281_st, hsa-miR-1299_st, hsa-miR-140-3p_st, hsa-miR-142-5p_st, hsa-miR-181a-star_st, hsa-miR-181a_st, hsa-miR-181b_st, hsa-miR-181c_st, hsa-miR-195-star_st, hsa-miR-223_st, hsa-miR-297_st, hsa-miR-29b-2-star_st, hsa-miR-342-3p_st, hsa-miR-342-5p_st, hsa-miR-34b_st, hsa-miR-541-star_st, hsa-miR-610_st, hsa-miR-647_st, hsa-miR-766_st, hsa-miR-768-3p_st, hsa-miR-768-5p_st, and/or hsa-miR-885-5p_st, said device allowing a determination of growth inhibition of cancer cells of said patient by melphalan; and/or

ix) one or more single-stranded nucleic acid molecules having at least 85% identity, preferably 100% identity, to a target nucleic acid molecule having a sequence that is complementary to or identical to at least 5 consecutive nucleotides of one or more biomarkers selected from U56_x_st, hsa-miR-106a_st, hsa-miR-126_st, hsa-miR-128_st, hsa-miR-148a-star_st, hsa-miR-148a_st, hsa-miR-153_st, hsa-miR-17_st, hsa-miR-18a-star_st, hsa-miR-18a_st, hsa-miR-18b_st, hsa-miR-19a_st, hsa-miR-19b_st, hsa-miR-20a_st, hsa-miR-20b_st, hsa-miR-25_st, hsa-miR-30c_st, hsa-miR-30e_st, hsa-miR-363-star_st, hsa-miR-363_st, hsa-miR-766_st, and/or hsa-miR-92a_st, said device allowing a determination of growth inhibition of cancer cells of said patient by suberoylanilide hydroxamic acid (SAHA, vorinostat); and/or

x) one or more single-stranded nucleic acid molecules having at least 85% identity, preferably 100% identity, to a target nucleic acid molecule having a sequence that is complementary to or identical to at least 5 consecutive nucleotides of one or more biomarkers selected from HBII-85-2_x_st, U104_st, U41_st, U52_st, U55_st, U55_x_st, U57_st, U74_x_st, hsa-miR-124_st, hsa-miR-1281_st, hsa-miR-1299_st, hsa-miR-140-3p_st, hsa-miR-142-5p_st, hsa-miR-181a-star_st, hsa-miR-181a_st, hsa-miR-181b_st, hsa-miR-181c_st, hsa-miR-195-star_st, hsa-miR-223_st, hsa-miR-297_st, hsa-miR-342-3p_st, hsa-miR-342-5p_st, hsa-miR-34b_st, hsa-miR-766_st, hsa-miR-768-5p_st, and/or hsa-miR-874_st, said device allowing a determination of growth inhibition of cancer cells of said patient by leukeran; and/or

xi) one or more single-stranded nucleic acid molecules having at least 85% identity, preferably 100% identity, to a target nucleic acid molecule having a sequence that is complementary to or identical to at least 5 consecutive nucleotides of one or more biomarkers selected from HBII-276_st, HBII-438A_s_st, HBII-52-32_x_st, HBII-85-11_st, hsa-miR-130a_st, hsa-miR-155_st, hsa-miR-181a-star_st, hsa-miR-181a_st, hsa-miR-181b_st, hsa-miR-20b-star_st, hsa-miR-20b_st, hsa-miR-34c-3p_st, hsa-miR-363_st, hsa-miR-424-star_st, hsa-miR-503_st, hsa-miR-554_st, and/or hsa-miR-766_st, said device allowing a determination of growth inhibition of cancer cells of said patient by fludarabine; and/or

xii) one or more single-stranded nucleic acid molecules having at least 85% identity, preferably 100% identity, to a target nucleic acid molecule having a sequence that is complementary to or identical to at least 5 consecutive nucleotides of one or more biomarkers selected from U55_st, U55_x_st, hsa-miR-1207-5p_st, hsa-miR-1246_st, hsa-miR-1281_st, hsa-miR-181a-star_st, hsa-miR-181b_st, hsa-miR-181c_st, hsa-miR-297_st, hsa-miR-766_st, and/or hsa-miR-923_st, said device allowing a determination of growth inhibition of cancer cells of said patient by busulfan; and/or

xiii) one or more single-stranded nucleic acid molecules having at least 85% identity, preferably 100% identity, to a target nucleic acid molecule having a sequence that is complementary to or identical to at least 5 consecutive nucleotides of one or more biomarkers selected from U13_st, U17a_st, U25_st, U29_st, U3-2_s_st, U41_st, U52_st, U55_st, hsa-miR-1207-5p_st, hsa-miR-1299_st, hsa-miR-140-3p_st, hsa-miR-140-5p_st, hsa-miR-17-star_st, hsa-miR-181d_st, hsa-miR-223_st, hsa-miR-297_st, hsa-miR-34b_st, hsa-miR-766_st, hsa-miR-92a-1-star_st, and/or hsa-miR-92a_st, said device allowing a determination of growth inhibition of cancer cells of said patient by dacarbazine; and/or

xiv) one or more single-stranded nucleic acid molecules having at least 85% identity, preferably 100% identity, to a target nucleic acid molecule having a sequence that is complementary to or identical to at least 5 consecutive nucleotides of one or more biomarkers selected from ACA7_s_st, ENSG00000199282_st, ENSG00000201859_x_st, HBII-202_st, HBII-336_st, HBII-429_st, U104_st, U25_st, U33_st, U34_st, U38A_st, U41_st, U49B_s_st, U52_st, U55_st, U55_x_st, U74_x_st, hsa-miR-1207-5p_st, hsa-miR-124_st, hsa-miR-1281_st, hsa-miR-1299_st, hsa-miR-140-3p_st, hsa-miR-155_st, hsa-miR-181a-star_st, hsa-miR-181b_st, hsa-miR-181c_st, hsa-miR-195-star_st, hsa-miR-223_st, hsa-miR-297_st, hsa-miR-34b_st, hsa-miR-491-3p_st, hsa-miR-595_st, hsa-miR-631_st, hsa-miR-766_st, and/or hsa-miR-768-5p_st, said device allowing a determination of growth inhibition of cancer cells of said patient by hydroxyurea; and/or

xv) one or more single-stranded nucleic acid molecules having at least 85% identity, preferably 100% identity, to a target nucleic acid molecule having a sequence that is complementary to or identical to at least 5 consecutive nucleotides of one or more biomarkers selected from ACA48_x_st, HBII-85-26_st, HBII-85-6_x_st, U104_st, U55_st, U55_x_st, U74_x_st, hsa-miR-106a-star_st, hsa-miR-106bstar_st, hsa-miR-106b_st, hsa-miR-124_st, hsa-miR-1281_st, hsa-miR-1299_st, hsa-miR-181a-star_st, hsa-miR-195-star_st, hsa-miR-297_st, hsa-miR-29b-2-star_st, hsa-miR-33b-star_st, hsa-miR-342-5p_st, hsa-miR-34b_st, hsa-miR-629-star_st, hsa-miR-652_st, hsa-miR-671-5p_st, hsa-miR-766_st, hsa-miR-768-3p_st, hsa-miR-768-5p_st, hsa-miR-877-star_st, hsa-miR-93-star_st, and/or hsa-miR-93_st, said device allowing a determination of growth inhibition of cancer cells of said patient by daunorubicin; and/or

xvi) one or more single-stranded nucleic acid molecules having at least 85% identity, preferably 100% identity, to a target nucleic acid molecule having a sequence that is complementary to or identical to at least 5 consecutive nucleotides of one or more biomarkers selected from ACA13_st, ACA21_st, ACA48_x_st, ACA7_s_st, ACA9_st, ENSG00000202252_st, HBII-202_st, HBII-239_st, HBII-336_st, HBII-429_st, U104_st, U33_st, U34_st, U52_st, U55_st, U55_x_st, U74_x_st, U75_st, U78_s_st, U78_x_st, U95_st, hsa-miR-124_st, hsa-miR-1281_st, hsa-miR-1299_st, hsa-miR-142-5p_st, hsa-miR-181a-star_st, hsa-miR-181a_st, hsa-miR-181b_st, hsa-miR-195-star_st, hsa-miR-297_st, hsa-miR-29b-2-star_st, hsa-miR-331-5p_st, hsa-miR-34b_st, hsa-miR-425_st, hsa-miR-766_st, hsa-miR-768-3p_st, and/or hsa-miR-768-5p_st, said device allowing a determination of growth inhibition of cancer cells of said patient by mechlorethamine; and/or

xvii) one or more single-stranded nucleic acid molecules having at least 85% identity, preferably 100% identity, to a target nucleic acid molecule having a sequence that is complementary to or identical to at least 5 consecutive nucleotides of one or more biomarkers selected from ACA10_s_st, ACA44_st, ACA52_st, ACA61_st, HBII-142_st, HBII-180A_x_st, HBII-382_s_st, HBII-429_st, U13_st, U17a_st, U17a_x_st, U17b_st, U17b_x_st, U38A_st, U41_st, U48_st, U52_st, U55_st, U55_x_st, U67_st, U67_x_st, U70_x_st, U74_x_st, U83B_st, hsa-miR-106a_st, hsa-miR-1274a_st, hsa-miR-1275_st, hsa-miR-1280_st, hsa-miR-130b_st, hsa-miR-146b-3p_st, hsa-miR-146b-5p_st, hsa-miR-17-star_st, hsa-miR-185-star_st, hsa-miR-18a-star_st, hsa-miR-18a_st, hsa-miR-18b_st, hsa-miR-19a_st, hsa-miR-19b_st, hsa-miR-202_st, hsa-miR-20a_st, hsa-miR-20b_st, hsa-miR-223_st, hsa-miR-25-star_st, hsa-miR-373_st, hsa-miR-378-star_st, hsa-miR-422a_st, hsa-miR-423-5p_st, hsa-miR-451_st, hsa-miR-486-3p_st, hsa-miR-486-5p_st, hsa-miR-504_st, hsa-miR-550_st, hsa-miR-616_st, hsa-miR-671-3p_st, hsa-miR-671-5p_st, hsa-miR-766_st, hsa-miR-92a-1-star_st, hsa-miR-92a_st, and/or hsa-miR-93-star_st, said device allowing a determination of growth inhibition of cancer cells of said patient by carmustine; and/or

xviii) one or more single-stranded nucleic acid molecules having at least 85% identity, preferably 100% identity, to a target nucleic acid molecule having a sequence that is complementary to or identical to at least 5 consecutive nucleotides of one or more biomarkers selected from U41_st, hsa-miR-106b-star_st, hsa-miR-1183_st, hsa-miR-1207-5p_st, hsa-miR-1281_st, hsa-miR-18b_st, hsa-miR-195-star_st, hsa-miR-615-3p_st, hsa-miR-631_st, hsa-miR-766_st, and/or hsa-miR-92a-2-star_st, said device allowing a determination of growth inhibition of cancer cells of said patient by lomustine; and/or

xix) one or more single-stranded nucleic acid molecules having at least 85% identity, preferably 100% identity, to a target nucleic acid molecule having a sequence that is complementary to or identical to at least 5 consecutive nucleotides of one or more biomarkers selected from ACA43_st, ACA57_st, ENSG00000199411_s_st, ENSG00000200879_st, ENSG00000202252_st, HBII-142_st, HBII-142_x_st, HBII-202_st, HBII-429_st, U13_st, U25_st, U27_st, U29_st, U30_st,
U31_x_st, U36C_st, U38A_st, U52_st, U55_st, U55_x_st, U59B_st, U74_x_st, U83B_st, U83_st, hsa-miR-106a_st, hsa-miR-1275_st, hsa-miR-17-star_st, hsa-miR-17_st, hsa-miR-18a-star_st, hsa-miR-18a_st, hsa-miR-18b_st, hsa-miR-19a_st, hsa-miR-19b_st, hsa-miR-20a_st, hsa-miR-20b_st, hsa-miR25-star_st, hsa-miR-378-star_st, hsa-miR-378_st, hsa-miR-422a_st, hsa-miR-423-5p_st, hsa-miR-663b_st, hsa-miR-766_st, hsa-miR-92a-1-star_st, and/or hsa-miR-92a_st, said device allowing a determination of growth inhibition of cancer cells of said patient by mercaptopurine; and/or

xx) one or more single-stranded nucleic acid molecules having at least 85% identity, preferably 100% identity, to a target nucleic acid molecule having a sequence that is complementary to or identical to at least 5 consecutive nucleotides of one or more biomarkers selected from ACA48_x_st, ACA7_s_st, HBII-239_st, HBII-429_st, HBII-85-26_st, U104_st, U17b_st, U17b_x_st, U55_st, U55_x_st, hsa-miR-106b-star_st, hsa-miR-124_st, hsa-miR-1281_st, hsa-miR-1299_st, hsa-miR-140-3p_st, hsa-miR-142-5p_st, hsa-miR-181a-star_st, hsa-miR-181a_st, hsa-miR-181b_st, hsa-miR-181c_st, hsa-miR-195-star_st, hsa-miR-297_st, hsa-miR-29b-2-star_st, hsa-miR-33b-star_st, hsa-miR-34b_st, hsa-miR-629-star_st, hsa-miR-766_st, hsa-miR-768-3p_st, hsa-miR-768-5p_st, and/or hsa-miR-92b_st, said device allowing a determination of growth inhibition of cancer cells of said patient by teniposide; and/or

xxi) one or more single-stranded nucleic acid molecules having at least 85% identity, preferably 100% identity, to a target nucleic acid molecule having a sequence that is complementary to or identical to at least 5 consecutive nucleotides of one or more biomarkers selected from hsa-miR-106b-star_st, hsa-miR-106b_st, hsa-miR-1275_st, hsa-miR-25-star_st, hsa-miR-25_st, hsa-miR-33b-star_st, hsa-miR-362-5p_st, hsa-miR-500-star_st, hsa-miR-500_st, hsa-miR-501-5p_st, hsa-miR-502-3p_st, hsa-miR-532-5p_st, hsa-miR-629-star_st, hsa-miR-629_st, hsa-miR-652_st, hsa-miR-671-5p_st, hsa-miR-766_st, hsa-miR-93-star_st, and/or hsa-miR-93_st, said device allowing a determination of growth inhibition of cancer cells of said patient by dactinomycin; and/or

xxii) one or more single-stranded nucleic acid molecules having at least 85% identity, preferably 100% identity, to a target nucleic acid molecule having a sequence that is complementary to or identical to at least 5 consecutive nucleotides of one or more biomarkers selected from U17b_st, U48_st, U49B_s_st, U55_st, hsa-miR-1207-5p_st, hsa-miR-128_st, hsa-miR-181a-star_st, hsa-miR-181c-star_st, hsa-miR-181d_st, hsa-miR-20b-star_st, hsa-miR-20b_st, hsa-miR-223_st, hsa-miR-363_st, and/or hsa-miR-766_st, said device allowing a determination of growth inhibition of cancer cells of said patient by ifosfamide; and/or

xxiii) one or more single-stranded nucleic acid molecules having at least 85% identity, preferably 100% identity, to a target nucleic acid molecule having a sequence that is complementary to or identical to at least 5 consecutive nucleotides of one or more biomarkers selected from hsa-miR-181a-star_st, hsa-miR-297_st, hsa-miR-432_st, hsa-miR-766_st, and/or hsa-miR-874_st, said device allowing a determination of growth inhibition of cancer cells of said patient by irinotecan; and/or

xxiv) one or more single-stranded nucleic acid molecules having at least 85% identity, preferably 100% identity, to a target nucleic acid molecule having a sequence that is complementary to or identical to at least 5 consecutive nucleotides of one or more biomarkers selected from ACA13_st, ENSG00000202093_x_st, ENSG00000202252_st, HBII-202_st, HBII-429_st, HBII-55_st, U104_st, U13_st, U17b_st, U17b_x_st, U25_st, U27_st, U29_st, U30_st, U31_st, U31_x_st, U36A_st, U36C_st, U38A_st, U52_st, U54_st, U55_st, U55_x_st, U56_x_st, U74_x_st, U75_st, U78_s_st, U78_x_st, U83B_st, U83_st, U95_st, hsa-miR-106a_st, hsa-miR-1183_st, hsa-miR-1246_st, hsa-miR-1281_st, hsa-miR-142-5p_st, hsa-miR-17-star_st, hsa-miR-17_st, hsa-miR-18a-star_st, hsa-miR-18a_st, hsa-miR-18b_st, hsa-miR-19a_st, hsa-miR-19b_st, hsa-miR-20a_st, hsa-miR-297_st, hsa-miR-34b_st, hsa-miR-378-star_st, hsa-miR-378_st, hsa-miR-491-3p_st, hsa-miR-766_st, hsa-miR-92a-1-star_st, and/or hsa-miR-92a_st, said device allowing a determination of growth inhibition of cancer cells of said patient by thioguanine.

4. The use according to claim 3, wherein said device comprises two or more of said single-stranded nucleic acid molecules of i) to xxiv).

**5.** The use according to any one of claims 1 to 4, wherein said single-stranded nucleic acid molecule(s) have a length in the range of 10 to 100 nucleotides in length, preferably wherein said nucleic acid molecule(s) have a length in the range of 20 to 60 nucleotides.

**6.** The use according to any one of claims 2 to 5, wherein the expression level of hsa-miR-766_st and, optionally, the one or more biomarkers is determined by detecting the level of mRNA transcribed from one or more genes encoding the one or more biomarkers.

**Patentansprüche**

**1.** Verwendung einer Vorrichtung zum Vorhersagen der Wachstumsinhibition von Krebszellen eines Krebspatienten durch mindestens eine Behandlung für Krebs,
wobei die Vorrichtung mindestens ein einzelsträngiges Nukleinsäuremolekül umfasst, das komplementär ist oder identisch ist zu mindestens 5 aufeinander folgenden Nukleotiden von hsa-miR-766_st,
wobei das mindestens eine einzelsträngige Nukleinsäuremolekül fähig ist, spezifisch an hsa-miR-766_st oder dessen Komplementär zu hybridisieren, wobei die Vorrichtung die Bestimmung eines Spiegels der Expression von hsa-miR-766_st in einer biologischen Probe von dem Patienten erlaubt,
wobei diese Verwendung ferner das Prüfen des Spiegels der Expression von hsa-miR-766_st in einer Probe von dem Patienten unter Verwendung der Vorrichtung umfasst, und wobei:

es vorhergesagt wird, dass der Patient Wachstumsinhibition von Krebszellen durch diese mindestens eine Behandlung aufweist, wenn der Spiegel der Expression von hsa-miR-766_st im Wesentlichen ähnlich ist zu dem Spiegel der Expression von hsa-miR-766_st in einer Zelle oder einem Gewebe, von dem bekannt ist, dass es Wachstumsinhibition von Krebszellen durch diese mindestens eine Behandlung aufweist,
wobei diese mindestens eine Behandlung für Krebs ausgewählt ist aus der Gruppe bestehend aus Etoposid, Adriamycin, Paclitaxel, Dexamethason, Methylprednisolon, Belinostat, 5-Aza-2'-deoxycytidin (Decitabin), Melphalan, Suberoylanilid-Hydroxamsäure (SAHA, Vorinostat), Leukeran, Fludarabin, Busulfan, Dacarbazin, Hydroxyharnstoff, Daunorubicin, Mechlorethamin, Carmustin, Lomustin, Mercaptopurin, Teniposid, Dactinomycin, Ifosfamid, Irinotecan und Thioguanin.

**2.** Verwendung gemäß Anspruch 1, wobei das einzelsträngige Nukleinsäuremolekül komplementär ist oder identisch ist zu mindestens 22 aufeinander folgenden Nukleotiden von hsa-miR-766_st.

**3.** Verwendung gemäß Anspruch 1 oder 2, wobei die Vorrichtung umfasst:

i) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus hsa-miR-124_st, hsa-miR-1281_st, hsa-miR-140-3p_st, hsa-miR-181a-star_st, hsa-miR-181b_st, hsa-miR-195-star_st, hsa-miR-342-3p_st, hsa-miR-766_st, hsa-miR-92b_st und / oder hsa-miR-938_st, wobei die Vorrichtung eine Bestimmung der Wachstumsinhibition von Krebszellen von diesem Patienten durch Etoposid erlaubt; und / oder
ii) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus U55_st, U55_x_st, hsa-miR-106b-star_st, hsa-miR-106b_st, hsa-miR-124_st, hsa-miR-1299_st, hsa-miR-29b-2-star_st, hsa-miR-33b-star_st, hsa-miR-629-star_st, hsa-miR-652_st, hsa-miR-671-5p_st, hsa-miR-766_st, hsa-miR-768-3p_st, hsa-miR-93-star_st und / oder hsa-miR-93_st, wobei die Vorrichtung eine Bestimmung der Wachstumsinhibition von Krebszellen von diesem Patienten durch Adriamycin erlaubt; und / oder
iii) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus hsa-miR-106b-star_st, hsa-miR-1228_st, hsa-miR-185_st, hsa-miR-188-5p_st, hsa-miR-18b_st, hsa-miR-20b_st, hsa-miR-25_st, hsa-miR-320c_st, hsa-miR-320d_st, hsa-miR-362-5p_st, hsa-miR-500-star_st, hsa-miR-500_st, hsa-miR-501-3p_st, hsa-miR-502-3p_st, hsa-miR-532-3p_st, hsa-miR-532-5p_st, hsa-miR-652_st und / oder hsa-miR-766_st, wobei die Vorrichtung eine Bestimmung der Wachstumsinhibition von Krebszellen von diesem Patienten durch Paclitaxel erlaubt; und / oder

iv) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus U49A_st, U49A_x_st, U49B_s_st, U49B_x_st, hsa-miR-10a-star_st, hsa-miR-1207-5p_st, hsa-miR-128_st, hsa-miR-150_st, hsa-miR-424-star_st, hsa-miR-424_st, hsa-miR-503_st, hsa-miR-766_st und / oder hsa-miR-768-5p_st, wobei die Vorrichtung eine Bestimmung der Wachstumsinhibition von Krebszellen von diesem Patienten durch Dexamethason erlaubt; und / oder

v) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus ACA23_st, ACA24_x_st, ACA54_st, U31_st, hsa-let-7d-star_st, hsa-miR-106b-star_st, hsa-miR-1183_st, hsa-miR-1207-5p_st, hsa-miR-1268_st, hsa-miR-1281_st, hsa-miR-128_st, hsa-miR-150_st, hsa-miR-15a_st, hsa-miR-181a-star_st, hsa-miR-181c_st, hsa-miR-18b_st, hsa-miR-198_st, hsa-miR-20b-star_st, hsa-miR-223_st, hsa-miR-297_st, hsa-miR-342-5p_st, hsa-miR-363_st, hsa-miR-588_st, hsa-miR-631_st, hsa-miR-766_st, hsa-miR-768-5p_st, hsa-miR-92a-2-star_st und / oder hsa-miR-940_st, wobei die Vorrichtung eine Bestimmung der Wachstumsinhibition von Krebszellen von diesem Patienten durch Methylprednisolon erlaubt; und / oder

vi) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus aACA18_x_st, ACA51_x_st, ACA9_st, ACA9_x_st, HBII-180A_x_st, HBII-202_st, HBII-336_st, HBII-55_st, U104_st, U27_st, U29_st, U30_st, U31_st, U31_x_st, U38A_st, U55_st, U55_x_st, U56_st, U56_x_st, U57_st, U60_st, U74_x_st, hsa-miR-106a_st, hsa-miR-1183_st, hsa-miR-1207-5p_st, hsa-miR-1246_st, hsa-miR-1268_st, hsa-miR-1299_st, hsa-miR-1307_st, hsa-miR-142-5p_st, hsa-miR-17-star_st, hsa-miR-17_st, hsa-miR-18a-star_st, hsa-miR-18a_st, hsa-miR-18b_st, hsa-miR-195-star_st, hsa-miR-19a_st, hsa-miR-19b_st, hsa-miR-20a_st, hsa-miR-20b_st, hsa-miR297_st, hsa-miR-330-3p_st, hsa-miR-346_st, hsa-miR-34b_st, hsa-miR-595_st, hsa-miR-629-star_st, hsa-miR-647_st, hsa-miR-766_st, hsa-miR-768-5p_st und / oder hsa-miR-92a_st, wobei die Vorrichtung eine Bestimmung der Wachstumsinhibition von Krebszellen von diesem Patienten durch Belinostat (PXD101) erlaubt; und / oder

vii) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus hsa-miR-106a_st, hsa-miR-106b-star_st, hsa-miR-1183_st, hsa-miR-195-star_st, hsa-miR-297_st, hsa-miR-324-3p_st, hsa-miR-34b_st, hsa-miR-371-5p_st, hsa-miR-766_st, hsa-miR-92a_st und / oder hsa-miR-93-star_st, wobei die Vorrichtung eine Bestimmung der Wachstumsinhibition von Krebszellen von diesem Patienten durch 5-Aza-2'-deoxycytidin (Decitabin) erlaubt; und / oder

viii) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus ACA7_s_st, ENSG00000202252_st, ENSG00000207002_x_st, HBII-202_st, HBII-429_st, HBII-438A_s_st, HBII-55_st, HBII-85-11_st, HBII-85-23_x_st, HBII-85-26_st, HBII-85-2_x_st, HBII-85-6_x_st, U104_st, U13_st, U17b_st, U17b_x_st, U33_st, U34_st, U41_st, U52_st, U55_st, U55_x_st, U56_st, U57_st, U68_st, U74_x_st, U78_s_st, U78_x_st, U83_st, U95_st, hsa-miR-1183_st, hsa-miR-1207-5p_st, hsa-miR-1228_st, hsa-miR-124_st, hsa-miR-1281_st, hsa-miR-1299_st, hsa-miR-140-3p_st, hsa-miR-142-5p_st, hsa-miR-181a-star_st, hsa-miR-181a_st, hsa-miR-181b_st, hsa-miR-181c_st, hsa-miR-195-star_st, hsa-miR-223_st, hsa-miR-297_st, hsa-miR-29b-2-star_st, hsa-miR-342-3p_st, hsa-miR-342-5p_st, hsa-miR-34b_st, hsa-miR-541-star_st, hsa-miR-610_st, hsa-miR-647_st, hsa-miR-766_st, hsa-miR-768-3p_st, hsa-miR-768-5p_st und / oder hsa-miR-885-5p_st, wobei die Vorrichtung eine Bestimmung der Wachstumsinhibition von Krebszellen von diesem Patienten durch Melphalan erlaubt; und /oder

ix) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus U56_x_st, hsa-miR-106a_st, hsa-miR-126_st, hsa-miR-128_st, hsa-miR-148a-star_st, hsa-miR-148a_st, hsa-miR-153_st, hsa-miR-17_st, hsa-miR-18a-star_st, hsa-miR-18a_st, hsa-miR-18b_st, hsa-miR-19a_st, hsa-miR-19b_st, hsa-miR-20a_st, hsa-miR-20b_st, hsa-miR-25_st, hsa-miR-30c_st, hsa-miR-30e_st, hsa-miR-363-star_st, hsa-miR-363_st, hsa-miR-766_st und / oder hsa-miR-92a_st, wobei die Vorrichtung eine Bestimmung der Wachstumsinhibition von Krebszellen von diesem Patienten durch Suberoylanilid-Hydroxamsäure (SAHA, Vorinostat) erlaubt; und / oder

x) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus HBII-85-2_x_st, U104_st, U41_st, U52_st, U55_st, U55_x_st, U57_st, U74_x_st, hsa-miR-124_st, hsa-miR-1281_st, hsa-miR-1299_st, hsa-miR-140-3p_st, hsa-miR-142-5p_st, hsa-miR-181a-star_st, hsa-miR-181a_st, hsa-miR-181b_st, hsa-miR-181c_st, hsa-miR-195-star_st, hsa-miR-223_st, hsa-miR-297_st, hsa-miR-342-3p_st, hsa-miR-342-5p_st, hsa-miR-34b_st, hsa-miR-766_st, hsa-miR-768-5p_st und / oder hsa-miR-874_st, wobei die Vorrichtung eine Bestimmung der Wachstumsinhibition von Krebszellen von diesem Patienten durch Leukeran erlaubt; und / oder

xi) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus HBII-276_st, HBII-438A_s_st, HBII-52-32_x_st, HBII-85-11_st, hsa-miR-130a_st, hsa-miR-155_st, hsa-miR-181a-star_st, hsa-miR-181a_st, hsa-miR-181b_st, hsa-miR-20b-star_st, hsa-miR-20b-st, hsa-miR-34c-3p_st, hsa-miR-363_st, hsa-miR-424-star_st, hsa-miR-503_st, hsa-miR-554_st und / oder hsa-miR-766_st, wobei die Vorrichtung eine Bestimmung der Wachstumsinhibition von Krebszellen von diesem Patienten durch Fludarabin erlaubt; und / oder

xii) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus U55_st, U55_x_st, hsa-miR-1207-5p_st, hsa-miR-1246_st, hsa-miR-1281_st, hsa-miR-181a-star_st, hsa-miR-181b_st, hsa-miR-181c_st, hsa-miR-297_st, hsa-miR-766_st und / oder hsa-miR-923_st, wobei die Vorrichtung eine Bestimmung der Wachstumsinhibition von Krebszellen von diesem Patienten durch Busulfan erlaubt; und /oder

xiii) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus U13_st, U17a_st, U25_st, U29_st, U3-2_s_st, U41_st, U52_st, U55_st, hsa-miR-1207-5p_st, hsa-miR-1299_st, hsa-miR-140-3p_st, hsa-miR-140-5p_st, hsa-miR-17-star_st, hsa-miR-181d_st, hsa-miR-223_st, hsa-miR-297_st, hsa-miR-34b_st, hsa-miR-766_st, hsa-miR-92a-1-star_st und / oder hsa-miR-92a_st, wobei die Vorrichtung eine Bestimmung der Wachstumsinhibition von Krebszellen von diesem Patienten durch Dacarbazin erlaubt; und / oder

xiv) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus ACA7_s_st, ENSG00000199282_st, ENSG00000201859_x_st, HBII-202_st, HBII-336_st, HBII-429_st, U104_st, U25_st, U33_st, U34_st, U38A_st, U41_st, U49B_s_st, U52_st, U55_st, U55_x_st, U74_x_st, hsa-miR-1207-5p_st, hsa-miR-124_st, hsa-miR-1281_st, hsa-miR-1299_st, hsa-miR-140-3p_st, hsa-miR-155_st, hsa-miR-181a-star_st, hsa-miR-181b_st, hsa-miR-181c_st, hsa-miR-195-star_st, hsa-miR-223_st, hsa-miR-297_st, hsa-miR-34b_st, hsa-miR-491-3p_st, hsa-miR-595_st, hsa-miR-631_st, hsa-miR-766_st und / oder hsa-miR-768-5p_st, wobei die Vorrichtung eine Bestimmung der Wachstumsinhibition von Krebszellen von diesem Patienten durch Hydroxyharnstoff erlaubt; und / oder

xv) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus ACA48_x_st, HBII-85-26_st, HBII-85-6_x_st, U104_st, U55_st, U55_x_st, U74_x_st, hsa-miR-106a-star_st, hsa-miR-106bstar_st, hsa-miR-106b_st, hsa-miR-124_st, hsa-miR-1281_st, hsa-miR-1299_st, hsa-miR-181a-star_st, hsa-miR-195-star_st, hsa-miR-297_st, hsa-miR-29b-2-star_st, hsa-miR-33b-star_st, hsa-miR-342-5p_st, hsa-miR-34b_st, hsa-miR-629-star_st, hsa-miR-652_st, hsa-miR-671-5p_st, hsa-miR-766_st, hsa-miR-768-3p_st, hsa-miR-768-5p_st, hsa-miR-877-star_st, hsa-miR-93-star_st und / oder hsa-miR-93_st, wobei die Vorrichtung eine Bestimmung der Wachstumsinhibition von Krebszellen von diesem Patienten durch Daunorubicin erlaubt; und / oder

xvi) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus ACA13_st, ACA21_st, ACA48_x_st, ACA7_s_st, ACA9_st, ENSG00000202252_st, HBII-202_st, HBII-239_st, HBII-336 st, HBII-429_st, U104_st, U33_st, U34_st, U52_st, U55_st, U55_x_st, U74_x_st, U75_st, U78_s_st, U78_x_st, U95_st, hsa-miR-124_st, hsa-miR-1281_st, hsa-miR-1299_st, hsa-miR-142-5p_st, hsa-miR-181a-star_st, hsa-miR-181a_st, hsa-miR-181b_st, hsa-miR-195-star_st, hsa-miR-297_st, hsa-miR-29b-2-star_st, hsa-miR-331-5p_st, hsa-miR-34b_st, hsa-miR-425_st, hsa-miR-766_st, hsa-miR-768-3p_st und / oder hsa-miR-768-5p_st, wobei die Vorrichtung eine Bestimmung der Wachstumsinhibition von Krebszellen von diesem Patienten durch Mechlorethamin erlaubt; und / oder

xvii) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus ACA10_s_st, ACA44_st, ACA52_st, ACA61_st, HBN-142_st, HBII-180A_x_st, HBII-382_s_st, HBII-429_st, U13_st, U17a_st, U17a_x_st, U17b_st, U17b_x_st, U38A_st, U41_st, U48_st, U52_st, U55_st, U55_x_st, U67_st, U67_x_st, U70_x_st, U74_x_st, U83B_st, hsa-miR-106a_st, hsa-miR-1274a_st, hsa-miR-1275_st, hsa-miR-1280_st, hsa-miR-130b_st, hsa-miR-146b-3p_st, hsa-miR-146b-5p_st, hsa-miR-17-star_st, hsa-miR-185-star_st, hsa-miR-18a-star_st, hsa-miR-18a_st, hsa-miR-18b_st, hsa-miR-19a_st, hsa-miR-19b_st, hsa-miR-202_st, hsa-miR-20a_st, hsa-miR-20b_st, hsa-miR-223_st, hsa-miR-25-star_st, hsa-miR-373_st, hsa-miR-378-star_st, hsa-miR-422a_st, hsa-miR-423-5p_st, hsa-miR-451_st, hsa-miR-486-3p_st, hsa-miR-486-5p_st, hsa-miR-504_st, hsa-miR-550_st, hsa-miR-616_st, hsa-miR-671-3p_st, hsa-miR-671-5p_st, hsa-miR-766_st, hsa-miR-92a-1-star_st, hsa-miR-92a_st und / oder hsa-miR-93-star_st, wobei die Vorrichtung eine Bestimmung der Wachstumsinhibition von Krebszellen von diesem Patienten durch Carmustin erlaubt; und / oder

xviii) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus U41_st, hsa-miR-106b-star_st, hsa-miR-1183_st, hsa-miR-1207-5p_st, hsa-miR-1281_st, hsa-miR-18b_st, hsa-miR-195-star_st, hsa-miR-615-3p_st, hsa-miR-631_st, hsa-miR-766_st und / oder hsa-miR-92a-2-star_st, wobei die Vorrichtung eine Bestimmung der Wachstumsinhibition von Krebszellen von diesem Patienten durch Lomustin erlaubt; und / oder

xix) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus ACA43_st, ACA57_st, ENSG00000199411_s_st, ENSG00000200879_st, ENBG00000202252_st, HBII-142_st, HBII-142_x_st, HBII-202_st, HBII-429_st, U13_st, U25_st, U27_st, U29_st, U30_st, U31_x_st, U36C_st, U38A_st, U52_st, U55_st, U55_x_st, U59B_st, U74_x_st, U83B_st, U83_st, hsa-miR-106a_st, hsa-miR-1275_st, hsa-miR-17-star_st, hsa-miR-17_st, hsa-miR-18a-star_st, hsa-miR-18a_st, hsa-miR-18b_st, hsa-miR-19a_st, hsa-miR-19b_st, hsa-miR-20a_st, hsa-miR-20b_st, hsa-miR25-star_st, hsa-miR-378-star_st, hsa-miR-378_st, hsa-miR-422a_st, hsa-miR-423-5p_st, hsa-miR-663b_st, hsa-miR-766_st, hsa-miR-92a-1-star_st und / oder hsa-miR-92a_st, wobei die Vorrichtung eine Bestimmung der Wachstumsinhibition von Krebszellen von diesem Patienten durch Mercaptopurin erlaubt; und / oder

xx) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus ACA48_x_st, ACA7_s_st, HBII-239-st, HBII-429_st, HBII-85-26_st, U104_st, U17b_st, U17b_x_st, U55_st, U55_x_st, hsa-miR-106b-star_st, hsa-miR-124_st, hsa-miR-1281_st, hsa-miR-1299_st, hsa-miR-140-3p_st, hsa-miR-142-5p_st, hsa-miR-181a-star_st, hsa-miR-181a_st, hsa-miR-181b_st, hsa-miR-181c_st, hsa-miR-195-star_st, hsa-miR-297_st, hsa-miR-29b-2-star_st, hsa-miR-33b-star_st, hsa-miR-34b_st, hsa-miR-629-star_st, hsa-miR-766_st, hsa-miR-768-3p_st, hsa-miR-768-5p_st und / oder hsa-miR-92b_st, wobei die Vorrichtung eine Bestimmung der Wachstumsinhibition von Krebszellen von diesem Patienten durch Teniposid erlaubt; und / oder

xxi) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus hsa-miR-106b-star_st, hsa-miR-106b_st, hsa-miR-1275_st, hsa-miR-25-star_st, hsa-miR-25_st, hsa-miR-33b-star_st, hsa-miR-362-5p_st, hsa-miR-500-star_st, hsa-miR-500_st, hsa-miR-501-5p_st, hsa-miR-502-3p_st, hsa-miR-532-5p_st, hsa-miR-629-star_st, hsa-miR-629_st, hsa-miR-652_st, hsa-miR-671-5p_st, hsa-miR-766_st, hsa-miR-93-star_st und / oder hsa-miR-93_st, wobei die Vorrichtung eine Bestimmung der Wachstumsinhibition von Krebszellen von diesem Patienten durch Dactinomycin erlaubt; und / oder

xxii) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus U17b_st, U48_st, U49B_s_st, U55_st, hsa-miR-1207-5p_st, hsa-miR-128_st, hsa-miR-181a-star_st, hsa-miR-181c-star_st, hsa-miR-181d_st, hsa-miR-20b-star_st, hsa-miR-20b_st, hsa-miR-223_st, hsa-miR-363_st und / oder hsa-miR-766_st, wobei die Vorrichtung eine Bestimmung der Wachstumsinhibition von Krebszellen von diesem Patienten durch Ifosfamid erlaubt; und / oder

xxiii) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus hsa-miR-181a-star_st, hsa-miR-297_st, hsa-miR-432_st, hsa-miR-766_st und / oder hsa-miR-874_st, wobei die Vorrichtung eine Bestimmung der Wachstumsinhibition von Krebszellen von diesem Patienten durch Irinotecan erlaubt; und / oder

xxiv) ein oder mehrere einzelsträngige(s) Nukleinsäuremolekül(e), das / die mindestens 85 % Identität, vorzugsweise 100 % Identität zu einem Zielnukleinsäuremolekül aufweist / aufweisen, das eine Sequenz aufweist, die komplementär ist zu oder identisch ist zu mindestens 5 aufeinander folgenden Nukleotiden von einem oder mehreren Biomarkern, ausgewählt aus ACA13_st, ENSG00000202093_x_st, ENSG00000202252_st, HBII-202_st, HBII-429-st, HBII-55_st, U104_st, U13_st, U17b_st, U17b_x_st, U25_st, U27_st, U29_st, U30_st, U31_st, U31_x_st, U36A_st, U36C_st, U38A_st, U52_st, U54_st, U55_st, U55_x_st, U56_x_st, U74_x_st, U75_st, U78_s_st, U78_x_st, U83B_st, U83_st, U95_st, hsa-miR-106a_st, hsa-miR-1183_st, hsa-miR-1246_st, hsa-miR-1281_st, hsa-miR-142-5p_st, hsa-miR-17-star_st, hsa-miR-17_st, hsa-miR-18a-star_st, hsa-miR-18a_st, hsa-miR-18b_st, hsa-miR-19a_st, hsa-miR-19b_st, hsa-miR-20a_st, hsa-miR-297_st, hsa-miR-34b_st, hsa-miR-378-star_st, hsa-miR-378_st, hsa-miR-491-3p_st, hsa-miR-766_st, hsa-miR-92a-1-star_st und / oder hsa-miR-92a_st, wobei die Vorrichtung eine Bestimmung der Wachstumsinhibition von Krebszellen von diesem Patienten durch Thioguanin erlaubt.

4. Verwendung gemäß Anspruch 3, wobei die Vorrichtung zwei oder mehr der einzelsträngigen Nukleinsäuremoleküle von i) bis xxiv) umfasst.

5. Verwendung gemäß einem beliebigen der Ansprüche 1 bis 4, wobei das / die einzelsträngige(n) Nukleinsäuremolekül(e) eine Länge in dem Bereich von 10 bis 100 Nukleotiden aufweisen, vorzugsweise wobei das / die Nukleinsäuremolekül(e) eine Länge in dem Bereich von 20 bis 60 Nukleotiden aufweisen.

6. Verwendung gemäß einem beliebigen der Ansprüche 2 bis 5, wobei der Expressionsspiegel von hsa-miR-766_st und, optional, von dem einen oder den mehreren Biomarkern bestimmt wird, durch Detektieren des Spiegels von mRNA, die von einem oder mehreren Genen transkribiert wird, das / die für den einen oder die mehreren Biomarker kodiert / kodieren.

**Revendications**

1. Utilisation d'un dispositif pour prédire l'inhibition de la croissance de cellules cancéreuses d'un patient cancéreux par au moins un traitement contre le cancer,
   ledit dispositif comprenant au moins une molécule d'acide nucléique simple brin qui est complémentaire ou identique à au moins 5 nucléotides consécutifs de hsa-miR-766_st,
   ladite au moins une molécule d'acide nucléique simple brin étant apte à s'hybrider spécifiquement avec hsa-miR-766_st ou son complément, ledit dispositif permettant la détermination d'un niveau d'expression de hsa-miR-766_st dans un échantillon biologique provenant dudit patient,
   ladite utilisation consistant en outre à évaluer un niveau d'expression de hsa-miR-766_st dans un échantillon provenant dudit patient à l'aide dudit dispositif, et dans laquelle :

   il est prédit que ledit patient présente une inhibition de la croissance de cellules cancéreuses par ledit au moins un traitement si ledit niveau d'expression de hsa-miR-766_st est sensiblement similaire au niveau d'expression de hsa-miR-766_st dans une cellule ou un tissu connu pour présenter une inhibition de la croissance de cellules cancéreuses par ledit au moins un traitement,
   ledit au moins un traitement contre le cancer étant sélectionné parmi le groupe constitué de l'étoposide, l'adriamycine, le paclitaxel, la dexaméthasone, la méthylprednisolone, le belinostat, la 5-aza-2'-désoxycytidine (décitabine), le melphalan, l'acide subéroylanilide-hydroxamique (SAHA, vorinostat), le leukeran, la fludarabine, le busulfan, la dacarbazine, l'hydroxyurée, la daunorubicine, la méchloréthamine, la carmustine, la lomustine, la mercaptopurine, le téniposide, la dactinomycine, l'ifosfamide, l'irinotécan, et la thioguanine.

2. Utilisation selon la revendication 1, dans laquelle ladite molécule d'acide nucléique simple brin est complémentaire ou identique à au moins 22 nucléotides consécutifs de hsa-miR-766_st.

3. Utilisation selon la revendication 1 ou 2, dans laquelle ledit dispositif comprend :

i) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi hsa-miR-124_st, hsa-miR-1281_st, hsa-miR-140-3p_st, hsa-miR-181a-star_st, hsa-miR-181b_st, hsa-miR-195-star_st, hsa-miR-342-3p_st, hsa-miR-766_st, hsa-miR-92b_st, et/ou hsa-miR-938_st, ledit dispositif permettant une détermination d'inhibition de la croissance de cellules cancéreuses dudit patient par l'étoposide ; et/ou

ii) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi U55_st, U55_x_st, hsa-miR-106b-star_st, hsa-miR-106b-st, hsa-miR-124_st, hsa-miR-1299_st, hsa-miR-29b-2-star_st, hsa-miR-33b-star_st, hsa-miR-629-star-st, hsa-miR-652_st, hsa-miR-671-5p_st, hsa-miR-766-st, hsa-miR-768-3p_st, hsa-miR-93-star_st, et/ou hsa-miR-93_st, ledit dispositif permettant une détermination d'inhibition de la croissance de cellules cancéreuses dudit patient par l'adriamycine ; et/ou

iii) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi hsa-miR-106b-star_st, hsa-miR-1228_st, hsa-miR-185_st, hsa-miR-188-5p_st, hsa-miR-18b_st, hsa-miR-20b_st, hsa-miR-25_st, hsa-miR-320c-st, hsa-miR-320d_st, hsa-miR-362-5p_st, hsa-miR-500-star_st, hsa-miR-500_st, hsa-miR-501-3p_st, hsa-miR-502-3p_st, hsa-miR-532-3p_st, hsa-miR-652_st, et/ou hsa-miR-766_st, ledit dispositif permettant une détermination d'inhibition de la croissance de cellules cancéreuses dudit patient par le paclitaxel ; et/ou

iv) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi U49A_st, U49A_x_st, U49B_s_st, U49B_x_st, hsa-miR-10a-star_st, hsa-miR-1207-5p_st, hsa-miR-128_st, hsa-miR-150_st, hsa-miR-424-star_st, hsa-miR-424_st, hsa-miR-503_st, hsa-miR-766_st, et/ou hsa-miR-768-5p_st, ledit dispositif permettant une détermination d'inhibition de la croissance de cellules cancéreuses dudit patient par la dexaméthasone ; et/ou

v) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi ACA23_st, ACA24_x_st, ACA54_st, U31_st, hsa-let-7d-star_st, hsa-miR-106b-star_st, hsa-miR-1183_st, hsa-miR-1207-5p_st, hsa-miR-1268_st, hsa-miR-1281_st, hsa-miR-128_st, hsa-miR-150-st, hsa-miR-15a_st, hsa-miR-181a-star_st, hsa-miR-181c_st, hsa-miR-18b_st, hsa-miR-198_st, hsa-miR-20b-star_st, hsa-miR-223_st, hsa-miR-297_st, hsa-miR-342-5p_st, hsa-miR-363_st, hsa-miR-588_st, hsa-miR-631_st, hsa-miR-766_st, hsa-miR-768-5p_st, hsa-miR-92a-2-star_st, et/ou hsa-miR-940_st, ledit dispositif permettant une détermination d'inhibition de la croissance de cellules cancéreuses dudit patient par la méthylprednisolone ; et/ou

vi) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi ACA18_x_st, ACA51_x_st, ACA9_st, ACA9_x_st, HBII-180A-x-st, HBII-202-st, HBII-336_st, HBII-55_st, U104_st, U27_st, U29_st, U30_st, U31_st, U31_x_st, U38A_st, U55_st, U55_x_st, U56_st, U56_x_st, U57_st, U60_st, U74_x_st, hsa-miR-106a_st, hsa-miR-1183_st, hsa-miR-1207-5p_st, hsa-miR-1246_st, hsa-miR-1268_st, hsa-miR-1299_st, hsa-miR-1307_st, hsa-miR-142-5p_st, hsa-miR-17-star_st, hsa-miR-17_st, hsa-miR-18a-star_st, hsa-miR-18a_st, hsa-miR-18b_st, hsa-miR-195-star_st, hsa-miR-19a_st, hsa-miR-19b_st, hsa-miR-20a_st, hsa-miR-20b_st, hsa-miR-297_st, hsa-miR-330-3p_st, hsa-miR-346_st, hsa-miR-34b_st, hsa-miR-595_st, hsa-miR-629-star_st, hsa-miR-647_st, hsa-miR-766_st, hsa-miR-768-5p_st, et/ou hsa-miR-92a_st, ledit dispositif permettant une détermination d'inhibition de la croissance de cellules cancéreuses dudit patient par le belinostat (PXD101) ; et/ou

vii) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi hsa-miR-106a_st, hsa-miR-106b_st, hsa-miR-1183_st, hsa-miR-195-star_st, hsa-miR-297_st, hsa-miR-324-3p_st, hsa-miR-34b_st, hsa-miR-371-5p_st, hsa-miR-766_st, hsa-miR-92a_st, et/ou hsa-miR-93-star_st, ledit dispositif permettant une détermination d'inhibition de la croissance de cellules cancéreuses dudit patient par la 5-aza-2'-désoxycytidine (décitabine) ; et/ou

viii) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi ACA7_s_st, ENSG00000202252_st, ENSG00000207002_x_st, HBII-202_st, HBII-429_st, HBII-438A_s_st, HBII-55_st, HBII-85-11_st, HBII-85-23_x_st, HBII-85-26_st, HBII-85-2_x_st, HBII-85-6_x_st, U104_st, U13_st, U17b_st, U17b_x_st, U33_st, U34_st, U41_st, U52_st, U55_st, U55_x_st, U56_st, U57_st, U68_st, U74_x_st, U78_s_st, U78_x_st, U83_st, U95_st, hsa-miR-1183_st, hsa-miR-1207-5p_st, hsa-miR-1228_st, hsa-miR-124_st, hsa-miR-1281_st, hsa-miR-1299_st, hsa-miR-140-3p_st, hsa-miR-142-5p_st, hsa-miR-181a-star_st, hsa-miR-181a_st, hsa-miR-181b_st, hsa-miR-181c_st, hsa-miR-195-star_st, hsa-miR-223_st, hsa-miR-297_st, hsa-miR-29b-2-star_st, hsa-miR-342-3p_st, hsa-miR-342-5p_st, hsa-miR-34b_st, hsa-miR-541-star_st, hsa-miR-610_st, hsa-miR-647_st, hsa-miR-766_st, hsa-miR-768-3p_st, hsa-miR-768-5p_st, et/ou hsa-miR-885-5p_st, ledit dispositif permettant une détermination d'inhibition de la croissance de cellules cancéreuses dudit patient par le melphalan ; et/ou

ix) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi U56_x_st, hsa-miR-106a_st, hsa-miR-126_st, hsa-miR-128_st, hsa-miR-148a-star_st, hsa-miR-148a_st, hsa-miR-153_st, hsa-miR-17_st, hsa-miR-18a-star_st, hsa-miR-18a_st, hsa-miR-18b_st, hsa-miR-19a_st, hsa-miR-19b_st, hsa-miR-20a-st, hsa-miR-20b_st, hsa-miR-25_st, hsa-miR-30c_st, hsa-miR-30e-st, hsa-miR-363-star_st, hsa-miR-363_st, hsa-miR-766_st, et/ou hsa-miR-92a_st, ledit dispositif permettant une détermination d'inhibition de la croissance de cellules cancéreuses dudit patient par l'acide subéroylanilide-hydroxamique (SAHA, vorinostat) ; et/ou

x) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi HBII-85-2_x_st, U104_st, U41_st, U52_st, U55_st, U55_x_st, U57_st, U74_x_st, hsa-miR-124_st, hsa-miR-1281_st, hsa-miR-1299_st, hsa-miR-140-3p_st, hsa-miR-142-5p_st, hsa-miR-181a-star_st, hsa-miR-181a_st, hsa-miR-181b_st, hsa-miR-181c_st, hsa-miR-195-star_st, hsa-miR-223_st, hsa-miR-297_st, hsa-miR-342-3p_st, hsa-miR-342-5p_st, hsa-miR-34b_st, hsa-miR-766_st, hsa-miR-768-5p_st, et/ou hsa-miR-874_st, ledit dispositif permettant une détermination d'inhibition de la croissance de cellules cancéreuses dudit patient par le leukeran ; et/ou

xi) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi HBII-276_st, HBII-438A_s_st, HBII-52-32_x_st, HBII-85-11_st, hsa-miR-130a_st, hsa-miR-155_st, hsa-miR-181a-star_st, hsa-miR-181a_st, hsa-miR-181b_st, hsa-miR-20b-star_st, hsa-miR-20b_st, hsa-miR-34c-3p_st, hsa-miR-363_st, hsa-miR-424-star_st, hsa-miR-503_st, hsa-miR-554_st, et/ou hsa-miR-766_st, ledit dispositif permettant une détermination d'inhibition de la croissance de cellules cancéreuses dudit patient par la fludarabine ; et/ou

xii) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi U55_st, U55_x_st, hsa-miR-1207-5p_st, hsa-miR-1246_st, hsa-miR-1281_st, hsa-miR-181a-star_st, hsa-miR-181b_st, hsa-miR-181c_st, hsa-miR-297_st, hsa-miR-766_st, et/ou hsa-miR-923_st, ledit dispositif permettant une détermination d'inhibition de la croissance de cellules cancéreuses dudit patient par le busulfan ; et/ou

xiii) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi U13_st, U17a_st, U25_st, U29_st, U3-2_s_st, U41_st, U52_st, U55_st, hsa-miR-1207-5p_st, hsa-miR-1299_st, hsa-miR-140-3p_st, hsa-miR-140-5p_st, hsa-miR-17-star_st, hsa-miR-181d_st, hsa-miR-223_st, hsa-miR-297_st, hsa-miR-34b_st, hsa-miR-766_st, hsa-miR- 92a-1-star_st, et/ou hsa-miR-92a_st, ledit dispositif permettant une détermination d'inhibition de la croissance de cellules cancéreuses dudit patient par la dacarbazine ; et/ou

xiv) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi ACA7_s_st, ENSG00000199282_st, ENSG00000201859_x_st, HBII-202_st, HBII-336_st, HBII-429_st, U104_st, U25_st, U33_st, U34_st, U38A_st, U41_st, U49B_s_st, U52_st, U55_st, U55_x_st, U74_x_st, hsa-miR-1207-5p_st, hsa-miR-124_st, hsa-miR-1281_st, hsa-miR-1299_st, hsa-miR-140-3p_st, hsa-miR-155_st, hsa-miR-181a-star_st, hsa-miR-181b_st, hsa-miR-181c_st, hsa-miR-195-star_st, hsa-miR-223_st, hsa-miR-297_st, hsa-miR-34b_st, hsa-miR-491-3p_st, hsa-miR-595_st, hsa-miR-631_st, hsa-miR-766_st, et/ou hsa-miR-768-5p_st, ledit dispositif permettant une détermination d'inhibition de la croissance de cellules cancéreuses dudit patient par l'hydroxyurée ; et/ou

xv) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi ACA48_x_st, HBII-85-26_st, HBII-85-6_x_st, U104_st, U55_st, U55_x_st, U74_x_st, hsa-miR-106a-star_st, hsa-miR-106b-star_st, hsa-miR-106b_st, hsa-miR-124_st, hsa-miR-1281_st, hsa-miR-1299_st, hsa-miR-181a-star_st, hsa-miR-195-star_st, hsa-miR-297_st, hsa-miR-29b-2-star_st, hsa-miR-33b-star_st, hsa-miR-342-5p_st, hsa-miR-34b_st, hsa-miR-629-star_st, hsa-miR-652_st, hsa-miR-671-5p_st, hsa-miR-766_st, hsa-miR-768-3p_st, hsa-miR-768-5p_st, hsa-miR-877-star_st, hsa-miR-93-star_st, et/ou hsa-miR-93_st, ledit dispositif permettant une détermination d'inhibition de la croissance de cellules cancéreuses dudit patient par la daunorubicine ; et/ou

xvi) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi ACA13_st, ACA21_st, ACA48_x_st, ACA7_s_st, ACA9_st, ENSG00000202252_st, HBII-202_st, HBII-239_st, HBII-336_st, HBII-429_st, U104_st, U33_st, U34_st, U52_st, U55_st, U55_x_st, U74_x_st, U75_st, U78_s_st, U78_x_st, U95_st, hsa-miR-124_st, hsa-miR-1281_st, hsa-miR-1299_st, hsa-miR-142-5p_st, hsa-miR-181a-star_st, hsa-miR-181a_st, hsa-miR-181b_st, hsa-miR-195-star_st, hsa-miR-297_st, hsa-miR-29b-2-star_st, hsa-miR-331-5p_st, hsa-miR-34b_st, hsa-miR-425_st, hsa-miR-766_st, hsa-miR-768-3p_st, et/ou hsa-miR-768-5p_st, ledit dispositif permettant une détermination d'inhibition de la croissance de cellules cancéreuses dudit patient par la méchloréthamine ; et/ou

xvii) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi ACA10_s_st, ACA44_st, ACA52_st, ACA61_st, HBII-142_st, HBII-180A_x_st, HBII-382_s_st, HBII-429_st, U13_st, U17a_st, U17a_x_st, U17b_st, U17b_x_st, U38A_st, U41_st, U48_st, U52_st, U55_st, U55_x_st, U67_st, U67_x_st, U70_x_st, U74_x_st, U83B_st, hsa-miR-106a_st, hsa-miR-1274a_st, hsa-miR-1275_st, hsa-miR-1280_st, hsa-miR-130b_st, hsa-miR-146b-3p_st, hsa-miR-146b-5p_st, hsa-miR-17-star_st, hsa-miR-185-star_st, hsa-miR-18a-star_st, hsa-miR-18a_st, hsa-miR-18b_st, hsa-miR-19a_st, hsa-miR-19b_st, hsa-miR-202_st, hsa-miR-20a_st, hsa-miR-20b_st, hsa-miR-223_st, hsa-miR-25-star_st, hsa-miR-373_st, hsa-miR-378-star_st, hsa-miR-422a_st, hsa-miR-423-5p_st, hsa-miR-451_st, hsa-miR-486-3p_st, hsa-miR-486-5p_st, hsa-miR-504_st, hsa-miR-550_st, hsa-miR-616_st, hsa-miR-671-3p_st, hsa-miR-671-5p_st, hsa-miR-766_st, hsa-miR-92a-1-star_st, hsa-miR-92a_st, et/ou hsa-miR-93-star_st, ledit dispositif permettant une détermination d'inhibition de la croissance de cellules cancéreuses dudit patient par la carmustine ; et/ou

xviii) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi U41_st, hsa-miR-106b-star_st, hsa-miR-1183_st, hsa-miR-1207-5p_st, hsa-miR-1281_st, hsa-miR-18b_st, hsa-miR-195-star_st, hsa-miR-615-3p_st, hsa-miR-631_st, hsa-miR-766_st, et/ou hsa-miR-92a-2-star_st, ledit dispositif permettant une détermination d'inhibition de la croissance de cellules cancéreuses dudit patient par la lomustine ; et/ou

xix) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi ACA43_st, ACA57_st, ENSG00000199411_s_st, ENSG00000200879_st, ENSG00000202252_st, HBII-142_st, HBII-142_x_st, HBII-202_st, HBII-429_st, U13_st, U25_st, U27_st, U29_st, U30_st, U31_x_st, U36C_st, U38A_st, U52_st, U55_st, U55_x_st, U59B_st, U74_x_st, U83B_st, U83_st, hsa-miR-106a_st, hsa-miR-1275_st, hsa-miR-17-star_st, hsa-miR-17_st, hsa-miR-18a-star_st, hsa-miR-18a_st, hsa-miR-18b_st, hsa-miR-19a_st, hsa-miR-19b_st, hsa-miR-20a_st, hsa-miR-20b_st, hsa-miR-25-star_st, hsa-miR-378-star_st, hsa-miR-378_st, hsa-miR-422a_st, hsa-miR-423-5p_st, hsa-miR-663b_st, hsa-miR-766_st, hsa-miR-92a-1-star_st, et/ou hsa-miR-92a_st, ledit dispositif permettant une détermination d'inhibition de la croissance de cellules cancéreuses dudit patient par la mercaptopurine ; et/ou

xx) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi ACA48_x_st, ACA7_s_st, HBII-239_st, HBII-429_st, HBII-85-26_st, U104_st, U17b_st, U17b_x_st, U55_st, U55_x_st, hsa-miR-106b-star_st, hsa-miR-124_st, hsa-miR-1281_st, hsa-miR-1299_st, hsa-miR-140-3p_st, hsa-miR-142-5p_st, hsa-miR-181a-star_st, hsa-miR-181a_st, hsa-miR-181b_st, hsa-miR-181c_st, hsa-miR-195-star_st, hsa-miR-297_st, hsa-miR-29b-2-star_st, hsa-miR-33b-star_st, hsa-miR-34b_st, hsa-miR-629-star_st, hsa-miR-766_st, hsa-miR-768-3p_st, hsa-miR-768-5p_st, et/ou hsa-miR-92b_st, ledit dispositif permettant une détermination d'inhibition de la croissance de cellules cancéreuses dudit patient par le téniposide ; et/ou

xxi) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi hsa-miR-106b-star_st, hsa-miR-106b_st, hsa-miR-1275_st, hsa-miR-25-star_st, hsa-miR-25_st, hsa-miR-33b-star_st, hsa-miR-362-5p_st, hsa-miR-500-star_st, hsa-miR-500_st, hsa-miR-501-5p_st, hsa-miR-502-3p_st, hsa-miR-532-5p_st, hsa-miR-629-star_st, hsa-miR-629_st, hsa-miR-652_st, hsa-miR-671-5p_st, hsa-miR-766_st, hsa-miR-93-star_st, et/ou hsa-miR-93_st, ledit dispositif permettant une détermination d'inhibition de la croissance de cellules cancéreuses dudit patient par la dactinomycine ; et/ou

xxii) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi U17b_st, U48_st, U49B_s_st, U55_st, hsa-miR-1207-5p_st, hsa-miR-128_st, hsa-miR-181a-star_st, hsa-miR-181c-star_st, hsa-miR-181d_st, hsa-miR-20b-star_st, hsa-miR-20b_st, hsa-miR-223_st, hsa-miR-363_st, et/ou hsa-miR-766_st, ledit dispositif permettant une détermination d'inhibition de la croissance de cellules cancéreuses dudit patient par l'ifosfamide ; et/ou

xxiii) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi hsa-miR-181a-star_st, hsa-miR-297_st, hsa-miR-432_st, hsa-miR-766_st, et/ou hsa-miR-874_st, ledit dispositif permettant une détermination d'inhibition de la croissance de cellules cancéreuses dudit patient par l'irinotécan ; et/ou

xxiv) une ou plusieurs molécules d'acide nucléique simple brin ayant au moins 85 % d'identité, de préférence 100 % d'identité, par rapport à une molécule d'acide nucléique cible ayant une séquence qui est complémentaire à ou identique à au moins 5 nucléotides consécutifs d'un ou plusieurs biomarqueurs sélectionnés parmi ACA13_st, ENSG00000202093_x_st, ENSG00000202252_st, HBII-202_st, HBII-429_st, HBII-55_st, U104_st, U13_st, U17b_st, U17b_x_st, U25_st, U27_st, U29_st, U30_st, U31_st, U31_x_st, U36A_st, U36C_st, U38A_st, U52_st, U54_st, U55_st, U55_x_st, U56_x_st, U74_x_st, U75_st, U78_s_st, U78_x_st, U83B_st, U83_st, U95_st, hsa-miR-106a_st, hsa-miR-1183_st, hsa-miR-1246_st, hsa-miR-1281_st, hsa-miR-142-5p_st, hsa-miR-17-star_st, hsa-miR-17_st, hsa-miR-18a-star_st, hsa-miR-18a_st, hsa-miR-18b_st, hsa-miR-19a_st, hsa-miR-19b_st, hsa-miR-20a_st, hsa-miR-297_st, hsa-miR-34b_st, hsa-miR-378-star_st, hsa-miR-378_st, hsa-miR-491-3p_st, hsa-miR-766_st, hsa-miR-92a-1-star_st, et/ou hsa-miR-92a_st, ledit dispositif permettant une détermination d'inhibition de la croissance de cellules cancéreuses dudit patient par la thioguanine.

4. Utilisation selon la revendication 3, dans laquelle ledit dispositif comprend deux ou plus desdites molécules d'acide nucléique simple brin de i) à xxiv).

5. Utilisation selon l'une quelconque des revendications 1 à 4, dans laquelle ladite ou lesdites molécule(s) d'acide nucléique simple brin ont une longueur dans la plage de 10 à 100 nucléotides en longueur, de préférence dans laquelle ladite ou lesdites molécule(s) d'acide nucléique ont une longueur dans la plage de 20 à 60 nucléotides.

6. Utilisation selon l'une quelconque des revendications 2 à 5, dans laquelle le niveau d'expression de hsa-miR-766_st et, facultativement, desdits un ou plusieurs biomarqueurs est déterminé par détection du niveau d'ARNm transcrit à partir d'un ou plusieurs gènes codant pour lesdits un ou plusieurs biomarqueurs.

Figure 1:

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 21112235 A
- WO 2008112283 A
- WO 2009080437 A
- FR 901228
- US 6391623 B
- US 6383754 B
- US 6383749 B
- US 6380377 B
- US 6379897 B
- US 6376191 B
- US 6372431 B
- US 6351712 B
- US 6344316 B
- US 6316193 B
- US 6312906 B
- US 6309828 B
- US 6309824 B
- US 6306643 B
- US 6300063 B
- US 6287850 B
- US 6284497 B
- US 62844656280954 A
- US 6262216 B
- US 6251601 B
- US 6245518 B
- US 6263287 B
- US 6238866 B
- US 6228575 B
- US 6214587 B
- US 6203989 B
- US 6171797 B
- US 6103474 A
- US 6083726 A
- US 6054274 A
- US 6040138 A
- US 6004755 A
- US 6001309 A
- US 5958342 A
- US 5952180 A
- US 5936731 A
- US 5843655 A
- US 5814454 A
- US 5837196 A
- US 5436327 A
- US 5412087 A
- US 5405783 A
- WO 0231463 A
- WO 0225288 A
- WO 0194946 A
- WO 0188162 A
- WO 0168671 A
- WO 0157259 A
- WO 0061806 A
- WO 0054046 A
- WO 0047774 A
- WO 9940434 A
- WO 9939210 A
- WO 9742507 A
- US 6268210 B
- US 5766960 A
- US 5143854 A
- US 5822715 A
- US 20030073083 A
- US 20050227266 A
- US 20050208512 A
- US 20050123945 A
- US 20030129629 A
- US 20020006613 A
- US 7101663 B
- US 20060177837 A
- US 20060088856 A

**Non-patent literature cited in the description**

- **PRADERVAND et al.** *Biotechniques,* 2010, vol. 48, 219-222
- **LI et al.** *DNA and Cell Biology,* 2007, vol. 26 (4), 195-207
- **FRIIS-HANSES et al.** *Gastroenterology,* 2009, vol. 136, A165
- DNA Microarrays: A Practical Approach. Practical Approach Series. Oxford University Press, 1999
- *Nature Genet.,* 1999, vol. 21 (1), 1-60
- Microarray Biochip: Tools and Technology. Eaton Publishing Company/BioTechniques Books Division, 2000
- **BRENNER et al.** *Proc. Natl. Acad. Sci. USA,* 2000, vol. 97 (4), 1665-1670
- **VAPNIK V N.** Statistical Learning Theory. John Wiley & Sons, 1998

- **HASTIE et al.** The Elements of Statistical Learning: Data Mining, Inference, and Prediction. Springer, 2001
- **AGRESTI.** Introduction to Categorical Data Analysis. John Wiley & Sons, 1996
- Neural Network Modeling of Physiological Processes. **V. TRESP et al.** Computational Learning Theory and Natural Learning Systems. MIT Press, 1994, vol. 2